# EUROPÄISCHE PATENTSCHRIFT

(11) **EP 1 910 381 B1**
(45) Veröffentlichungstag und Bekanntmachung des Hinweises auf die Patenterteilung: **03.06.2009**
(21) Anmeldenummer: 06754597.0
(22) Anmeldetag: 27.06.2006
(51) Int. Cl.: C07D 498/10, A61K 31/438, A61P 25/04

(54) **SUBSTITUIERTE 1-OXA-3,8-DIAZASPIRO[4.5]-DECAN-2-ON-VERBINDUNGEN UND DEREN VERWENDUNG ZUR HERSTELLUNG VON ARZNEIMITTELN**
SUBSTITUTED 1-OXA-3,8-DIAZASPIRO[4.5]-DECAN-2-ON- COMPOUNDS AND THE USE THEREOF FOR PRODUCING DRUGS
COMPOSES DE 1-OXA-3,8-DIAZASPIRO[4.5]-DECANE-2-ONE SUBSTITUES ET UTILISATION DE CEUX-CI POUR PRODUIRE DES MEDICAMENTS

(30) Priorität: 27.06.2005 DE 102005030051
(43) Veröffentlichungstag der Anmeldung: 16.04.2008
(73) Patentinhaber: Grünenthal GmbH, 52078 Aachen (DE)
(72) Erfinder: Dr.SUNDERMANN, Corinna, 61381 Friedrichsdorf (DE); PRZEWOSNY, Michael, 52064 Aachen (DE); Dr.SUNDERMANN, Bernd, 61381 Friedrichsdorf (DE)
(74) Vertreter: Brosch, Oliver
(86) Internationale Anmeldenummer: PCT/EP2006/006215
(87) Internationale Veröffentlichungsnummer: WO 2007/000325

(56) Entgegenhaltungen:
- EP-A1- 1 484 327
- WO-A-99/65494
- WO-A2-03/057698
- US-A- 4 255 432
- US-A- 5 739 336

## Beschreibung

Die vorliegende Erfindung betrifft substituierte 1-Oxa-3,8-diazaspiro[4.5]-decan-2-on-Verbindungen, Verfahren zu ihrer Herstellung, Arzneimittel enthaltend diese Verbindungen und die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des weiteren sind sie bei neuropathischen Schmerzen, unter denen insbesondere Tumorpatienten leiden, häufig nicht ausreichend wirksam.

Aus der EP 1 484 327 sind Stickstoff-haltige Heterocyclen sowie deren Verwendung als Inhibitoren der Natriumkanäle bekannt.

US 4 255 432 offenbart 8-[(2-3-Indolyl)ethyl)-1-oxa-3-,8-diazaspiro[4,5]-decan-2-on-Verbindungen sowie Arzneimittel enthaltend diese Verbindungen.

Der WO 99/65494 sind Inhibitoren der Prenyl-Protein-Transferase zu entnehmen.

WO 03/057698 beschreibt spiroazacyclische Verbindungen und deren Verwendung als Modulatoren für Monoamin-Rezeptoren.

Aus US 5 739 336 sind 1,3,8-Triaza- und 3,8-Diaza-1-oxaspiro[4,5]decanderivate und deren Verwendung in Arzneimitteln bekannt.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Eine weitere Aufgabe der vorliegenden Erfindung bestand darin, neue Verbindungen zur Verfügung zu stellen, die sich als pharmakologische Wirkstoffe in Arzneimitteln zur Behandlung von Störungen oder Krankheiten eignen, die zumindest teilweise durch Opioid-Rezeptoren, insbesondere µ-Opioid-Rezeptoren, und/oder Serotonin-(5-HT)-Rezeptoren und/oder Noradrenalin-Rezeptoren vermittelt werden.

Überraschenderweise wurde nun gefunden, dass substituierte 1-Oxa-3,8-diazaspiro[4.5]-decan-2-on-Verbindungen der nachstehend angegebenen allgemeinen Formel I eine ausgezeichnete Affinität zum Opioid-Rezeptor, insbesondere zum µ-Opioid-Rezeptor, zum Serotonin-(5-HT)-Rezeptor und zum Noradrenalin-Rezeptor aufweisen und sich daher insbesondere zur Prophylaxe und/oder Behandlung von Störungen oder Krankheiten eignen, die zumindest teilweise durch Opioid-Rezeptoren, insbesondere µ-Opioid-Rezeptoren, und/oder Serotonin-(5-HT)-Rezeptoren und/oder Noradrenalin-Rezeptoren vermittelt werden.

Ein Gegenstand der vorliegenden Erfindung sind daher 1-Oxa-3,8-diazaspiro[4.5]-decan-2-on-Verbindungen der nachstehend angegebenen allgemeinen Formel I, 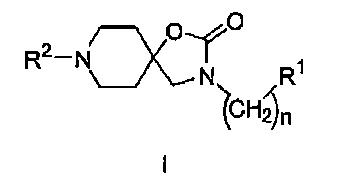 worin
- n: gleich 1, 2, 3, 4 oder 5 ist;
- R¹: für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R²: für -C(=S)-NH-R³;
für -C(=O)-NH-R⁴;
für -S(=O)₂-R⁵;
für -(CH₂)-C(=O)-NH-R⁶;
für -(CH₂)-Dₐₐ-(CH₂)_{bb}-E_{cc}-(CH₂)_{dd}-R⁷ mit aa = 0 oder 1; bb = 0, 1 oder
2; cc = 0 oder 1 und dd = 0 oder 1 steht; worin D und E unabhängig voneinander für O, S, NH, N(CH₃), N(C₂H₅) oder N[CH(CH₃)₂] stehen und wobei die Summe aus aa und cc ungleich 0 ist;
für -C(=O)-R⁸
oder für -S(=O)₂-NR⁹R¹⁰ steht;
- R³: für -(CHR¹¹)-(CH₂)_{w}-C(=O)-O-R¹² mit w = 0 oder 1 steht;
für -(CHR¹³)-(CH₂)ₐ-K_{b}-(CH₂)_{c}-L_{d}-R¹⁴ mit a = 0, 1 oder 2; b = 0 oder 1; c = 0, 1 oder 2 und d = 0 oder 1 steht; worin K und L unabhängig voneinander für O, S, NH, N(CH₃), N(C₂H₅) oder N[CH(CH₃)₂] stehen;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R⁴: für -(CHR¹⁵)-(CH₂)ₑ-M_{f}-(CH₂)_{g}-Pₕ-R¹⁶ mit e = 0, 1 oder 2; f = 0 oder 1; g = 0, 1 oder 2 und h = 0 oder 1 steht; worin M und P unabhängig voneinander für O, S, NH, N(CH₃), N(C₂H₅) oder N[CH(CH₃)₂] stehen;
für einen linearen oder verzweigten, gesättigten oder ungesättigten, ggf. substituierten C₁₋₁₀ aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R⁵: für -(CHR¹⁷)-(CH₂)ₖ-Qₗ-(CH₂)ₘ-Tₒ-R¹⁸ mit k = 0, 1 oder 2; I = 0 oder 1; m = 0, 1 oder 2 und o = 0 oder 1 steht; worin Q und T unabhängig voneinander für O, S, NH, N(CH₃), N(C₂H₅) oder N[CH(CH₃)₂] stehen;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R⁶: für einen linearen oder verzweigten, gesättigten oder ungesättigten, ggf. substituierten C₁₋₁₀ aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R⁷: für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann; oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Imidazolyl, Indolyl, Isoindolyl, Benzo[b]furanyl, Benzo[b]thlophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅,-S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-H, -C(=O)-CH₃, -C(=O)-C₂H₅, -C(=O)-C(CH₃)₃,-C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂,-S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
- R⁸: für -(CHR¹⁹)-Vₚ-(CH₂)_{q}-(CH₂)ᵣ-Wₛ-R²⁰ mit p = 0 oder 1; q = 0, 1 oder 2; r = 0, 1 oder 2 und s = 0 oder 1 steht; worin V und W unabhängig voneinander jeweils für O, S, NH, N(CH₃), N(C₂H₅) oder N[CH(CH₃)₂] stehen;
für -(CH=CH)-R²¹ steht;
für -(CR²²R²³)-Yₜ-(CR²⁴R²⁵)ᵤ-(CH₂)ᵥ-C(=O)-OR²⁶ mit t = 0 oder 1, u = 0 oder 1 und v = 0 oder 1 steht, worin Y für O, S, NH, N(CH₃), N(C₂H₅) oder N[CH(CH₃)₂] steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
- R⁹ und R¹⁰,: unabhängig voneinander, jeweils
für einen linearen oder verzweigten, gesättigten oder ungesättigten, ggf. substituierten C₁₋₁₀ aliphatischen Rest stehen;
- R¹¹, R¹³, R¹⁵, R¹⁷, R¹⁹, R²²,:
- R²³, R²⁴, R²⁵ und R²⁶,: unabhängig voneinander, jeweils
für einen Wasserstoff-Rest stehen
oder für einen linearen oder verzweigten, gesättigten oder ungesättigten, ggf. substituierten C₁₋₁₀ aliphatischen Rest stehen;
- R¹², R²⁸ und R³²,: unabhängig voneinander, jeweils
für einen linearen oder verzweigten, gesättigten oder ungesättigten, ggf. substituierten C₁₋₁₀ aliphatischen Rest stehen;
- R¹⁴, R¹⁶, R¹⁸ und R²⁰,: unabhängig voneinander, jeweils
für einen linearen oder verzweigten, gesättigten oder ungesättigten, ggf. substituierten C₁₋₁₀ aliphatischen Rest stehen;
für einen ggf. substituierten cycloaliphatischen Rest stehen, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest stehen, wobei der Aryl-Rest mit einem gesättigten oder ungesättigten, ggf. substituierten mono- oder bizyklischen Ringsystem kondensiert sein kann;
und
- R²¹, R²⁷, R²⁹, R³⁰ und R³¹,: unabhängig voneinander, jeweils
für einen ggf. substituierten cycloaliphatischen Rest stehen, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest stehen, wobei der Aryl-Rest jeweils mit einem gesättigten oder ungesättigten, ggf. substituierten mono- oder bizyklischen Ringsystem kondensiert sein kann;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Die vorstehend genannten C₁₋₁₀ aliphatischen Reste können jeweils ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein.

Sofern einer oder mehrere der vorstehend genannten Substituenten für einen linearen oder verzweigten C₁₋₁₀ aliphatischen Rest stehen, kann dieser Rest bevorzugt ausgewählt werden aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl.

Besonders bevorzugte, ggf. substituierte C₁₋₁₀ aliphatische Reste können ausgewählt werden aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, n-Hexyl, 3-Heptyl, 4-Heptyl, -CF₃, -CFH₂, -CF₂H, -CBr₃, -CCl₃, -CF₂-CF₃, -CH₂-CF₃, -CH₂-CN, -CH₂-NO₂, -CF₂-CF₂-CF₃, -CH₂-CH₂-CF₃, -CH₂-CH₂-CN, -CH₂-CH₂-NO₂, -CF₂-CF₂-CF₂-CF₃ und -CH₂-CH₂-CH₂-CN.

Die vorstehend genannten cycloaliphatischen Reste können jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -S-CF₂H, -S-CFH₂, -SH, -S-C₁₋₅-Alkyl, -C₁₋₅-Alkyl, -C(=O)-OH, -(CH₂)-C(=O)-OH; -C(=O)-O-C₁₋₅-Alkyl, -(CH₂)-C(=O)-O-C₁₋₅-Alkyl, -O-C(=O)-C₁₋₅-Alkyl, -NH-C₁₋₅-Alkyl, -N(C₁₋₆-Alkyl)₂, -NH-Phenyl, -NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H, -C(=O)-C₁₋₅-Alkyl, -C(=O)-C₁₋₅-Perfluoralkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, -S(=O)₂-NH₂, -S(=O)₂-NH-Phenyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl substituiert sein, wobei jeweils der zyklische Teil der Reste -S(=O)₂-NH-Phenyl, -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, -S(=O)₂-Phenyl, - O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein.

Sofern einer der vorstehend genannten Substituenten für einen cycloaliphatischen Rest steht, der ggf. mit 1 oder 2 linearen oder verzweigten C₁₋₅-Alkylen-Gruppen überbrückt sein kann, ist dieser ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl.

Besonders bevorzugt können die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, - C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, Phenyl und -S(=O)₂-NH₂ substituiert sein, wobei der Phenyl-Rest jeweils mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃ und - O-CH₂-CH₂-CH₂-CH₃ substituiert sein kann.

Die vorstehend genannten C₁₋₅-Alkylen-Gruppen können jeweils ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein.

Sofern einer der vorstehend genannten Substituenten eine C₁₋₅-Alkylen-Gruppe aufweist, kann diese bevorzugt ausgewählt werden aus der Gruppe bestehend aus - (CH₂)-, -(CH₂)₂-, -C(H)(CH₃)-, -C(CH₃)₂-, -(CH₂)₃-, -(CH₂)₄-, -(CH₂)₅-, - C(H)(C(H)(CH₃)₂)- und -C(C₂H₅)(H)-.

Ebenfalls bevorzugt können die Ringe der vorstehend genannten mono- oder polyzyklischen Ringsysteme jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -S-CF₂H, -S-CFH₂, -SH, -S-C₁₋₅-Alkyl, -C₁₋₅-Alkyl, -C(=O)-OH, -(CH₂)-C(=O)-OH; -C(=O)-O-C₁₋₅-Alkyl, -(CH₂)-C(=O)-O-C₁₋₅-Alkyl, -O-C(=O)-C₁₋₅-Alkyl, -NH-C₁₋₅-Alkyl, - N(C₁₋₅-Alkyl)₂, -NH-Phenyl, -NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H, -C(=O)-C₁₋₅-Alkyl, -C(=O)-C₁₋₅-Perfluoralkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, -S(=O)₂-NH₂, - S(=O)₂-NH-Phenyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl substituiert sein, wobei jeweils der zyklische Teil der Reste -S(=O)₂-NH-Phenyl, -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Besonders bevorzugt können die Ringe der vorstehend genannten ggf. substituierten mono- oder polyzyklischen Ringsysteme unsubstituiert oder ggf. jeweils mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, - (CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂-C₂H₅, - NH-S(=O)₂-CH₃, -S(=O)₂-NH-CH₃, Phenyl und -S(=O)₂-NH₂ substituiert sein; wobei der Phenyl-Rest jeweils mit 1, 2, 3. 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃ und -O-CH₂-CH₂-CH₂-CH₃ substituiert sein kann.

Aryl-Reste, die mit einem mono- bzw. polyzyklischen Ringsystem kondensiert sind, sind ausgewählt aus der Gruppe bestehend aus [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl und [3,4]-Dihydro-2H-1,4-benzoxazinyl.

Ebenfalls bevorzugt können die vorstehend genannten ggf. substituierten Aryl- oder Heteroaryl-Reste unsubstituiert oder ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-C₁₋₅-Alkyl, -NH₂, -NO₂, -O-CF₃, -S-CF₃, -S-CF₂H, -S-CFH₂, SH, -S-C₁₋₅-Alkyl, -C₁₋₁₀-Alkyl, -C(=O)-OH, -(CH₂)-C(=O)-OH, -C(=O)-O-C₁₋₅-Alkyl, - (CH₂)-C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Perfluoralkyl, -C(=O)-NH₂, -C(=O)-NH-C₁₋₅-Alkyl, -C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl. -S(=O)₂-NH₂, - S(=O)₂-NH-Phenyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein, wobei jeweils der zyklische Teil der Reste Pyridinyl, Cyclopentyl, Cyclohexyl, Pyridazinyl, - S(=O)₂-Phenyl, -S(=O)₂-NH-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - NO₂, -C₁₋₅-Alkyl, -O-C₁₋₅-Alkyl, -O-CF₃, -S-CF₃, Phenyl und -O-Benzyl substituiert sein kann.

Sofern einer oder mehrere der vorstehend genannten Substituenten für einen Aryl-Rest stehen, kann dieser ausgewählt werden aus der Gruppe bestehend aus Phenyl und Naphthyl (1-Naphthyl und 2-Naphthyl).

Sofern einer oder mehrere der vorstehend genannten Substituenten für einen Heteroaryl-Rest stehen, ist dieser ausgewählt aus der Gruppe bestehend aus 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, Oxazolyl, Isoxazolyl, Pyridazinyl, Pyrazinyl, Pyrimidinyl, Indazolyl, Chinazolinyl, Chinolinyl und Isochinolinyl.

Besonders bevorzugt können die Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H,-S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃,-N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅,-C(=O)-NH-C(CH₃)₃, -C(=O)-N(CH₃)₂, -C(=O)-N(C₂H₅)₂, -S(=O)₂-CH₃, -S(=O)₂-C₂H₅,-NH-S(=O)₂-CH₃, -S(=O)₂-NH-CH₃, -S(=O)₂-NH₂, -S(=O)₂-NH-Phenyl, Phenyl, -O-Phenyl, -O-Benzyl und Benzyl substituiert sein, wobei jeweils der zyklische Teil der Reste -S(=O)₂-NH-Phenyl, Phenyl, -O-Phenyl, -O-Benzyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅,-O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann.

Ganz besonders bevorzugt kann ein substituierter Phenyl-Rest ausgewählt werden aus der Gruppe bestehend aus Biphenyl, 2-Trifluoromethyl-phenyl, 2-Butoxy-phenyl, 2-(1,1)-Dimethyl-propyl-phenyl, 2-Nitro-phenyl, 2-Ethyl-benzoat, 2-Acetamid-phenyl, 2-Difluoromethylsulfanyl-phenyl, 2-Dimethylamino-phenyl, 2-Diethylamino-phenyl, 2-Amino-phenyl, 2-Benzol-sulfonamid, 2-Trifluoromethylsulfanyl-phenyl, 2-Ethyl-phenyl, 2-Methyl-benzoat, 2-Methansulfonyl-phenyl, 2-Bromo-phenyl, 2-Chloro-phenyl, 2-Fluoro-phenyl, 2-Methyl-phenyl, 2-Trifluoromethoxy, 2-Methoxy-phenyl, 2-Ethoxy-phenyl, 2-Propyl-phenyl, 2-Cyano-phenyl, 2-Acetylphenyl, 2-Dimethylamino-sulfonyl-phenyl, 3-Chloro-phenyl, 3-Methyl-phenyl, 3-Butoxy-phenyl, 3-Nitro-phenyl, 3-Trifluoromethylsulfanyl-phenyl, 3-Trifluoromethyl-phenyl, 3-Methansulfonyl-phenyl, 3-Benzol-sulfonamid, 3-Ethyl-benzoat, 3-Fluoro-phenyl, 3-Difluoromethylsulfanyl-phenyl, 3-Propyl-phenyl, 3-Bromo-phenyl, 3-Dimethylamino-phenyl, 3-(1,1)-Dimethylpropyl-phenyl, 3-Acetamind-phenyl, 3-Diethylamino-phenyl, 3-Amino-phenyl, 3-Methoxy-phenyl, 3-Ethyl-phenyl, 3-Ethoxy-phenyl, 3-Cyano-phenyl, 3-Trifluoromethoxy-phenyl, 3-Acetylphenyl, 3-Phenyl-phenyl, 3-Dimethylamino-sulfonyl-phenyl, 4-Methansulfonyl-phenyl, 4-Bromo-phenyl, 4-Methoxy-phenyl, 4-Chloro-phenyl, 4-Benzol-sulfonamid, 4-Difluoromethylsulfanyl-phenyl, 4-Fluoro-phenyl, 4-tert-Butyl-phenyl, 4-Cyano-phenyl, 4-Butoxy-phenyl, 4-Nitro-phenyl, 4-Trifluoromethylsulfanyl-phenyl, 4-Methyl-phenyl, 4-Phenyl-phenyl, 4-Trifluoromethyl-phenyl, 4-Dimethylamino-phenyl, 4-Propyl-phenyl, 4-Diethylamino-phenyl, 4-Ethyl-benzoat, 4-Amino-phenyl, 4-Iodo-phenyl, 4-Trifluoromethoxy-phenyl, 4-(1,1)-Dimethyl-propyl-phenyl, 4-n-Propyl-phenyl, 4-Di-n-propyl-amino-sulfonyl-phenyl, 4-(3,5-Dichloro-phenylsulfamoyl)-phenyl, 4-Acetamid-phenyl, 4-Diethylamino-sulfonyl-phenyl, 4-Dimethylamino-sulfonyl-phenyl, 4-Ethyl-phenyl, 4-Ethoxy-phenyl, 4-Methyl-benzoat, 4-Acetyl-phenyl, 2-Fluoro-3-trifluoromethylphenyl, (2,3)-Difluorophenyl, (2,3)-Dimethyl-phenyl, (2,3)-Dichlorophenyl, 3-Fluoro-2-trifluoromethylphenyl, (2,4)-Dichloro-phenyl, (2,4)-Difluorophenyl, 4-Fluoro-2-trifluoromethyl-phenyl, (2,4)-Dimethoxyphenyl, 2-Chloro-4-fluoro-phenyl, 2-Chloro-4-nitro-phenyl, (2,4)-Dibromo-phenyl, 2-Fluoro-4-trifluoromethyl-phenyl, (2,5)-Difluoro-phenyl, 2-Fluoro-5-trifluoromethyl-phenyl, 5-Fluoro-2-trifluoromethyl-phenyl, 5-Chloro-2-trifluoromethyl-phenyl, 5-Bromo-2-trifluoromethyl-phenyl, (2,5)-Dimethoxy-phenyl, (2,5)-Bistrifluoromethyl-phenyl, (2,5)-Dichloro-phenyl, (2,5)-Dibromo-phenyl, 2-Methoxy-5-nitro-phenyl, 2-Fluor-6-trifluoromethyl-phenyl, (2,6)-Dimethoxy-phenyl, (2,6)-Dimethyl-phenyl, (2,6)-Dichloro-phenyl, 2-Chloro-6-fluoro-phenyl, 2-Bromo-6-chloro-phenyl, 2-Bromo-6-fluoro-phenyl, (2,6)-Difluoro-phenyl, (2,6)-Difluoro-3-methyl-phenyl, (2,6)-Dibromo-phenyl, (2,6)-Dichlorophenyl, 3-Chloro-2-fluoro-phenyl, (3,4)-Dichlorophenyl, 4-Chloro-3-nitro-phenyl, (3,4)-Dimethoxy-phenyl, 4-Fluoro-3-trifluoromethylphenyl, 3-Fluoro-4-trifluoromethyl-phenyl, (3,4)-Difluoro-phenyl, 4-Chloro-3-trifluoromethyl, 4-Bromo-3-methyl-phenyl, 4-Bromo-5-methyl-phenyl, 3-Chloro-4-fluoro-phenyl, 4-Fluoro-3-nitro-phenyl, 4-Bromo-3-nitro-phenyl, (3,4)-Dibromo-phenyl, 4-Chlor-3-methyl-phenyl, 4-Bromo-3-methyl-phenyl, 4-Fluoro-3-methyl-phenyl, 4-Methyl-3-nitro-phenyl, (3,5)-Dimethoxy-phenyl, (3,5)-Bis-trifluoromethyl-phenyl, (3,5)-Difluoro-phenyl, (3,5)-Dinitro-phenyl, (3,5)-Dichlorophenyl, 3-Fluoro-5-trifluoromethyl-phenyl, 5-Fluoro-3-trifluoromethyl-phenyl, (3,5)-Dibromo-phenyl, 5-Chloro-4-fluoro-phenyl, 5-Chloro-4-fluoro-phenyl, 5-Bromo-4-methyl-phenyl, (2,3,4)-Trifluorophenyl, (2,3,4)-Trichlorophenyl, (2,3,6)-Trifluorophenyl, 5-Chloro-2-methoxy-phenyl, (2,3)-Difluoro-4-methyl, (2,4,5)-Trifluoro-phenyl, (2,4,5)-Trichloro-phenyl, (2,4)-Dichloro-5-fluoro-phenyl, (2,4,6)-Trichloro-phenyl, (2,4,6)-Trimethylphenyl, (2,4,6)-Trifluoro-phenyl, (2,4,6)-Trimethoxy-phenyl, (3,4,5)-Trimethoxy-phenyl, (2,3,4,5)-Tetrafluoro-phenyl, 4-Methoxy-2,3,6-trimethyl-phenyl, 4-Methoxy-2,3,6-trimethyl-phenyl, 4-Chloro-2,5-dimethyl-phenyl, 2-Chloro-6-fluoro-3-methyl-phenyl, 6-Chloro-2-fluoro-3-methyl und (2,3,4,5,6)-Pentafluoro-phenyl.

Ganz besonders bevorzugt kann ein ggf. substituierter Heteroaryl-Rest ausgewählt werden aus der Gruppe bestehend aus Benzo[1,2,3]oxadiazol-5-yl, 5-tert-Butyl-2-methyl-2H-pyrazol-3-yl, 5-tert-Butyl-2-methyl-furan-3-yl, 3-(2-Chlorphenyl)-5-methyl-isoxazol-4-yl, 2-Chlor-pyridin-3-yl, 2-Chlor-pyridin-4-yl, 6-Chlor-2H-chromen-3-yl, 6-Chlor-pyridin-3-yl, 5-Chlor-benzo[b]thiophen-3-yl, 3-Chlor-thiophen-2-yl, 3-Cyano-4-methyl-thiophen-2-yl, (4,5)-Dichlor-thiophen-2-yl, (2,5)-Dimethyl-2H-pyrazol-3-yl, 2-Ethylsulfanyl-pyridin-3-yl, 3-(2-Fluorphenyl)-5-methyl-isoxazol-4-yl, 2-Furanyl, 3-Furanyl, 1H-Indol-3-yl, Isoxazol-5-yl, 3-(4-Methoxy-phenyl)-[1,2,4]oxadiazol-5-yl, 2-Methylsulfanyl-pyridin-3-yl, 5-Methyl-isoxazol-4-yl, 5-Methyl-isoxazol-3-yl, 5-Methyl-2-phenyl-2H-[1,2,3]triazol-4-yl, 4-Methyl-[1,2,3]thiadiazol-5-yl, 2-Methyl-6-trifluormethyl-pyridin-3-yl, 1-Phenyl-5-propyl-1 H-pyrazol-4-yl, 4-Phenyl-5-trifluormethyl-thiophen-3-yl, 2-Phenoxy-pyridin-3-yl, 4-Phenyl-thiazol-2-yl, 2-Pyridinyl, 3-Pyridinyl, 4-Pyridinyl, 2-Thiophenyl, 3-Thiophenyl, (1,2,3,4)-Tetrahydro-isochinolin-7-yl und 5-Trifluormethyl-1 H-pyrazol-4-yl.

Ebenfalls bevorzugt sind substituierte 1-Oxa-3,8-diazaspiro[4.5]-decan-2-on-Verbindungen der vorstehend angegebenen allgemeinen Formel I, worin

R¹ für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, [3,4]-Dihydro-2H-1,4-benzoxazinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(-O)-C(CH₃)₃, -C(=O)-CF₃, - C(=O)-C₂F₅, -C(=O)-NH₂, -C(=O)-NH-CH₃, -C(=O)-NH-C₂H₅, -C(=O)-NH-C(CH₃)₃, - C(=O)-N(CH₃)₂, -C(=O)-N(C₂H₅)₂, -S(=O)₂-CH₃, -S(=O)₂-C₂H₅, -NH-S(=O)₂-CH₃, - S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;

und jeweils R² bis R³², aa, bb, cc, dd, a, b, c, d, e, f, g, h, k, l, m, n, o, p, q, r, s, t, u, v, w, D, E, K, L, M, P, Q, T, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin
R² für -C(=S)-NH-R³; für -C(=O)-NH-R⁴; für -S(=O)₂-R⁵; für -(CH₂)-C(=O)-NH-R⁶; für -(CH₂)-O-R⁷, für -(CH₂)-S-R⁷, für -(CH₂)-NH-R⁷, für -(CH₂)-N(CH₃)-R⁷, für -(CH₂)-(CH₂)-O-R⁷, für -(CH₂)-(CH₂)-S-R¹, für -(CH₂)-NH-R⁷, für-(CH₂)-N(CH₃)-R⁷, für -(CH₂)-(CH₂)-(CH₂)-O-R⁷, für -(CH₂)-(CH₂)-(CH₂)-S-R⁷, für - (CH₂)-(CH₂)-(CH₂)-NH-R⁷, für -(CH₂)-(CH₂)-(CH₂)-N(CH₃)-R⁷, für -(CH₂)-O-(CH₂)-R⁷, für -(CH₂)-S-(CH₂)-R⁷; für -(CH₂)-NH-(CH₂)-R⁷; für -C(=O)-R⁸ oder für -S(=O)₂-NR⁹R¹⁰ steht;
und jeweils R¹, R³ bis R¹⁰ und n die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin
R³ für -(CHR¹¹)-C(=O)-O-R¹² oder -(CHR¹¹)-(CH₂)-C(=O)-O-R¹² steht;
für -(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, - (CHR¹³)-S-R¹⁴, -(CHR¹³)-NH-R¹⁴, -(CHR¹³)-N(CH₃)-R¹⁴, -(CHR¹³)-(CH₂)-OR¹⁴, - (CHR¹³)-(CH₂)-S-R¹⁴, -(CHR¹³)-NH-R¹⁴, -(CHR¹³)-N(CH₃)-R¹⁴, -(CHR¹³)-(CH₂)-(CH₂)-O-R¹⁴, -(CHR¹³)-(CH₂)-(CH₂)-S-R¹⁴, -(CHR¹³)-(CH₂)-(CH₂)-NH-R¹⁴, -(CHR¹³)-(CH₂)-(CH₂)-N(CH₃)-R¹⁴, -(CHR¹³)-O-(CH₂)-R¹⁴, -(CHR¹³)-S-(CH2)-R¹⁴ oder -(CHR¹³)-NH-(CH₂)-R¹⁴ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂,-S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl; Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H,-S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-CF₃, -C(=O)-C₂F₅, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-NH₂, -C(=O)-NH-CH₃, -C(=O)-NH-C₂H₅, -C(=O)-NH-C(CH₃)₃,-C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R², R¹¹ bis R¹⁴, a, b, c, d, n, w, K und L die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin
R⁴ für -(CHR¹⁵)-R¹⁶, -(CHR¹⁵)-(CH₂)-R¹⁶, -(CHR¹⁵)-(CH₂)-(CH₂)-R¹⁶, -(CHR¹⁵)-O-R¹⁶, -(CHR¹⁵)-S-R¹⁶, -(CHR¹⁵)-NH-R¹⁶, -(CHR¹⁵)-N(CH₃)-R¹⁶, -(CHR¹⁵)-(CH₂)-O-R¹⁶,-(CHR¹⁵)-(CH₂)-S-R¹⁶, -(CHR¹⁵)-NH-R¹⁶, -(CHR¹⁵)-N(CH₃)-R¹⁶, -(CHR¹⁵)-(CH₂)-(CH₂)-O-R¹⁶, -(CHR¹⁵)-(CH₂)-(CH₂)-S-R¹⁶, -(CHR¹⁵)-(CH₂)-(CH₂)-NH-R¹⁶, -(CHR¹⁵)-(CH₂)-(CH₂)-N(CH₃)-R¹⁶, -(CHR¹⁵)-O-(CH₂)-R¹⁶, -(CHR¹⁵)-S-(CH₂)-R¹⁶ oder -(CHR¹⁵)-NH-(CH₂)-R¹⁶ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, - S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, - S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, - (CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, - N(CH₃)(C₂H₅), -C(=O)-H, -C(=O)-CH₃, -C(=O)-C₂H₅, -C(=O)-C(CH₃)₃, -C(=O)-CF₃, - C(=O)-C₂F₅, -C(=O)-NH₂, -C(=O)-NH-CH₃, -C(=O)-NH-C₂H₅, -C(=O)-NH-C(CH₃)₃, - C(=O)-N(CH₃)₂, -C(=O)-N(C₂H₅)₂, -S(=O)₂-CH₃, -S(=O)₂-C₂H₅, -NH-S(=O)₂-CH₃, - S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R², R¹⁵, R¹⁶, e, f, g, h, n, M und P die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R⁵ für -(CHR¹⁷)-R¹⁸, -(CHR¹⁷)-(CH₂)-R¹⁸, -(CHR¹⁷)-(CH₂)-(CH₂)-R¹⁸, -(CHR¹⁷)-O-R¹⁸, -(CHR¹⁷)-S-R¹⁸, -(CHR¹⁷)-NH-R¹⁸, -(CHR¹⁷)-N(CH₃)-R¹⁸, -(CHR¹⁷)-(CH₂)-O-R¹⁸, - (CHR¹⁷)-(CH₂)-S-R¹⁸, -(CH-R¹⁷)-NH-R¹⁸, -(CHR¹⁷)-N(CH₃)-R¹⁸, -(CHR¹⁷)-(CH₂)-(CH₂)-O-R¹⁸, -(CHR¹⁷)-(CH₂)-(CH₂)-S-R¹⁸, -(CHR¹⁷)-(CH₂)-(CH₂)-NH-R¹⁸, -(CHR¹⁷)-(CH₂)-(CH₂)-N(CH₃)-R¹⁸, -(CHR¹⁷)-O-(CH₂)-R¹⁸, -(CHR¹⁷)-S-(CH₂)-R¹⁸ oder -(CHR¹⁷)-NH-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂,-S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I,-CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl,-NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-H, -C(=O)-CH₃, -C(=O)-C₂H₅, -C(=O)-C(CH₃)₃, -C(=O)-CF₃,-C(=O)-C₂F₅, -C(=O)-NH₂, -C(=O)-NH-CH₃, -C(=O)-NH-C₂H₅, -C(=O)-NH-C(CH₃)₃,-C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R², R¹⁷, R¹⁸, k, l, m, n, o, Q und T die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin

R⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, - S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I,-CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl,-NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-H, -C(=O)-CH₃, -C(=O)-C₂H₅, -C(=O)-C(CH₃)₃, -C(=O)-CF₃,-C(=O)-C₂F₅, -C(=O)-NH₂, -C(=O)-NH-CH₃, -C(=O)-NH-C₂H₅, -C(=O)-NH-C(CH₃)₃,-C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃, -S(=O)₂-NH₂, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R² und n die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R⁷ für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, - C(=O)-CH₃, -C(=O)-C₂H₅, -C(=O)-C(CH₃)₃, -C(=O)-CF₃, -C(=O)-C₂F₅, -C(=O)-NH₂, - C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und-S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I,-CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, - NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-H, -C(=O)-CH₃, -C(=O)-C₂H₅, -C(=O)-C(CH₃)₃, -C(=O)-CF₃,-C(=O)-C₂F₅, -C(=O)-NH₂, -C(=O)-NH-CH₃, -C(=O)-NH-C₂H₅, -C(=O)-NH-C(CH₃)₃,-C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃, -S(=O)₂-NH₂, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R², D, E, aa, bb, cc, dd und n die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R⁸ für -(CHR¹⁹)-R²⁰_{,} -(CHR¹⁹)-(CH₂)-R²⁰, -(CHR¹⁹)-(CH₂)-(CH₂)-R²⁰, -(CHR¹⁹)-O-R²⁰, -(CHR¹⁹)-S-R²⁰, -(CHR¹⁹)-NH-R²⁰, -(CHR¹⁹)-N(CH₃)-R²⁰, -(CHR¹⁹)-(CH₂)-O-R²⁰,-(CHR¹⁹)-(CH₂)-S-R²⁰, -(CHR¹⁹)-NH-R²⁰, -(CHR¹⁹)-N(CH₃)-R²⁰, -(CHR¹⁹)-(CH₂)-(CH₂)-O-R²⁰, -(CHR¹⁹)-(CH₂)-(CH₂)-S-R²⁰, -(CHR¹⁹)-(CH₂)-(CH₂)-NH-R²⁰, -(CHR¹⁹)-(CH₂)-(CH₂)-N(CH₃)-R²⁰, -(CHR¹⁹)-O-(CH₂)-R²⁰, -(CHR¹⁹)-S-(CH₂)-R²⁰, -(CHR¹⁹)-NH-(CH₂)-R²⁰, -(CHR¹⁹)-O-(CH₂)-(CH₂)-O-R²⁰, -(CHR¹⁹)-O-(CH₂)-(CH₂)-S-R²⁰ oder -(CHR¹⁹)-S-(CH₂)-(CH₂)-S-R²⁰ steht;
für -(CH=CH)-R²¹ steht;
für -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶, -(CR²²R²³)-O-(CR²⁴R²⁵)-C(=O)-O-R²⁶, -(CR²²R²³)-S-(CR²⁴R²⁵)-C(=O)-O-R²⁶ oder -(CR²²R²³)-NH-(CR²⁴R²⁵)-C(=O)-O-R²⁶ steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl,-(CH₂)-(CH₂)-(C(CH₃)₃), n-Hexyl, 2-Hexyl, 3-Hexyl, n-Heptyl, 2-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂,-(CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅,-C(=O)-NH-C(CH₃)₃, -C(=O)-N(CH₃)₂, -C(=O)-N(C₂H₅)₂, -S(=O)₂-CH₃, -S(=O)₂-C₂H₅,-NH-S(=O)₂-CH₃, -S(=O)₂-NH-CH₃, Phenyl und -S(=O)₂-NH₂ substituiert sein kann; wobei der Phenyl-Rest jeweils mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl,-O-CH₃, -O-C₂H₅, -O-CH(CH₃)₂, -O-CH₂-CH₂-CH₃, -O-C(CH₃)₃ und -O-CH₂-CH₂-CH₂-CH₃ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, - NH-C(=O)-O-C(CH₃)₃, -O-CH₂-CH₂-CH₂-CH₃, -NH-C(=O)-CH₃, -NH-C(=O)-C₂H₅, - NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, - NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, - C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-Phenyl, Phenyl, -O-Phenyl, -O-Benzyl und Benzyl substituiert sein kann, wobei jeweils der zyklische Teil der Reste -S(=O)₂-NH-Phenyl, Phenyl, -O-Phenyl, -O-Benzyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
und jeweils R¹, R², R¹⁹ bis R³², p, q, n, r, s, t, u, v, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin
R⁹ und R¹⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
und jeweils R¹, R² und n die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R¹¹, R¹³, R¹⁵, R¹⁷, R¹⁹, R²², R²³, R²⁴, R²⁵ und R²⁶, unabhängig voneinander, jeweils
für einen Wasserstoff-Rest oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
und jeweils R¹ bis R¹⁰, R¹², R¹⁴, R¹⁶, R¹⁸, R²⁰, R²¹, R²⁷ bis R³², a, b, c, d, e, f, g, h, k, l, m, n, o, p, q, r, s, t, u, v, w, K, L, Q, T, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1-Oxa-3,8-diazaspiro[4.5]-decan-2-on-Verbindungen der vorstehend angegebenen allgemeinen Formel I, worin
R¹², R²⁸ und R³², unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
und jeweils R¹ bis R¹¹, R¹³ bis R²⁷, R²⁹, R³⁰, R³¹, a, b, c, d, e, f, g, h, k, l, m, n, o, p, q, r, s, t, u, v, w, K, L, Q, T, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R¹⁴, R¹⁶, R¹⁸ und R²⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl stehen, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, - C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) und S(=O)₂-NH₂ substituiert sein kann;
und jeweils R¹ bis R¹³, R¹⁵, R¹⁷, R¹⁹, R²¹ bis R³², a, b, c, d, e, f, g, h, k, l, m, n, o, p, q, r, s, t, u, v, w, K, L, Q, T, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1-Oxa-3,8-diazaspiro[4.5]-decan-2-on-Verbindungen der vorstehend angegebenen allgemeinen Formel I, worin
R²¹, R²⁷, R²⁹, R³⁰ und R³¹, unabhängig voneinander, jeweils für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, - NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) und S(=O)₂-NH₂ substituiert sein kann;
und jeweils R¹ bis R²⁰, R²² bis R²⁶, R²⁸, R³², a, b, c, d, e, f, g, h, k, l, m, n, o, p, q, r, s, t, u, v, w, K, L, Q, T, V, W und Y die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 1-Oxa-3,8-diazaspiro[4.5]-decan-2-on-Verbindungen der vorstehend angegebenen allgemeinen Formel I, worin
- n: gleich 1, 2 oder 3 ist;
- R¹: für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Oxazolyl, Isoxazolyl, Pyridazinyl, Pyrazinyl, Pyrimidinyl, Indazolyl, Chinazolinyl. Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, Methyl, Ethyl, n-Propyl, Isopropyl, n-Butyl, sec-Butyl, Isobutyl, tert-Butyl, n-Pentyl, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl, - O-CH₃, -O-C₂H₅, -O-CH(CH₃)₂, -O-CH₂-CH₂-CH₃ und -O-C(CH₃)₃ substituiert sein kann;
- R²: für -C(=S)-NH-R³;
für -C(=O)-NH-R⁴;
für -S(=O)₂-R⁵;
für -(CH₂)-C(=O)-NH-R⁶;
für -(CH₂)-O-R⁷, für -(CH₂)-S-R⁷, für -(CH₂)-NH-R⁷, für -(CH₂)-N(CH₃)-R⁷, für - (CH₂)-(CH₂)-O-R⁷, für -(CH₂)-(CH₂)-S-R⁷, für -(CH₂)-NH-R⁷, für -(CH₂)-N(CH₃)-R⁷, für -(CH₂)-(CH₂)-(CH₂)-O-R⁷, für -(CH₂)-(CH₂)-(CH₂)-S-R⁷, für -(CH₂)-(CH₂)-(CH₂)-NH-R⁷, für -(CH₂)-(CH₂)-(CH₂)-N(CH₃)-R⁷, für -(CH₂)-O-(CH₂)-R⁷,
für -(CH₂)-S-(CH₂)-R⁷; für -(CH₂)-NH-(CH₂)-R⁷;
für -C(=O)-R⁸
oder für -S(=O)₂-NR⁹R¹⁰ steht;
- R³: für -(CHR¹¹)-C(=O)-O-R¹² oder -(CHR¹¹)-(CH₂)-C(=O)-O-R¹² steht;
für -(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-(CH₂)-O-R¹⁴, -(CHR¹³)-(CH₂)-(CH₂)-O-R¹⁴ oder -(CHR¹³)-O-(CH₂)-R¹⁴ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃ und -C(=O)-C₂F₅ substituiert sein kann;
- R⁴: für -(CHR¹⁵)-R¹⁶, -(CHR¹⁵)-(CH₂)-R¹⁶ oder -(CHR¹⁵)-(CH₂)-(CH₂)-R¹⁶ steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -NO₂, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CH₃, -C(=O)-C₂H₅, -C(=O)-C(CH₃)₃, - C(=O)-CF₃ und -C(=O)-C₂F₅ substituiert sein kann;
- R⁵: für -(CHR¹⁷)-R¹⁸, -(CHR¹⁷)-(CH₂)-R¹⁸ oder -(CHR¹⁷)-(CH₂)-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, - CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ und-S(=O)₂-C₂H₅ substituiert sein kann;
- R⁶: für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl und Br substituiert sein kann;
- R⁷: für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,4]-Oxadiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃,-SF₅, -OH, -O-CH₃, -O-C₂H₅, -NO₂, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl,-C(CH₃)₂-C₂H₅, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃,-O-C(CH₃)₃ und -O-CH₂-CH₂-CH₂-CH₃ substituiert sein kann;
- R⁸: für -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ oder -(CHR¹⁹)-O-(CH₂)-(CH₂)-O-R²⁰ steht;
für -(CH=CH)-R²¹ steht;
für -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ oder -(CR²²R²³)-O-(CR²⁴R²⁵)-C(=O)-O-R²⁶ steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl steht, wobei der Rest jeweils ggf. mit 1, 2, 3, 4 oder 5 Substituenten unabhängig voneinander ausgewählt aus der Gruppe bestehend aus -C(=O)-CH₃, -C(=O)-C₂H₅, -C(=O)-C(CH₃)₃ und Phenyl substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, Phenyl, -O-Phenyl, -O-Benzyl und Benzyl substituiert sein kann, wobei jeweils der zyklische Teil der Reste Phenyl, -O-Phenyl, -O-Benzyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl,-O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
- R⁹ und R¹⁰,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
- R¹¹, R¹³, R¹⁵, R¹⁷, R¹⁹,:
- R²², R²³, R²⁴, R²⁵ und R²⁶,: unabhängig voneinander, jeweils für einen Wasserstoff-Rest oder für einen Rest ausgewählt aus der Gruppe bestehend aus Methyl, Ethyl, n-Propyl, Isopropyl, n-Butyl, sec-Butyl, Isobutyl und tert-Butyl stehen;
- R¹², R²⁸ und R³²,: unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
- R¹⁴, R¹⁶, R¹⁸ und R²⁰,: unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl, Cyclohexyl, Cycloheptyl, Tetrahydrofuranyl, Tetrahydrothiophenyl, Tetrahydropyranyl und Bicyclo[2.2.1]heptyl stehen, wobei der Rest jeweils ggf. mit 1, 2, 3, 4 oder 5 Substituenten unabhängig voneinander ausgewählt aus der Gruppe bestehend aus Oxo (=O), Thioxo (=S), Methyl, Ethyl, n-Propyl, Isopropyl, n-Butyl, sec-Butyl, Isobutyl, tert-Butyl, n-Pentyl, n-Hexyl, n-Heptyl, -(CH₂)-C(=O)-OH und-C(=O)-OH substituiert sein kann;
oder für einen Rest ausgewählt aus der Gruppe bestehend aus Phenyl und Naphthyl stehen, wobei der Rest jeweils mit 1, 2, 3, 4 oder 5 Substituenten unabhängig voneinander ausgewählt aus der Gruppe bestehend aus F, Cl, Br, -O-CH₃, -O-C₂H₅,-O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl und n-Heptyl substituiert sein kann;
und
- R²¹, R²⁷, R²⁹, R³⁰ und R³¹,: unabhängig voneinander, jeweils für einen Rest ausgewählt aus der Gruppe bestehend aus Phenyl, Naphthyl, Indolyl und Isoindolyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃,-O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, -C(CH₃)₂-C₂H₅, n-Hexyl und n-Heptyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte 1-Oxa-3,8-diazaspiro[4.5]-decan-2-on-Verbindungen der vorstehend angegebenen allgemeinen Formel I, worin
- n: gleich 1 oder 2 ist;
- R¹: für einen Rest ausgewählt aus der Gruppe bestehend aus Phenyl und Naphthyl
steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, Methyl, Ethyl, n-Propyl, Isopropyl, n-Butyl, sec-Butyl, Isobutyl, tert-Butyl, n-Pentyl, n-Hexyl, n-Heptyl, Phenyl und Benzyl substituiert sein kann;
- R²: für -C(=S)-NH-R³:
für -C(=O)-NH-R⁴;
für -S(=O)₂-R⁵;
für -(CH₂)-C(=O)-NH-R⁶;
für -(CH₂)-O-R⁷, für -(CH₂)-(CH₂)-O-R⁷, für -(CH₂)-(CH₂)-(CH₂)-O-R⁷;
für -C(=O)-R⁸
oder für -S(=O)₂-NR⁹R¹⁰ steht;
- R³: für-(CHR¹¹)-(CH₂)-C(=O)-O-R¹² steht;
für -(CHR¹³)-R¹⁴, -(CHR¹³)-(CH₂)-R¹⁴, -(CHR¹³)-(CH₂)-(CH₂)-R¹⁴ oder - (CHR¹³)-(CH₂)-O-R¹⁴ steht;
für einen Rest ausgewählt aus der Gruppe bestehend aus Vinyl, 1-Propenyl, 2-Propenyl, 1-Butenyl, 2-Butenyl und 3-Butenyl steht;
oder für einen Phenyl-Rest steht, der Rest mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃,-O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅,-C(=O)-C(CH₃)₃, -C(=O)-CF₃ und -C(=O)-C₂F₅ substituiert sein kann;
- R⁴: für -(CHR¹⁵)-R¹⁶, -(CHR¹⁵)-(CH₂)-R¹⁶ oder -(CHR¹⁵)-(CH₂)-(CH₂)-R¹⁶ steht;
oder für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus F, Cl, Br, -NO₂, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-O-CH₃,-C(=O)-O-CH₂-CH₃, -C(=O)-O-CH(CH₃)₂, -C(=O)-O-C(CH₃)₃, -C(=O)-CH₃,-C(=O)-C₂H₅, -C(=O)-C(CH₃)₃, -C(=O)-CF₃ und -C(=O)-C₂F₅ substituiert sein kann;
- R⁵: für -(CHR¹⁷)-R¹⁸, -(CHR¹⁷)-(CH₂)-R¹⁸ oder -(CHR¹⁷)-(CH₂)-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
oder für einen Rest ausgewählt aus der Gruppe bestehend aus Phenyl, Naphthyl, Pyrazolyl, Thiophenyl, [1,2,3,4]-Tetrahydrochinolinyl und [1,2,3,4]-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ und-S(=O)₂-C₂H₅ substituiert sein kann;
- R⁶: für einen Rest ausgewählt aus der Gruppe bestehend aus Phenyl, Naphthyl, Pyrazolyl, Thiophenyl und Thiazolyl steht, wobei der Rest jeweils ggf. mit 1, 2, 3, 4 oder 5 Substituenten unabhängig voneinander ausgewählt aus der Gruppe bestehend aus F, Cl, Br, -CF₃, -CN, -O-CH₃, -O-C₂H₅, -O-CH(CH₃)₂,-O-CH₂-CH₂-CH₃, -O-C(CH₃)₃, -O-CH₂-CH₂-CH₂-CH₃, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl und Br substituiert sein kann;
- R⁷: für einen Rest ausgewählt aus der Gruppe bestehend aus Phenyl, Naphthyl, Benzo[b]furanyl, Benzo[b]thiophenyl und [1,2,4]-Oxadiazolyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -SF₅, -OH,-O-CH₃, -O-C₂H₅, -NO₂, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃,-O-C(CH₃)₃ und -O-CH₂-CH₂-CH₂-CH₃ substituiert sein kann;
- R⁸: für -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ oder -(CHR¹⁹)-O-(CH₂)-(CH₂)-O-R²⁰ steht;
für -(CH=CH)-R²¹ steht;
für -(CR²²R²³)-C(=O)-O-R²⁶, -(CR²²R²¹)-(CR²⁴R²⁵)-C(=O)-O-R²⁶, -(CR²²R²³)-(CR²⁴R²⁵)-(CH₂)-C(=O)-O-R²⁶ oder -(CR²²R²³)-O-(CR²⁴R²⁵)-C(=O)-O-R²⁶ steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl steht;
für einen Rest ausgewählt aus der Gruppe bestehend aus Cyclopropyl, Cyclobutyl, Cyclopentyl, Cyclohexyl, Cycloheptyl, Pyrrolidinyl, Piperidinyl und Adamantyl steht, wobei der Rest jeweils ggf. mit 1, 2, 3, 4 oder 5 Substituenten unabhängig voneinander ausgewählt aus der Gruppe bestehend aus -C(=O)-CH₃, -C(=O)-C₂H₅, -C(=O)-C(CH₃)₃ und Phenyl substituiert sein kann;
oder für einen Rest ausgewählt aus der Gruppe bestehend aus Phenyl, Naphthyl, 2H-Chromenyl, Thiophenyl, Furanyl, Pyrazolyl, Triazolyl, Pyridinyl, [1,2,3]-Thiadiazolyl, [2,1,3]-Benzoxadiazolyl, [1,2,3]-Benzoxadiazolyl, Oxazolyl und Isoxazolyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, Phenyl, -O-Phenyl, -O-Benzyl und Benzyl substituiert sein kann, wobei jeweils der zyklische Teil der Reste Phenyl, -O-Phenyl, -O-Benzyl und Benzyl mit 1, 2, 3, 4 oder 5 Substituenten unabhängig voneinander ausgewählt aus der Gruppe bestehend aus F, Cl und Br substituiert sein kann;
- R⁹ und R¹⁰,: unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
- R¹¹, R¹³, R¹⁵, R¹⁷, R¹⁹, R²²,:
- R²³, R²⁴, R²⁵ und R²⁶,: unabhängig voneinander, jeweils für einen Wasserstoff-Rest oder für einen Rest ausgewählt aus der Gruppe bestehend aus Methyl, Ethyl und n-Propyl stehen;
- R¹², R²⁸ und R³²,: unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
- R¹⁴: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Tetrahydrofuranyl, Tetrahydrothiophenyl und Tetrahydropyranyl steht;
oder für einen Phenyl-Rest steht, der mit 1, 2, 3, 4 oder 5 Substituenten ausgewählt aus der Gruppe bestehend aus F, Cl und Br substituiert sein kann;
- R¹⁶: für einen Rest ausgewählt aus der Gruppe bestehend aus Phenyl und Naphthyl steht, wobei der Rest jeweils mit 1, 2, 3, 4 oder 5 Substituenten unabhängig voneinander ausgewählt aus der Gruppe bestehend aus F, Cl und Br substituiert sein kann;
- R¹⁸: für einen 7,7-Dimethyl-2-oxo-bicyclo[2.2.1]hept-1-yl-Rest steht;
- R²⁰: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl und Cycloheptyl steht; wobei der Rest jeweils mit einem Substituenten ausgewählt aus der Gruppe bestehend aus -(CH₂)-C(=O)-OH und-C(=O)-OH substituiert sein kann;
oder für Phenyl-Rest steht, der mit 1, 2, 3, 4 oder 5 Substituenten unabhängig voneinander ausgewählt aus der Gruppe bestehend aus F, Cl, Br, -O-CH₃, -O-C₂H₅,-O-CH(CH₃)₂, -O-CH₂-CH₂-CH₃, -O-C(CH₃)₃ und -O-CH₂-CH₂-CH₂-CH₃, substituiert sein kann;
- R²¹: für einen Phenyl-Rest steht, der mit 1, 2, 3, 4 oder 5 Substituenten ausgewählt aus der Gruppe bestehend aus F, Cl und Br substituiert sein kann;
- R²⁷: für einen Phenyl-Rest steht;
- R²⁹: für einen Phenyl-Rest steht;
- R³⁰: für einen Phenyl-Rest steht, der mit 1, 2, 3, 4 oder 5 Substituenten ausgewählt aus der Gruppe bestehend aus Methyl, Ethyl, n-Propyl, Isopropyl, n-Butyl, sec-Butyl und tert-Butyl substituiert sein kann;
und
- R³¹: für einen Indolyl-Rest steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind 1-Oxa-3,8-diazaspiro[4.5]-decan-2-on-Verbindungen ausgewählt aus der Gruppe bestehend aus
[1] 3-(4-Methyl-benzyl)-2-oxo-1-oxa-3,8-diaza-spiro[4.5]decan-8-carbothiosäure-pentafluorphenyl-amid
[2] 3-(3-Brom-benzyl)-2-oxo-1-oxa-3,8-diaza-spiro[4.5]decan-8-carbothiosäure-(2,5-dichlor-phenyl)-amid
[3] 2-Oxo-3-(4-trifluormethyl-benzyl)-1-oxa-3,8-diaza-spiro[4.5]decan-8-carbothiosäurebenzylamid
[4] 3-(3,5-Dimethyl-benzyl)-2-oxo-1-oxa-3,8-diaza-spiro[4.5]decan-8-carbothiosäure-(tetrahydro-furan-2-ylmethyl)-amid
[5] 3-(3-Brom-benzyl)-2-oxo-1-oxa-3,8-diaza-spiro[4.5]decan-8-carbothiosäure-(2-methoxy-ethyl)-amid
[6] 3-(3-Brom-benzyl)-2-oxo-1-oxa-3,8-diaza-spiro[4.5]decan-8-carbothiosäure-(2-methoxy-phenyl)-amid
[7] 3-(3,5-Dimethyl-benzyl)-2-oxo-1-oxa-3,8-diaza-spiro[4.5]decan-8-carbothiosäure-(3-acetyl-phenyl)-amid
[8] 3-Benzyl-2-oxo-1-oxa-3,8-diaza-spiro[4.5]decan-8-carbothiosäure-o-tolylamid
[9] 3-{[3-(3-Brom-benzyl)-2-oxo-1-oxa-3,8-diaza-spiro[4.5]decan-8-carbothioyl]-amino}-butansäureethylester
[10] 3-Naphthalen-2-ylmethyl-2-oxo-1-oxa-3,8-diaza-spiro[4.5]decan-8-carbothiosäureallylamid
[11] 3-(3,4-Difluor-benzyl)-2-oxo-1-oxa-3,8-diaza-spiro[4.5]decan-8-carbothiosäurebenzylamid
[12] 3-Biphenyl-2-ylmethyl-2-oxo-1-oxa-3,8-diaza-spiro[4.5]decan-8-carbothiosäure-(1-phenyl-ethyl)-amid
[13] 3-(3,4-Difluor-benzyl)-2-oxo-1-oxa-3,8-diaza-spiro[4.5]decan-8-carbothiosäure-(4-ethoxy-phenyl)-amid
[14] 3-(3-Cyano-benzyl)-2-oxo-1-oxa-3,8-diaza-spiro[4.5]decan-8-carbothiosäure-(3,5-dichlor-phenyl)-amid
[15] 3-(3-Cyano-benzyl)-2-oxo-1-oxa-3,8-diaza-spiro[4.5]decan-8-carbothiosäure-(3-trifluormethyl-phenyl)-amid
[16] 3-(3-Brom-benzyl)-2-oxo-1-oxa-3,8-diaza-spiro[4.5]decan-8-carbothiosäure-benzylamid
[17] 3-(2-Methoxy-5-nitro-benzyl)-2-oxo-1-oxa-3,8-diaza-spiro[4.5]decan-8-carbothiosäure-(tetrahydro-furan-2-ylmethyl)-amid
[18] 3-Biphenyl-2-ylmethyl-2-oxo-1-oxa-3,8-diaza-spiro[4.5]decan-8-carbothiosäure-phenylamid
[19] 3-(3-Cyano-benzyl)-2-oxo-1-oxa-3,8-diaza-spiro[4.5]decan-8-carbothiosäure-cyclohexylmethyl-amid
[20] 3-(2-Fluor-benzyl)-2-oxo-1-oxa-3,8-diaza-spiro[4.5]decan-8-carbothiosäure-(3-acetyl-phenyl)-amid
[21] 3-(2-Methoxy-5-nitro-benzyl)-2-oxo-1-oxa-3,8-diaza-spiro[4.5]decan-8-carbothiosäure-cyclohexylmethyl-amid
[22] 3-(3-Cyano-benzyl)-2-oxo-1-oxa-3,8-diaza-spiro[4.5]decan-8-carbothiosäure-4-chlor-benzylamid
[23] 3-(2-Methoxy-5-nitro-benzyl)-2-oxo-1-oxa-3,8-diaza-spiro[4.5]decan-8-carbothiosäure-benzylamid
[24] 3-(2-Fluor-benzyl)-2-oxo-1-oxa-3,8-diaza-spiro[4.5]decan-8-carbonsäure-(4-ethoxy-phenyl)-amid
[25] 3-Biphenyl-2-ylmethyl-2-oxo-1-oxa-3,8-diaza-spiro[4.5]decan-8-carbonsäure-(2,5-dichlor-phenyl)-amid
[26] 3-(2-Methoxy-5-nitro-benzyl)-2-oxo-1-oxa-3,8-diaza-spiro[4.5]decan-8-carbonsäure-(2,5-difluor-phenyl)-amid
[27] 3-(4-Methyl-benzyl)-2-oxo-1-oxa-3,8-diaza-spiro[4.5]decan-8-carbonsäure-(4-methyl-3-nitro-phenyl)-amid
[28] 3-(3,4-Difluor-benzyl)-2-oxo-1-oxa-3,8-diaza-spiro[4.5]decan-8-carbonsäure-4-fluor-benzylamid
[29] 3-(3-Brom-benzyl)-2-oxo-1-oxa-3,8-diaza-spiro[4.5]decan-8-carbonsäure-(2,5-difluor-phenyl)-amid
[30] 4-[(3-Naphthalen-2-ylmethyl-2-oxo-1-oxa-3,8-diaza-spiro[4.5]decan-8-carbonyl)-amino]-benzoesäure-ethyl-ester
[31] 2-Oxo-3-(4-trifluormethyl-benzyl)-1-oxa-3,8-diaza-spiro[4.5]decan-8-carbonsäure-(4-chlor-3-trifluormethyl-phenyl)-amid
[32] 3-(2-Cyano-benzyl)-2-oxo-1-oxa-3,8-diaza-spiro[4.5]decan-8-carbonsäure-(2,5-difluor-phenyl)-amid
[33] 3-Benzyl-2-oxo-1-oxa-3,8-diaza-spiro[4.5]decan-8-carbonsäure-(3-cyano-phenyl)-amid
[34] 3-(3,4-Difluor-benzyl)-2-oxo-1-oxa-3,8-diaza-spiro[4.5]decan-8-carbonsäure-(4-methyl-3-nitro-phenyl)-amid
[35] 3-(3,4-Difluor-benzyl)-2-oxo-1-oxa-3,8-diaza-spiro[4.5]decan-8-carbonsäure-(3-methoxy-phenyl)-amid
[36] 3-(4-Methyl-benzyl)-2-oxo-1-oxa-3,8-diaza-spiro[4.5]decan-8-carbonsäure-3,4-dichlor-benzylamid
[37] 3-Benzyl-2-oxo-1-oxa-3,8-diaza-spiro[4.5]decan-8-carbonsäure-(4-chlor-3-trifluormethyl-phenyl)-amid
[38] 2-Oxo-3-(4-trifluormethylsulfanyl-benzyl)-1-oxa-3,8-diaza-spiro[4.5]decan-8-carbonsäure-(3-cyano-phenyl)-amid
[39] 3-Benzyl-2-oxo-1-oxa-3,8-diaza-spiro[4.5]decan-8-carbonsäure-(4-methoxy-phenyl)-amid
[40] 2-Oxo-3-(4-trifluormethylsulfanyl-benzyl)-1-oxa-3,8-diaza-spiro[4.5]decan-8-carbonsäure-(4-methyl-3-nitro-phenyl)-amid
[41] 3-(4-lod-benzyl)-2-oxo-1-oxa-3,8-diaza-spiro[4.5]decan-8-carbonsäure-(4-butoxy-phenyl)-amid
[42] 3-(3-Brom-benzyl)-2-oxo-1-oxa-3,8-diaza-spiro[4.5]decan-8-carbonsäure-3,4-dichlor-benzylamid
[43] 3-Benzyl-2-oxo-1-oxa-3,8-diaza-spiro[4.5]decan-8-carbonsäure-(1-phenylethyl)-amid
[44] 3-Naphthalen-2-ylmethyl-2-oxo-1-oxa-3,8-diaza-spiro[4.5]decan-8-carbonsäure-3,4-dichlor-benzylamid
[45] 3-(4-Methyl-benzyl)-2-oxo-1-oxa-3,8-diaza-spiro[4.5]decan-8-carbonsäure-phenethyl-amid
[46] 2-Oxo-3-(2-trifluormethyl-benzyl)-1-oxa-3,8-diaza-spiro[4.5]decan-8-carbonsäure-(3-methoxy-phenyl)-amid
[47] 3-(4-Cyano-benzyl)-2-oxo-1-oxa-3,8-diaza-spiro[4.5]decan-8-carbonsäure-(4-methoxy-phenyl)-amid
[48] 2-Oxo-3-(4-trifluormethyl-benzyl)-1-oxa-3,8-diaza-spiro[4.5]decan-8-carbonsäure-(1-naphthalen-1-yl-ethyl)-amid
[49] 2-Oxo-3-(2-trifluormethyl-benzyl)-1-oxa-3,8-diaza-spiro[4.5]decan-8-carbonsäure-phenethyl-amid
[50] 3-(4-lod-benzyl)-2-oxo-1-oxa-3,8-diaza-spiro[4.5]decan-8-carbonsäure-(4-methyl-3-nitro-phenyl)-amid
[51] 3-(2-Methoxy-5-nitro-benzyl)-2-oxo-1-oxa-3,8-diaza-spiro[4.5]decan-8-carbonsäure-(3-cyano-phenyl)-amid
[52] 3-Naphthalen-2-ylmethyl-2-oxo-1-oxa-3,8-diaza-spiro[4.5]decan-8-c carbonsäure-(4-methoxy-phenyl)-amid
[53] 3-{[3-(4-Methyl-benzyl)-2-oxo-1-oxa-3,8-diaza-spiro[4.5]decan-8-carbonyl]-amino}-benzoesäure-ethyl-ester
[54] 2-Oxo-3-(2-trifluormethyl-benzyl)-1-oxa-3,8-diaza-spiro[4.5]decan-8-carbonsäure-(1-naphthalen-1-yl-ethyl)-amid
[55] 3-Naphthalen-2-ylmethyl-2-oxo-1-oxa-3,8-diaza-spiro[4.5]decan-8-carbonsäure-(2,5-dimethoxy-phenyl)-amid
[56] 3-(4-Cyano-benzyl)-2-oxo-1-oxa-3,8-diaza-spiro[4.5]decan-8-carbonsäure-(3-acetyl-phenyl)-amid
[57] 3-Biphenyl-2-ylmethyl-2-oxo-1-oxa-3,8-diaza-spiro[4.5]decan-8-carbonsäure-phenethyl-amid
[58] 3-(4-Cyano-benzyl)-2-oxo-1-oxa-3,8-diaza-spiro[4.5]decan-8-carbonsäure-(5-chlor-2-methoxy-phenyl)-amid
[59] 3-{[2-Oxo-3-(2-trifluormethyl-benzyl)-1-oxa-3,8-diaza-spiro[4.5]decan-8-carbonyl]-amino}-benzoesäure-ethyl-ester
[60] 3-Benzyl-2-oxo-1-oxa-3,8-diaza-spiro[4.5]decan-8-carbonsäure-(3-fluorphenyl)-amid
[61] 3-{[3-(3-Cyano-benzyl)-2-oxo-1-oxa-3,8-diaza-spiro[4.5]decan-8-carbonyl]-amino}-benzoesäure-ethyl-ester
[62] 3-Biphenyl-2-ylmethyl-2-oxo-1-oxa-3,8-diaza-spiro[4.5]decan-8-carbonsäure-(3-methoxy-phenyl)-amid
[63] 3-(2-Methoxy-5-nitro-benzyl)-2-oxo-1-oxa-3,8-diaza-spiro[4.5]decan-8-carbonsäure-(1-naphthalen-1-yl-ethyl)-amid
[64] {2-[3-(3,4-Difluor-benzyl)-2-oxo-1-oxa-3,8-diaza-spiro[4.5]dec-8-yl]-2-oxo-ethoxy}-essigsäure
[65] 4-[3-(3,4-Difluor-benzyl)-2-oxo-1-oxa-3,8-diaza-spiro[4.5]dec-8-yl]-3,3-dimethyl-4-oxo-butansäure
[66] [2-[3-(3-Brom-benzyl)-2-oxo-1-oxa-3,8-diaza-spiro[4.5]dec-8-yl]-1-(1H-indol-3-ylmethyl)-2-oxo-ethyl]-carbaminsäure-tert-butyl-ester
[67] 5-[3-(2-Fluor-benzyl)-2-oxo-1-oxa-3,8-diaza-spiro[4.5]dec-8-yl]-3-methyl-5-oxo-pentansäure
[68] 3,3-Dimethyl-5-[3-(4-methyl-benzyl)-2-oxo-l-oxa-3,8-diaza-spiro[4.5]dec-8-yl]-5-oxo-pentansäure
[69] 5-[3-(3.4-Difluor-benzyl)-2-oxo-1-oxa-3,8-diaza-spiro[4.5]dec-8-yl]-3-methyl-5-oxo-pentansäure
[70] {2-oxo-2-[2-oxo-3-(4-trifluormethyl-benzyl)-1-oxa-3,8-diaza-spiro[4.5]dec-8-yl]-ethoxy}-essigsäure
[71] (1-{2-[3-(2-Cyano-benzyl)-2-oxo-1-oxa-3,8-diaza-spiro[4.5]dec-8-yl]-2-oxo-ethyl}-cyclopentyl)-essigsäure
[72] (1-{2-[3-(3,4-Difluor-benzyl)-2-oxo-1-oxa-3,8-diaza-spiro[4.5]dec-8-yl]-2-oxo-ethyl}-cyclopentyl)-essigsäure
[73] 5-(3-Biphenyl-2-ylmethyl-2-oxo-1-oxa-3,8-diaza-spiro[4.5]dec-8-yl)-3-methyl-5-oxo-pentansäure
[75] N-[4-(3,5-Dichlor-phenylsulfamoyl)-phenyl]-2-[3-(4-methyl-benyl)-2-oxo-1-oxa-3,8-diaza-spiro[4.5]dec-8-yl]-acetamid
[76] N-(2,5-Dimethyl-2H-pyrazol-3-yl)-2-[3-(2-methoxy-5-nitro-benzyl)-2-oxo-1-oxa-3,8-diaza-spiro[4.5]dec-8-yl]-acetamid
[80] N-(3-Cyano-4-methyl-thiophen-2-yl)-2-[3-(3,4-difluor-benzyl)-2-oxo-1-oxa-3,8-diaza-spiro[4.5]dec-8-yl]-acetamid
[85] 2-[2-Oxo-3-(4-trifluormethylsulfanyl-benzyl)-1-oxa-3,8-diaza-spiro[4.5]dec-8-yl]-N-(4-phenyl-5-trifluormethyl-thiophen-3-yl)-acetamid
[86] 2-[3-(2-Fluor-benzyl)-2-oxo-1-oxa-3,8-diaza-spiro[4.5]dec-8-yl]-N-(4-phenylthiazol-2-yl)-acetamid
[87] 2-[2-Oxo-3-(2-trifluonnethyl-benzyl)-1-oxa-3,8-diaza-spiro[4,5]dec-8-yl]-N-(4-trifluormethoxy-phenyl)-acetamid
[91] N{4-[3-(3-Cyano-benzyl)-2-oxo-1-oxa-3,8-diaza-spiro[4.5]decan-8-carbonyl]-phenyl}-acetamid
[92] 8-(4-Ethoxy-benzoyl)-3-(4-methyl-benzyl)-1-oxa-3,8-diaza-spiro[4.5]decan-2-on
[93] 3-[3-(4-Cyano-benzyl)-2-oxo-1-oxa-3,8-diaza-spiro[4.5]dec-8-yl]-3-oxo-propionsäuremethylester
[94] 2-[8-(2,4-Difluor-benzoyl)-2-oxo-1-oxa-3,8-diaza-spiro[4.5]dec-3-ylmethyl]-benzonitril
[95] 8-(3-Dimethylamino-benzoyl)-3-(3,5-dimethyl-benzyl)-1-oxa-3,8-diaza-spiro[4.5]decan-2-on
[96] N-{4-[3-(3,4-Difluor-benzyl)-2-oxo-1-oxa-3,8-diaza-spiro[4.5]decan-8-carbonyl]-phenyl}-acetamid
[97] 8-(4-Brom-benzoyl)-3-(4-fluor-benzyl)-1-oxa-3,8-diaza-spiro[4.5]decan-2-on
[98] 4-[8-(Adamantan-1-carbonyl)-2-oxo-1-oxa-3,8-iaza-spiro[4.5]dec-3-ylmethyl]-benzonitril
[99] 3-(3,5-Dlmethyl-benzyl)-8-(2-ethylsulfanyl-pyridin-3-carbonyl)-1-oxa-3,8-diaza-spiro[4.5]decan-2-on
[100] 3-(3-Brom-benzyl)-8-(4-chlor-benzoyl)-1-oxa-3,8diaza-spiro[4.5]decan-2-on
[101] 8-[3-(2-Chlorphenyl)-5-methyl-isoxazol-4-carbonyl]-3-(3,5-dimethyl-benzyl)-1-oxa-3,8-diaza-spiro[4.5]decan-2-on
[102] 3-(3-Brom-benzyl)-8-(4-ethyl-benzylsulfanyl)-1-oxa-3,8-diaza-spiro[4.5]decan-2-on
[103] 8-(4-Butoxy-benzylsulfonyl)-3-(3,5-dimethyl-benzyl)-1-oxa-3,8-diaza-spiro[4.5]decan-2-on
[104] 8-(Biphenyl-4-carbonyl)-3-(3-brom-benzyl)-1-oxa-3,8-diaza-spiro[4.5]decan-2-on
[105] Essigsäure-2-[3-(3,5-dimethyl-benzyl)-2-oxo-1-oxa-3,8-diaza-spiro[4.5]dec-8-yl]-2-oxo-1-phenyl-ethylester
[106] 4-[2-Oxo-8-(2-phenyl-cyclopropancarbonyl)-1-oxa-3,8-diaza-spiro[4.5]dec-3-ylmethyl]-benzonitril
[107] 8-(2,5-Dimethoxy-benzylsulfonyl)-3-(3,5-dimethyl-benzyl)-1-oxa-3,8-diaza-spiro[4.5]decan-2-on
[108] 8-(2-Chlor-benzoyl)-3-(3,5-dimethyl-benzyl)-1-oxa-3,8-diaza-spiro[4.5]decan-2-on
[109] 3-(3-Brom-benzyl)-8-(4-nitro-benzoyl)-1-oxa-3,8-diaza-spiro[4.5]decan-2-on
[110] N-{1-Benzyl-2-[3-(3-cyano-benzyl)-2-oxo-1-oxa-3,8-diaza-spiro[4.5]dec-8-yl]-2-oxo-ethyl}-4-methyl-benzylsulfonamid
[111] 8-(2-Ethylsulfanyl-pyridin-3-carbonyl)-3-(3-methoxy-benzyl)-1-oxa-3,8-diaza-spiro[4.5]decan-2-on
[112] 8-(2,4-Dimethoxy-benzoyl)-3-(3-methoxy-benzyl)-1-oxa-3,8-diaza-spiro[4.5]decan-2-on
[113] 3-(3,5-Dimethyl-benzyl)-8-[2-(3-methoxy-phenyl)-acetyl]-1-oxa-3,8-diaza-spiro[4.5]decan-2-on
[114] 3-(3,5-Dimethyl-benzyl)-8-(4-methoxy-2,3,6-trimethyl-benzylsulfonyl)-1-oxa-3,8-diaza-spiro[4.5]decan-2-on
[115] 4-[8-(Biphenyl-4-carbonyl)-2-oxo-1-oxa-3,8-diaza-spiro[4.5]dec-3-ylmethyl]-benzonitril
[116] 8-(2-Chlor-benzylsulfonyl)-3-(3,5-dimethyl-benzyl)-1-oxa-3,8-diaza-spiro[4.5]decan-2-on
[117] 8-(Benzo[1,2,5]oxadiazol-5-carbonyl)-3-(3,5-dimethyl-benzyl)-1-oxa-3,8-diaza-spiro[4.5]decan-2-on
[118] 8-(Biphenyl-4-carbonyl)-3-(4-iod-benzyl)-1-oxa-3,8-diaza-spiro[4.5]decan-2-on
[119] 3-(3,5-Dimethyl-benzyl)-8-(pyridin-2-carbonyl)-1-oxa-3,8-diaza-spiro[4.5]decan-2-on
[120] 8-(6-Chlor-pyridin-3-carbonyl)-3-naphthalen-2-ylmethyl-1-oxa-3,8-diaza-spiro[4.5]decan-2-on
[121] 3-(3-Brom-benzyl)-8-(2-ethylsulfanyl-pyridin-3-carbonyl)-1-oxa-3,8-diaza-spiro[4.5]decan-2-on
[122] 3-(3,5-Dimethyl-benzyl)-8-(5-methyl-isoxazol-3-carbonyl)-1-oxa-3,8-diaza-spiro[4.5]decan-2-on
[123] 3-(2-Methoxy-5-nitro-benzyl)-8-pentanoyl-1-oxa-3,8-diaza-spiro[4.5]decan-2-on
[124] 8-(4-Brom-benzoyl)-3-(3,5-dimethyl-benzyl)-1-oxa-3,8-diaza-spiro[4.5]decan-2-on
[125] 8-(5-tert-Butyl-2-methyl-furan-3-carbonyl)-3-(2-methoxy-5-nitro-benzyl)-1-oxa-3,8-diaza-spiro[4.5]decan-2-on
[126] 8-(3-Chlor-benzoyl)-3-(3,5-dimethyl-benzyl)-1-oxa-3,8-diaza-spiro[4.5]decan-2-on
[127] 8-(2-Ethylsulfanyl-pyridin-3-carbonyl)-3-(2-trifluormethyl-benzyl)-1-oxa-3,8-diaza-spiro[4.5]decan-2-on
[128] 3-(3,5-Dimethyl-benzyl)-8-(isoxazol-5-carbonyl)-1-oxa-3,8-diaza-spiro[4.5]decan-2-on
[129] 3-(3-Brom-benzyl)-8-(pyridin-2-carbonyl)-1-oxa-3,8-diaza-spiro[4.5]decan-2-on
[130] 3-(3,5-Dimethyl-benzyl)-8-(thiophen-2-carbonyl)-1-oxa-3,8-diaza-spiro[4.5]decan-2-on
[131] 8-(3-Chlor-4-fluor-benzoyl)-3-(3,5-dimethyl-benzyl)-1-oxa-3,8-diaza-spiro[4.5]decan-2-on
[132] 8-(2,6-Difluor-3-methyl-benzoyl)-3-(3,5-dimethyl-benzyl)-1-oxa-3,8-diaza-spiro[4.5]decan-2-on
[133] 4-[3-(3-Brom-benzyl)-2-oxo-1-oxa-3,8-diaza-spiro[4.5]dec-8-yl]-4-oxo-butansäuremethylester
[134] 3-[8-(2-Ethyl-butyryl)-2-oxo-1-oxa-3,8-diaza-spiro[4.5]dec-3-ylmethyl]-benzonitril
[135] 3-[8-(3-Brom-benzylsulfonyl)-2-oxo-1-oxa-3,8-diaza-spiro[4.5]dec-3-ylmethyl]-benzonitril
[136] 3-(3,4-Difluor-benzyl)-8-(4-methyl-benzoyl)-1-oxa-3,8-diaza-spiro[4.5]decan-2-on
[137] 3-(3-Brom-benzyl)-8-(3,5-dimethoxy-benzoyl)-1-oxa-3,8-diaza-spiro[4.5]decan-2-on
[138] 8-(3-Dimethylamino-benzoyl)-3-(2-methoxy-5-nitro-benzyl)-1-oxa-3,8-diaza-spiro[4.5]decan-2-on
[139] 8-(2,6-Dichlor-benzoyl)-3-(2-fluor-benzyl)-1-oxa-3,8-diaza-spiro[4.5]decan-2-on
[140] 3-(2-Methoxy-5-nitro-benzyl)-8-(2-phenoxy-pyridin-3-carbonyl)-1-oxa-3,8-diaza-spiro[4.5]decan-2-on
[141] 8-[2-(3-Methoxy-phenyl)-acetyl]-3-naphthalen-2-ylmethyl-1-oxa-3,8-diaza-spiro[4.5]decan-2-on
[142] 4-{2-Oxo-8-[2-(2,2,2-trifluoro-acetyl)-1,2,3,4-tetrahydro-isochinolin-7-sulfonyl]-1-oxa-3,8-diaza-spiro[4.5]dec-3-ylmethyl}-benzonitril
[143] 8-(3,5-Bis-trifluormethyl-benzoyl)-3-(4-trifluormethyl-benzyl)-1-oxa-3,8-diaza-spiro[4.5]decan-2-on
[144] 3-(3-Brom-benzyl)-8-(2-phenoxy-pyridin-3-carbonyl)-1-oxa-3,8-diaza-spiro[4.5]decan-2-on
[145] 3-(2-Methoxy-5-nitro-benzyl)-8-[2-(3-methoxy-phenyl)-acetyl]-1-oxa-3,8-diaza-spiro[4.5]decan-2-on
[146] 3-(3,4-Difluor-benzyl)-8-(2-phenoxy-pyridin-3-carbonyl)-1-oxa-3,8-diaza-spiro[4.5]decan-2-on
[147] 3-[8-(4-Brom-3-methyl-benzoyl)-2-oxo-1-oxa-3,8-diaza-spiro[4.5]dec-3-ylmethyl]-benzonitril
[148] 3-(3-Brom-benzyl)-8-(4-propyl-benzoyl)-1-oxa-3,8-diaza-spiro[4.5]decan-2-on
[149] 3-Biphenyl-2-ylmethyl-8-(3-chlor-benzylsulfonyl)-1-oxa-3,8-diaza-spiro[4.5]decan-2-on
[150] 8-[2-(3,4-Dimethoxy-phenyl)-acetyl]-3-(2-methoxy-5-nitro-benzyl)-1-oxa-3,8-diaza-spiro[4.5]decan-2-on
[151] 2-[8-(2-Chlor-4-nitro-benzoyl)-2-oxo-1-oxa-3,8-diaza-spiro[4.5]dec-3-ylmethyl]-benzonitril
[152] 3-[2-Oxo-8-(2,4,6-trimethyl-benzylsulfonyl)-1-oxa-3;8-diaza-spiro[4.5]dec-3-ylmethyl]-benzonitril
[153] 3-Benzyl-8-(4-fluor-benzylsulfonyl)-1-oxa-3,8-diaza-spiro[4.5]decan-2-on
[154] 3-(4-Fluor-benzyl)-8-(toluol-4-sulfonyl)-1-oxa-3,8-diaza-spiro[4.5]decan-2-on
[155] 4-[2-Oxo-8-(toluol-4-sulfonyl)-1-oxa-3,8-diaza-spiro[4.5]dec-3-ylmethyl]-benzonitril
[156] 3-(2-Fluor-benzyl)-8-(toluol-4-sulfonyl)-1-oxa-3,8-diaza-spiro[4.5]decan-2-on
[157] 3-(3,4-Difluor-benzyl)-8-(toluol-4-sulfonyl)-1-oxa-3,8-diaza- spiro[4.5]decan-2-on
[158] 3-(4-tert-Butyl-benzyl)-8-(toluol-4-sulfonyl)-1-oxa-3,8-diaza-spiro[4.5]decan-2-on
[159] 8-(Toluol-4-sulfonyl)-3-(4-trifluormethylsulfanyl-benzyl)-1-oxa-3,8-diaza-spiro[4.5]decan-2-on
[160] 2-[3-(4-Fluor-benzyl)-2-oxo-1-oxa-3,8-diaza-spiro[4.5]decan-8-sulfonyl]-benzoesäuremethylester
[161] 2-[3-(3-Methoxy-benzyl)-2-oxo-1-oxa-3,8-diaza-spiro[4.5]decan-8-sulfonyl]-benzoesäuremethylester
[162] 2-[3-(3-Brom-benzyl)-2-oxo-1-oxa-3,8-diaza-spiro[4.5]decan-8-sulfonyl]-benzoesäuremethylester
[163] 3-Benzyl-8-(2-methansulfonyl-benzylsulfonyl)-1-oxa-3,8-diaza-spiro[4.5]decan-2-on
[164] 8-(2-Methansulfonyl-benzylsulfonyl)-3-(2-methoxy-5-nitro-benzyl)-1-oxa-3,8-diaza-spiro[4.5]decan-2-on
[165] 3-(4-Methyl-benzyl)-2-oxo-1-oxa-3,8-diaza-spiro[4.5]decan-8-sulfonsäure-dimethylamid
[166] 4-[8-(7,7-Dimethyl-2-oxo-bicyclo[2.2.1]hept-1-ylmethansulfonyl)-2-oxo-1-oxa-3,8-diaza-spiro[4.5]dec-3-ylmethyl]-benzonitril
[167] 3-(4-Fluor-benzyl)-8-(4-methoxy-benzylsulfonyl)-1-oxa-3,8-diaza-spiro[4.5]decan-2-on
[168] 3-(4-Methyl-benzyl)-8-(4-trifluormethoxy-benzylsulfonyl)-1-oxa-3,8-diaza-spiro[4.5]decan-2-on
[169] 8-(4-Trifluormethoxy-benzylsulfonyl)-3-(4-trifluormethylsulfanyl-benzyl)-1-oxa-3,8-diaza-spiro[4.5]decan-2-on
[170] 8-(Propan-1-sulfonyl)-3-(4-trifluormethylsulfanyl-benzyl)-1-oxa-3,8-diaza-spiro[4.5]decan-2-on
[171] 3-(2-Methoxy-5-nitro-benzyl)-8-(4-propyl-benzylsulfonyl)-1-oxa-3,8-diaza-spiro[4.5]decan-2-on
[172] 3-Biphenyl-2-ylmethyl-8-(4-propyl-benzylsulfonyl)-1-oxa-3,8-diaza-spiro[4.5]decan-2-on
[173] 8-(3-Brom-benzylsulfonyl)-3-(2-fluor-benzyl)-1-oxa-3,8-diaza-spiro[4.5]decan-2-on
[174] 8-(3-Brom-benzylsulfonyl)-3-(3-brom-benzyl)-1-oxa-3,8-diaza-spiro[4.5]decan-2-on
[175] 2-{8-[4-(1,1-Dimethyl-propyl)-benzylsulfonyl]-2-oxo-1-oxa-3,8-diaza-spiro[4.5]dec-3-ylmethyl}-benzonitril
[176] 3-Benzyl-8-[4-(1,1-dimethyl-propyl)-benzylsulfonyl]-1-oxa-3,8-diaza-spiro[4.5]decan-2-on
[177] 8-[4-(1,1-Dimethyl-propyl)-benzylsulfonyl]-3-(4-iod-benzyl)-1-oxa-3,8-diaza-spiro[4.5]decan-2-on
[178] 8-[4-(1,1-Dimethyl-propyl)-benzylsulfonyl]-3-(2-methoxy-5-nitro-benzyl)-1-oxa-3,8-diaza-spiro[4.5]decan-2-on
[179] 8-[4-(1,1-Dimethyl-propyl)-benzylsulfonyl]-3-(4-trifluormethyl-benzyl)-1-oxa-3,8-diaza-spiro[4.5]decan-2-on
[180] 3-Biphenyl-2-ylmethyl-8-[4-(1,1-dimethyl-propyl)-benzylsulfonyl]-1-oxa-3,8-diaza-spiro[4.5]decan-2-on
[181] 8-(4,5-Dichlor-thiophen-2-sulfonyl)-3-(3-methoxy-benzyl)-1-oxa-3,8-diaza-spiro[4.5]decan-2-on
[182] 8-(4,5-Dichlor-thiophen-2-sulfonyl)-3-(4-trifluormethyl-benzyl)-1-oxa-3,8-diaza-spiro[4.5]decan-2-on
[183] 3-(3-Brom-benzyl)-8-(4,5-dichlor-thiophen-2-sulfonyl)-1-oxa-3,8-diaza-spiro[4.5]decan-2-on
[184] 2-[8-(4-Methoxy-2,3,6-trimethyl-benzylsulfonyl)-2-oxo-1-oxa-3,8-diaza-spiro[4.5]dec-3-ylmethyl]-benzonitril
[185] 3-(2-Fluor-benzyl)-8-(4-methoxy-2,3,6-trimethyl-benzylsulfonyl)-1-oxa-3,8-diaza-spiro[4.5]decan-2-on
[186] 3-(4-tert-Butyl-benzyl)-8-[2-(2,2,2-trifluor-acetyl)-1,2,3,4-tetrahydroisochinolin-7-sulfonyl]-1-oxa-3,8-diaza-spiro[4.5]decan-2-on
[187] 8-(Butan-1-sulfonyl)-3-(3-methoxy-benzyl)-1-oxa-3,8-diaza-spiro[4.5]decan-2-on
[188] 3-(4-Methyl-benzyl)-8-(thiophen-2-sulfonyl)-1-oxa-3,8-diaza-spiro[4.5]decan-2-on
[189] 8-(4-Chlor-2,5-dimethyl-benzylsulfonyl)-3-(4-methyl-benzyl)-1-oxa-3,8-diaza-spiro[4.5]decan-2-on
[190] 3-Benzyl-8-(4-chlor-2,5-dimethyl-benzylsulfonyl)-1-oxa-3,8-diaza-spiro[4.5]decan-2-on
[191] 8-(4-Chlor-2,5-dimethyl-benzylsulfonyl)-3-(4-trifluormethyl-benzyl)-1-oxa-3,8-diaza-spiro[4.5]decan-2-on
[192] 3-Biphenyl-2-ylmethyl-8-(4-chlor-2,5-dimethyl-benzylsulfonyl)-1-oxa-3,8-diaza-spiro[4.5]decan-2-on
[193] 8-(4-Chlor-2,5-dimethyl-benzylsulfonyl)-3-(4-trifluormethylsulfanyl-benzyl)-1-oxa-3,8-diaza-spiro[4.5]decan-2-on
[194] 3-(4-Fluor-benzyl)-8-(2-trifluormethyl-benzylsulfonyl)-1-oxa-3,8-diaza-spiro[4.5]decan-2-on
[195] 3-(3-Methoxy-benzyl)-8-(2-trifluormethyl-benzylsulfonyl)-1-oxa-3,8-diaza-spiro[4.5]decan-2-on
[196] 3-Benzyl-8-(2-methyl-5-nitro-benzylsulfonyl)-1-oxa-3,8-diaza-spiro[4.5]decan-2-on
[197] 3-(4-tert-Butyl-benzyl)-8-(2-methyl-5-nitro-benzylsulfonyl)-1-oxa-3,8-diaza-spiro[4.5]decan-2-on
[198] 3-(4-tert-Butyl-benzyl)-8-(toluol-3-sulfonyl)-1-oxa-3,8-diaza-spiro[4.5]decan-2-on
[199] 8-(Furan-2-carbonyl)-3-(4-trifluormethyl-benzyl)-1-oxa-3,8-diaza-spiro[4.5]decan-2-on
[200] 3-(3-Brom-benzyl)-8-(furan-2-carbonyl)-1-oxa-3,8-diaza-spiro[4.5]decan-2-on
[201] 4-[8-(Naphthalen-1-carbonyl)-2-oxo-1-oxa-3,8-diaza-spiro[4.5]dec-3-ylmethyl]-benzonitril
[202] 3-(3,4-Difluor-benzyl)-8-(naphthalen-1-carbonyl)-1-oxa-3,8-diaza-spiro[4.5]decan-2-on
[203] 3-(3-Brom-benzyl)-8-(naphthalen-1-carbonyl)-1-oxa-3,8-diaza-spiro[4.5]decan-2-on
[204] 2-[8-(3-Nitro-benzoyl)-2-oxo-1-oxa-3,8-diaza-spiro[4.5]dec-3-ylmethyl]-benzonitril
[205] 4-[8-(3-Nitro-benzoyl)-2-oxo-1-oxa-3,8-diaza-spiro[4.5]dec-3-ylmethyl]-benzonitril
[206] 3-(3-Brom-benzyl)-8-(3,5-difluor-benzoyl)-1-oxa-3,8-diaza-spiro[4.5]decan-2-one
[207] 8-(2-Benzyloxy-acetyl)-3-(2-fluor-benzyl)-1-oxa-3,8-diaza-spiro[4.5]decan-2-on
[208] 8-(2-Chlor-benzoyl)-3-(2-trifluormethyl-benzyl)-1-oxa-3,8-diaza-spiro[4.5]decan-2-on
[209] 8-(2-Chlor-benzoyl)-3-(4-iod-benzyl)-1-oxa-3,8-diaza-spiro[4.5]decan-2-on
[210] 8-(2-Chlor-benzoyl)-3-(2-methoxy-5-nitro-benzyl)-1-oxa-3,8-diaza-spiro[4.5]decan-2-on
[211] 3-Biphenyl-2-ylmethyl-8-(2-chlor-benzoyl)-1-oxa-3,8-diaza-spiro[4.5]decan-2-on
[212] 8-(2,6-Dichlor-benzoyl)-3-naphthalen-2-ylmethyl-1-oxa-3,8-diaza-spiro[4.5]decan-2-on
[213] 3-(4-tert-Butyl-benzyl)-8-(2,6-dichlor-benzoyl)-1-oxa-3,8-diaza-spiro[4.5]decan-2-on
[214] 3-(3-Brom-benzyl)-8-(2,6-dichlor-benzoyl)-1-oxa-3,8-diaza-spiro[4.5]decan-2-on
[215] 8-(2,6-Dichlor-benzoyl)-3-(4-trifluormethylsulfanyl-benzyl)-1-oxa-3,8-diaza-spiro[4.5]decan-2-on
[216] 2-[8-(2-Methyl-benzoyl)-2-oxo-1-oxa-3,8-diaza-spiro[4.5]dec-3-ylmethyl]-benzonitril
[217] 8-(2-Methyl-benzoyl)-3-(4-methyt-benzyl)-1-oxa-3,8-diaza-spiro[4.5]decan-2-on
[218] 8-(2-Methyl-benzoyl)-3-(2-trifluormethyl-benzyl)-1-oxa-3,8-diaza-spiro[4.5]decan-2-on
[219] 3-(2-Methoxy-5-nitro-benzyl)-8-(2-methyl-benzoyl)-1-oxa-3,8-diaza-spiro[4.5]decan-2-on
[220] 3-(3,4-Difluor-benzyl)-8-(2-methyl-benzoyl)-1-oxa-3,8-diaza-spiro[4.5]decan-2-on
[221] 8-(2-Methyl-benzoyl)-3-(4-trifluormethyl-benzyl)-1-oxa-3,8-diaza-spiro[4.5]decan-2-on
[222] 8-(2-Methyl-benzoyl)-3-(4-trifluormethylsulfanyl-benzyl)-1-oxa-3,8-diaza-spiro[4.5]decan-2-on
[223] 8-(3-Brom-benzoyl)-3-(4-fluor-benzyl)-1-oxa-3,8-diaza-spiro[4.5]decan-2-on
[224] 8-(3-Brom-benzoyl)-3-(2-trifluormethyl-benzyl)-1-oxa-3,8-diaza-spiro[4.5]decan-2-on
[225] 8-(3-Brom-benzoyl)-3-(2-fluor-benzyl)-1-oxa-3,8-diaza-spiro[4.5]decan-2-on
[226] 8-(3-Brom-benzoyl)-3-(3,4-difluor-benzyl)-1-oxa-3,8-diaza-spiro[4.5]decan-2-on
[227] 8-(3-Brom-benzoyl)-3-naphthalen-2-ylmethyl-1-oxa-3,8-diaza-spiro[4.5]decan-2-on
[228] 8-(3-Brom-benzoyl)-3-(4-tert-butyl-behzyl)-1-oxa-3,8-diaza-spiro[4.5]decan-2-on
[229] 3-Benzyl-8-(3-fluor-benzoyl)-1-oxa-3,8-diaza-spiro[4.5]decan-2-on
[230] 8-(3-Fluor-benzoyl)-3-(2-fluor-benzyl)-1-oxa-3,8-diaza-spiro[4.5]decan-2-on
[231] 3-(3,4-Difluor-benzyl)-8-(3-fluor-benzoyl)-1-oxa-3,8-diaza-spiro[4.5]decan-2-on
[232] 8-(3-Fluor-benzoyl)-3-(4-trifluormethyl-benzyl)-1-oxa-3,8-diaza-spiro[4.5]decan-2-on
[233] 3-(3-Brom-benzyl)-8-(3-fluor-benzoyl)-1-oxa-3,8-diaza-spiro[4.5]decan-2-on
[234] 8-(3-Chlor-benzoyl)-3-(3,5-dimethyl-benzyl)-1-oxa-3,8-diaza-spiro[4.5]decan-2-on
[235] 3-Benzyl-8-(3-chlor-benzoyl)-1-oxa-3,8-diaza-spiro[4.5]decan-2-on
[236] 4-[8-(3,4-Dichlor-benzoyl)-2-oxo-1-oxa-3,8-diaza-spiro[4.5]dec-3-ylmethyl]-benzonitril
[237] 3-(4-lod-benzyl)-8-(3-methoxy-benzoyl)-1-oxa-3,8-diaza-spiro[4.5]decan-2-on
[238] 3-(2-Fluor-benzyl)-8-(3-methoxy-benzoyl)-1-oxa-3,8-diaza-spiro[4.5]decan-2-on
[239] 3-(4-tert-Butyl-benzyl)-8-(3-methoxy-benzoyl)-1-oxa-3,8-diaza-spiro[4.5]decan-2-on
[240] 3-Benzyl-8-(3-methyl-benzoyl)-1-oxa-3,8-diaza-spiro[4.5]decan-2-on
[241] 3-(2-Methoxy-5-nitro-benzyl)-8-(3-methyl-benzoyl)-1-oxa-3,8-diaza-spiro[4.5]decan-2-on
[242] 3-(4-tert-Butyl-benzyl)-8-(3-methyl-benzoyl)-1-oxa-3,8-diaza-spiro[4.5]decan-2-on
[243] 8-(3-Methyl-benzoyl)-3-(4-trifluormethyl-benzyl)-1-oxa-3,8-diaza-spiro[4.5]decan-2-on
[244] 3-(3,4-Difluor-benzyl)-8-(2-phenyl-butyryl)-1-oxa-3,8-diaza-spiro[4.5]decan-2-on
[245] 3-Benzyl-8-[3-(2-chlor-phenyl)-5-methyl-isoxazol-4-carbonyl]-1-oxa-3,8-diaza-spiro[4.5]decan-2-on
[246] 4-[8-(2,3-Dichlor-benzoyl)-2-oxo-1-oxa-3,8-diaza-spiro[4.5]dec-3-ylmethyl]-benzonitril
[247] 8-[2-(4-Chlor-phenyl)-acetyl]-3-(3,4-difluor-benzyl)-1-oxa-3,8-diaza-spiro[4.5]decan-2-on
[248] 3-Biphenyl-2-ylmethyl-8-[2-(4-chlor-phenyl)-acetyl]-1-oxa-3,8-diaza-spiro[4.5]decan-2-on
[249] 8-[2-(4-Chlor-phenyl)-acetyl]-3-(4-trifluormethylsulfanyl-benzyl)-1-oxa-3,8-diaza-spiro[4.5]decan-2-on
[250] 3-(4-tert-Butyl-benzyl)-8-[3-(2-chlor-phenyl)-acryloyl]-1-oxa-3,8-diaza-spiro[4.5]decan-2-on
[251] 3-Biphenyl-2-ylmethyl-8-[3-(2-chlor-phenyl)-acryloyl]-1-oxa-3,8-diaza-spiro[4.5]decan-2-on
[252] 3-(3-Brom-benzyl)-8-[3-(2-chlor-phenyl)-acryloyl]-1-oxa-3,8-diaza-spiro[4.5]decan-2-on
[253] 4-[8-(2,3-Difluor-benzoyl)-2-oxo-1-oxa-3,8-diaza-spiro[4.5]dec-3-ylmethyl]-benzonitril
[254] 8-(2,3-Difluor-benzoyl)-3-(2-trifluormethyl-benzyl)-1-oxa-3,8-diaza-spiro[4.5]decan-2-on
[255] 8-(2,3-Difluor-benzoyl)-3-(4-iod-benzyt)-1-oxa-3,8-diaza-spiro[4.5]decan-2-on
[256] 2-[2-Oxo-8-(2-propyl-pentanoyl)-1-oxa-3,8-diaza-spiro[4.5]dec-3-ylmethyl]-benzonitril
[257] 3-(3,4-Difluor-benzyl)-8-(2-propyl-pentanoyl)-1-oxa-3,8-diaza-spiro[4.5]decan-2-on
[258] 8-(2-Chlor-4-nitro-benzoyl)-3I-(4-methyl-benzyl)-1-oxa-3,8-diaza-spiro[4.5]decan-2-on
[259] 8-(2-Chlor-4-nitro-benzoyl)-3I-(2-trifluormethyt-benzyl)-1-oxa-3,8-diaza-spiro[4.5]decan-2-on
[260] 3-(4-tert-Butyl-benzyl)-8-(2-chlor-4-nitro-benzoyl)-1-oxa-3,8-diaza-spiro[4.5]decan-2-on
[261] 8-(2-Chlor-4-nitro-benzoyl)-3I-(4-trifluormethyl-benzyl)-1-oxa-3,8-diaza-spiro[4.5]decan-2-on
[262] 3-Biphenyl-2-ylmethyl-8-(2-chlor-4-nitro-benzoyl)-1-oxa-3,8-diaza-spiro[4.5]decan-2-on
[263] 3-(3-Brom-benzyl)-8-(2-chlor-4-nitro-benzoyl)-1-oxa-3,8-diaza-spiro[4.5]decan-2-on
[264] 8-(2-Chlor-4-nitro-benzoyl)-3I-(4-trifluormethylsulfanyl-benzyl)-1-oxa-3,8-diaza-spiro[4.5]decan-2-on
[265] 8-(2-Chlor-pyridin-3-carbonyl)-3-(4-fluor-benzyl)-1-oxa-3,8-diaza-spiro[4.5]decan-2-on
[266] 2-[8-(2-Chlor-pyridin-3-carbonyl)-2-oxo-1-oxa-3,8-diaza-spiro[4.5]dec-3-ylmethyl]-benzonitril
[267] 8-(2-Chlor-pyridin-3-carbonyl)-3-(4-methyl-benzyl)-1-oxa-3,8-diaza-spiro[4.5]decan-2-on
[268] 8-(2-Chlor-pyridin-3-carbonyl)-3-(4-trifluormethyl-benzyl)-1-oxa-3,8-diaza-spiro[4.5]decan-2-on
[269] 3-(3-Methoxy-benzyl)-8-(2-methytsulfanyl-pyridin-3-carbonyl)-1-oxa-3,8-diaza-spiro[4.5]decan-2-on
[270] 3-(4-Methyl-benzyl)-8-(2-methylsulfanyl-pyridin-3-carbonyl)-1-oxa-3,8-diaza-spiro[4.5]decan-2-on
[271] 3-(4-lod-benzyl)-8-(2-methylsulfanyl-pyridin-3-carbonyl)-1-oxa-3,8-diaza-spiro[4.5]decan-2-on
[272] 3-(3,4-Difluor-benzyl)-8-(2-methylsulfanyl-pyridin-3-carbonyl)-1-oxa-3,8-diaza-spiro[4.5]decan-2-on
[273] 3-(4-tert-Butyl-benzyl)-8-(2-methylsulfanyl-pyridin-3-carbonyl)-1-oxa-3,8-diaza-spiro[4.5]decan-2-on
[274] 8-(2-Methylsulfanyl-pyridin-3-carbonyl)-3-(4-trifluormethyl-benzyl)-1-oxa-3,8-diaza-spiro[4.5]decan-2-on
[275] 8-(2-Ethylsulfanyl-pyridin-3-carbonyl)-3-(4-fluor-benzyl)-1-oxa-3,8-diaza-spiro[4.5]decan-2-on
[276] 3-[8-(2-Ethylsulfanyl-pyridin-3-carbonyl)-2-oxo-1-oxa-3,8-diaza-spiro[4.5]dec-3-ylmethyl]-benzonitril
[277] 2-[8-(2-Ethylsulfanyl-pyridin-3-carbonyl)-2-oxo-1-oxa-3,8-diaza-spiro[4.5]dec-3-ylmethyl]-benzonitril
[278] 8-(2-Ethylsulfanyl-pyridin-3-carbonyl)-3-(4-methyl-benzyl)-1-oxa-3,8-diaza-spiro[4.5]decan-2-on
[279] 3-Benzyl-8-(2-ethylsulfanyl-pyridin-3-carbonyl)-1-oxa-3,8-diaza-spiro[4.5]decan-2-on
[280] 8-(2-Ethylsulfanyl-pyridin-3-carbonyl)-3-(2-fluor-benzyl)-1-oxa-3,8-diaza-spiro[4.5]decan-2-on
[281] 4-[8-(6-Chlor-pyridin-3-carbonyl)-2-oxo-1-oxa-3,8-diaza-spiro[4.5]dec-3-ylmethyl]-benzonitril
[282] 8-(6-Chlor-pyridin-3-carbonyl)-3-(2-trifluormethyt-benzyl)-1-oxa-3,8-diaza-spiro[4.5]decan-2-on
[283] 8-(6-Chlor-pyridin-3-carbonyl)-3-(4-iod-benzyl)-1-oxa-3,8-diaza-spiro[4.5]decan-2-on
[284] 3-(4-tert-Butyl-benzyl)-8-(6-chlor-pyridin-3-carbonyl)-1-oxa-3,8-diaza-spiro[4.5]decan-2-on
[285] 3-Biphenyl-2-ylmethyl-8-(6-chlor-pyridin-3-carbonyl)-1-oxa-3,8-diaza-spiro[4.5]decan-2-on
[286] 3-(3-Methoxy-benzyl)-8-(4-trifluormethoxy-benzoyl)-1-oxa-3,8-diaza-spiro[4.5]decan-2-on
[287] 3-(3,4-Difluor-benzyl)-8-(4-trifluormethoxy-benzoyl)-1-oxa-3,8-diaza-spiro[4.5]decan-2-on
[288] 8-[3-(2-Chlor-6-fluor-phenyl)-5-methyl-isoxazol-4-carbonyl]-3-(4-fluor-benzyl)-1-oxa-3,8-diaza-spiro[4.5]decan-2-on
[289] 8-[3-(2-Chlor-6-fluor-phenyl)-5-methyl-isoxazol-4-carbonyl]-3-(3-methoxybenzyl)-1-oxa-3,8-diaza-spiro[4.5]decan-2-on
[290] 8-[3-(2-Chlor-6-fluor-phenyl)-5-methyl-isoxazol-4-carbonyl]-3-(4-trifluormethyl-benzyl)-1-oxa-3,8-diaza-spiro[4.5]decan-2-on
[291] 8-[3-(2-Chlor-6-fluor-phenyl)-5-methyl-isoxazol-4-carbonyl]-3-(4-trifluormethylsulfanyl-benzyl)-1-oxa-3,8-diaza-spiro[4.5]decan-2-on
[292] 3-(4-Methyl-benzyl)-8-(3-trifluormethoxy-benzoyl)-1-oxa-3,8-diaza-spiro[4.5]decan-2-on
[293] 3-(4-lod-benzyl)-8-(3-trifluormethoxy-benzoyl)-1-oxa-3,8-diaza-spiro[4.5]decan-2-on
[294] 3-(3,4-Difluor-benzyl)-8-(3-trifluormethoxy-benzoyl)-1-oxa-3,8-diaza-spiro[4.5]decan-2-on
[295] 3-Naphthalen-2-ylmethyl-8-(3-trifluormethoxy-benzoyl)-1-oxa-3,8-diaza-spiro[4.5]decan-2-on
[296] 4-[8-(Isoxazol-5-carbonyl)-2-oxo-1-oxa-3,8-diaza-spiro[4.5]dec-3-ylmethyl]-benzonitril
[297] 8-(Isoxazol-5-carbonyl)-3-(2-trifluormethyl-benzyl)-1-oxa-3,8-diaza-spiro[4.5]decan-2-on
[298] 8-(Isoxazol-5-carbonyl)-3-(2-methoxy-5-nitro-benzyl)-1-oxa-3,8-diaza-spiro[4.5]decan-2-on
[299] 3-(3-Brom-benzyl)-8-(isoxazol-5-carbonyl)-1-oxa-3,8-diaza-spiro[4.5]decan-2-on
[300] 2-[8-(2-Chlor-6-fluor-benzoyl)-2-oxo-1-oxa-3,8-diaza-spiro[4.5]dec-3-ylmethyl]-benzonitril
[301] 4-[8-(2-Chlor-6-fluor-benzoyl)-2-oxo-1-oxa-3,8-diaza-spiro[4.5]dec-3-ylmethyl]-benzonitril
[302] 8-(2-Chlor-6-fluor-benzoyl)-3-(4-methyl-benzyl)-1-oxa-3,8-diaza-spiro[4.5]decan-2-on
[303] 3-Biphenyl-2-ylmethyl-8-(2-chlor-6-fluor-benzoyl)-1-oxa-3,8-diaza-spiro[4.5]decan-2-on
[304] 8-(2,5-Dimethyl-furan-3-carbonyl)-3-(3-methoxy-benzyl)-1-oxa-3,8-diaza-spiro[4.5]decan-2-on
[305] 4-[8-(2,5-Dimethyl-furan-3-carbonyl)-2-oxo-1-oxa-3,8-diaza-spiro[4.5]dec-3-ylmethyl]-benzonitril
[306] 4-[8-(4-Brom-3-methyl-benzoyl)-2-oxo-1-oxa-3,8-diaza-spiro[4.5]dec-3-ylmethyl]-benzonitril
[307] 8-(4-Brom-3-methyl-benzoyl)-3-(4-methyl-benzyl)-1-oxa-3,8-diaza-spiro[4.5]decan-2-on
[308] 3-Benzyl-8-(4-brom-3-methyl-benzoyl)-1-oxa-3,8-diaza-spiro[4.5]decan-2-on
[309] 8-(4-Brom-3-methyl-benzoyl)-3-(2-trifluormethyl-benzyl)-1-oxa-3,8-diaza-spiro[4.5]decan-2-on
[310] 8-(4-Brom-3-methyl-benzoyl)-3-(3,4-difluor-benzyl)-1-oxa-3,8-diaza-spiro[4.5]decan-2-on
[311] 8-(4-Brom-3-methyl-benzoyl)-3-(4-tert-butyl-benzyl)-1-oxa-3,8-diaza-spiro[4.5]decan-2-on
[312] 3-Biphenyl-2-ylmethyl-8-(4-brom-3-methyl-benzoyl)-1-oxa-3,8-diaza-spiro[4.5]decan-2-on
[313] 8-(4-Brom-3-methyl-benzoyl)-3-(4-trifluormethylsulfanyl-benzyl)-1-oxa-3,8-diaza-spiro[4.5]decan-2-on
[314] 8-(2,6-Difluor-3-methyl-benzoyl)-3-(3-methoxy-benzyl)-1-oxa-3,8-diaza-spiro[4.5]decan-2-on
[315] 2-[8-(2,6-Difluor-3-methyl-benzoyl)-2-oxo-1-oxa-3,8-diaza-spiro[4.5]dec-3-ylmethyl]-benzonitril
[316] 4-[8-(2,6-Difluor-3-methyl-benzoyl)-2-oxo-1-oxa-3,8-diaza-spiro[4.5]dec-3-ylmethyl]-benzonitril
[317] 8-(2,6-Difluor-3-methyl-benzoyl)-3-(2-trifluormethyl-benzyl)-1-oxa-3,8-diaza-spiro[4.5]decan-2-on
[318] 8-(2,6-Difluor-3-methyl-benzoyl)-3-(2-fluor-benzyl)-1-oxa-3,8-diaza-spiro[4.5]decan-2-on
[319] 8-(2-Chlor-6-fluor-3-methyl-benzoyl)-3-(4-fluor-benzyl)-1-oxa-3,8-diaza-spiro[4.5]decan-2-on
[320] 8-(2-Chlor-6-fluor-3-methyl-benzoyl)-3-(3-methoxy-benzyl)-1-oxa-3,8-diaza-spiro[4.5]decan-2-on
[321] 8-(2-Chlor-6-fluor-3-methyl-benzoyl)-3-(4-trifluormethylsulfanyl-benzyl)-1-oxa-3,8-diaza-spiro[4.5]decan-2-on
[322] 3-Biphenyl-2-ylmethyl-8-(6-chlor-2-fluor-3-methyl-benzoyl)-1-oxa-3,8-diaza-spiro[4.5]decan-2-on
[323] 3-Benzyl-8-(3-difluormethylsulfanyl-benzoyl)-1-oxa-3,8-diaza-spiro[4.5]decan-2-on
[324] 8-(3-Difluormethylsulfanyl-benzoyl)-3-naphthalen-2-ylmethyl-1-oxa-3,8-diaza-spiro[4.5]decan-2-on
[325] 8-(3-Difluormethylsulfanyl-benzoyl)-3-(4-trifluormethyl-benzyl)-1-oxa-3,8-diaza-spiro[4.5]decan-2-on
[326] 3-(3-Brom-benzyl)-8-(3-difluormethylsulfanyl-benzoyl)-1-oxa-3,8-diaza-spiro[4.5]decan-2-on
[327] 3-[8-(3-Chlor-2-fluor-benzoyl)-2-oxo-1-oxa-3,8-diaza-spiro[4.5]dec-3-ylmethyl]-benzonitril
[328] 8-(3-Chlor-2-fluor-benzoyl)-3-(4-iod-benzyl)-1-oxa-3,8-diaza-spiro[4.5]decan-2-on
[329] 8-(3-Chlor-2-fluor-benzoyl)-3-naphthalen-2-ylmethyl-1-oxa-3,8-diaza-spiro[4.5]decan-2-on
[330] 3-(3-Methoxy-benzyl)-8-(4-trifluormethylsulfanyl-benzoyl)-1-oxa-3,8-diaza-spiro[4.5]decan-2-on
[331] 2-[2-Oxo-8-(4-trifluormethylsulfanyl-benzoyl)-1-oxa-3,8-diaza-spiro[4.5]dec-3-ylmethyl]-benzonitril
[332] 4-[2-Oxo-8-(4-trifluormethylsulfanyl-benzoyl)-1-oxa-3,8-diaza-spiro[4.5]dec-3-ylmethyl]-benzonitril
[333] 3-(4-Iod-benzyl)-8-(4-trifluormethylsulfanyl-benzoyl)-1-oxa-3,8-diaza-spiro[4.5]decan-2-on
[334] 3-Naphthalen-2-ylmethyl-8-(4-trifluormethylsulfanyl-benzoyl)-1-oxa-3,8-diaza-spiro[4.5]decan-2-on
[335] 3-(4-Trifluormethyl-benzyl)-8-(4-trifluormethylsulfanyl-benzoyl)-1-oxa-3,8-diaza-spiro[4.5]decan-2-on
[336] 8-(2-Chlor-5-trifluormethyl-benzoyl)-3-(2-trifluormethyl-benzyl)-1-oxa-3,8-diaza-spiro[4.5]decan-2-on
[337] 8-(2-Chlor-5-trifluormethyl-benzoyl)-3-(2-fluor-benzyl)-1-oxa-3,8-diaza-spiro[4.5]decan-2-on
[338] 8-(2-Chlor-5-trifluormethyl-benzoyl)-3-(3,4-difluor-benzyl)-1-oxa-3,8-diaza-spiro[4.5]decan-2-on
[339] 8-(2,3-Difluor-4-methyl-benzoyl)-3-(3-methoxy-benzyl)-1-oxa-3,8-diaza-spiro[4.5]decan-2-on
[340] 4-[8-(2,3-Difluor-4-methyl-benzoyl)-2-oxo-1-oxa-3,8-diaza-spiro[4.5]dec-3-ylmethyl]-benzonitril
[341] 3-Benzyl-8-(2,3-difluor-4-methyl-benzoyl)-1-oxa-3,8-diaza-spiro[4.5]decan-2-on
[342] 8-(2,3-Difluor-4-methyl-benzoyl)-3-(4-iod-benzyl)-1-oxa-3,8-diaza-spiro[4.5]decan-2-on
[343] 3-(3,4-Difluor-benzyl)-8-(2,3-difluor-4-methyl-benzoyl)-1-oxa-3,8-diaza-spiro[4.5]decan-2-on
[344] 8-(2,3-Difluor-4-methyl-benzoyl)-3-naphthalen-2-ylmethyl-1-oxa-3,8-diaza-spiro[4.5]decan-2-on
[345] 3-Biphenyl-2-ylmethyl-8-(2,3-difluor-4-methyl-benzoyl)-1-oxa-3,8-diaza-spiro[4.5]decan-2-on
[346] 3-(3-Brom-benzyl)-8-(2,3-difluor-4-methyl-benzoyl)-1-oxa-3,8-diaza-spiro[4.5]decan-2-on
[347] 3-Benzyl-8-[2-(2-methoxy-ethoxy)-acetyl]-1-oxa-3,8-diaza-spiro[4.5]decan-2-on
[348] 3-(2-Fluor-benzyl)-8-[2-(2-methoxy-ethoxy)-acetyl]-1-oxa-3,8-diaza-spiro[4.5]decan-2-on
[349] 3-(3,4-Difluor-benzyl)-8-[2-(2-methoxy-ethoxy)-acetyl]-1-oxa-3,8-diaza-spiro[4.5]decan-2-on
[350] 3-(4-tert-Butyl-benzyl)-8-[2-(2-methoxy-ethoxy)-acetyl]-1-oxa-3,8-diaza-spiro[4.5]decan-2-on
[351] 8-[2-(2-Methoxy-ethoxy)-acetyl]-3-(4-trifluormethylsulfanyl-benzyl)-1-oxa-3,8-diaza-spiro[4.5]decan-2-on
[352] 8-(1-Acetyl-piperidin-4-carbonyl)-3-(3,4-difluor-benzyl)-1-oxa-3,8-diaza-spiro[4.5]decan-2-on
[353] 4-{8-[2-(3-Chlor-phenoxy)-acetyl]-2-oxo-1-oxa-3,8-diaza-spiro[4.5]dec-3-ylmethyl}-benzonitril
[354] 8-[2-(3-Chlor-phenoxy)-acetyl]-3-(3,4-difluor-benzyl)-1-oxa-3,8-diaza-spiro[4.5]decan-2-on
[355] 3-(3-Brom-benzyl)-8-[2-(3-chlor-phenoxy)-acetyl]-1-oxa-3,8-diaza-spiro[4.5]decan-2-on
[356] 8-[2-(3-Chlor-phenoxy)-acetyl]-3-(4-trifluormethylsulfanyl-benzyl)-1-oxa-3,8-diaza-spiro[4.5]decan-2-on
[357] 4-[3-(3-Methoxy-benzyl)-2-oxo-1-oxa-3,8-diaza-spiro[4.5]decan-8-carbonyl]-benzylsulfonamid
[358] N-{4-[3-(3,4-Difluor-benzyl)-2-oxo-1-oxa-3,8-diaza-spiro[4.5]decan-8-carbonyl]-phenyl}-acetamid
[359] 8-(3-Dimethylamino-benzoyl)-3-(3,5-dimethyl-benzyl)-1-oxa-3,8-diaza-spiro[4.5]decan-2-on
[360] 3-(4-tert-Butyl-benzyl)-8-(3-dimethylamino-benzoyl)-1-oxa-3,8-diaza-spiro[4.5]decan-2-on
[361] 3-(3-Brom-benzyl)-8-(3-dimethylamino-benzoyl)-1-oxa-3,8-diaza-spiro[4.5]decan-2-on
[362] 3-(4-Methyl-benzyl)-8-(4-phenoxy-butyryl)-1-oxa-3,8-diaza-spiro[4.5]decan-2-on
[363] 8-(4-Phenoxy-butyryl)-3-(2-trifluormethyl-benzyl)-1-oxa-3,8-diaza-spiro[4.5]decan-2-on
[364] 3-(2-Fluor-benzyl)-8-(4-phenoxy-butyryl)-1-oxa-3,8-diaza-spiro[4.5]decan-2-on
[365] 8-(2,3-Dimethyl-benzoyl)-3-(4-fluor-benzyl)-1-oxa-3,8-diaza-spiro[4.5]decan-2-on
[366] 3-[8-(2,3-Dimethyl-benzoyl)-2-oxo-1-oxa-3,8-diaza-spiro[4.5]dec-3-ylmethyl]-benzonitril
[367] 8-(2,3-Dimethyl-benzoyl)-3-(3,5-dimethyl-benzyl)-1-oxa-3,8-diaza-spiro[4.5]decan-2-on
[368] 8-(2,3-Dimethyl-benzoyl)-3-(4-methyl-benzyl)-1-oxa-3,8-diaza-spiro[4.5]decan-2-on
[369] 8-(2,3-Dimethyl-benzoyl)-3-(2-trifluormethyl-benzyl)-1-oxa-3,8-diaza-spiro[4.5]decan-2-on
[370] 8-(2,3-Dimethyl-benzoyl)-3-(2-methoxy-5-nitro-benzyl)-1-oxa-3,8-diaza-spiro[4.5]decan-2-on
[371] 8-(2,3-Dimethyl-benzoyl)-3-(4-trifluormethyl-benzyl)-1-oxa-3,8-diaza-spiro[4.5]decan-2-on
[372] 3-Biphenyl-2-ylmethyl-8-(2,3-dimethyl-benzoyl)-1-oxa-3,8-diaza-spiro[4.5]decan-2-on
[373] 4-[8-(5-Methyl-2-phenyl-2H-[1,2,3]triazol-4-carbonyl)-2-oxo-1-oxa-3,8-diaza-spiro[4.5]dec-3-ylmethyl]-benzonitril
[374] 3-Benzyl-8-(5-methyl-2-phenyl-2H-[1,2,3]truazol-4-carbonyl)-1-oxa-3,8-diaza-spiro[4.5]decan-2-on
[375] 3-(3,4-Difluor-benzyl)-8-(5-methyl-2-phenyl-2H-[1,2,3]triazol-4-carbonyl)-1-oxa-3,8-diaza-spiro[4.5]decan-2-on
[376] 8-(5-Methyl-2-phenyl-2H-[1,2,3]triazol-4-carbonyl)-3-naphthalen-2-ylmethyl-1-oxa-3,8-diaza-spiro[4.5]decan-2-on
[377] 3-Biphenyl-2-ylmethyl-8-(5-methyl-2-phenyl-2H-[1,2,3]triazol-4-carbonyl)-1-oxa-3,8-diaza-spiro[4.5]decan-2-on
[378] 3-(4-tert-Butyl-benzyl)-8-[3-(2,6-dichlor-phenyl)-5-methyl-isoxazol-4-carbonyl]-1-oxa-3,8-diaza-spiro[4.5]decan-2-on
[379] 3-[2-Oxo-8-(2-phenoxy-pyridin-3-carbonyl)-1-oxa-3,8-diaza-spiro[4.5]dec-3-ylmethyl]-benzonitril
[380] 2-[2-Oxo-8-(2-phenoxy-pyridin-3-carbonyl)-1-oxa-3,8-diaza-spiro[4.5]dec-3-ylmethyl]-benzonitril
[381] 3-(4-Fluor-benzyl)-8-(pyridin-2-carbonyl)-1-oxa-3,8-diaza-spiro[4.5]decan-2-on
[382] 3-(3-Methoxy-benzyl)-8-(pyridin-2-carbonyl)-1-oxa-3,8-diaza-spiro[4.5]decan-2-on
[383] 3-(3,5-Dimethyl-benzyl)-8-(pyridin-2-carbonyl)-1-oxa-3,8-diaza-spiro[4.5]decan-2-on
[384] 2-[2-Oxo-8-(pyridin-2-carbonyl)-1-oxa-3,8-diaza-spiro[4.5]dec-3-ylmethyl]-benzonitril
[385] 3-(2-Fluor-benzyl)-8-(pyridin-2-carbonyl)-1-oxa-3,8-diaza-spiro[4.5]decan-2-on
[386] 3-(3,4-Difluor-benzyl)-8-(pyridin-2-carbonyl)-1-oxa-3,8-diaza-spiro[4.5]decan-2-on
[387] 3-Biphenyl-2-ylmethyl-8-(pyridin-2-carbonyl)-1-oxa-3,8-diaza-spiro[4.5]decan-2-on
[388] 4-[8-(3-Chlor-thiophen-2-carbonyl)-2-oxo-1-oxa-3,8-diaza-spiro[4.5]dec-3-ylmethyl]-benzonitril
[389] 8-(3-Chlor-thiophen-2-carbonyl)-3-(3,4-difluor-benzyl)-oxa-3,8-diaza-spiro[4.5]decan-2-on
[390] 3-Biphenyl-2-ylmethyl-8-(3-chlor-thiophen-2-carbonyl)-1-oxa-3,8-diaza-spiro[4.5]decan-2-on
[391] 8-[1-(4-Chlor-phenyl)-5-trifluormethyl-1H-pyrazol-4-carbonyl]-3-(2-fluorbenzyl)-1-oxa-3,8-diaza-spiro[4.5]decan-2-on
[392] 8-[1-(4-Chlor-phenyl)-5-trifluormethyl-1H-pyrazol-4-carbonyl]-3-(3,4-difluorbenzyl)-1-oxa-3,8-diaza-spiro[4.5]decan-2-on
[393] 8-[1-(4-Chlor-phenyl)-5-trifluormethyl-1H-pyrazol-4-carbonyl]-3-(4-trifluormethylsulfanyl-benzyl)-1-oxa-3,8-diaza-spiro[4.5]decan-2-on
[394] 3-(4-tert-Butyl-benzyl)-8-(5-tert-butyl-2-methyl-2H-pyrazol-3-carbonyl)-1-oxa-3,8-diaza-spiro[4.5]decan-2-on
[395] 3-[8-(5-Methyl-isoxazol-3-carbonyl)-2-oxo-1-oxa-3,8-diaza-spiro[4.5]dec-3-ylmethyl]-benzonitril
[396] 3-(3,5-Dimethyl-benzyl)-8-(5-methyl-isoxazol-3-carbonyl)-1-oxa-3,8-diaza-spiro[4.5]decan-2-on
[397] 2-[8-(5-Methyl-isoxazol-3-carbonyl)-2-oxo-1-oxa-3,8-diaza-spiro[4.5]dec-3-ylmethyl]-benzonitril
[398] 4-[8-(5-Methyl-isoxazol-3-carbonyl)-2-oxo-1-oxa-3,8-diaza-spiro[4.5]dec-3-ylmethyl]-benzonitril
[399] 3-Benzyl-8-(5-methyl-isoxazol-3-carbonyl)-1-oxa-3,8-diaza-spiro[4.5]decan-2-on
[400] 3-(3,4-Difluor-benzyl)-8-(5-methyl-isoxazol-3-carbonyl)-1-oxa-3,8-diaza-spiro[4.5]decan-2-on
[401] 3-Biphenyl-2-ylmethyl-8-(5-methyl-isoxazol-3-carbonyl)-1-oxa-3,8-diaza-spiro[4.5]decan-2-on
[402] 8-(4-Methyl-[1,2,3]thiadiazol-5-carbonyl)-3-(2-trifluormethyl-benzyl)-1-oxa-3,8-diaza-spiro[4.5]decan-2-on
[403] 3-(4-tert-Butyl-benzyl)-8-(4-methyl-[1,2,3]thiadiazol-5-carbonyl)-1-oxa-3,8-diaza-spiro[4.5]decan-2-on
[404] 8-(4-Methyl-[1,2,3]thiadiazol-5-carbonyl)-3-(4-trifluormethylsulfanyl-benzyl)-1-oxa-3,8-diaza-spiro[4.5]decan-2-on
[405] 4-[2-Oxo-8-(1-phenyl-5-propyl-1H-pyrazöl-4-carbonyl)-1-oxa-3,8-diaza-spiro[4.5]dec-3-ylmethyl]-benzonitril
[406] 3-(3,4-Difluor-benzyl)-8-(1-phenyl-5-propyl-1H-pyrazo)-4-carbonyl)-1-oxa-3,8-diaza-spiro[4.5]decan-2-on
[407] 3-Naphthalen-2-ylmethyl-8-(1-ohenyl-5-propyl-1H-pyrazo)-4-carbonyl)-1-oxa-3,8-diaza-spiro[4.5]decan-2-on
[408] 8-(1-Phenyl-5-propyl-1H-pyrazol-4-carbonyl)-3-(4-trifluormethylsulfanyl-benzyl)-1-oxa-3,8-diaza-spiro[4.5]decan-2-on
[409] 3-[8-(2-Chlor-pyridin-4-carbonyl)-2-oxo-1-oxa-3,8-diaza-spiro[4.5]dec-3-ylmethyl]-benzonitril
[410] 8-(2-Chlor-pyridin-4-carbonyl)-3-(3-methoxy-benzyl)-1-oxa-3,8-diaza-spiro[4.5]decan-2-on
[411] 8-(5-tert-Butyl-2-methyl-furan-3-carbonyl)-3-(4-ftuor-benzyl)-1-oxa-3.8-diaza-spiro[4.5]decan-2-on
[412] 3-[8-(5-tert-Butyl-2-methyl-furan-3-carbonyl)-2-oxo-1-oxa-3,8-diaza-spiro[4.5]dec-3-ylmethyl]-benzonitril
[413] 2-[8-(5-tert-Butyl-2-methyl-furan-3-carbonyl)-2-oxo-1-oxa-3,8-diaza-spiro[4.5]dec-3-ylmethyl]-benzonitril
[414] 4-[8-(5-tert-Butyl-2-methyl-furan-3-carbonyl)-2-oxo-1-oxa-3,8-diaza-spiro[4.5]dec-3-ylmethyl]-benzonitril
[415] 8-(5-tert-Butyl-2-methyl-furan-3-carbonyl)-3-(2-trifluormethyl-benzyl)-1-oxa-3,8-diaza-spiro[4.5]decan-2-on
[416] 8-(5-tert-Butyl-2-methyl-furan-3-carbonyl)-3-naphthalen-2-ylmethyl-1-oxa-3,8-diaza-spiro[4.5]decan-2-on
[417] 3-Biphenyl-2-ylmethyl-8-(5-tert-butyl-2-methyl-furan-3-carbonyl)-1-oxa-3,8-diaza-spiro[4.5]decan-2-on
[418] 3-(3-Brom-benzyl)-8-(5-tert-butyl-2-methyl-furan-3-carbonyl)-1-oxa-3,8-diaza-spiro[4.5]decan-2-on
[419] 8-(Benzo[1,2,5]oxadiazol-5-carbonyl)-3-(3,4-difluor-benzyl)-1-oxa-3,8-diaza-spiro[4.5]decan-2-on
[420] 8-(Benzo[1,2,5]oxadiazol-5-carbonyl)-3-(4-trifluormethyl-benzyl)-1-oxa-3,8-diaza-spiro[4.5]decan-2-on
[421] 3-[8-(2-Methyl-6-trifluormethyl-pyridin-3-carbonyl)-2-oxo-1-oxa-3,8-diaza-spiro[4.5]dec-3-ylmethyl]-benzonitril
[422] 2-[8-(2-Methyl-6-trifluormethyl-pyridin-3-carbonyl)-2-oxo-1-oxa-3,8-diaza-spiro[4.5]dec-3-ylmethyl]-benzonitril
[423] 4-[8-(2-Methyl-6-trifluormethyl-pyridin-3-carbonyl)-2-oxo-1-oxa-3,8-diaza-spiro[4.5]dec-3-ylmethyl]-benzonitril
[424] 8-(2-Methyl-6-trifluormethyl-pyridin-3-carbonyl)-3-(2-trifluormethyl-benzyl)-1-oxa-3,8-diaza-spiro[4.5]decan-2-on
[425] 3-(3,4-Difluor-benzyl)-8-(2-methyl-6-trifluormethyl-pyridin-3-carbonyl)-1-oxa-3,8-diaza-spiro[4.5]decan-2-on
[426] 3-(3-Brom-benzyl)-8-(2-methyl-6-trifluormethyl-pyridin-3-carbonyl)-1-oxa-3,8-diaza-spiro[4.5]decan-2-on
[427] 8-(6-Chlor-2H-chromen-3-carbonyl)-3-(3,4-difluor-benzyl)-1-oxa-3,8-diaza-spiro[4.5]decan-2-on
[428] 8-(2-Chlor-pyridin-4-carbonyl)-3-(2-fluor-benzyl)-1-oxa-3,8-diaza-spiro[4.5]decan-2-on
[429] 8-(2-Chlor-pyridin-4-carbonyl)-3-naphthalen-2-ylmethyl-1-oxa-3,8-diaza-spiro[4.5]decan-2-on
[430]8-Acetyl-3-(2-fluor-benzyl)-1-oxa-3,8-diaza-spiro[4.5]decan-2-on
[431] 8-Acetyl-3-(3-brom-benzyl)-1-oxa-3,8-diaza-spiro[4.5]decan-2-on
   und
[432] 3-(2-Methoxy-5-nitro-benzyl)-8-(3-phenoxy-propyl)-1-oxa-3,8-diaza-spiro[4.5]decan-2-on;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung der erfindungsgemäßen Verbindungen der vorstehend angegebenen allgemeinen Formel I gemäß dem wenigstens einer Verbindung der allgemeinen Formel II, 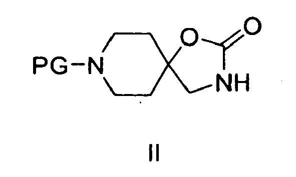 worin PG für eine Schutzgruppe, bevorzugt eine tert-Butyloxy-carbonyl- oder Benzyloxycarbonyl-Gruppe steht, in einem Reaktionsmedium, in Gegenwart wenigstens einer Base, bevorzugt in Gegenwart wenigstens eines Metallhydridsalzes, besonders bevorzugt in Gegenwart von Kalium- und/oder Natriumhydrid, mit wenigstens einer Verbindung der allgemeinen Formel R¹-(CH₂)ₙ-X, worin R¹ und n die vorstehend angegebene Bedeutung haben und X für eine Abgangsgruppe, bevorzugt ein Halogenatom, besonders bevorzugt ein Chlor- oder Bromatom steht, zu wenigstens einer Verbindung der allgemeinen Formel III, 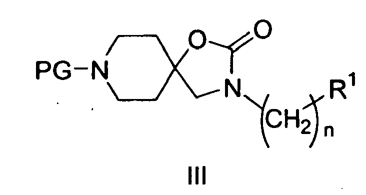 worin R¹, PG und n die vorstehend genannte Bedeutung haben, umgesetzt wird, und diese ggf. gereinigt und/oder isoliert wird,
und wenigstens eine Verbindung der allgemeinen Formel III durch Abspaltung der Schutzgruppe, vorzugsweise der tert-Butoxycarbonyl-Gruppe in einem Reaktionsmedium, vorzugsweise in Gegenwart wenigstens einer Säure oder in Gegenwart wenigstens einer Base oder durch Abspaltung der Benzyloxycarbonyl-Gruppe in einem Reaktionsmedium, vorzugsweise in Gegenwart von Wasserstoff und Katalysator, bevorzugt Palladium auf Kohle, zu wenigstens einer Verbindung der allgemeinen Formel IV, ggf. in Form eines entsprechenden Salzes, bevorzugt in Form eines entsprechenden Hydrochlorids, 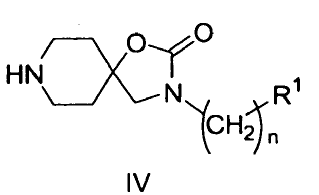 worin R¹ und n die vorstehend genannte Bedeutung haben, umgesetzt wird, und diese ggf. gereinigt und/oder isoliert wird,
und wenigstens eine Verbindung der allgemeinen Formel IV in einem Reaktionsmedium mit wenigstens einem Isothiocyanat der allgemeinen Formel R³-N=C=S, worin R³ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, zu wenigstens einer Verbindung der allgemeinen Formel umgesetzt wird, worin R¹ und n die vorstehend genannte Bedeutung haben und R² für-C(=S)-NH-R³, worin R³ die vorstehend genannte Bedeutung hat, steht, und diese ggf. gereinigt und/oder isoliert wird;
oder
wenigstens eine Verbindung der allgemeinen Formel IV in einem Reaktionsmedium mit wenigstens einem Isocyanat der allgemeinen Formel R⁴-N=C=O, worin R⁴ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, zu wenigstens einer Verbindung der allgemeinen Formel umgesetzt wird, worin R¹ und n die vorstehend genannte Bedeutung haben und R² für -C(=O)-NH-R⁴, worin R⁴ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Sulfonsäure-Derivat der allgemeinen Formel R⁵-S(=O)₂-X, worin R⁵ die vorstehend angegebene Bedeutung hat und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -S(=O)₂-R⁵, worin R⁵ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Verbindung der allgemeinen Formel X-(CH₂)-C(=O)-NHR⁶, worin R⁶ die vorstehend angegebene Bedeutung hat und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -(CH₂)-C(=O)-NH-R⁶, worin R⁶ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Verbindung der allgemeinen Formel X-(CH₂)-R⁷ oder wenigstens einer Verbindung der allgemeinen Formel X-(CH₂)-Dₐₐ-(CH₂)_{bb}-E_{cc}-(CH₂)_{dd}-R⁷, worin R⁷, D, E, aa, bb, cc und dd die vorstehend angegebene Bedeutung haben und X für eine Abgangsgruppe, bevorzugt ein Halogenatom, besonders bevorzugt ein Chlor oder Bromatom steht, zu wenigstens einer Verbindung der allgemeinen Formel I umgesetzt wird, worin R¹ und n die vorstehend genannte Bedeutung haben und R² für -(CH₂)-R⁷ oder -(CH₂)-Dₐₐ-(CH₂)_{bb}-E_{cc}-(CH₂)_{dd}-R⁷, worin R⁷, D, E, aa, bb, cc und dd die vorstehend genannte Bedeutung haben, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Carbonsäure-Derivat der allgemeinen Formel R⁸-C(=O)-X, worin R⁸ die vorstehend angegebene Bedeutung hat und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für-C(=O)-R⁸, worin R⁸ die vorstehend genannte Bedeutung hat, steht, und diese ggf. gereinigt und/oder isoliert wird;
oder
wenigstens eine Verbindung der allgemeinen Formel IV in einem Reaktionsmedium, in Gegenwart wenigstens eines Kupplungsreagenzes, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Carbonsäure der allgemeinen Formel R⁸-C(=O)-OH, worin R⁸ die vorstehend angegebene Bedeutung hat, zu wenigstens einer Verbindung der allgemeinen Formel I umgesetzt wird, worin R¹ und n die vorstehend genannte Bedeutung haben und R² für -C(=O)-R⁸, worin R⁸ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Sulfonsäure-Derivat der allgemeinen Formel X-S(=O)₂-NR⁹R¹⁰, worin R⁹ und R¹⁰ die vorstehend angegebene Bedeutung haben und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -S(=O)₂-NR⁹R¹⁰, worin R⁹ und R¹⁰ die vorstehend genannte Bedeutung haben, steht, und diese ggf. gereinigt und/oder isoliert wird.

Das erfindungsgemäße Verfahren zur Herstellung substituierter 1-Oxa-3,8-diazaspiro[4.5]-decan-2-on-Verbindungen der vorstehend angegebenen allgemeinen Formel I ist auch in dem folgenden Schema 1 wiedergegeben.

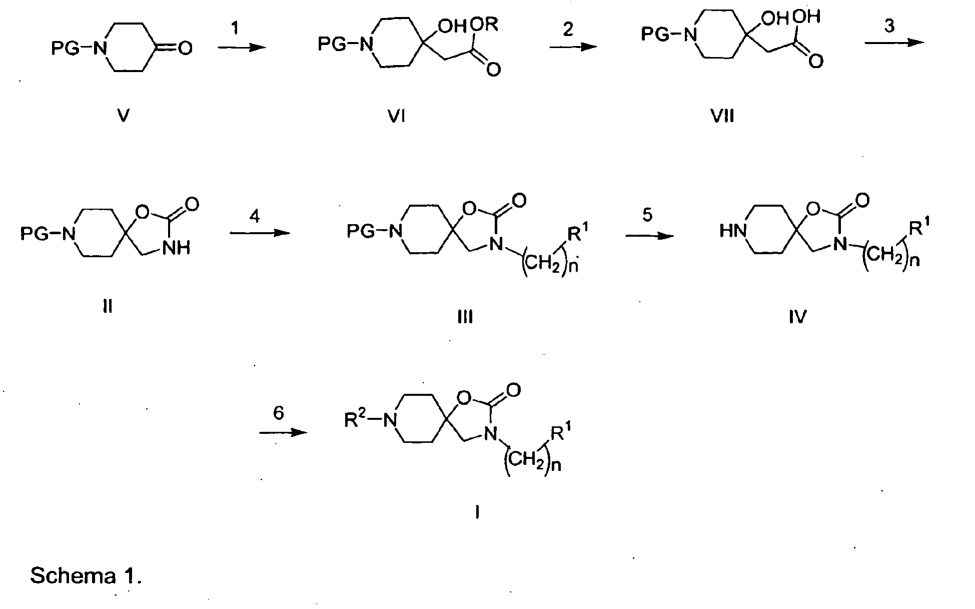

In Stufe 1 werden Verbindungen der allgemeinen Formel V, worin PG für eine tert-Butoxycarbonyl-Gruppe oder Benzyloxycarbonyl-Gruppe steht, in einem Reaktionsmedium vorzugsweise ausgewählt aus der Gruppe bestehend aus Dichlormethan, Acetonitril, Toluol, Diethylether, Tetrahydrofuran sowie entsprechenden Mischungen in Gegenwart wenigstens einer Base, vorzugsweise in Gegenwart von Lithium- und/oder Kaliumhexamethyldisilazid mit Verbindungen der allgemeinen Formel CH₃-C(=O)-O-R, worin R für einen linearen oder verzweigten C₁₋₁₀-Alkyl-Rest steht; bei Temperaturen von vorzugsweise -80 bis 20 °C zu Verbindungen der allgemeinen Formel VI umgesetzt.

In Stufe 2 werden Verbindungen der allgemeinen Formel VI in einem Reaktionsmedium, vorzugsweise ausgewählt aus der Gruppe bestehend aus Methanol, Ethanol, Isopropanol, Wasser und entsprechenden Mischungen, mit einer anorganischen Base, vorzugsweise mit wenigstens einer Base ausgewählt aus der Gruppe bestehend aus Lithiumhydroxid, Kaliumhydroxid und Natriumhydroxid bei Temperaturen von vorzugsweise 20 bis 30 °C zu Verbindungen der allgemeinen Formel VII umgesetzt.

In Stufe 3 werden Verbindungen der allgemeinen Formel VII in einem Reaktionsmedium vorzugsweise ausgewählt aus der Gruppe bestehend aus Dichlormethan, Acetonitril, Toluol, Dioxan, Diethylether, Tetrahydrofuran sowie entsprechenden Mischungen in Gegenwart wenigstens einer organischen Base, vorzugsweise in Gegenwart von Triethylamin, 4,4-Dimethylaminopyridin, Pyridin, N-Methylmorpholin und Diisopropylethylamin mit Diphenylphosphorylazid bei Temperaturen von vorzugsweise 20 bis 120 °C zu Verbindungen der allgemeinen Formel II umgesetzt.

In Stufe 4 werden Verbindungen der allgemeinen Formel II in einem Reaktionsmedium, vorzugsweise ausgewählt aus der Gruppe bestehend aus Dichlormethan, Dimethylformamid, Toluol, Tetrahydrofuran, Acetonitril, Diethylether, Dioxan und entsprechenden Mischungen in Gegenwart wenigstens einer Base, bevorzugt in Gegenwart wenigstens eines Metallhydridsalzes, besonders bevorzugt in Gegenwart von Kalium- und/oder Natriumhydrid, mit Verbindungen der allgemeinen Formel R¹-(CH₂)ₙ-X, worin R¹ und n die vorstehend angegebene Bedeutung haben und X für eine Abgangsgruppe, bevorzugt ein Halogenatom, besonders bevorzugt ein Chlor- oder Bromatom steht, zu Verbindungen der allgemeinen Formel III umgesetzt.

In Stufe 5 werden Verbindungen der allgemeinen Formel III, worin PG für eine tert-Butyloxycarbonyl-Gruppe steht, in einem Reaktionsmedium vorzugsweise ausgewählt aus der Gruppe bestehend aus Dichlormethan, Acetonitril, Methanol, Ethanol, Isopropanol, Wasser, Diethylether, Tetrahydrofuran sowie entsprechenden Mischungen in Gegenwart wenigstens einer Säure vorzugsweise ausgewählt aus der Gruppe bestehend aus Salzsäure, Schwefelsäure, Trifluoressigsäure und Essigsäure bei Temperaturen von vorzugsweise 0 bis 50 °C zu Verbindungen der allgemeinen Formel IV umgesetzt. Besonders bevorzugt erfolgt die Umsetzung der Verbindung der allgemeinen Formel III mit Trifluoressigsäure in Dichlormethan bei Temperaturen von vorzugsweise 20 bis 30 °C zu einer Verbindung der allgemeinen Formel IV.

Alternativ werden Verbindungen der allgemeinen Formel III, worin PG für eine Benzyloxycarbonyl-Gruppe steht, in einem Reaktionsmedium vorzugsweise ausgewählt aus der Gruppe bestehend aus Diethylether, Tetrahydrofuran, Dioxan, Acetonitril, Toluol und entsprechenden Mischungen in Gegenwart von Wasserstoff und Palladium auf Kohle bei Temperaturen von vorzugsweise 20 bis 80 °C zu einer Verbindung der allgemeinen Formel IV umgesetzt.

Sofern Verbindungen der allgemeinen Formel IV in Form eines entsprechenden Hydrochlorids oder als Salz der Trifluoressigsäure vorliegen, werden diese in einem Reaktionsmedium, vorzugsweise ausgewählt aus der Gruppe bestehend aus Dioxan, Tetrahydrofuran, Diethylether, Methanol, Ethanol, Isopropanol, Wasser und entsprechenden Mischungen, in Gegenwart einer anorganischen Base, vorzugsweise unter Zusatz eines Metallhydroxids, beispielsweise Natriumhydroxid, Kaliumhydroxid oder Lithiumhydroxid, bei Temperaturen von vorzugsweise 0°C bis 30°C in die entsprechenden Basen der allgemeinen Formel IV umgewandelt.

In Stufe 6 werden Verbindungen der allgemeinen Formel IV mit Isothiocyanaten der allgemeinen Formel S=C=N-R³, worin R³ die vorstehend angegebene Bedeutung hat, in einem Reaktionsmedium, vorzugsweise ausgewählt aus der Gruppe bestehend aus Acetonitril, Toluol, Tetrahydrofuran, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, 4,4-Dimethylaminopyridin, Pyridin, N-Methylmorpholin und Diisopropylethylamin, zu Verbindungen der allgemeinen Formel I, worin R² für C(=S)-NH-R³ steht, umgesetzt.

Alternativ werden in Stufe 6 Verbindungen der allgemeinen Formel IV mit einem Isocyanat der allgemeinen Formel R⁴-N=C=O, worin R⁴ die vorstehend angegebene Bedeutung hat, in einem Reaktionsmedium, vorzugsweise ausgewählt aus der Gruppe bestehend aus Acetonitril, Tetrahydrofuran, Toluol, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, N-Methylmorpholin, Pyridin, 4,4-Dimethylaminopyridin und Diisopropylethylamin, zu Verbindungen der allgemeinen Formel I umgesetzt, worin R² für -C(=O)-NHR¹⁴ steht.

Ebenfalls können Verbindungen der allgemeinen Formel IV mit Sulfonsäure-Derivaten der allgemeinen Formel X-S(=O)₂-R⁵ oder X-S(=O)₂-NR⁹R¹⁰, worin R⁵, R⁹ und R¹⁰ die vorstehend angegebene Bedeutung haben und X für eine Abgangsgruppe, bevorzugt für ein Halogen-Atom, besonders bevorzugt für ein Chlor- oder Bromatom steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimeihylaminopyridin, Pyridin, N-Methylmorpholin und Diisopropylethylamin, bei Temperaturen von vorzugsweise 70°C bis 100°C zu Verbindungen der allgemeinen Formel I, worin R² für S(=O)₂-R⁵ oder -S(=O)₂-NR⁹R¹⁰ steht, umgesetzt werden.

Alternativ werden in Stufe 6 Verbindungen der allgemeinen Formel IV in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit Verbindungen der allgemeinen Formel X-(CH₂)-R⁷ oder X-(CH₂)-C(=O)-NH-R⁶ oder X-(CH₂)-Dₐₐ-(CH₂)_{bb}-E_{cc}-(CH₂)_{dd}-R⁷, worin R⁶, R⁷, D, E, aa, bb, cc und dd die vorstehend angegebene Bedeutung haben und X für eine Abgangsgruppe, bevorzugt ein Halogenatom, besonders bevorzugt ein Chlor- oder Bromatom steht, zu Verbindungen der allgemeinen Formel I umgesetzt, worin R² für -(CH₂)-R⁷, (CH₂)-C(=O)-NH-R⁶ oder -(CH₂)-Dₐₐ-(CH₂)_{bb}-E_{cc}-(CH₂)_{dd}-R⁷ steht.

In Stufe 6 werden Verbindungen der allgemeinen Formel IV mit CarbonsäureDerivaten der allgemeinen Formel X-C(=O)-R⁸, worin R⁸ die vorstehend angegebene Bedeutung hat und X für eine Abgangsgruppe, bevorzugt für ein Halogen-Atom, besonders bevorzugt für ein Chlor- oder Bromatom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, N-Methylmorpholin, Pyridin und Diisopropylethylamin, bei Temperaturen von vorzugsweise 70°C bis 100°C zu Verbindungen der allgemeinen Formel. I, worin R² für -C(=O)-R⁸ steht, umgesetzt.

Alternativ werden Verbindungen der vorstehend angegebenen allgemeinen Formel IV mit Carbonsäuren der allgemeinen Formel OH-C(=O)-R⁸, worin R⁸ die vorstehend angegebene Bedeutung hat, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart einer Base, vorzugsweise ausgewählt aus der Gruppe bestehend aus Triethylamin, Pyridin, N-Methylmorpholin, 4,4-Dimethylaminopyridin und Diisopropylethylamin bei Temperaturen von vorzugsweise 70°C bis 100°C zu Verbindungen der allgemeinen Formel I, worin R² für -C(=O)-R⁸ steht, umgesetzt.

Die vorstehend beschriebenen Umsetzungen können jeweils unter den üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten 1-Oxa-3,8-diazaspiro[4.5]-decan-2-on-Verbindungen der vorstehend genannten allgemeinen Formel I sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten 1-Oxa-3,8-diazaspiro[4.5]-decan-2-on-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen substituierten 1-Oxa-3,8-diazaspiro[4.5]-decan-2-on-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten 1-Oxa-3,8-diazaspiro[4.5]-decan-2-on-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-AlkylRest steht, genannt.

Die erfindungsgemäßen substituierten 1-Oxa-3,8-diazaspiro[4.5]-decan-2-on-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten 1-Oxa-3,8-diazaspiro[4.5]-decan-2-on-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 1-Oxa-3,8-diazaspiro[4.5]-decan-2-on-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße 1-Oxa-3,8-diazaspiro[4.5]-decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Die erfindungsgemäßen Arzneimittel eignen sich insbesondere zur Noradrenalin-Rezeptor-Regulation, insbesondere zur Hemmung der Noradrenalin-Wiederaufnahme (NA-Uptake), zur 5-HT-Rezeptor-Regulation, insbesondere zur Hemmung der 5-Hydroxy-Tryptophan-Wiederaufnahme (5-HT-Uptake) und/oder zur µ-Opioid-Rezeptor-Regulation, insbesondere zur Hemmung des µ-Opioid-Rezeptors.

Ebenfalls bevorzugt eignen sich die erfindungsgemäßen Arzneimittel zur Prophylaxe und/oder Behandlung von Störungen oder Krankheiten, die zumindest teilweise durch Noradrenalin-Rezeptoren, 5-HT-Rezeptoren und/oder µ-Opioid-Rezeptoren vermittelt werden.

Bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Wasserspeicher-Krankheiten; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Katalepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Unterdrückung des Miktionsreflexes; zur Regulation des kardiovaskulären Systems, bevorzugt zur Vasodilatation der Arterien; zur Lokalanästhesie; zur Vigilanzsteigerung; zur Libidosteigerung; zur Diurese und/oder zur Antinatriurese.

Besonders bevorzugt eignet sich das erfindungsgemäße Arzneimittel zur Behandlung und/oder Prophylaxe von einer oder mehrerer Erkrankungen ausgewählt aus der Gruppe bestehend aus Schmerz, vorzugsweise von Schmerz ausgewählt aus der Gruppe bestehend aus akutem Schmerz, chronischem Schmerz und neuropathischem Schmerz, Harninkontinenz, Depressionen und Angstzuständen.

Ein weiterer Gegenstand der vorliegenden Erfindung ist die Verwendung wenigstens einer erfindungsgemäßen substituierten 1-Oxa-3,8-diazaspiro[4.5]-decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Noradrenalin-Rezeptor-Regulation, insbesondere zur Hemmung der Noradrenalin-Wiederaufnahme (NA-Uptake), zur 5-HT-Rezeptor-Regulation, insbesondere zur Hemmung der 5-Hydroxy-Tryptophan-Wiederaufnahme (5-HT-Uptake) und/oder zur µ-Opioid-Rezeptor-Regulation, insbesondere zur µ-Opioid-Rezeptor-Hemmung.

Bevorzugt ist die Verwendung wenigstens einer erfindungsgemäßen substituierten 1-Oxa-3,8-diazaspiro[4.5]-decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Noradrenalin-Rezeptoren, 5-HT-Rezeptoren und/oder µ-Opioid-Rezeptoren vermittelt werden.

Besonders bevorzugt ist die Verwendung wenigstens einer erfindungsgemäßen substituierten 1-Oxa-3,8-diazaspiro[4.5]-decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Wasserspeicher-Krankheiten; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Katalepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Unterdrückung des Miktionsreflexes; zur Regulation des kardiovaskulären Systems, bevorzugt zur Vasodilatation der Arterien; zur Lokalanästhesie; zur Vigilanzsteigerung; zur Libidosteigerung; zur Diurese und/oder zur Antinatriurese.

Ganz besonders bevorzugt ist die Verwendung wenigstens einer erfindungsgemäßen substituierten 1-Oxa-3,8-diazaspiro[4.5]-decan-2-on-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehrerer Erkrankungen ausgewählt aus der Gruppe bestehend aus Schmerz, vorzugsweise von Schmerz ausgewählt aus der Gruppe bestehend aus akutem Schmerz, chronischem Schmerz und neuropathischem Schmerz, Harninkontinenz, Depressionen und Angstzuständen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten 1-Oxa-3,8-diazaspiro[4.5]-decan-2-on-Verbindung der vorstehend angegebenen allgemeinen Formel I, ggf. in Form eines ihrer reinen Stereoisomeren, insbesondere Enantiomeren oder Diastereomeren, ihrer Racemate oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten 1-Oxa-3,8-diazaspiro[4.5]-decan-2-on-Verbindungen können in einem Depot in gelöster Form oder in einem Pflaster, gegebenenfalls unter Zusatz von die Hautpenetration fördernden Mitteln, als geeignete perkutane Applikationszubereitungen vorliegen.

Oral oder perkutan anwendbare Zubereitungsformen können die jeweiligen erfindungsgemäßen substituierten 1-Oxa-3,8-diazaspiro[4.5]-decan-2-on-Verbindungen auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten 1-Oxa-3,8-diazaspiro[4.5]-decan-2-on-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden:

### a) Methode zur Bestimmung der Noradrenalin- und der 5-HT-Uptake-Inhibierung:

Für in vitro Studien werden Synaptosomen aus Rattenhirnarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1ml) in einem Glas-Homogenisierer mit Teflonstößel homogenisiert, indem fünf volle Auf- und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert werden.
Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 ml inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:
NA-Uptake : Km = 0,32 ± 0,11 µM
5HT-Uptake : Km = 0,084 ± 0,011 µM

### b) Methode zur Bestimmung der Affinität zum humanen µ-Opioid-Rezeptor

Die Rezeptoraffinität zum humanen µ-Opioid-Rezeptor wird in einem homogenen Ansatz in Mikrotiterplatten bestimmt. Hierzu werden Verdünnungsreihen des jeweils zu prüfenden substituierten 1-Oxa-3,8-diazaspiro[4.5]-decan-2-on-Verbindungen mit einer Rezeptormembranpräparation (15-40 µg Protein pro 250 µl Inkubationsansatz) von CHO-K1-Zellen, welche den humanen µ-Opioid-Rezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wird die prozentuale Verdrängung des radioaktiven Liganden aus seiner Bindung zum humanen µ-Opioid-Rezeptor bei einer Konzentration der Prüfsubstanzen von 1 µmol/l bestimmt und als Prozent Hemmung der spezifischen Bindung angegeben.

Im Folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Ether" bedeutet Diethylether, "EtOAc" Ethylacetat, "DCM" Dichlormethan, "DMF" N,N-Dimethylformamid, "EtOH" Ethanol, "MeOH" Methanol Die Angabe "Äquivalente" bedeutet Stoffmengenäquivalente, "Smp." Schmelzpunkt bzw. Schmelzbereich, "RT" Raumtemperatur, d.h. ca 20 °C, "min" Minuten, "h" Stunden, "ges." gesättigt und aq. "wäßrig".

Die eingesetzten Chemikalien und Lösungsmittel wurden kommerziell bei den herkömmlichen Anbietern (Acros, Acocado, Aldrich, Bachem, Fluka, Lancaster, Maybridge, Merck, Sigma, TCi etc.) erworben oder nach üblichen, dem Fachmann geläufigen Methoden synthetisiert.

Als stationäre Phase für die Säulenchromathographie wurde Kieselgel 60 (0.04- - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben. Die Analytik erfolgte über NMR und HPLC-MS.

### Synthese von 2-Oxo-1-oxa-3,8-diaza-spiro[4.5]decan-8-carbonsäure-tert-butylester (A3)

### 1. Synthese von 4-Ethoxycarbonylmethyl-4-hydroxy-piperidin-1-carbonsäure-tert-butylester (A1)

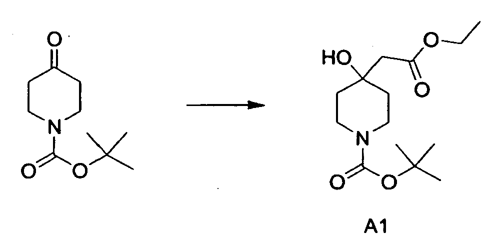

Eine 1.0 M Lösung von Lithiumhexamethyldisilazid (LiHMDS) in THF (40 mL, 40 mmol) wurde unter einer Stickstoffatmosphäre auf -70 °C gekühlt. Es wurde EtOAc (3.91 mL, 40 mmol) langsam hinzu getropft. Das Reaktionsgemisch wurde 10 min bei - 70 °C gerührt und eine Lösung von 1-(tert-Butyloxycarbonyl)-4-piperidon (7.34 g, 36.84 mmol, Lancaster, Bestellnummer: 13361) in THF (16 mL) wird langsam hinzu getropft. Nach Erwärmen der Reaktionslösung auf 0 °C wurde Wasser (50 mL) hinzugefügt und die Reaktionsmischung mehrfach mit Ether extrahiert. Die vereinigten organischen Phasen wurden mit ges. aq. NaCl-Lösung gewaschen, über Na₂SO₄ getrocknet und das Lösungsmittel wurde im Vakuum entfernt. Es wurden 11.31 g des gewünschten Produkts 4-Ethoxycarbonylmethyl-4-hydroxy-piperidin-1-carbonsäure-tert-butylester (A1) erhalten, das ohne Aufreinigung weiter umgesetzt wurde.

### 2. Synthese von 4-Carboxymethyl-4-hydroxy-piperidin-1-carbonsäure-tert-butylester (A2)

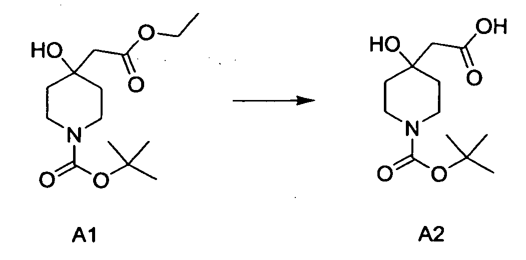

Zu einer Lösung von A1 (11.31 g) in Methanol (40 mL) wurde eine 2.0 M Lösung von Natriumhydroxid in Wasser (28 mL) gegeben. Das Reaktionsgemisch wurde 1 h bei RT gerührt. Das organische Lösungsmittel wurde im Vakuum entfernt und das verbliebene wäßrige Reaktionsgemisch wurde mehrfach mit Ether extrahiert. Die wäßrige Phase wurde mit 2.0 M Salzsäure-Lösung in Wasser angesäuert und mehrfach mit DCM extrahiert. Die vereinigten organischen Phasen aus DCM wurden mit ges. aq. NaCl-Lösung gewaschen, über Na₂SO₄ getrocknet und das Lösungsmittel wurde im Vakuum entfernt. Es wurden 7.9 g des gewünschten Produkts 4-Carboxymethyl-4-hydroxy-piperidin-1-carbonsäure-tert-butylester (A2) erhalten, das ohne Aufreinigung weiter umgesetzt wurde.

### 3. Synthese von 2-Oxo-1-oxa-3,8-diaza-spiro[4.5]decan-8-carbonsäure-tert-butylester (A3)

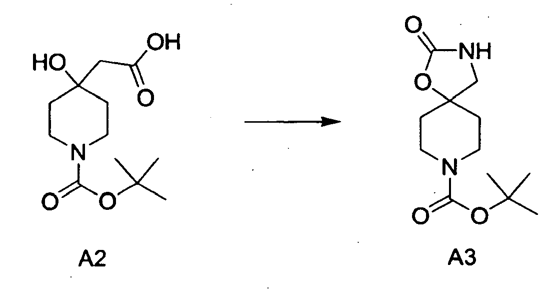

Zu einer Lösung von A2 (7.9 g) in trockenem Toluol (205 mL) wurden unter einer Stickstoffatmosphäre nacheinander Triethylamin (5.59 mL, 39.78 mmol) und Diphenylphosphorylazid (10.86 mL, 5.1 mmol) gegeben. Das Reaktionsgemisch wurde auf 80 bis 90 °C erhitzt, wobei eine Gasentwicklung beobachtet wurde, die nach 90 min aufhörte. Es wurde weitere 2 h bei 115 °C gerührt. Nach Abkühlen wurde das Reaktionsgemisch mit EtOAc verdünnt und mehrfach mit Wasser gewaschen. Die vereinigten wäßrigen Phasen wurden mit EtOAc extrahiert und die vereinigten organischen Phasen wurden mit ges. aq. NaCl-Lösung gewaschen, über Na₂SO₄ getrocknet und das Lösungsmittel wurde im Vakuum entfernt. Der Rückstand wurde mehrfach mit Ether gewaschen. Es blieben 5.72 g (60.6 % der Theorie über drei Stufen ausgehend von 1-(tert-Butyloxycarbonyl)-4-piperidon) des gewünschten Produkts 2-Oxo-1-oxa-3,8-diaza-spiro[4.5]decan-8-carbonsäure-tert-butylester (A3) als weißer Feststoff zurück.
Verbindungen der vorstehend angegebenen allgemeinen Formel I, in denen R² für - C(=S)-NH-R³ oder-C(=O)-NH-R⁴ steht, lassen sich analog den Beispielen 29, 33 und 52 herstellen. Dem Fachmann sind die dafür benötigten Ausgangsstoffe bekannt.

### Beispiel 33: 3-Benzyl-2-oxo-1-oxa-3,8-diaza-spiro[4.5]decan-8-carbonsäure-(3-cyano-phenyl)-amid

### 1. Synthese von 3-Benzyl-2-oxo-1-oxa-3,8-diaza-spiro[4.5]decan-8-carbonsäure-tert-butylester (A4)

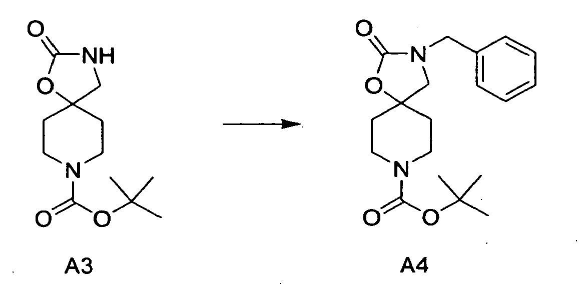

Natriumhydrid (82 mg, 60%-ige Dispersion in Mineralöl, Gewichtsprozent) wurde in einer Stickstoffatmosphäre in DMF (5 mL) suspendiert. Die Verbindung A3 (0.5 g, 1.95 mmol) wurde hinzu gegeben und das Reaktionsgemisch wurde 10 min bei RT gerührt. Es wurde Benzylbromid (235 µL, 1.97 mmol) hinzu gegeben. Das Reaktionsgemisch wurde 18 h bei RT gerührt, mit Wasser versetzt und mit EtOAc extrahiert. Die vereinigten organischen Phasen wurden mit ges. aq. NaCl-Lösung gewaschen, über Na₂SO₄ getrocknet und das Lösungsmittel wurde im Vakuum entfernt. Der Rückstand wurde in einer Mischung aus Ether und Heptan gelöst. Nach Entfernen des Ethers wurde das gewünschte Produkt 3-Benzyl-2-oxo-1-oxa-3,8-diaza-spiro[4.5]decan-8-carbonsäure-tert-butylester (A4) (0.54 g, 80 % der Theorie) als weißer Feststoff durch Filtration erhalten.

### 2. Synthese von 3-Benzyl-2-oxo-1-oxa-3,8-diaza-spiro[4.5]decan-8-carbonsäure-(3-cyano-phenyl)-amid (33)

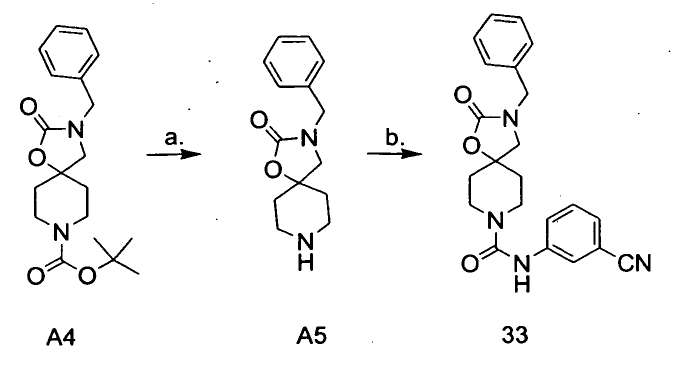

a. Die Verbindung A4 (0.57 g, 1.65 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden über Na₂SO₄ getrocknet und das Lösungsmittel im Vakuum entfernt.
b. Der Rückstand aus Stufe a. wurde in wasserfreiem THF (5 mL) aufgenommen und mit wenig Triethylamin versetzt. Das Reaktionsgemisch wurde mit 3-Cyanophenylisocyanat (249 mg, 1.73 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden über Na₂SO₄ getrocknet und das Lösungsmittel im Vakuum entfernt. Der Rückstand wurde säulenchromatographisch (SiO₂, Heptan/EtOAc 1:1) gereinigt. Nach Kristallisation aus Heptan und DCM wurden 242 mg (37 % der Theorie) des gewünschten Produkts 3-Benzyl-2-oxo-1-oxa-3,8-diaza-spiro[4.5]decan-8-carbonsäure-(3-cyano-phenyl)-amid (33) erhalten

### Beispiel 29: 3-(3-Brom-benzyl)-2-oxo-1-oxa-3,8-diaza-spiro[4.5]decan-8-carbonsäure-(2,5-difluor-phenyl)-amid

### 1. Synthese von 3-(3-Brom-benzyl)-2-oxo-1-oxa-3,8-diaza-spiro[4.5]decan-8-carbonsäure-tert-butylester (A6)

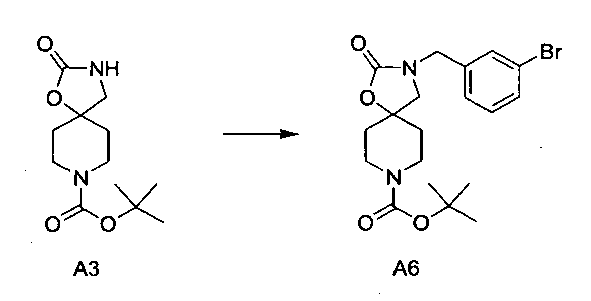

Natriumhydrid (246 mg, 60%-ige Dispersion in Mineralöl, Gewichtsprozent) wurde in einer Stickstoffatmosphäre in DMF (15 mL) suspendiert. Die Verbindung A3 (1.5 g, 5.85 mmol) wurde hinzu gegeben und das Reaktionsgemisch wurde 10 min bei RT gerührt. Es wurde 3-Brom-benzylbromid (1.48 g, 5.91 mmol) hinzu gegeben. Das Reaktionsgemisch wurde 18 h bei RT gerührt, mit Wasser versetzt und mit EtOAc extrahiert. Die vereinigten organischen Phasen wurden mit ges. aq. NaCl-Lösung gewaschen, über Na₂SO₄ getrocknet und das Lösungsmittel wurde im Vakuum entfernt. Der Rückstand wurde in einer Mischung aus Ether und Heptan gelöst. Nach Entfernen des Ethers wurde das gewünschte Produkt 3-(3-Brom-benzyl)-2-oxo-1-oxa-3,8-diaza-spiro[4.5]decan-8-carbonsäure-tert-butylester (A6) (2.33 g, 93 % der Theorie) als weißer Feststoff durch Filtration erhalten.

### 2. Synthese von 3-(3-Brom-benzyl)-2-oxo-1-oxa-3,8-diaza-spiro[4.5]decan-8-carbonsäure-(2,5-difluor-phenyl)-amid (29)

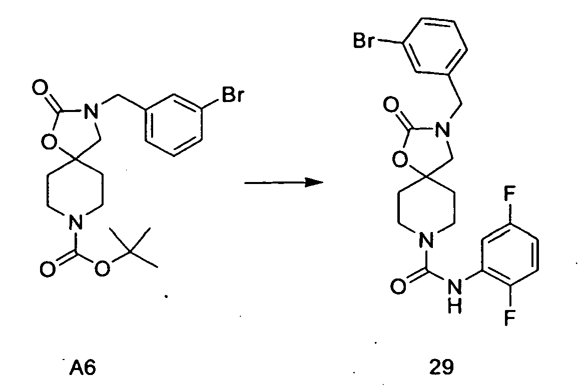

Die Verbindung A6 (0.50 g, 1.18 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden über Na₂SO₄ getrocknet und das Lösungsmittel im Vakuum entfernt. Der Rückstand wurde in wasserfreiem THF (5 mL) aufgenommen und mit wenig Triethylamin versetzt. Das Reaktionsgemisch wurde mit 2,5-Dilfluorphenylisocyanat (145 µL, 1.23 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden mit ges. aq. NaCl-Lösung gewaschen, über Na₂SO₄ getrocknet und das Lösungsmittel im Vakuum entfernt. Der Rückstand wurde säulenchromatographisch (SiO₂, Heptan/EtOAc 2:1) gereinigt. Nach Kristallisation aus Heptan und DCM wurden 483 mg (85 % der Theorie) des gewünschten Produkts 3-(3-Brom-benzyl)-2-oxo-1-oxa-3,8-diaza-spiro[4.5]decan-8-carbonsäure-(2,5-difluor-phenyl)-amid (29) erhalten.

### Beispiel 52: 3-Naphthalen-2-ylmethyl-2-oxo-1-oxa-3,8-diaza-spiro[4.5]decan-8-c carbonsäure-(4-methoxy-phenyl)-amid

### 1. Synthese von 3-Naphthalen-2-ylmethyl-2-oxo-1-oxa-3,8-diaza-spiro[4.5]decan-8-carbonsäure-tert-butylester (A7)

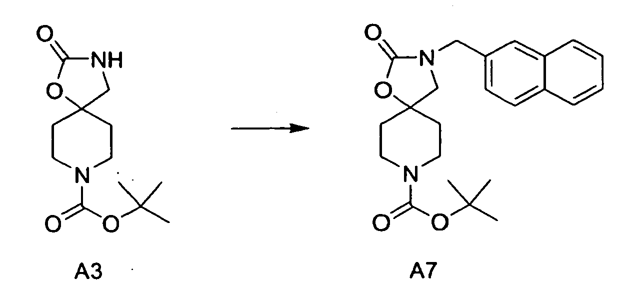

Natriumhydrid (82 mg, 60%-ige Dispersion in Mineralöl, Gewichtsprozent) wurde in einer Stickstoffatmosphäre in DMF (5 mL) suspendiert. Die Verbindung A3 (0.5 g, 1.95 mmol) wurde hinzu gegeben und das Reaktionsgemisch wurde 10 min bei RT gerührt. Es wurde 2-Bromomethylnaphthol (436 mg, 1.97 mmol) hinzu gegeben. Das Reaktionsgemisch wurde 18 h bei RT gerührt, mit Wasser versetzt und mit EtOAc extrahiert. Die vereinigten organischen Phasen wurden mit ges. aq. NaCl-Lösung gewaschen, über Na₂SO₄ getrocknet und das Lösungsmittel wurde im Vakuum entfernt. Der Rückstand wurde in einer Mischung aus Ether und Heptan gelöst. Nach Entfernen des Ethers wurde das gewünschte Produkt 3-Naphthalen-2-ylmethyl-2-oxo-1-oxa-3,8-diaza-spiro[4.5]decan-8-carbonsäure-tert-butylester (A7) (260 mg, 34 % der Theorie) als weißer Feststoff durch Filtration erhalten.

### 2. Synthese von 3-Naphthalen-2-ylmethyl-2-oxo-1-oxa-3,8-diaza-spiro[4.5]decan-8-carbonsäure-(4-methoxy-phenyl)-amid (52)

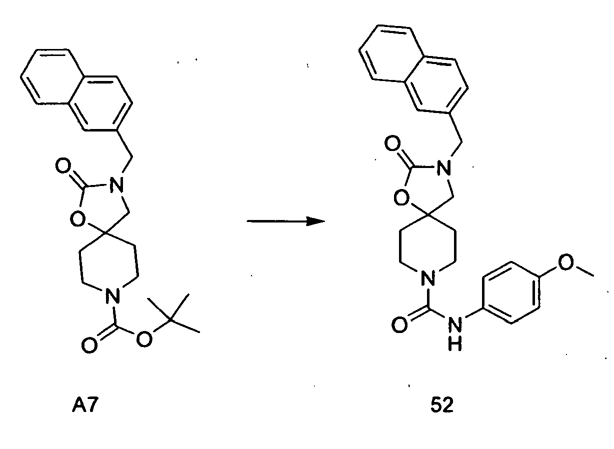

Die Verbindung A7 (0.50 g, 1.26 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden über Na₂SO₄ getrocknet und das Lösungsmittel im Vakuum entfernt. Der Rückstand wurde in wasserfreiem THF (5 mL) aufgenommen und mit wenig Triethylamin versetzt. Das Reaktionsgemisch wurde mit 4-Methoxphenylisocyanat (202 mg, 1.35 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden über Na₂SO₄ getrocknet und das Lösungsmittel im Vakuum entfernt. Der Rückstand wurde säulenchromatographisch (SiO₂, Heptan/EtOAc 2:1) gereinigt. Nach Kristallisation aus Heptan und DCM wurden 237 mg (42 % der Theorie) des gewünschten Produkts 3-Naphthalen-2-ylmethyl-2-oxo-1-oxa-3,8-diaza-spiro[4.5]decan-8-carbonsäure-(4-methoxy-phenyl)-amid (52) erhalten.

Verbindungen der vorstehend angegebenen allgemeinen Formel I, in denen R² für - S(=O)₂-R⁵, -S(=O)2-NR⁹R¹⁰ oder für -C(=O)-R⁸ steht, lassen sich analog den Beispielen 137 und 144 herstellen. Dem Fachmann sind die dafür benötigten Ausgangsstoffe bekannt.

### Beispiel 137: 3-(3-Brom-benzyl)-8-(3,5-dimethoxy-benzoyl)-1-oxa-3,8-diaza-spiro[4.5]decan-2-on

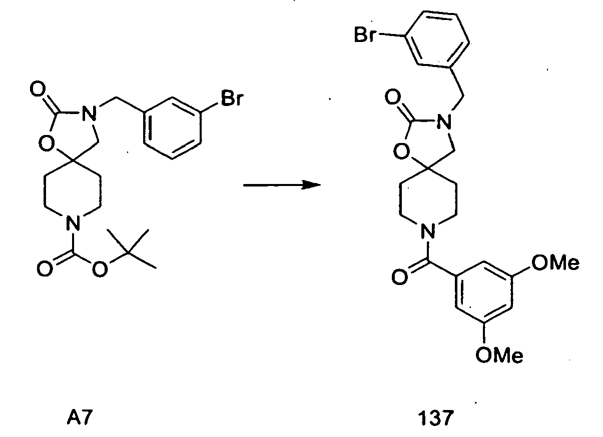

Die Verbindung A7 (0.50 g, 1.18 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden mit ges. aq. NaCl-Lösung gewaschen, über Na₂SO₄ getrocknet und das Lösungsmittel im Vakuum entfernt. Der Rückstand wurde in wasserfreiem DCM (5 mL) aufgenommen und mit Triethylamin (148 µL, 1.76 mmol) versetzt. Das Reaktionsgemisch wurde mit 3,5-Dimethoxybenzoylchlorid (technisch (92%-ig), 282 mg, 1.29 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden mit ges. aq. NaCl-Lösung gewaschen, über Na₂SO₄ getrocknet und das Lösungsmittel im Vakuum entfernt. Der Rückstand wurde säulenchromatographisch (SiO₂, Heptan/EtOAc 1:2) gereinigt. Es wurden 350 mg (60 % der Theorie) des gewünschten Produkts 3-(3-Brom-benzyl)-8-(3,5-dimethoxy-benzoyl)-1-oxa-3,8-diaza-spiro[4.5]decan-2-on (137) erhalten.

### Beispiel 144: 3-(3-Brom-benzyl)-8-(2-phenoxy-pyridin-3-carbonyl)-1-oxa-3,8-diaza-spiro[4.5]decan-2-on

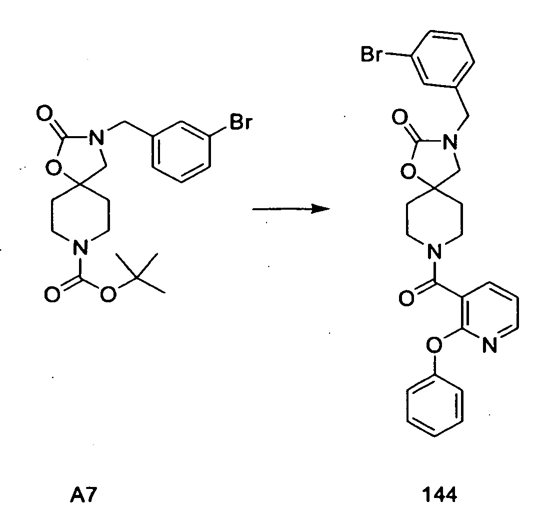

Die Verbindung A7 (0.50 g, 1.18 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden über Na₂SO₄ getrocknet und das Lösungsmittel im Vakuum entfernt. Der Rückstand wurde in wasserfreiem DCM (5 mL) aufgenommen und mit Triethylamin (148 µL, 1.76 mmol) versetzt. Das Reaktionsgemisch wurde mit 2-Phenoxy-pyridin-3-carbonyl-chlorid (technisch (80%-ig), 377 mg, 1.29 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden mit ges. aq. NaCl-Lösung gewaschen, über Na₂SO₄ getrocknet und das Lösungsmittel im Vakuum entfernt. Der Rückstand wurde säulenchromatographisch (SiO₂, Heptan/EtOAc 1:2) gereinigt. Es wurden 539 mg (88 % der Theorie) des gewünschten Produkts 3-(3-Brom-benzyl)-8-(2-phenoxy-pyridin-3-carbonyl)-1-oxa-3,8-diaza-spiro[4.5]decan-2-on (144) erhalten.

Verbindungen der vorstehend angegebenen allgemeinen Formel I, in denen R² für - (CH₂)-R⁷, -(CH₂)-C(=O)-NH-R⁶ und -(CH₂)-Dₐₐ-(CH₂)_{bb}-E_{cc}-(CH₂)_{dd}-R⁷ steht, lassen sich analog dem Beispiel 432 herstellen. Dem Fachmann sind die dafür benötigten Ausgangsstoffe bekannt.

### Beispiel 432: 3-(2-Methoxy-5-nitro-benzyl)-8-(3-phenoxy-propyl)-1-oxa-3,8-diaza-spiro[4.5]decan-2-on

### 1. Synthese von 3-(2-Methoxy-5-nitrobenzyl)-2-oxo-1-oxa-3,8-diaza-spiro[4.5]decan-8-carbonsäure-tert-butylester (A8)

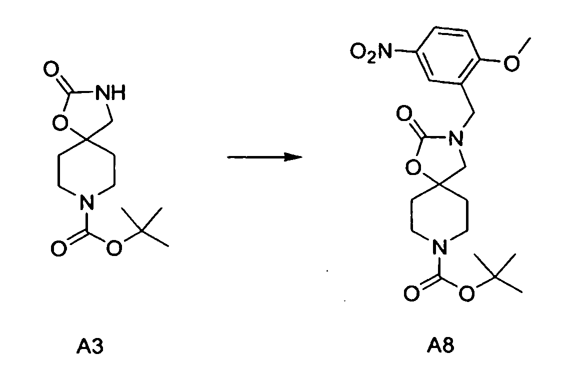

Natriumhydrid (82 mg, 60%-ige Dispersion in Mineralöl, Gewichtsprozent) wurde in einer Stickstoffatmosphäre in DMF (5 mL) suspendiert. Die Verbindung A3 (0.5 g, 1.95 mmol) wurde hinzu gegeben und das Reaktionsgemisch wurde 10 min bei RT gerührt. Es wurde 2-Methoxy-5-nitrobenzylbromid (485 mg, 1.97 mmol) hinzu gegeben. Das Reaktionsgemisch wurde 18 h bei RT gerührt, mit Wasser versetzt und mit EtOAc extrahiert. Die vereinigten organischen Phasen wurden mit ges. aq. NaCl-Lösung gewaschen, über Na₂SO₄ getrocknet und das Lösungsmittel wurde im Vakuum entfernt. Der Rückstand wurde in einer Mischung aus Ether und Heptan gelöst. Nach Entfernen des Ethers wurde das gewünschte Produkt 3-(2-Methoxy-5-nitrobenzyl)-2-oxo-1-oxa-3,8-diaza-spiro[4.5]decan-8-carbonsäure-tert-butylester (A87) (670 mg, 81 % der Theorie) als weißer Feststoff durch Filtration erhalten.

### 2. Synthese von 3-(2-Methoxy-5-nitro-benzyl)-8-(3-phenoxy-propyl)-1-oxa-3,8-diaza-spiro[4.5]decan-2-on (432)

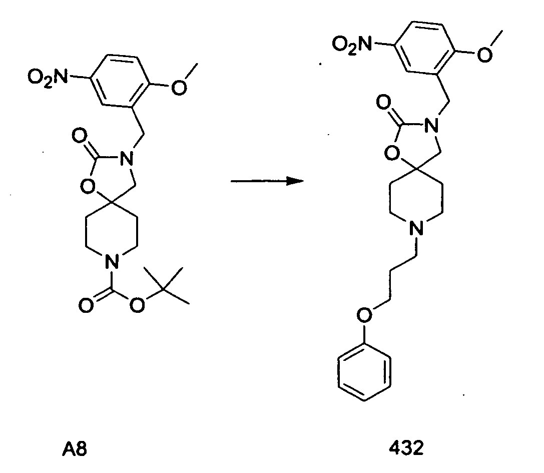

Die Verbindung A8 (0.67 g, 1.59 mmol) wurde in DCM (3 mL) gelöst und mit Trifluoressigsäure (3 mL) versetzt. Das Reaktionsgemisch wurde 5 min bei RT gerührt und anschließend das Lösungsmittel im Vakuum entfernt. Der Rückstand wurde mehrfach mit Toluol versetzt und anschließend das Lösungsmittel wieder entfernt. Anschließend wurde der Rückstand mit ges. aq. NaHCO₃-Lösung versetzt und mehrfach mit EtOAc extrahiert. Die vereinigten organischen Phasen wurden über Na₂SO₄ getrocknet und das Lösungsmittel im Vakuum entfernt. Der Rückstand wurde in wasserfreiem THF (5 mL) aufgenommen und mit Diisopropylethylamin (333 µL, 1.91 mmol) versetzt. Das Reaktionsgemisch wurde mit 3-Phenoxy-propylbromid (277 µL, 1.75 mmol) versetzt, 18 h bei RT gerührt und mit Wasser und EtOAc versetzt. Die organische Phase wurde abgetrennt und die wäßrige Phase mehrfach mit EtOAc extrahiert. Die vereinigten organischen Phasen wurden über Na₂SO₄ getrocknet und das Lösungsmittel im Vakuum entfernt. Der Rückstand wurde säulenchromatographisch (SiO₂, DCM/MeOH 98:2) gereinigt. Es wurden 433 mg (58 % der Theorie) des gewünschten Produkts 3-(2-Methoxy-5-nitro-benzyl)-8-(3-phenoxy-propyl)-1-oxa-3,8-diaza-spiro[4.5]decan-2-on (432) erhalten.

Die zuvor nicht detailliert beschriebene Herstellung der übrigen Verbindungen gemäß den nachstehend angegebenen Beispielen erfolgte ebenfalls analog den vorstehend angegebenen Herstellungsvorschriften, wobei dem Fachmann aufgrund dieser Vorschriften die jeweils eingesetzten Edukte bekannt sind.

| | |
|---|---|
| [1] | 3-(4-Methyl-benzyl)-2-oxo-1-oxa-3,8-diaza-spiro[4.5]decan-8-carbothiosäure-pentafluorphenyl-amid |
| [2] | 3-(3-Brom-benzyl)-2-oxo-1-oxa-3,8-diaza-spiro[4.5]decan-8-carbothiosäure-(2,5-dichlor-phenyl)-amid |
| [3] | 2-Oxo-3-(4-trifluormethyl-benzyl)-1-oxa-3,8-diaza-spiro[4.5]decan-8-carbothiosäurebenzylamid |
| [4] | 3-(3,5-Dimethyl-benzyl)-2-oxo-1-oxa-3,8-diaza-spiro[4.5]decan-8-carbothiosäure-(tetrahydro-furan-2-ylmethyl)-amid |
| [5] | 3-(3-Brom-benzyl)-2-oxo-1-oxa-3,8-diaza-spiro[4.5]decan-8-carbothiosäure-(2-methoxy-ethyl)-amid |
| [6] | 3-(3-Brom-benzyl)-2-oxo-1-oxa-3,8-diaza-spiro[4.5]decan-8-carbothiosäure-(2-methoxy-phenyl)-amid |
| [7] | 3-(3,5-Dimethyl-benzyl)-2-oxo-1-oxa-3,8-diaza-spiro[4.5]decan-8-carbothiosäure-(3-acetyl-phenyl)-amid |
| [8] | 3-Benzyl-2-oxo-1-oxa-3,8-diaza-spiro[4.5]decan-8-carbothiosäure-o-tolylamid |
| [9] | 3-{[3-(3-Brom-benzyl)-2-oxo-1-oxa-3,8-diaza-spiro[4.5]decan-8-carbothioyl]-amino}-butansäureethylester |
| [10] | 3-Naphthalen-2-ylmethyl-2-oxo-1-oxa-3,8-diaza-spiro[4.5]decan-8-carbothiosäureallylamid |
| [11] | 3-(3,4-Difluor-benzyl)-2-oxo-1-oxa-3,8-diaza-spiro[4.5]decan-8-carbothiosäurebenzylamid |
| [12] | 3-Biphenyl-2-ylmethyl-2-oxo-1-oxa-3,8-diaza-spiro[4.5]decan-8-carbothiosäure-(1-phenyl-ethyl)-amid |
| [13] | 3-(3,4-Difluor-benzyl)-2-oxo-1-oxa-3,8-diaza-spiro[4.5]decan-8-carbothiosäure-(4-ethoxy-phenyl)-amid |
| [14] | 3-(3-Cyano-benzyl)-2-oxo-1-oxa-3,8-diaza-spiro[4.5]decan-8-carbothiosäure-(3,5-dichlor-phenyl)-amid |
| [15] | 3-(3-Cyano-benzyl)-2-oxo-1-oxa-3,8-diaza-spiro[4.5]decan-8-carbothiosäure-(3-trifluormethyl-phenyl)-amid |
| [16] | 3-(3-Brom-benzyl)-2-oxo-1-oxa-3,8-diaza-spiro[4.5]decan-8-carbothiosäure-benzylamid |
| [17] | 3-(2-Methoxy-5-nitro-benzyl)-2-oxo-1-oxa-3,8-diaza-spiro[4.5]decan-8-carbothiosäure-(tetrahydro-furan-2-ylmethyl)-amid |
| [18] | 3-Biphenyl-2-ylmethyl-2-oxo-1-oxa-3,8-diaza-spiro[4.5]decan-8-carbothiosäure-phenylamid |
| [19] | 3-(3-Cyano-benzyl)-2-oxo-1-oxa-3,8-diaza-spiro[4.5]decan-8-carbothiosäure-cyclohexylmethyl-amid |
| [20] | 3-(2-Fluor-benzyl)-2-oxo-1-oxa-3,8-diaza-spiro[4.5]decan-8-carbothiosäure-(3-acetyl-phenyl)-amid |
| [21] | 3-(2-Methoxy-5-nitro-benzyl)-2-oxo-1-oxa-3,8-diaza-spiro[4.5]decan-8-carbothiosäure-cyclohexylmethyl-amid |
| [22] | 3-(3-Cyano-benzyl)-2-oxo-1-oxa-3,8-diaza-spiro[4.5]decan-8-carbothiosäure-4-chlor-benzylamid |
| [23] | 3-(2-Methoxy-5-nitro-benzyl)-2-oxo-1-oxa-3,8-diaza-spiro[4.5]decan-8-carbothiosäure-benzylamid |
| [24] | 3-(2-Fluor-benzyl)-2-oxo-1-oxa-3,8-diaza-spiro[4.5]decan-8-carbonsäure-(4-ethoxy-phenyl)-amid |
| [25] | 3-Biphenyl-2-ylmethyl-2-oxo-1-oxa-3,8-diaza-spiro[4.5]decan-8-carbonsäure-(2,5-dichlor-phenyl)-amid |
| [26] | 3-(2-Methoxy-5-nitro-benzyl)-2-oxo-1-oxa-3,8-diaza-spiro[4.5]decan-8-carbonsäure-(2,5-difluor-phenyl)-amid |
| [27] | 3-(4-Methyl-benzyl)-2-oxo-1-oxa-3,8-diaza-spiro[4.5]decan-8-carbonsäure-(4-methyl-3-nitro-phenyl)-amid |
| [28] | 3-(3,4-Difluor-benzyl)-2-oxo-1-oxa-3,8-diaza-spiro[4.5]decan-8-carbonsäure-4-fluor-benzylamid |
| [29] | 3-(3-Brom-benzyl)-2-oxo-1-oxa-3,8-diaza-spiro[4.5]decan-8-carbonsäure-(2,5-difluor-phenyl)-amid |
| [30] | 4-[(3-Naphthalen-2-ylmethyl-2-oxo-1-oxa-3,8-diaza-spiro[4.5]decan-8-carbonyl)-amino]-benzoesäure-ethyl-ester |
| [31] | 2-Oxo-3-(4-trifluormethyl-benzyl)-1-oxa-3,8-diaza-spiro[4.5]decan-8-carbonsäure-(4-chlor-3-trifluormethyl-phenyl)-amid |
| [32] | 3-(2-Cyano-benzyl)-2-oxo-1-oxa-3,8-diaza-spiro[4.5]decan-8-carbonsäure-(2,5-difluor-phenyl)-amid |
| [33] | 3-Benzyl-2-oxo-1-oxa-3,8-diaza-spiro[4.5]decan-8-carbonsäure-(3-cyano-phenyl)-amid |
| [34] | 3-(3,4-Difluor-benzyl)-2-oxo-1-oxa-3,8-diaza-spiro[4.5]decan-8-carbonsäure-(4-methyl-3-nitro-phenyl)-amid |
| [35] | 3-(3,4-Difluor-benzyl)-2-oxo-1-oxa-3,8-diaza-spiro[4.5]decan-8-carbonsäure-(3-methoxy-phenyl)-amid |
| [36] | 3-(4-Methyl-benzyl)-2-oxo-1-oxa-3,8-diaza-spiro[4.5]decan-8-carbonsäure-3,4-dichlor-benzylamid |
| [37] | 3-Benzyl-2-oxo-1-oxa-3,8-diaza-spiro[4.5]decan-8-carbonsäure-(4-chlor-3-trifluormethyl-phenyl)-amid |
| [38] | 2-Oxo-3-(4-trifluormethylsulfanyl-benzyl)-1-oxa-3,8-diaza-spiro[4.5]decan-8-carbonsäure-(3-cyano-phenyl)-amid |
| [39] | 3-Benzyl-2-oxo-1-oxa-3,8-diaza-spiro[4.5]decan-8-carbonsäure-(4-methoxy-phenyl)-amid |
| [40] | 2-Oxo-3-(4-trifluormethylsulfanyl-benzyl)-1-oxa-3,8-diaza-spiro[4.5]decan-8-carbonsäure-(4-methyl-3-nitro-phenyl)-amid |
| [41] | 3-(4-Iod-benzyl)-2-oxo-1-oxa-3,8-diaza-spiro[4.5]decan-8-carbonsäure-(4-butoxy-phenyl)-amid |
| [42] | 3-(3-Brom-benzyl)-2-oxo-1-oxa-3,8-diaza-spiro[4.5]decan-8-carbonsäure-3,4-dichlor-benzylamid |
| [43] | 3-Benzyl-2-oxo-1-oxa-3,8-diaza-spiro[4.5]decan-8-carbonsäure-(1-phenyl-ethyl)-amid |
| [44] | 3-Naphthalen-2-ylmethyl-2-oxo-1-oxa-3,8-diaza-spiro[4.5]decan-8-carbonsäure-3,4-dichlor-benzylamid |
| [45] | 3-(4-Methyl-benzyl)-2-oxo-1-oxa-3,8-diaza-spiro[4.5]decan-8-carbonsäure-phenethyl-amid |
| [46] | 2-Oxo-3-(2-trifluormethyl-benzyl)-1-oxa-3,8-diaza-spiro[4.5]decan-8-carbonsäure-(3-methoxy-phenyl)-amid |
| [47] | 3-(4-Cyano-benzyl)-2-oxo-1-oxa-3,8-diaza-spiro[4.5]decan-8-carbonsäure-(4-methoxy-phenyl)-amid |
| [48] | 2-Oxo-3-(4-trifluormethyl-benzyl)-1-oxa-3,8-diaza-spiro[4.5]decan-8-carbonsäure-(1-naphthalen-1-yl-ethyl)-amid |
| [49] | 2-Oxo-3-(2-trifluormethyl-benzyl)-1-oxa-3,8-diaza-spiro[4.5]decan-8-carbonsäure-phenethyl-amid |
| [50] | 3-(4-lod-benzyl)-2-oxo-1-oxa-3,8-diaza-spiro[4.5]decan-8-carbonsäure-(4-methyl-3-nitro-phenyl)-amid |
| [51] | 3-(2-Methoxy-5-nitro-benzyl)-2-oxo-1-oxa-3,8-diaza-spiro[4.5]decan-8-carbonsäure-(3-cyano-phenyl)-amid |
| [52] | 3-Naphthalen-2-ylmethyl-2-oxo-1-oxa-3,8-diaza-spiro[4.5]decan-8-c carbonsäure-(4-methoxy-phenyl)-amid |
| [53] | 3-{[3-(4-Methyl-benzyl)-2-oxo-1-oxa-3,8-diaza-spiro[4.5]decan-8-carbonyl]-amino}-benzoesäure-ethyl-ester |
| [54] | 2-Oxo-3-(2-trifluormethyl-benzyl)-1-oxa-3,8-diaza-spiro[4.5]decan-8-carbonsäure-(1-naphthalen-1-yl-ethyl)-amid |
| [55] | 3-Naphthalen-2-ylmethyl-2-oxo-1-oxa-3,8-diaza-spiro[4.5]decan-8-carbonsäure-(2,5-dimethoxy-phenyl)-amid |
| [56] | 3-(4-Cyano-benzyl)-2-oxo-1-oxa-3,8-diaza-spiro[4.5]decan-8-carbonsäure-(3-acetyl-phenyl)-amid |
| [57] | 3-Biphenyl-2-ylmethyl-2-oxo-1-oxa-3,8-diaza-spiro[4.5]decan-8-carbonsäure-phenethyl-amid |
| [58] | 3-(4-Cyano-benzyl)-2-oxo-1-oxa-3,8-diaza-spiro[4.5]decan-8-carbonsäure-(5-chlor-2-methoxy-phenyl)-amid |
| [59] | 3-{[2-Oxo-3-(2-trifluormethyl-benzyl)-1-oxa-3,8-diaza-spiro[4.5]decan-8-carbonyl]-amino}-benzoesäure-ethyl-ester |
| [60] | 3-Benzyl-2-oxo-1-oxa-3,8-diaza-spiro[4.5]decan-8-carbonsäure-(3-fluor-phenyl)-amid |
| [61] | 3-{[3-(3-Cyano-benzyl)-2-oxo-1-oxa-3,8-diaza-spiro[4.5]decan-8-carbonyl]-amino}-benzoesäure-ethyl-ester |
| [62] | 3-Biphenyl-2-ylmethyl-2-oxo-1-oxa-3,8-diaza-spiro[4.5]decan-8-carbonsäure-(3-methoxy-phenyl)-amid |
| [63] | 3-(2-Methoxy-5-nitro-benzyl)-2-oxo-1-oxa-3,8-diaza-spiro[4.5]decan-8-carbonsäure-(1-naphthalen-1-yl-ethyl)-amid |
| [64] | {2-[3-(3,4-Difluor-benzyl)-2-oxo-1-oxa-3,8-diaza-spiro[4.5]dec-8-yl]-2-oxo-ethoxy}-essigsäure |
| [65] | 4-[3-(3,4-Difluor-benzyl)-2-oxo-1-oxa-3,8-diaza-spiro[4.5]dec-8-yl]-3,3-dimethyl-4-oxo-butansäure |
| [66] | [2-[3-(3-Brom-benzyl)-2-oxo-1-oxa-3,8-diaza-spiro[4.5]dec-8-yl]-1-(1H-indol-3-ylmethyl)-2-oxo-ethyl]-carbaminsäure-tert-butyl-ester |
| [67] | 5-[3-(2-Fluor-benzyl)-2-oxo-1-oxa-3,8-diaza-spiro[4.5]dec-8-yl]-3-methyl-5-oxo-pentansäure |
| [68] | 3,3-Dimethyl-5-[3,(4-methyl-benzyl)-2-oxo-1-oxa-3,8-diaza-spira[4.5]dec-8-yl]-5-oxo-pentansäure |
| [69] | 5-[3-(3,4-Difluor-benzyl)-2-oxo-1-oxa-3,8-diaza-spiro[4.5]dec-8-yl]-3-methyl-5-oxo-pentansäure |
| [70] | {2-Oxo-2-[2-oxo-3-(4-trifluormethyl-benzyl)-1-oxa-3,8-diaza-spiro[4.5]dec-8-yl]-ethoxy}-essigsäure |
| [71] | (1-{2-[3-(2-Cyano-benzyl)-2-oxo-1-oxa-3,8-diaza-spiro[4.5]dec-8-yl]-2-oxo-ethyl}-cyclopentyl)-essigsäure |
| [72] | (1-{2-[3-(3,4-Difluor-benzyl)-2-oxo-1-oxa-3,8-diaza-spiro[4,5]dec-8-yl]-2-oxo-ethyl}-cyclopentyl)-essigsäure |
| [73] | 5-(3-Biphenyl-2-ylmethyl-2-oxo-1-oxa-3,8-diaza-spiro[4.5]dec-8-yl)-3-methyl-5-oxo-pentansäure |
| [75] | N-[4-(3,5-Dichlor-phenylsulfamoyl)-phenyl]-2-[3-(4-methyl-benzyl)-2-oxo-1-oxa-3,8-diaza-spiro[4.5]dec-8-yl]-acetamid |
| [76] | N-(2,5-Dimethyl-2H-pyrazol-3-yl)-2-[3-(2-methoxy-5-nitro-benzyl)-2-oxo-1-oxa-3,8-diaza-spiro[4.5]dec-8-yl]-acetamid |
| [80] | N-(3-Cyano-4-methyl-thiophen-2-yl)-2-[3-(3,4-difluor-benzyl)-2-oxo-1-oxa-3,8-diaza-spiro[4.5]dec-8-yl]-acetamid ylmethyl]-benzonitril |
| [85] | 2-[2-Oxo-3-(4-trifluormethylsulfanyl-benzyl)-1-oxa-3,8-diaza-spiro[4.5]dec-8-yl]-N-(4-phenyl-5-trifluormethyl-thiophen-3-yl)-acetamid |
| [86] | 2-[3-(2-Fluor-benzyl)-2-oxo-1-oxa-3,8-diaza-spiro[4.5]dec-8-yl]-N-(4-phenyl-thiazol-2-yl)-acetamid |
| [87] | 2-[2-Oxo-3-(2-trifluormethyl-benzyl)-1-oxa-3,8-diaza-spiro[4.5]dec-8-yl]-N-(4-trifluormethvxy-phenyl)-acetamid |
| [91] | N-{4-[3-(3-Cyano-benzyl)-2-oxo-1-oxa-3,8-diaza-spim[4.5]decan-8-carbonyl]-phenyl}-acetamid |
| [92] | 8-(4-Ethoxy-benzoyl)-3-(4-methyl-benzyl)-1-oxa-3,8-diaza-spiro[4.5]decan-2-on |
| [93] | 3-[3-(4-Cyano-benzyl)-2-oxo-1-oxa-3,8-diaza-spiro[4.5]dec-8-yl]-3-oxo-propionsäuremethylester |
| [94] | 2-[8-(2,4-Difluor-benzoyl)-2-oxo-1-oxa-3,8-diaza-spiro[4.5]dec-3-ylmethyl]-benzonitril |
| [95] | 8-(3-Dimethylamino-benzoyl)-3-(3,5-dimethyl-benzyl)-1-oxa-3,8-diaza-spiro[4.5]decan-2-on |
| [96] | N-{4-[3-(3,4-Difluor-benzyl)-2-oxo-1-oxa-3,8-diaza-spiro[4.5]decan-8-carbonyl]-phenyl}-acetamid |
| [97] | 8-(4-Brom-benzoyl)-3-(4-fluor-benzyl)-1-oxa-3,8-diaza-spiro[4.5]decan-2-on |
| [98] | 4-[8-(Adamantan-1-carbonyl)-2-oxo-1-oxa-3,8-diaza-spiro[4.5]dec-3-ylmethyl]-benzonitril |
| [99] | 3-(3,5-Dimethyl-benzyl)-8-(2-ethylsulfanyl-pyridin-3-carbonyl)-1-oxa-3,8-diaza-spiro[4.5]decan-2-on |
| [100] | 3-(3-Brom-benzyl)-8-(4-chlor-benzoyl)-1-oxa-3,8-diaza-spiro[4.5]decan-2-on |
| [101] | 8-[3-(2-Chlorphenyl)-5-methyl-isoxazol-4-carbonyl]-3-(3,5-dimethyl-benzyl)-1-oxa-3,8-diaza-spiro[4.5]decan-2-on |
| [102] | 3-(3-Brom-benzyl)-8-(4-ethyl-benzylsulfonyl)-1-oxa-3,8-diaza-spiro[4.5]decan-2-on |
| [103] | 8-(4-Butoxy-benzylsulfonyl)-3-(3,5-dimethyl-benzyl)-1-oxa-3,8-diaza-spiro[4.5]decan-2-on |
| [104] | 8-(Biphenyl-4-carbonyl)-3-(3-brom-benzyl)-1-oxa-3,8-diaza-spiro[4.5]decan-2-on |
| [105] | Essigsäure-2-[3-(3,5-dimethyl-benzyl)-2-oxo-1-oxa-3,8-diaza-spiro[4.5]dec-8-yl]-2-oxo-1-phenyl-ethylester |
| [106] | 4-[2-Oxo-8-(2-phenyl-cyclopropancarbonyl)-1-oxa-3,8-diaza-spiro[4.5]dec-3-ylmethyl]-benzonitril |
| [107] | 8-(2,5-Dimethoxy-benzylsulfonyl)-3-(3,5-dimethyl-benzyl)-1-oxa-3,8-diaza-spiro[4.5]decan-2-on |
| [108] | 8-(2-Chlor-benzoyl)-3-(3,5-dimethyl-benzyl)-1-oxa-3,8-diaza-spiro[4.5]decan-2-on |
| [109] | 3-(3-Brom-benzyl)-8-(4-nitro-benzoyl)-1-oxa-3,8-diaza-spiro[4.5]decan-2-on |
| [110] | N-{1-Benzyl-2-[3-(3-cyano-benzyl)-2-oxo-1-oxa-3,8-diaza-spiro[4.5]dec-8-yl]-2-oxo-ethyl}-4-methyl-benzylsulfonamid |
| [111] | 8-(2-Ethylsulfanyl-pyridin-3-carbonyl)-3-(3-methoxy-benzyl)-1-oxa-3,8-diaza-spiro[4.5]decan-2-on |
| [112] | 8-(2,4-Dimethoxy-benzoyl)-3-(3-methoxy-benzyl)-1-oxa-3,8-diaza-spiro[4.5]decan-2-on |
| [113] | 3-(3,5-Dimethyl-benzyl)-8-[2-(3-methoxy-phenyl)-acetyl]-1-oxa-3,8-diaza-spiro[4.5]decan-2-on |
| [114] | 3-(3,5-Dimethyl-benzyl)-8-(4-methoxy-2,3,6-trimethyl-benzylsulfonyl)-1-oxa-3,8-diaza-spiro[4.5]decan-2-on |
| [115] | 4-[8-(Biphenyl-4-carbonyl)-2-oxo-1-oxa-3,8-diaza-spiro[4.5]dec-3-ylmethyl]-benzonitril |
| [116] | 8-(2-Chlor-benzylsulfonyl)-3-(3,5-dimethyl-benzyl)-1-oxa-3,8-diaza-spiro[4.5]decan-2-on |
| [117] | 8-(Benzo[1,2,5]oxadiazol-5-carbonyl)-3-(3,5-dimethyl-benzyl)-1-oxa-3,8-diaza-spiro[4.5]decan-2-on |
| [118] | 8-(Biphenyl-4-carbonyl)-3-(4-iod-benzyl)-1-oxa-3,8-diaza-spiro[4.5]decan-2-on |
| [119] | 3-(3,5-Dimethyl-benzyl)-8-(pyridin-2-carbonyl)-1-oxa-3,8-diaza-spiro[4.5]decan-2-on |
| [120] | 8-(6-Chlor-pyridin-3-carbonyl)-3-naphthalen-2-ylmethyl-1-oxa-3,8-diaza-spiro[4.5]decan-2-on |
| [121] | 3-(3-Brom-benzyl)-8-(2-ethylsulfanyl-pyridin-3-carbonyl)-1-oxa-3,8-diaza-spiro[4.5]decan-2-on |
| [122] | 3-(3,5-Dimethyl-benzyl)-8-(5-methyl-isoxazol-3-carbonyl)-1-oxa-3,8-diaza-spiro[4.5]decan-2-on |
| [123] | 3-(2-Methoxy-5-nitro-benzyl)-8-pentanoyl-1-oxa-3,8-diaza-spiro[4.5]decan-2-on |
| [124] | 8-(4-Brom-benzoyl)-3-(3,5-dimethyl-benzyl)-1-oxa-3,8-diaza-spiro[4.5]decan-2-on |
| [125] | 8-(5-tert-Butyl-2-methyl-furan-3-carbonyl)-3-(2-methoxy-5-nitro-benzyl)-1-oxa-3,8-diaza-spiro[4.5]decan-2-on |
| [126] | 8-(3-Chlor-benzoyl)-3-(3,5-dimethyl-benzyl)-1-oxa-3,8-diaza-spiro[4.5]decan-2-on |
| [127] | 8-(2-Ethylsulfanyl-pyridin-3-carbonyl)-3-(2-trifluormethyl-benzyl)-1-oxa-3,8-diaza-spiro[4.5]decan-2-on |
| [128] | 3-(3,5-Dimethyl-benzyl)-8-(isoxazol-5-carbonyl)-1-oxa-3,8-diaza-spiro[4.5]decan-2-on |
| [129] | 3-(3-Brom-benzyl)-8-(pyridin-2-carbonyl)-1-oxa-3,8-diaza-spiro[4.5]decan-2-on |
| [130] | 3-(3,5-Dimethyl-benzyl)-8-(thiophen-2-carbonyl)-1-oxa-3,8-diaza-spiro[4.5]decan-2-on |
| [131] | 8-(3-Chlor-4-fluor-benzoyl)-3-(3,5-dimethyl-benzyl)-1-oxa-3,8-diaza- spiro[4.5]decan-2-on |
| [132] | 8-(2,6-Difluor-3-methyl-benzoyl)-3-(3,5-dimethyl-benzyl)-1-oxa-3,8-diaza-spiro[4.5]decan-2-on |
| [133] | 4-[3-(3-Brom-benzyl)-2-oxo-1-oxa-3,8-diaza-spiro[4.5]dec-8-yl]-4-oxo-butansäuremethylester |
| [134] | 3-[8-(2-Ethyl-butyryl)-2-oxo-1-oxa-3,8-diaza-spiro[4.5]dec-3-ylmethyl]-benzonitril |
| [135] | 3-[8-(3-Brom-benzylsulfonyl)-2-oxo-1-oxa-3,8-diaza-spiro[4.5]dec-3-ylmethyl]-benzonitril |
| [136] | 3-(3,4-Difluor-benzyl)-8-(4-methyl-benzoyl)-1-oxa-3,8-diaza-spiro[4.5]decan-2-on |
| [137] | 3-(3-Brom-benzyl)-8-(3,5-dimethoxy-benzoyl)-1-oxa-3,8-diaza-spiro[4.5]decan-2-on |
| [138] | 8-(3-Dimethylamino-benzoyl)-3-(2-methoxy-5-nitro-benzyl)-1-oxa-3,8-diaza-spiro[4.5]decan-2-on |
| [139] | 8-(2,6-Dichlor-benzoyl)-3-(2-fluor-benzyl)-1-oxa-3,8-diaza-spiro[4.5]decan-2-on |
| [140] | 3-(2-Methoxy-5-nitro-benzyl)-8-(2-phenoxy-pyridin-3-carbonyl)-1-oxa-3,8-diaza-spiro[4.5]decan-2-on |
| [141] | 8-[2-(3-Methoxy-phenyl)-acetyl]-3-naphthalen-2-ylmethyl-1-oxa-3,8-diaza-spiro[4.5]decan-2-on |
| [142] | 4-{2-Oxo-8-[2-(2,2,2-trifluoro-acetyl)-1,2,3,4-tetrahydro-isochinolin-7-sulfonyl]-1-oxa-3,8-diaza-spiro[4.5]dec-3-ylmethyl}-benzonitril |
| [143] | 8-(3,5-Bis-trifluormethyl-benzoyl)-3-(4-trifluormethyl-benzyl)-1-oxa-3,8-diaza-spiro[4.5]decan-2-on |
| [144] | 3-(3-Brom-benzyl)-8-(2-phenoxy-pyridin-3-carbonyl)-1-oxa-3,8-diaza-spiro[4.5]decan-2-on |
| [145] | 3-(2-Methoxy-5-nitro-benzyl)-8-[2-(3-methoxy-phenyl)-acetyl]-1-oxa-3,8-diaza-spiro[4.5]decan-2-on |
| [146] | 3-(3,4-Difluor-benzyl)-8-(2-phenoxy-pyridin-3-carbonyl)-1-oxa-3,8-diaza-spiro[4.5]decan-2-on |
| [147] | 3-[8-(4-Brom-3-methyl-benzoyl)-2-oxo-1-oxa-3,8-diaza-spiro[4.5]dec-3-ylmethyl]-benzonitril |
| [148] | 3-(3-Brom-benzyl)-8-(4-propyl-benzoyl)-1-oxa-3,8-diaza-spiro[4.5]decan-2-on |
| [149] | 3-Biphenyl-2-ylmethyl-8-(3-chlor-benzylsulfonyl)-1-oxa-3,8-diaza-spiro[4.5]decan-2-on |
| [150] | 8-[2-(3,4-Dimethoxy-phenyl)-acetyl]-3-(2-methoxy-5-nitro-benzyl)-1-oxa-3,8-diaza-spiro[4.5]decan-2-on |
| [151] | 2-[8-(2-Chlor-4-nitro-benzoyl)-2-oxo-1-oxa-3,8-diaza-spiro[4.5]dec-3-ylmethyl]-benzonitril |
| [152] | 3-[2-Oxo-8-(2,4,6-trimethyl-benzylsulfonyl)-1-oxa-3,8-diaza-spiro[4.5]dec-3-ylmethyl]-benzonitril |
| [153] | 3-Benzyl-8-(4-fluor-benzylsulfonyl)-1-oxa-3,8-diaza-spiro[4.5]decan-2-on |
| [154] | 3-(4-Fluor-benzyl)-8-(toluol-4-sulfonyl)-1-oxa-3,8-diaza-spiro[4.5]decan-2-on |
| [155] | 4-[2-Oxo-8-(toluol-4-sulfonyl)-1-oxa-3,8-diaza-spiro[4.5]dec-3-ylmethyl]-benzonitril |
| [156] | 3-(2-Fluor-benzyl)-8-(toluol-4-sulfonyl)-1-oxa-3,8-diaza-spiro[4.5]decan-2-on |
| [157] | 3-(3,4-Difluor-benzyl)-8-(toluol-4-sulfonyl)-1-oxa-3,8-diaza- spiro[4.5]decan-2-on |
| [158] | 3-(4-tert-Butyl-benzyl)-8-(toluol-4-sulfonyl)-1-oxa-3,8-diaza-spiro[4.5]decan-2-on |
| [159] | 8-(Toluol-4-sulfonyl)-3-(4-trifluormethylsulfanyl-benzyl)-1-oxa-3,8-diaza-spiro[4.5]decan-2-on |
| [160] | 2-[3-(4-Fluor-benzyl)-2-oxo-1-oxa-3,8-diaza-spiro[4.5]decan-8-sulfonyl]-benzoesäuremethylester |
| [161] | 2-[3-(3-Methoxy-benzyl)-2-oxo-1-oxa-3,8-diaza-spiro[4.5]decan-8-sulfonyl]-benzoesäuremethylester |
| [162] | 2-[3-(3-Brom-benzyl)-2-oxo-1-oxa-3,8-diaza-spiro[4.5]decan-8-sulfonyl]-benzoesäuremethylester |
| [163] | 3-Benzyl-8-(2-methansulfonyl-benzylsulfonyl)-1-oxa-3,8-diaza-spiro[4.5]decan-2-on |
| [164] | 8-(2-Methansulfonyl-benzylsulfonyl)-3-(2-methoxy-5-nitro-benzyl)-1-oxa-3,8-diaza-spiro[4.5]decan-2-on |
| [165] | 3-(4-Methyl-benzyl)-2-oxo-1-oxa-3,8-diaza-spiro[4.5]decan-8-sulfonsäure-dimethylamid |
| [166] | 4-[8-(7,7-Dimethyl-2-oxo-bicyclo[2.2.1]hept-1-ylmethansulfonyl)-2-oxo-1-oxa-3,8-diaza-spiro[4.5]dec-3-ylmethyl]-benzonitril |
| [167] | 3-(4-Fluor-benzyl)-8-(4-methoxy-benzylsulfonyl)-1-oxa-3,8-diaza-spiro[4.5]decan-2-on |
| [168] | 3-(4-Methyl-benzyl)-8-(4-trifluormethoxy-benzylsulfonyl)-1-oxa-3,8-diaza-spiro[4.5]decan-2-on |
| [169] | 8-(4-Trifluormethoxy-benzylsulfonyl)-3-(4-trifluormethylsulfanyl-benzyl)-1-oxa-3,8-diaza-spiro[4.5]decan-2-on |
| [170] | 8-(Propan-1-sulfonyl)-3-(4-trifluormethylsulfanyl-benzyl)-1-oxa-3,8-diaza-spiro[4.5]decan-2-on |
| [171] | 3-(2-Methoxy-5-nitro-benzyl)-8-(4-propyl-benzylsulfonyl)-1-oxa-3,8-diaza-spiro[4.5]decan-2-on |
| [172] | 3-Biphenyl-2-ylmethyl-8-(4-propyl-benzylsulfonyl)-1-oxa-3,8-diaza-spiro[4.5]decan-2-on |
| [173] | 8-(3-Brom-benzylsulfonyl)-3-(2-fluor-benzyl)-1-oxa-3,8-diaza-spiro[4.5]decan-2-on |
| [174] | 8-(3-Brom-benzylsulfonyl)-3-(3-brom-benzyl)-1-oxa-3,8-diaza-spiro[4.5]decan-2-on |
| [175] | 2-{8-[4-(1,1-Dimethyl-propyl)-benzylsulfonyl]-2-oxo-1-oxa-3,8-diaza-spiro[4.5]dec-3-ylmethyl}-benzonitril |
| [176] | 3-Benzyl-8-[4-(1,1-dimethyl-propyl)-benzylsulfonyl]-1-oxa-3,8-diaza-spiro[4.5]decan-2-on |
| [177] | 8-[4-(1,1-Dimethyl-propyl)-benzylsulfonyl]-3-(4-iod-benzyl)-1-oxa-3,8-diaza-spiro[4.5]decan-2-on |
| [178] | 8-[4-(1,1-Dimethyl-propyl)-benzylsulfonyl]-3-(2-methoxy-5-nitro-benzyl)-1-oxa-3,8-diaza-spiro[4.5]decan-2-on |
| [179] | 8-[4-(1,1-Dimethyl-propyl)-benzylsulfonyl]-3-(4-trifluormethyl-benzyl)-1-oxa-3,8-diaza-spiro[4.5]decan-2-on |
| [180] | 3-Biphenyl-2-ylmethyl-8-[4-(1,1-dimethyl-propyl)-benzylsulfonyl]-1-oxa-3,8-diaza-spiro[4.5]decan-2-on |
| [181] | 8-(4,5-Dichlor-thiophen-2-sulfonyl)-3-(3-methoxy-benzyl)-1-oxa-3,8-diaza-spiro[4.5]decan-2-on |
| [182] | 8-(4,5-Dichlor-thiophen-2-sulfonyl)-3-(4-trifluormethyl-benzyl)-1-oxa-3,8-diaza-spiro[4.5]decan-2-on |
| [183] | 3-(3-Brom-benzyl)-8-(4,5-dichlor-thiophen-2-sulfonyl)-1-oxa-3,8-diaza-spiro[4.5]decan-2-on |
| [184] | 2-[8-(4-Methoxy-2,3,6-trimethyl-benzylsulfonyl)-2-oxo-1-oxa-3,8-diaza-spiro[4.5]dec-3-ylmethyl]-benzonitril |
| [185] | 3-(2-Fluor-benzyl)-8-(4-methoxy-2,3,6-trimethyl-benzylsulfonyl)-1-oxa-3,8-diaza-spiro[4.5]decan-2-on |
| [186] | 3-(4-tert-Butyl-benzyl)-8-[2-(2,2,2-trifluor-acetyl)-1,2,3,4-tetrahydro-isochinolin-7-sulfonyl]-1-oxa-3,8-diaza-spiro[4.5]decan-2-on |
| [187] | 8-(Butan-1-sulfonyl)-3-(3-methoxy-benzyl)-1-oxa-3,8-diaza-spiro[4.5]decan-2-on |
| [188] | 3-(4-Methyl-benzyl)-8-(thiophen-2-sulfonyl)-1-oxa-3,8-diaza-spiro[4.5]decan-2-on |
| [189] | 8-(4-Chlor-2,5-dimeth-yl-benzylsulfonyl)-3-(4-methyl-benzyl)-1-oxa-3,8-diaza-spiro[4.5]decan-2-on |
| [190] | 3-Benzyl-8-(4-chlor-2,5-dimethyl-benzylsulfonyl)-1-oxa-3,8-diaza-spiro[4.5]decan-2-on |
| [191] | 8-(4-Chlor-2,5-dimethyl-benzylsulfonyl)-3-(4-trifluormethyl-benzyl)-1-oxa-3,8-diaza-spiro[4.5]decan-2-on |
| [192] | 3-Biphenyl-2-ylmethyl-8-(4-chlor-2,5-dimethyl-benzylsulfonyl)-1-oxa-3,8-diaza-spiro[4.5]decan-2-on |
| [193] | 8-(4-Chlor-2,5-dimethyl-benzylsulfonyl)-3-(4-trifluormethylsulfanyl-benzyl)-1-oxa-3,8-diaza-spiro[4.5]decan-2-on |
| [194] | 3-(4-Fluor-benzyl)-8-(2-trifluormethyl-benzylsulfonyl)-1-oxa-3,8-diaza-spiro[4.5]decan-2-on |
| [195] | 3-(3-Methoxy-benzyl)-8-(2-trifluormethyl-benzylsulfonyl)-1-oxa-3,8-diaza-spiro[4.5]decan-2-on |
| [196] | 3-Benzyl-8-(2-methyl-5-nitro-benzylsulfonyl)-1-oxa-3,8-diaza-spiro[4.5]decan-2-on |
| [197] | 3-(4-tert-Butyl-benzyl)-8-(2-methyl-5-nitro-benzylsulfonyl)-1-oxa-3,8-diaza-spiro[4.5]decan-2-on |
| [198] | 3-(4-tert-Butyl-benzyl)-8-(toluol-3-sulfonyl)-1-oxa-3,8-diaza-spiro[4.5]decan-2-on |
| [199] | 8-(Furan-2-carbonyl)-3-(4-trifluormethyl-benzyl)-1-oxa-3,8-diaza-spiro[4.5]decan-2-on |
| [200] | 3-(3-Brom-benzyl)-8-(furan-2-carbonyl)-1-oxa-3,8-diaza-spiro[4.5]decan-2-on |
| [201] | 4-[8-(Naphthalen-1-carbonyl)-2-oxo-1-oxa-3,8-diaza-spiro[4.5]dec-3-ylmethyl]-benzonitril |
| [202] | 3-(3,4-Difluor-benzyl)-8-(naphthalen-1-carbonyl)-1-oxa-3,8-diaza-spiro[4.5]decan-2-on |
| [203] | 3-(3-Brom-benzyl)-8-(naphthalen-1-carbonyl)-1-oxa-3,8-diaza-spiro[4.5]decan-2-on |
| [204] | 2-[8-(3-Nitro-benzoyl)-2-oxo-1-oxa-3,8-diaza-spiro[4.5]dec-3-ylmethyl]-benzonitril |
| [205] | 4-[8-(3-Nitro-benzoyl)-2-oxo-1-oxa-3,8-diaza-spiro[4.5]dec-3-ylmethyl]-benzonitril |
| [206] | 3-(3-Brom-benzyl)-8-(3,5-difluor-benzoyl)-1-oxa-3,8-diaza-spiro[4.5]decan-2-on |
| [207] | 8-(2-Benzyloxy-acetyl)-3-(2-fluor-benzyl)-1-oxa-3,8-diaza-spiro[4.5]decan-2-on |
| [208] | 8-(2-Chlor-benzoyl)-3-(2-trifluormethyl-benzyl)-1-oxa-3,8-diaza-spiro[4.5]decan-2-on |
| [209] | 8-(2-Chlor-benzoyl)-3-(4-iod-benzyl)-1-oxa-3,8-diaza-spiro[4.5]decan-2-on |
| [210] | 8-(2-Chlor-benzoyl)-3-(2-methoxy-5-nitro-benzyl)-1-oxa-3,8-diaza-spiro[4.5]decan-2-on |
| [211] | 3-Biphenyl-2-ylmethyl-8-(2-chlor-benzoyl)-1-oxa-3,8-diaza-spiro[4.5]decan-2-on |
| [212] | 8-(2,6-Dichlor-benzoyl)-3-naphthälen-2-ylmethyl-1-oxa-3,8-diaza-spiro[4.5]decan-2-on |
| [213] | 3-(4-tert-Butyl-benzyl)-8-(2,6-dichlor-benzoyl)-1-oxa-3,8-diaza-spiro[4.5]decan-2-on |
| [214] | 3-(3-Brom-benzyl)-8-(2,6-dichlor-benzoyl)-1-oxa-3,8-diaza-spiro[4.5]decan-2-on |
| [215] | 8-(2,6-Dichlor-benzoyl)-3-(4-trifluormethylsulfanyl-benzyl)-1-oxa-3,8-diaza-spiro[4.5]decan-2-on |
| [216] | 2-[8-(2-Methyl-benzoyl)-2-oxo-1-oxa-3,8-diaza-spiro[4.5]dec-3-ylmethyl]-benzonitril |
| [217] | 8-(2-Methyl-benzoyl)-3-(4-methyl-benzyl)-1-oxa-3,8-diaza-spiro[4.5]decan-2-on |
| [218] | 8-(2-Methyl-benzoyl)-3-(2-trifluormethyl-benzyl)-1-oxa-3,8-diaza-spiro[4.5]decan-2-on |
| [219] | 3-(2-Methoxy-5-nitro-benzyl)-8-(2-methyl-benzoyl)-1-oxa-3,8-diaza-spiro[4.5]decan-2-on |
| [220] | 3-(3,4-Difluor-benzyl)-8-(2-methyl-benzoyl)-1-oxa-3,8-diaza-spiro[4.5]decan-2-on |
| [221] | 8-(2-Methyl-benzoyl)-3-(4-trifluormethyl-benzyl)-1-oxa-3,8-diaza-spiro[4.5]decan-2-on |
| [222] | 8-(2-Methyl-benzoyl)-3-(4-trifluormethylsulfanyl-benzyl)-1-oxa-3,8-diaza-spiro[4.5]decan-2-on |
| [223] | 8-(3-Brom-benzoyl)-3-(4-fluor-benzyl)-1-oxa-3,8-diaza-spiro[4.5]decan-2-on |
| [224] | 8-(3-Brom-benzoyl)-3-(2-trifluormethyl-benzyl)-1-oxa-3,8-diaza-spiro[4.5]decan-2-on |
| [225] | 8-(3-Brom-benzoyl)-3-(2-fluor-benzyl)-1-oxa-3,8-diaza-spiro[4.5]decan-2-on |
| [226] | 8-(3-Brom-benzoyl)-3-(3,4-difluor-benzyl)-1-oxa-3,8-diaza-spiro[4.5]decan-2-on |
| [227] | 8-(3-Brom-benzoyl)-3-naphthalen-2-ylmethyl-1-oxa-3,8-diaza-spiro[4.5]decan-2-on |
| [228] | 8-(3-Brom-benzoyl)-3-(4-tert-butyl-benzyl)-1-oxa-3,8-diaza-spiro[4.5]decan-2-on |
| [229] | 3-Benzyl-8-(3-fluor-benzoyl)-1-oxa-3,8-diaza-spiro[4.5]decan-2-on |
| [230] | 8-(3-Fluor-benzoyl)-3-(2-fluor-benzyl)-1-oxa-3,8-diaza-spiro[4.5]decan-2-on |
| [231] | 3-(3,4-Difluor-benzyl)-8-(3-fluor-benzoyl)-1-oxa-3,8-diaza-spiro[4.5]decan-2-on |
| [232] | 8-(3-Fluor-benzoyl)-3-(4-trifluormethyl-benzyl)-1-oxa-3,8-diaza-spiro[4.5]decan-2-on |
| [233] | 3-(3-Brom-benzyl)-8-(3-fluor-benzoyl)-1-oxa-3,8-diaza-spiro[4.5]decan-2-on |
| [234] | 8-(3-Chlor-benzoyl)-3-(3,5-dimethyl-benzyl)-1-oxa-3,8-diaza-spiro[4.5]decan-2-on |
| [235] | 3-Benzyl-8-(3-chlor-benzoyl)-1-oxa-3,8-diaza-spiro[4.5]decan-2-on |
| [236] | 4-[8-(3,4-Dichlor-benzoyl)-2-oxo-1-oxa-3,8-diaza-spiro[4.5]dec-3-ylmethyl]-benzonitril |
| [237] | 3-(4-Iod-benzyl)-8-(3-methoxy-benzoyl)-1-oxa-3,8-diaza-spiro[4.5]decan-2-on |
| [238] | 3-(2-Fluor-benzyl)-8-(3-methoxy-benzoyl)-1-oxa-3,8-diaza-spiro[4.5]decan-2-on |
| [239] | 3-(4-tert-Butyl-benzyl)-8-(3-methoxy-benzoyl)-1-oxa-3,8-diaza-spiro[4.5]decan-2-on |
| [240] | 3-Benzyl-8-(3-methyl-benzoyl)-1-oxa-3,8-diaza-spiro[4.5]decan-2-on |
| [241] | 3-(2-Methoxy-5-nitro-benzyl)-8-(3-methyl-benzoyl)-1-oxa-3,8-diaza-spiro[4.5]decan-2-on |
| [242] | 3-(4-tert-Butyl-benzyl)-8-(3-methyl-benzoyl)-1-oxa-3,8-diaza-spiro[4.5]decan-2-on |
| [243] | 8-(3-Methyl-benzoyl)-3-(4-trifluormethyl-benzyl)-1-oxa-3,8-diaza-spiro[4.5]decan-2-on |
| [244] | 3-(3,4-Difluor-benzyl)-8-(2-phenyl-butyryl)-1-oxa-3,8-diaza-spiro[4.5]decan-2-on |
| [245] | 3-Benzyl-8-[3-(2-chlor-phenyl)-5-methyl-isoxazol-4-carbonyl]-1-oxa-3,8-diaza-spiro[4.5]decan-2-on |
| [246] | 4-[8-(2,3-Dichlor-benzoyl)-2-oxo-1-oxa-3,8-diaza-spiro[4.5]dec-3-ylmethyl]-benzonitril |
| [247] | 8-[2-(4-Chlor-phenyl)-acetyl]-3-(3,4-difluor-benzyl)-1-oxa-3,8-diaza-spiro[4.5]decan-2-on |
| [248] | 3-Biphenyl-2-ylmethyl-8-[2-(4-chlor-phenyl)-acetyl]-1-oxa-3,8-diaza-spiro[4.5]decan-2-on |
| [249] | 8-[2-(4-Chlor-phenyl)-acetyl]-3-(4-trifluormethylsulfanyl-benzyl)-1-oxa-3,8-diaza-spiro[4.5]decan-2-on |
| [250] | 3-(4-tert-Butyl-benzyl)-8-[3-(2-chlor-phenyl)-acryloyl]-1-oxa-3,8-diaza-spiro[4.5]decan-2-on |
| [251] | 3-Biphenyl-2-ylmethyl-8-[3-(2-chlor-phenyl)-acryloyl]-1-oxa-3,8-diaza-spiro[4.5]decan- 2-on |
| [252] | 3-(3-Brom-benzyl)-8-[3-(2-chlor-phenyl)-acryloyl]-1-oxa-3,8-diaza-spiro[4.5]decan-2-on |
| [253] | 4-[8-(2,3-Difluor-benzoyl)-2-oxo-1-oxa-3,8-diaza-spiro[4.5]dec-3-ylmethyl]-benzonitril |
| [254] | 8-(2,3-Difluor-benzoyl)-3-(2-trifluormethyl-benzyl)-1-oxa-3,8-diaza-spira[4.5]decan-2-on |
| [255] | 8-(2,3-Difluor-benzoyl)-3-(4-iod-benzyl)-1-oxa-3,8-diaza-spiro[4.5]decan-2-on |
| [256] | 2-[2-Oxo-8-(2-propyl-pentanoyl)-1-oxa-3,8-diaza-spiro[4.5]dec-3-ylmethyl]-benzonitril |
| [257] | 3-(3,4-Difluor-benzyl)-8-(2-propyl-pentanoyl)-1-oxa-3,8-diaza-spiro[4.5]decan-2-on |
| [258] | 8-(2-Chlor-4-nitro-benzoyl)-3I-(4-methyl-benzyl)-1-oxa-3,8-diaza-spiro[4.5]decan-2-on |
| [259] | 8-(2-Chlor-4-nitro-benzoyl)-3I-(2-trifluormethyl-benzyl)-1-oxa-3,8-diaza-spiro[4.5]decan-2-on |
| [260] | 3-(4-tert-Butyl-benzyl)-8-(2-chlor-4-nitro-benzoyl)-1-oxa-3,8-diaza-spiro[4.5]decan-2-on |
| [261] | 8-(2-Chlor-4-nitro-benzoyl)-3I-(4-trifluormethyl-benzyl)-1-oxa-3,8-diaza-spiro[4.5]decan-2-on |
| [262] | 3-Biphenyl-2-ylmethyl-8-(2-chlor-4-nitro-benzoyl)-1-oxa-3,8-diaza-spiro[4.5]decan-2-on |
| [263] | 3-(3-Brom-benzyl)-8-(2-chlor-4-nitro-benzoyl)-1-oxa-3,8-diaza-spiro[4.5]decan-2-on |
| [264] | 8-(2-Chlor-4-nitro-benzoyl)-3I-(4-trifluormethylsulfanyl-benzyl)-1-oxa-3,8- diaza-spiro[4.5]decan-2-on |
| [265] | 8-(2-Chlor-pyridin-3-carbonyl)-3-(4-fluor-benzyl)-1-oxa-3,8-diaza-spiro[4.5]decan-2-on |
| [266] | 2-[8-(2-Chlor-pyridin-3-carbonyl)-2-oxo-1-oxa-3,8-diaza-spiro[4.5]dec-3-ylmethyl]-benzonitril |
| [267] | 8-(2-Chlor-pyridin-3-carbonyl)-3-(4-methyl-benzyl)-1-oxa-3,8-diaza-spiro[4.5]decan-2-on |
| [268] | 8-(2-Chlor-pyridin-3-carbonyl)-3-(4-trifluormethyl-benzyl)-1-oxa-3,8-diaza-spiro[4.5]decan-2-on |
| [269] | 3-(3-Methoxy-benzyl)-8-(2-methylsulfanyl-pyridin-3-carbonyl)-1-oxa-3,8-diaza-spiro[4.5]decan-2-on |
| [270] | 3-(4-Methyl-benzyl)-8-(2-methylsulfanyl-pyridin-3-carbonyl)-1-oxa-3,8-diaza-spiro[4.5]decan-2-on |
| [271] | 3-(4-lod-benzyl)-8-(2-methylsulfanyl-pyridin-3-carbonyl)-1-oxa-3,8-diaza-spiro[4.5]decan-2-on |
| [272] | 3-(3,4-Difluor-benzyl)-8-(2-methylsulfanyl-pyridin-3-carbonyl)-1-oxa-3,8-diaza-spiro[4.5]decan-2-on |
| [273] | 3-(4-tert-Butyl-benzyl)-8-(2-methylsulfanyl-pyridin-3-carbonyl)-1-oxa-3,8-diaza- spiro[4.5]decan-2-on |
| [274] | 8-(2-Methylsulfanyl-pyridin-3-carbonyl)-3-(4-trifluormethyl-benzyl)-1-oxa-3,8-diaza-spiro[4.5]decan-2-on |
| [275] | 8-(2-Ethylsulfanyl-pyridin-3-carbonyl)-3-(4-fluor-benzyl)-1-oxa-3,8-diaza-spiro[4.5]decan-2-on |
| [276] | 3-[8-(2-Ethylsulfanyl-pyridin-3-carbonyl)-2-oxo-1-oxa-3,8-diaza-spiro[4.5]dec-3-ylmethyl]-benzonitril |
| [277] | 2-[8-(2-Ethylsulfanyl-pyridin-3-carbonyl)-2-oxo-1-oxa-3,8-diaza-spiro[4.5]dec-3-ylmethyl]-benzonitril |
| [278] | 8-(2-Ethylsulfanyl-pyridin-3-carbonyl)-3-(4-methyl-benzyl)-1-oxa-3,8-diaza-spiro[4.5]decan-2-on |
| [279] | 3-Benzyl-8-(2-ethylsulfanyl-pyridin-3-carbonyl)-1-oxa-3,8-diaza-spiro[4.5]decan-2-on |
| [280] | 8-(2-Ethylsulfanyl-pyridin-3-carbonyl)-3-(2-fluor-benzyl)-1-oxa-3,8-diaza-spiro[4.5]decan-2-on |
| [281] | 4-[8-(6-Chlor-pyridin-3-carbonyl)-2-oxo-1-oxa-3,8-diaza-spiro[4.5]dec-3-ylmethyl]-benzonitril |
| [282] | 8-(6-Chlor-pyridin-3-carbonyl)-3-(2-trifluormethyl-benzyl)-1-oxa-3,8-diaza-spiro[4.5]decan-2-on |
| [283] | 8-(6-Chlor-pyridin-3-carbonyl)-3-(4-iod-benzyl)-1-oxa-3,8-diaza-spiro[4.5]decan-2-on |
| [284] | 3-(4-tert-Butyl-benzyl)-8-(6-chlor-pyridin-3-carbonyl)-1-oxa-3,8-diaza-spiro[4.5]decan-2-on |
| [285] | 3-Biphenyl-2-ylmethyl-8-(6-chlor-pyridin-3-carbonyl)-1-oxa-3,8-diaza-spiro[4.5]decan-2-on |
| [286] | 3-(3-Methoxy-benzyl)-8-(4-trifluormethoxy-benzoyl)-1-oxa-3,8-diaza-spiro[4.5]decan-2-on |
| [287] | 3-(3,4-Difluor-benzyl)-8-(4-trifluormethoxy-benzoyl)-1-oxa-3,8-diaza-spiro[4.5]decan-2-on |
| [288] | 8-[3-(2-Chlor-6-fluor-phenyl)-5-methyl-isoxazol-4-carbonyl]-3-(4-fluor-benzyl)-1-oxa-3,8-diaza-spiro[4.5]decan-2-on |
| [289] | 8-[3-(2-Chlor-6-fluor-phenyl)-5-methyl-isoxazol-4-carbonyl]-3-(3-methoxy-benzyl)-1-oxa-3,8-diaza-spiro[4.5]decan-2-on |
| [290] | 8-[3-(2-Chlor-6-fluor-phenyl)-5-methyl-isoxazol-4-carbonyl]-3-(4-trifluormethyl-benzyl)-1-oxa-3,8-diaza-spiro[4.5]decan-2-on |
| [291] | 8-[3-(2-Chlor-6-fluor-phenyl)-5-methyl-isoxazol-4-carbonyl]-3-(4-trifluormethylsulfanyl-benzyl)-1-oxa-3,8-diaza-spiro[4.5]decan-2-on |
| [292] | 3-(4-Methyl-benzyl)-8-(3-trifluormethoxy-benzoyl)-1-oxa-3,8-diaza-spiro[4.5]decan-2-on |
| [293] | 3-(4-lod-benzyl)-8-(3-trifluormethoxy-benzoyl)-1-oxa-3,8-diaza-spiro[4.5]decan-2-on |
| [294] | 3-(3,4-Difluor-benzyl)-8-(3-trifluormethoxy-benzoyl)-1-oxa-3,8-diaza-spiro[4.5]decan-2-on |
| [295] | 3-Naphthalen-2-ylmethyl-8-(3-trifluormethoxy-benzoyl)-1-oxa-3,8-diaza-spiro[4.5]decan-2-on |
| [296] | 4-[8-(Isoxazol-5-carbonyl)-2-oxo-1-oxa-3,8-diaza-spiro[4.5]dec-3-ylmethyl]-benzonitril |
| [297] | 8-(Isoxazol-5-carbonyl)-3-(2-trifluormethyl-benzyl)-1-oxa-3,8-diaza-spiro[4.5]decan-2-on |
| [298] | 8-(Isoxazol-5-carbonyl)-3-(2-methoxy-5-nitro-benzyl)-1-oxa-3,8-diaza-spiro[4.5]decan-2-on |
| [299] | 3-(3-Brom-benzyl)-8-(isoxazol-5-carbonyl)-1-oxa-3,8-diaza-spiro[4.5]decan-2-on |
| [300] | 2-[8-(2-Chlor-6-fluor-benzoyl)-2-oxo-1-oxa-3,8-diaza-spiro[4.5]dec-3-ylmethyl]-benzonitril |
| [301] | 4-[8-(2-Chlor-6-fluor-benzoyl)-2-oxo-1-oxa-3,8-diaza-spiro[4.5]dec-3-ylmethyl]-benzonitril |
| [302] | 8-(2-Chlor-6-fluor-benzoyl)-3-(4-methyl-benzyl)-1-oxa-3,8-diaza-spiro[4.5]decan-2-on |
| [303] | 3-Biphenyl-2-ylmethyl-8-(2-chlor-6-fluor-benzoyl)-1-oxa-3,8-diaza-spiro[4.5]decan-2-on |
| [304] | 8-(2,5-Dimethyl-furan-3-carbonyl)-3-(3-methoxy-benzyl)-1-oxa-3,8-diaza-spiro[4.5]decan-2-on |
| [305] | 4-[8-(2,5-Dimethyl-furan-3-carbonyl)-2-oxo-1-oxa-3,8-diaza-spiro[4.5]dec-3-ylmethyl]-benzonitril |
| [306] | 4-[8-(4-Brom-3-methyl-benzoyl)-2-oxo-1-oxa-3,8-diaza-spiro[4.5]dec-3-ylmethyl]-benzonitril |
| [307] | 8-(4-Brom-3-methyl-benzoyl)-3-(4-methyl-benzyl)-1-oxa-3,8-diaza-spiro[4.5]decan-2-on |
| [308] | 3-Benzyl-8-(4-brom-3-methyl-benzoyl)-1-oxa-3,8-diaza-spiro[4.5]decan-2-on |
| [309] | 8-(4-Brom-3-methyl-benzoyl)-3-(2-trifluormethyl-benzyl)-1-oxa-3,8-diaza-spiro[4.5]decan-2-on |
| [310] | 8-(4-Brom-3-methyl-benzoyl)-3-(3,4-difluor-benzyl)-1-oxa-3,8-diaza-spiro[4.5]decan-2-on |
| [311] | 8-(4-Brom-3-methyl-benzoyl)-3-(4-tert-butyl-benzyl)-1-oxa-3,8-diaza-spiro[4.5]decan-2-on |
| [312] | 3-Biphenyl-2-ylmethyl-8-(4-brom-3-methyl-benzoyl)-1-oxa-3,8-diaza-spiro[4.5]decan-2-on |
| [313] | 8-(4-Brom-3-methyl-benzoyl)-3-(4-trifluormethylsulfanyl-benzyl)-1-oxa-3,8-diaza-spiro[4.5]decan-2-on |
| [314] | 8-(2,6-Difluor-3-methyl-benzoyl)-3-(3-methoxy-benzyl)-1-oxa-3,8-diaza-spiro[4.5]decan-2-on |
| [315] | 2-[8-(2,6-Difluor-3-methyl-benzoyl)-2-oxo-1-oxa-3,8-diaza-spiro[4.5]dec-3-ylmethyl]-benzonitril |
| [316] | 4-[8-(2,6-Difluor-3-methyl-benzoyl)-2-oxo-1-oxa-3,8-diaza-spiro[4.5]dec-3-ylmethyl]-benzonitril |
| [317] | 8-(2,6-Difluor-3-methyl-benzoyl)-3-(2-trifluormethyl-benzyl)-1-oxa-3,8-diaza-spiro[4.5]decan-2-on |
| [318] | 8-(2,6-Difluor-3-methyl-benzoyl)-3-(2-fluor-benzyl)-1-oxa-3,8-diaza-spiro[4.5]decan-2-on |
| [319] | 8-(2-Chlor-6-fluor-3-methyl-benzoyl)-3-(4-fluor-benzyl)-1-oxa-3,8-diaza-spiro[4.5]decan-2-on |
| [320] | 8-(2-Chlor-6-fluor-3-methyl-benzoyl)-3-(3-methoxy-benzyl)-1-oxa-3,8-diaza-spiro[4.5]decan-2-on |
| [321] | 8-(2-Chlor-6-fluor-3-methyl-benzoyl)-3-(4-trifluormethylsulfanyl-benzyl)-1-oxa-3,8-diaza-spiro[4.5]decan-2-on |
| [322] | 3-Biphenyl-2-ylmethyl-8-(6-chlor-2-fluor-3-methyl-benzoyl)-1-oxa-3,8-diaza-spiro[4.5]decan-2-on |
| [323] | 3-Benzyl-8-(3-difluormethylsulfanyl-benzoyl)-1-oxa-3,8-diaza-spiro[4.5]decan-2-on |
| [324] | 8-(3-Difluormethylsulfanyl-benzoyl)-3-naphthalen-2-ylmethyl-1-oxa-3,8-diaza-spiro[4.5]decan-2-on |
| [325] | 8-(3-Difluormethylsulfanyl-benzoyl)-3-(4-trifluormethyl-benzyl)-1-oxa-3,8-diaza-spiro[4.5]decan-2-on |
| [326] | 3-(3-Brom-benzyl)-8-(3-difluormethylsulfanyl-benzoyl)-1-oxa-3,8-diaza-spiro[4.5]decan-2-on |
| [327] | 3-[8-(3-Chlor-2-fluor-benzoyl)-2-oxo-1-oxa-3,8-diaza-spiro[4.5]dec-3-ylmethyl]-benzonitril |
| [328] | 8-(3-Chlor-2-fluor-benzoyl)-3-(4-iod-benzyl)-1-oxa-3,8-diaza-spiro[4.5]decan-2-on |
| [329] | 8-(3-Chlor-2-fluor-benzoyl)-3-naphthalen-2-ylmethyl-1-oxa-3,8-diaza-spiro[4.5]decan-2-on |
| [330] | 3-(3-Methoxy-benzyl)-8-(4-trifluormethylsulfanyl-benzoyl)-1-oxa-3,8-diaza-spiro[4.5]decan-2-on |
| [331] | 2-[2-Oxo-8-(4-trifluormethylsulfanyl-benzoyl)-1-oxa-3,8-diaza-spiro[4.5]dec-3-ylmethyl]-benzonitril |
| [332] | 4-[2-Oxo-8-(4-trifluormethylsulfanyl-benzoyl)-1-oxa-3,8-diaza-spiro[4.5]dec-3-ylmethyl]-benzonitril |
| [333] | 3-(4-lod-benzyl)-8-(4-trifluormethylsulfanyl-benzoyl)-1-oxa-3,8-diaza-spiro[4.5]decan-2-on |
| [334] | 3-Naphthalen-2-ylmethyl-8-(4-trifluormethylsulfanyl-benzoyl)-1-oxa-3,8-diaza-spiro[4.5]decan-2-on |
| [335] | 3-(4-Trifluormethyl-benzyl)-8-(4-trifluormethylsulfanyl-benzoyl)-1-oxa-3,8-diaza-spiro[4.5]decan-2-on |
| [336] | 8-(2-Chlor-5-trifluormethyl-benzoyl)-3-(2-trifluormethyl-benzyl)-1-oxa-3,8-diaza-spiro[4.5]decan-2-on |
| [337] | 8-(2-Chlor-5-trifluormethyl-benzoyl)-3-(2-fluor-benzyl)-1-oxa-3,8-diaza-spiro[4.5]decan-2-on |
| [338] | 8-(2-Chlor-5-trifluormethyl-benzoyl)-3-(3,4-difluor-benzyl)-1-oxa-3,8-diaza-spiro[4.5]decan-2-on |
| [339] | 8-(2,3-Difluor-4-methyl-benzoyl)-3-(3-methoxy-benzyl)-1-oxa-3,8-diaza-spiro[4.5]decan-2-on |
| [340] | 4-[8-(2,3-Difluor-4-methyl-benzoyl)-2-oxo-1-oxa-3,8-diaza-spiro[4.5]dec-3-ylmethyl]-benzonitril |
| [341] | 3-Benzyl-8-(2,3-difluor-4-methyl-benzoyl)-1-oxa-3,8-diaza-spiro[4.5]decan-2-on |
| [342] | 8-(2,3-Difluor-4-methyl-benzoyl)-3-(4-iod-benzyl)-1-oxa-3,8-diaza-spiro[4.5]decan-2-on |
| [343] | 3-(3,4-Difluor-benzyl)-8-(2,3-difluor-4-methyl-benzoyl)-1-oxa-3,8-diaza-spiro[4.5]decan-2-on |
| [344] | 8-(2,3-Difluor-4-methyl-benzoyl)-3-naphthalen-2-ylmethyl-1-oxa-3,8-diaza-spiro[4.5]decan-2-on |
| [345] | 3-Biphenyl-2-ylmethyl-8-(2,3-difluor-4-methyl-benzoyl)-1 -oxa-3,8-diaza-spiro[4.5]decan-2-on |
| [346] | 3-(3-Brom-benzyl)-8-(2,3-difluor-4-methyl-benzoyl)-1-oxa-3,8-diaza-spiro[4.5]decan-2-on |
| [347] | 3-Benzyl-8-[2-(2-methoxy-ethoxy)-acetyl]-1-oxa-3,8-diaza-spiro[4.5]decan-2-on |
| [348] | 3-(2-Fluor-benzyl)-8-[2-(2-methoxy-ethoxy)-acetyl]-1-oxa-3,8-diaza-spiro[4.5]decan-2-on |
| [349] | 3-(3,4-Difluor-benzyl)-8-[2-(2-methoxy-ethoxyfacetyl]-1-oxa-3,8-diaza-spiro[4.5]decan-2-on |
| [350] | 3-(4-tert-Butyl-benzyl)-8-[2-(2-methoxy-ethoxy)-acetyl]-1-oxa-3,8-diaza-spiro[4.5]decan-2-on |
| [351] | 8-[2-(2-Methoxy-ethoxy)-acetyl]-3-(4-trifluormethylsulfanyl-benzyl)-1-oxa-3,8-diaza-spiro[4.5]decan-2-on |
| [352] | 8-(1-Acetyl-piperidin-4-carbonyl)-3-(3,4-difluor-benzyl)-1-oxa-3,8-diaza-spiro[4.5]decan-2-on |
| [353] | 4-{8-[2-(3-Chlor-phenoxy)-acetyl]-2-oxo-1-oxa-3,8-diaza-spiro[4.5]dec-3-ylmethyl}-benzonitril |
| [354] | 8-[2-(3-Chlor-phenoxy)-acetyl]-3-(3,4-difluor-benzyl)-1-oxa-3,8-diaza-spiro[4.5]decan-2-on |
| [355] | 3-(3-Brom-benzyl)-8-[2-(3-chlor-phenoxy)-acetyl]-1-oxa-3,8-diaza-spiro[4.5]decan-2-on |
| [356] | 8-[2-(3-Chlor-phenoxy)-acetyl]-3-(4-trifluormethylsulfanyl-benzyl)-1-oxa-3,8-diaza-spiro[4.5]decan-2-on |
| [357] | 4-[3-(3-Methoxy-benzyl)-2-oxo-1-oxa-3,8-diaza-spiro[4.5]decan-8-carbonyl]-benzylsulfonamid |
| [358] | N-{4-[3-(3,4-Difluor-benzyl)-2-oxo-1-oxa-3,8-diaza-spiro[4.5]decan-8-carbonyl]-phenyl}-acetamid |
| [359] | 8-(3-Dimethylamino-benzoyl)-3-(3,5-dimethyl-benzyl)-1-oxa-3,8-diaza-spiro[4.5]decan-2-on |
| [360] | 3-(4-tert-Butyl-benzyl)-8-(3-dimethylamino-benzoyl)-1-oxa-3,8-diaza-spiro[4.5]decan-2-on |
| [361] | 3-(3-Brom-benzyl)-8-(3-dimethylamino-benzoyl)-1-oxa-3,8-diaza-spiro[4.5]decan-2-on |
| [362] | 3-(4-Methyl-benzyl)-8-(4-phenoxy-butyryl)-1-oxa-3,8-diaza-spiro[4.5]decan-2-on |
| [363] | 8-(4-Phenoxy-butyryl)-3-(2-trifluormethyl-benzyl)-1-oxa-3,8-diaza-spiro[4.5]decan-2-on |
| [364] | 3-(2-Fluor-benzyl)-8-(4-phenoxy-butyryl)-1-oxa-3,8-diaza-spiro[4.5]decan-2-on |
| [365] | 8-(2,3-Dimethyl-benzoyl)-3-(4-fluor-benzyl)-1-oxa-3,8-diaza-spiro[4.5]decan-2-on |
| [366] | 3-[8-(2,3-Dimethyl-benzoyl)-2-oxo-1-oxa-3,8-diaza-spiro[4.5]dec-3-ylmethyl]-benzonitril |
| [367] | 8-(2,3-Dimethyl-benzoyl)-3-(3,5-dimethyl-benzyl)-1-oxa-3,8-diaza-spiro[4.5]decan-2-on |
| [368] | 8-(2,3-Dimethyl-benzoyl)-3-(4-methyl-benzyl)-1-oxa-3,8-diaza-spiro[4.5]decan-2-on |
| [369] | 8-(2,3-Dimethyl-benzoyl)-3-(2-trifluormethyl-benzyl)-1-oxa-3,8-diaza-spiro[4.5]decan-2-on |
| [370] | 8-(2,3-Dimethyl-benzoyl)-3-(2-methoxy-5-nitro-benzyl)-1-oxa-3,8-diaza-spiro[4.5]decan-2-on |
| [371] | 8-(2,3-Dimethyl-benzoyl)-3-(4-trifluormethyl-benzyl)-1-oxa-3,8-diaza-spiro[4.5]decan-2-on |
| [372] | 3-Biphenyl-2-ylmethyl-8-(2,3-dimethyl-benzoyl)-1-oxa-3,8-diaza-spiro[4.5]decan-2-on |
| [373] | 4-[8-(5-Methyl-2-phenyl-2H-[1,2,3]triazol-4-carbonyl)-2-oxo-1-oxa-3,8-diaza-spiro[4.5]dec-3-ylmethyl]-benzonitril |
| [374] | 3-Benzyl-8-(5-methyl-2-phenyl-2H-[1,2,3]triazol-4-carbonyl)-1-oxa-3,8- diaza-spiro[4.5]decan-2-on |
| [375] | 3-(3,4-Difluor-benzyl)-8-(5-methyl-2-phenyl-2H-[1,2,3]triazol-4-carbonyl)-1-oxa-3,8-diaza-spiro[4.5]decan-2-on |
| [376] | 8-(5-Methyl-2-phenyl-2H-[1,2,3]triazol-4-carbonyl)-3-naphthalen-2-ylmethyl-1-oxa-3,8-diaza-spiro[4.5]decan-2-on |
| [377] | 3-Biphenyl-2-ylmethyl-8-(5-methyl-2-phenyl-2H-[1,2,3]triazol-4-carbonyl)-1-oxa-3,8-diaza-spiro[4.5]decan-2-on |
| [378] | 3-(4-tert-Butyl-benzyl)-8-[3-(2,6-dichlor-phenyl)-5-methyl-isoxazol-4-carbonyl]-1-oxa-3,8-diaza-spiro[4.5]decan-2-on |
| [379] | 3-[2-Oxo-8-(2-phenoxy-pyridin-3-carbonyl)-1-oxa-3,8-diaza-spiro[4.5]dec-3-ylmethyl]-benzonitril |
| [380] | 2-[2-Oxo-8-(2-phenoxy-pyridin-3-carbonyl)-1-oxa-3,8-diaza-spiro[4.5]dec-3-ylmethyl]-benzonitril |
| [381] | 3-(4-Fluor-benzyl)-8-(pyridin-2-carbonyl)-1-oxa-3,8-diaza-spiro[4.5]decan-2-on |
| [382] | 3-(3-Methoxy-benzyl)-8-(pyridin-2-carbonyl)-1-oxa-3,8-diaza-spiro[4.5]decan-2-on |
| [383] | 3-(3,5-Dimethyl-benzyl)-8-(pyridin-2-carbonyl)-1-oxa-3,8-diaza-spiro[4.5]decan-2-on |
| [384] | 2-[2-Oxo-8-(pyridin-2-carbonyl)-1-oxa-3,8-diaza-spiro[4.5]dec-3-ylmethyl]-benzonitril |
| [385] | 3-(2-Fluor-benzyl)-8-(pyridin-2-carbonyl)-1-oxa-3,8-diaza-spiro[4.5]decan-2-on |
| [386] | 3-(3,4-Difluor-benzyl)-8-(pyridin-2-carbonyl)-1-oxa-3,8-diaza-spiro[4.5]decan-2-on |
| [387] | 3-Biphenyl-2-ylmethyl-8-(pyridin-2-carbonyl)-oxa-3,8-diaza-spira[4.5]decan-2-on |
| [388] | 4-[8-(3-Chlor-thiophen-2-carbonyl)-2-oxo-1-oxa-3,8-diaza-spiro[4.5]dec-3-ylmethyl]-benzonitril |
| [389] | 8-(3-Chlor-thiophen-2-carbonyl)-3-(3,4-difluor-benzyl)-1-oxa-3,8-diaza-spiro[4.5]decan-2-on |
| [390] | 3-Biphenyl-2-ylmethyl-8-(3-chlor-thiophen-2-carbonyl)-1-oxa-3,8-diaza-spiro[4.5]decan-2-on |
| [391] | 8-[1-(4-Chlor-phenyl)-5-trifluormethyl-1H-pyrazol-4-carbonyl]-3-(2-fluor-benzyl)-1-oxa-3,8-diaza-spiro[4.5]decan-2-on |
| [392] | 8-[1-(4-Chlor-phenyl)-5-trifluormethyl-1H-pyrazol-4-carbonyl]-3-(3,4-difluor-benzyl)-1-oxa-3,8-diaza-spiro[4.5]decan-2-on |
| [393] | 8-[1-(4-Chlor-phenyl)-5-trifluormethyl-1H-pyrazol-4-carbonyl]-3-(4-trifluormethylsulfanyl-benzyl)-1-oxa-3,8-diaza-spiro[4.5]decan-2-on |
| [394] | 3-(4-tert-Butyl-benzyl)-8-(5-tert-butyl-2-methyl-2H-pyrazol-3-carbonyl)-1-oxa-3,8-diaza-spiro[4.5]decan-2-on |
| [395] | 3-[8-(5-Methyl-isoxazol-3-carbonyl)-2-oxo-1-oxa-3,8-diaza-spiro[4.5]dec-3-ylmethyl]-benzonitril |
| [396] | 3-(3,5-Dimethyl-benzyl)-8-(5-methyl-isoxazol-3-carbonyl)-1-oxa-3,8-diaza-spiro[4.5]decan-2-on |
| [397] | 2-[8-(5-Methyl-isoxazol-3-carbonyl)-2-oxo-1-oxa-3,8-diaza-spiro[4.5]dec-3-ylmethyl]-benzonitril |
| [398] | 4-[8-(5-Methyl-isoxazol-3-carbonyl)-2-oxo-1-oxa-3,8-diaza-spiro[4.5]dec-3-ylmethyl]-benzonitril |
| [399] | 3-Benzyl-8-(5-methyl-isoxazol-3-carbonyl)-1-oxa-3,8-diaza-spiro[4.5]decan-2-on |
| [400] | 3-(3,4-Difluor-benzyl)-8-(5-methyl-isoxazol-3-carbonyl)-1-oxa-3,8-diaza-spiro[4.5]decan-2-on |
| [401] | 3-Biphenyl-2-ylmethyl-8-(5-methyl-isoxazol-3-carbonyl)-1-oxa-3,8-diaza-spiro[4.5]decan-2-on |
| [402] | 8-(4-Methyl-[1,2,3]thiadiazol-5-carbonyl)-3-(2-trifluormethyl-benzyl)-1-oxa-3,8-diaza-spiro[4.5]decan-2-on |
| [403] | 3-(4-tert-Butyl-benzyl)-8-(4-methyl-[1,2,3]thiadiazol-5-carbonyl)-1-oxa-3,8-diaza-spiro[4.5]decan-2-on |
| [404] | 8-(4-Methyl-[1,2,3]thiadiazol-5-carbonyl)-3-(4-trifluormethylsulfanyl-benzyl)-1-oxa-3,8-diaza-spiro[4.5]decan-2-on |
| [405] | 4-[2-Oxo-8-(1-phenyl-5-propyl-1H-pyrazol-4-carbonyl)-1-oxa-3,8-diaza-spiro[4.5]dec-3-ylmethyl]-benzonitril |
| [406] | 3-(3,4-Difluor-benzyl)-8-(1-phenyl-5-propyl-1H-pyrazol-4-carbonyl)-1-oxa-3,8-diaza-spiro[4.5]decan-2-on |
| [407] | 3-Naphthalen-2-ylmethyl-8-(1-phenyl-5-propyl-1H-pyrazol-4-carbonyl)-1-oxa-3,8-diaza-spiro[4.5]decan-2-on |
| [408] | 8-(1-Phenyl-5-propyl-1H-pyrazol-4-carbonyl)-3-(4-trifluormethylsulfanyl-benzyl)-1-oxa-3,8-diaza-spiro[4.5]decan-2-on |
| [409] | 3-[8-(2-Chlor-pyridin-4-carbonyl)-2-oxo-1-oxa-3,8-diaza-spiro[4.5]dec-3-ylmethyl]-benzonitril |
| [410] | 8-(2-Chlor-pyridin-4-carbonyl)-3-(3-methoxy-benzyl)-1-oxa-3,8-diaza-spiro[4.5]decan-2-on |
| [411] | 8-(5-tert-Butyl-2-methyl-furan-3-carbonyl)-3-(4-fluor-benzyl)-1-oxa-3,8-diaza-spiro[4.5]decan-2-on |
| [412] | 3-[8-(5-tert-Butyl-2-methyl-furan-3-carbonyl)-2-oxo-1-oxa-3,8-diaza-spiro[4.5]dec-3-ylmethyl]-benzonitril |
| [413] | 2-[8-(5-tert-Butyl-2-methyl-furan-3-carbonyl)-2-oxo-1-oxa-3,8-diaza-spiro[4.5]dec-3-ylmethyl]-benzonitril |
| [414] | 4-[8-(5-tert-Butyl-2-methyl-furan-3-carbonyl)-2-oxo-1-oxa-3,8-diaza-spiro[4.5]dec-3-ylmethyl]-benzonitril |
| [415] | 8-(5-tert-Butyl-2-methyl-furan-3-carbonyl)-3-(2-trifluormethyl-benzyl)-1-oxa-3,8-diaza-spiro[4.5]decan-2-on |
| [416] | 8-(5-tert-Butyl-2-methyl-furan-3-carbonyl)-3-naphthalen-2-ylmethyl-1-oxa-3,8-diaza-spiro[4.5]decan-2-on |
| [417] | 3-Biphenyl-2-ylmethyl-8-(5-tert-butyl-2-methyl-furan-3-carbonyl)-1-oxa-3,8-diaza-spiro[4.5]decan-2-on |
| [418] | 3-(3-Brom-benzyl)-8-(5-tert-butyl-2-methyl-furan-3-carbonyl)-1-oxa-3,8-diaza-spiro[4.5]decan-2-on |
| [419] | 8-(Benzo[1,2,5]oxadiazol-5-carbonyl)-3-(3,4-difluor-benzyl)-1-oxa-3,8-diaza-spiro[4.5]decan-2-on |
| [420] | 8-(Benzo[1,2,5]oxadiazol-5-carbonyl)-3-(4-trifluormethyl-benzyl)-1-oxa-3,8-diaza-spiro[4.5]decan-2-on |
| [421] | 3-[8-(2-Methyl-6-trifluormethyl-pyridin-3-carbonyl)-2-oxo-1-oxa-3,8-diaza-spiro[4.5]dec-3-ylmethyl]-benzonitril |
| [422] | 2-[8-(2-Methyl-6-trifluormethyl-pyridin-3-carbonyl)-2-oxo-1-oxa-3,8-diaza-spiro[4.5]dec-3-ylmethyl]-benzonitril |
| [423] | 4-[8-(2-Methyl-6-trifluormethyl-pyridin-3-carbonyl)-2-oxo-1-oxa-3,8-diaza-spiro[4.5]dec-3-ylmethyl]-benzonitril |
| [424] | 8-(2-Methyl-6-trifluormethyl-pyridin-3-carbonyl)-3-(2-trifluormethyl-benzyl)-1-oxa-3,8-diaza-spiro[4.5]decan-2-on |
| [425] | 3-(3,4-Difluor-benzyl)-8-(2-methyl-6-trifluormethyl-pyridin-3-carbonyl)-1-oxa-3,8-diaza-spiro[4.5]decan-2-on |
| [426] | 3-(3-Brom-benzyl)-8-(2-methyl-6-trifluormethyl-pyridin-3-carbonyl)-1-oxa-3,8-diaza-spiro[4.5]decan-2-on |
| [427] | 8-(6-Chlor-2H-chromen-3-carbonyl)-3-(3,4-difluor-benzyl)-1-oxa-3,8-diaza-spiro[4.5]decan-2-on |
| [428] | 8-(2-Chlor-pyridin-4-carbonyl)-3-(2-fluor-benzyl)-1-oxa-3,8-diaza-spiro[4.5]decan-2-on |
| [429] | 8-(2-Chlor-pyridin-4-carbonyl)-3-naphthalen-2-ylmethyl-1-oxa-3,8-diaza-spiro[4.5]decan-2-on |
| [430] | 8-Acetyl-3-(2-fluor-benzyl)-1-oxa-3,8-diaza-spiro[4.5]decan-2-on |
| [431] | 8-Acetyl-3-(3-brom-benzyl)-1-oxa-3,8-diaza-spiro[4.5]decan-2-on |
| [432] | 3-(2-Methoxy-5-nitro-benzyl)-8-(3-phenoxy-propyl)-1-oxa-3,8-diaza-spiro[4.5]decan-2-on; |

### Pharmakologische Daten:

Die 5-HT-Uptake-Inhibierung und Noradrenalin-Uptake-Inhibierung der erfindungsgemäßen substituierten 1-Oxa-3,8-diazaspiro[4.5]-decan-2-on-Verbindungen der allgemeinen Formel I wurde wie vorstehend beschrieben bestimmt.

Die untersuchten 1-Oxa-3,8-diazaspiro[4.5]-decan-2-on-Verbindungen der allgemeinen Formel I zeigen eine ausgezeichnete Hemmung der 5-HT- und Noradrenalin-Wiederaufnahme.

| **Verbindung gemäß Beispiel** | **5-HT-Uptake, % Hemmung, 10 µM** | **NA-Uptake, Hemmung, 10 µM** |
|---|---|---|
| 1 | 66 | |
| 2 | 60 | 64 |
| 3 | 69 | |
| 4 | 76 | |
| 5 | | 70 |
| 6 | 63 | 56 |
| 7 | 55 | 50 |
| 8 | 62 | 50 |
| 9 | | 81 |
| 10 | 50 | 78 |
| 11 | 58 | |
| 12 | 60 | |
| 13 | 44 | 53 |
| 14 | 57 | 49 |
| 15 | 57 | 55 |
| 16 | 54 | |
| 17 | 50 | 45 |
| 18 | | 55 |
| 19 | 69 | 48 |
| 20 | | 48 |
| 21 | 55 | |
| 22 | 48 | |
| 23 | 60 | |
| 24 | | 58 |
| 25 | 53 | |
| 26 | 70 | |
| 27 | 62 | 68 |
| 28 | 45 | 53 |
| 29 | 84 | 82 |
| 30 | 65 | |
| 32 | 56 | |
| 33 | 59 | 93 |
| 34 | | 41 |
| 35 | 56 | 63 |
| 36 | 56 | 60 |
| 37 | | 47 |
| 38 | 52 | |
| 39 | 42 | 76 |
| 40 | 54 | |
| 41 | 49 | |
| 42 | 60 | 83 |
| 43 | 49 | |
| 44 | 57 | 61 |
| 45 | 51 | |
| 46 | 43 | |
| 47 | 49 | |
| 48 | 43 | |
| 49 | | 48 |
| 50 | 64 | |
| 51 | 89 | |
| 52 | 94 | 96 |
| 53 | 68 | 81 |
| 54 | 75 | |
| 55 | 61 | |
| 56 | 60 | |
| 57 | 50 | |
| 58 | 49 | |
| 59 | 47 | |
| 60 | | 58 |
| 61 | 69 | 77 |
| 62 | 57 | 67 |
| 63 | 84 | 41 |
| 65 | 55 | |
| 66 | 53 | |
| 71 | 62 | |
| 72 | 45 | |
| 73 | 57 | |
| 75 | | 67 |
| 76 | 51 | |
| 80 | 47 | 45 |
| 85 | 90 | |
| 86 | 56 | 79 |
| 87 | 72 | 59 |
| 91 | 68 | |
| 92 | 67 | 73 |
| 99 | 73 | |
| 101 | 84 | |
| 102 | 67 | |
| 103 | 83 | |
| 104 | 89 | 73 |
| 105 | 74 | |
| 106 | 63 | |
| 107 | 76 | |
| 108 | 77 | |
| 109 | 81 | 78 |
| 110 | 64 | |
| 111 | 71 | |
| 112 | 66 | |
| 113 | 68 | |
| 114 | 73 | |
| 115 | 84 | |
| 116 | 76 | |
| 117 | 73 | |
| 118 | 66 | |
| 119 | 67 | |
| 120 | 66 | |
| 121 | 77 | |
| 122 | 68 | |
| 123 | 71 | |
| 124 | 72 | |
| 125 | 63 | |
| 126 | 64 | |
| 127 | 63 | |
| 128 | 62 | |
| 129 | 77 | |
| 130 | 70 | |
| 131 | 63 | |
| 132 | 65 | |
| 133 | 64 | |
| 134 | 64 | |
| 135 | 69 | |
| 136 | 67 | |
| 137 | 74 | 84 |
| 138 | 65 | |
| 139 | 70 | |
| 140 | 64 | |
| 141 | 78 | |
| 142 | 67 | |
| 143 | 66 | |
| 144 | 81 | |
| 145 | 77 | |
| 146 | 63 | |
| 147 | 73 | |
| 148 | 73 | 75 |
| 150 | 84 | |
| 151 | 71 | |

Ebenfalls wurde die Affinität der erfindungsgemäßen substituierten 1-Oxa-3,8-diazaspiro[4.5]-decan-2-on-Verbindungen für den humanen µ-Opioid-Rezeptor wie vorstehend beschrieben bestimmt.

Die erfindungsgemäßen Verbindungen zeigen auch eine ausgezeichnete Affinität für den humanen µ-Opioid-Rezeptor.

| **Verbindung gemäß Beispiel** | **µ-Opioid Rezeptor, % Hemmung (1 µM)** |
|---|---|
| 64 | 48 |
| 65 | 56 |
| 67 | 41 |
| 68 | 46 |
| 69 | 50 |
| 70 | 42 |
| 92 | 70 |
| 93 | 65 |
| 94 | 62 |
| 95 | 75 |
| 96 | 78 |
| 97 | 67 |
| 98 | 75 |
| 100 | 64 |
| 149 | 61 |

## Patentansprüche

1. Substituierte 1-Oxa-3,8-diazaspiro[4.5]-decan-2-on-Verbindungen der allgemeinen Formel I, 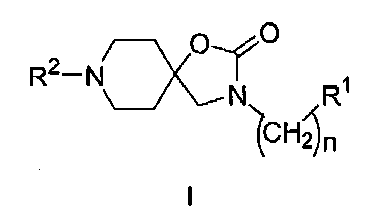 worin
n gleich 1, 2, 3, 4 oder 5 ist;
R¹ für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R² für -C(=S)-NH-R³;
für -C(=O)-NH-R⁴;
für -S(=O)₂-R⁵;
für -(CH₂)-C(=O)-NH-R⁶;
für -(CH₂)-Dₐₐ-(CH₂)_{bb}-E_{cc}-(CH₂)_{dd}-R⁷ mit aa = 0 oder 1; bb = 0,1 oder 2; cc = 0 oder 1 und dd = 0 oder 1 steht; worin D und E unabhängig voneinander für O, S, NH, N(CH₃), N(C₂H₅) oder N[CH(CH₃)₂] stehen und wobei die Summe aus aa und cc ungleich 0 ist;
für -C(=O)-R⁸
oder für -S(=O)₂-NR⁹R¹⁰ steht;
R³ für-(CHR¹¹)-(CH₂)_{w}C(=O)-O-R¹² mit w = 0 oder 1 steht;
für-(CHR¹³)(CH₂)ₐ-K_{b}-(CH₂)_{c}-L_{d}-R¹⁴ mit a = 0.1 oder 2; b = 0 oder 1; c = 0, 1 oder 2 und d = 0 oder 1 steht; worin K und L unabhängig voneinander für O, S, NH, N(CH₃), N(C₂H₅) oder N[CH(CH₃)₂] stehen;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R⁴ für -(CHR¹⁵)-(CH₂)ₑ-Mᵣ-(CH₂)_{g}-Pₕ-R¹⁶ mit e = 0, 1 oder 2; f = 0 oder 1; g = 0, 1 oder 2 und h = 0 oder 1 steht; worin M und P unabhängig voneinanderfür O, S, NH, N(CH₃), N(C₂H₅) oder N[CH(CH₃)₂] stehen;
für einen linearen oder verzweigten, gesättigten oder ungesättigten, ggf. substituierten C₁₋₁₀ aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R⁵ für -(CHR¹⁷)-(CH₂)ₖ-Qₗ-(CH₂)ₘ-Tₒ-R¹⁸ mit k = 0, 1 oder 2; I = 0 oder 1; m = 0, 1 oder 2 und o = 0 oder 1 steht; worin Q und T unabhängig voneinander für O, S, NH, N(CH₃), N(C₂H₅) oder N[CH(CH₃)₂] stehen;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R⁶ für einen linearen oder verzweigten, gesättigten oder ungesättigten, ggf. substituierten C₁₋₁₀ aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R⁷ für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃. -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃. -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅,-S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(-O)-O-C(CH₃)₃, -NH-CH₃, -NH-C₂H₅, -NH-CH(CH₃)₂, -NH-C(CH₃)₃, -N(CH₃)₂, -N(C₂H₅)₂,-N(CH₃)(C₂H₅), -C(=O)-H, -C(=O)-CH₃, -C(=O)-C₂H₅, -C(=O)-C(CH₃)₃,-C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S-=O)₂-NH₂,-S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
R⁸ für-(CHR¹⁹)-VP-(CH₂)_{q}-(CH₂)ᵣWₛ-R²⁰ mit p = 0 oder 1; q = 0, 1 oder 2; r = 0, 1 oder 2 und s = 0 oder 1 steht; worin V und W unabhängig voneinander jeweils für O, S, NH, N(CH₃), N(C₂H₅) oder N[CH(CH₃)₂] stehen;
für -(CH=CH)-R²¹ steht;
für -(CR²²R²³)-Yₜ-(CR²⁴R²⁵)ᵤ-(CH₂)ᵥ-C(=O)-OR²⁶ mit t = 0 oder 1, u = 0 oder 1 und v = 0 oder 1 steht, worin Y für O, S, NH, N(CH₃), N(C₂H₅) oder N[CH(CH₃)₂] steht;
für -(CHR²⁷)-Q-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen linearen oder verzweigten, gesättigten oder ungesättigten, ggf. substituierten C₁₋₁₀aliphatischen Rest steht;
für einen ggf. substituierten cycloaliphatischen Rest steht, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest steht, wobei der Aryl-Rest mit einem gesättigten oder ungesättigten, ggf. substituierten mono- oder bizyklischen Ringsystem kondensiert sein kann;
R⁹ und R¹⁰, unabhängig voneinander, jeweils
für einen linearen oder verzweigten, gesättigten oder ungesättigten, ggf. substituierten C₁₋₁₀ aliphatischen Rest stehen;
R¹¹, R¹³, R¹⁵, R¹⁷, R¹⁹, R²², R²³, R²⁴, R²⁵ und R²⁶, unabhängig voneinander, jeweils
für einen Wasserstoff-Rest stehen
oder für einen linearen oder verzweigten, gesättigten oder ungesättigten, ggf. substituierten C₁₋₁₀ aliphatischen Rest stehen;
R¹², R²⁸ und R³², unabhängig voneinander, jeweils
für einen linearen oder verzweigten, gesättigten oder ungesättigten, ggf. substituierten C₁₋₁₀ aliphatischen Rest stehen;
R¹⁴, R¹⁶, R¹⁸ und R²⁰, unabhängig voneinander, jeweils
für einen linearen oder verzweigten, gesättigten oder ungesättigten, ggf. substituierten C₁₋₁₀ aliphatischen Rest stehen;
für einen ggf. substituierten cycloaliphatischen Rest stehen, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest stehen, wobei der Aryl-Rest mit einem gesättigten oder ungesättigten, ggf. substituierten mono- oder bizyklischen Ringsystem kondensiert sein kann;
und
R²¹, R²⁷, R²⁹, R³⁰ und R³¹, unabhängig voneinander, jeweils
für einen ggf. substituierten cycloaliphatischen Rest stehen, der mit 1 oder 2 linearen oder verzweigten, ggf. substituierten C₁₋₅-Alkylen-Gruppen überbrückt sein kann;
oder für einen ggf. substituierten Aryl-Rest oder für einen ggf. substituierten Heteroaryl-Rest stehen, wobei der Aryl-Rest jeweils mit einem gesättigten oder ungesättigten, ggf. substituierten mono- oder bizyklischen Ringsystem kondensiert sein kann;
wobei
die vorstehend genannten C₁₋₁₀ aliphatischen Reste jeweils ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein können;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, 1, -CN, -CF₃, -SF₅, -OH, -O-C₁₋₅-Alkyl, -NH₂, -NO₂, -O-CF₃, -S-CF₃, -S-CF₂H, -S-CFH₂, -SH, -S-C₁₋₅-Alkyl,-C₁₋₅-Alkyl, -C(=O)-OH, -(CH₂)-C(=O)-OH; -C(=O)-O-C₁₋₅-Alkyl, -(CH₂)-C(=O)-O-C₁₋₅-Alkyl, -O-C(=O)-C₁₋₅-Alkyl, -NH-C₁₋₅-Alkyl, -N(C₁₋₅-Alkyl)₂, -NH-Phenyl,-NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H. -C(=O)-C₁₋₅-Alkyl, -C(=O)-C₁₋₅-Perfluoralkyl, -C(=O)-NH₂,-C(=O)-NH-C₁₋₅-Alkyl, C(=O)-N-(C₁₋₅-Alkyl)_{2,} -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, -S(=O)₂-NH₂, -S(=O)₂-NH-Phenyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl substituiert sein können, wobei jeweils der zyklische Teil der Reste -S(=O)₂-NH-Phenyl, -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, - S(=O)₂-Phenyt, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN,-NO₂, -C₁₋₅-Alkyl, -O-C₁₋₅-Alkyl, -O-CF₃, -S-CF₃, Phenyl und -O-Benzyl substituiert sein kann,
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein können,
die Ringe der vorstehend genannten mono- oder polyzyklischen Ringsysteme jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -S-CF₂H, -S-CFH₂, -SH, -S-C₁₋₅-Alkyl, -C₁₋₅-Alkyl, -C(=O)-OH, -(CH₂)-C(=O)-OH; -C(=O)-O-C₁₋₅-Alkyl, -(CH₂)-C(=O)-O-C₁₋₅-Alkyl, -O-C(=O)-C₁₋₅-Alkyl, -NH-C₁₋₅-Alkyl,-N(C₁₋₅-Alkyl)₂, -NH-Phenyl, -NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H, -C(=OFC₁₋₅-Alkyl, -C(=O)-C₁₋₅-Perfluoralkyl, -C(=O)-NH₂, -C(=O)-NH-C₁₋₅-Alkyl, C(=O)-N-(C₁₋₅-Alkyl)₂,-S(=O)₂-C₁₋₅-Alkyl, -S(=O)₂-Phenyl, -NH-S(=O)₂-C₁₋₅-Alkyl, -S(=O)₂-NH-C₁₋₅-Alkyl, -S(=O)₂-NH₂, -S(=O)₂-NH-Phenyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl substituiert sein können, wobei jeweils der zyklische Teil der Reste -S(=O)₂-NH-Phenyl, -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, -S(=O)₂-Phenyl, -O-Phenyl), -O-Benzyl, Phenyl,-(CH₂)-BenZQ[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO_{2,} -O-CF₃, -S-CF₃, -S-CF₂H, -S-CFH₂, SH, -S-C₁₋₅-Alkyl, -C₁₋₁₀-Alkyl, -C(=O)-OH, - (CH₂)-C(=O)-OH, -C(=O)-O-C₁₋₅-Alkyl, -(CH₂)-C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-C₁₋₅-Pertluoralkyl, -C(=O)-NH₂, -C(=O)-NH-C₁₋₅-Alkyl, -C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, -S(=O)₂-NH₂, -S(=O)₂-NH-Phenyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -S(=O)₂-NH-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, -C₁₋₅-Alkyl, -O-C₁₋₅-Alkyl, -O-CF₃, -S-CF₃, Phenyl und -O-Benzyl substituiert sein kann,
die vorstehend genannten cycloaliphatischen Reste ausgewählt sind aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl,
die vorstehend genannten Heteroaryl-Reste ausgewählt sind aus der Gruppe bestehend aus 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazalyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, Oxazolyl, Isoxazolyl, Pyridazinyl, Pyrazinyl, Pyrimidinyl, Indazolyl, Chinazolinyl, Chinolinyl und Isochinolinyl, die vorstehend genannten Aryl-Reste ausgewählt sind aus der Gruppe bestehend aus Phenyl und Naphthyl, und
die Aryl-Reste, die mit einem mono-bzw. polyzklischen Ringsystem kondensiert sind, ausgewählt sind aus der Gruppe bestehend aus [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl und [3,4]-Dihydro-2H-1,4-benzoxazinyl,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
R¹ für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, [3,4]-Dihydro-2H-1,4-benzoxazinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH,-O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-G-C(CH₃)₃, -NH-C(=O)-CH₃, -NH-C(=O)-C₂H₅, -NH-C(=O)-C(CH₃)₃, -C(=O)-H. -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃. -C(=O)-NH-C₂H₅, -C(=O)-NH-C(CH₃)₃, -C(=O)-N(CH₃)₂, -C(=O)-N(C₂H₅)₂, -S(=O)₂-CH₃, - S(=O)₂-C₂H₅, -NH-S(=O)₂-CH₃, -S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, - O-C(CH₃)₃, -O-CH₂-CH₂-CH₂-CH₃, -O-CF₃, -S-CF₃, Phenyl und -O-Benzyl substituiert sein kann.

3. Verbindungen gemäß Anspruch 1 oder 2, **dadurch gekennzeichnet, dass**
R² für -C(=S)-NH-R³; für -C(=O)-NH-R⁴; für -S(=O)₂-R⁵; für -(CH₂)-C(=O)-NH-R⁶; für -(CH₂)-O-R⁷, für -(CH₂)-S-R⁷, für -(CH₂)NH-R⁷, für -(CH₂)-N(CH₃)-R⁷, für -(CH₂)-(CH₂)-O-R⁷, für -(CH₂)-(CH₂)-S-R⁷, für -(CH₂)-NH-R⁷, für -(CH₂)-N(CH₃)-R⁷, für -(CH₂)-(CH₂)-(CH₂)-O-R⁷, für -(CH₂)-(CH₂)-(CH₂)-S-R⁷, für - (CH₂)-(CH₂)-(CH₂)-NH-R⁷, für -(CH₂)-(CH₂)-(CH₂)-N(CH₃)-R⁷, für -(CH₂)-O-(CH₂)-R⁷, für -(CH₂)-S-(CH₂)-R⁷; für -(CH₂)-NH-(CH₂)-R⁷; für -C(=O)-R⁸ oder für -S(=O)₂-NR⁹R¹⁰ steht.

4. Verbindungen gemäß einem oder mehreren der Ansprüche 1 bis 3, **dadurch gekennzeichnet, dass**
R³ für -(CHR¹¹)-C(=O)-O-R¹² oder -(CHR¹¹)-(CH₂)-C(=O)-O-R¹² steht;
für -CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-S-R¹⁴, -(CHR¹³)-NH-R¹⁴, -(CHR¹³)N(CH₃)-R¹⁴, -(CHR¹³)-(CH₂)-O-R¹⁴, -(CHR¹³)-(CH₂)-S-R¹⁴, -(CHR¹³)-NH-R¹⁴, -(CHR¹³)-N(CH₃)-R¹⁴, - (CHR¹³)-(CH₂)-(CH₂)-O-R¹⁴, -(CHR¹³)-(CH₂)-(CH₂)-S-R¹⁴, -(CHR¹³)-(CH₂)-(CH₂)-NH-R¹⁴, -(CHR¹³)-(CH₂)-(CH₂)-N(CH₃)-R¹⁴, -(CHR¹³)-O-(CH₂)-R¹⁴, - (CHR¹³)-S-(CH₂)-R¹⁴ oder-(CHR¹³)-NH-(CH²)-R¹⁴ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, 1, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-GF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂M₅, -C(=O)-C(CH₃)₃, -C(=O)-CF₃,-C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₆)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃,-C(=O)-H, -C(=O)-CH₃, -C(=O)-C₂H₅, -c(=O)-C(CH₃)₃, -C(=O)-CF₃, -C(=O)-C₂F₅, -C(=O)-OH, -(CH₂)-C(=O)-OH, -C(=O)-O-CH₃, -C(=O)-O-CH₂-CH₃,-C(=O)-O-CH(CH₃)₂, -C(=O)-O-C(CH₃)₃, -NH-CH₃, -NH-C₂H₅, -NH-CH(CH₃)₂,-NH-C(CH₃)₃, -N(CH₃)₂, -N(C₂H₅)₂, -N(CH₃)(C₂H₅), -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃,-SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann.

5. Verbindungen gemäß einem oder mehreren der Ansprüche 1 bis 4, **dadurch gekennzeichnet, dass**
R⁴ für -(CHR¹⁵)-R¹⁶, -(CHR¹⁵)-(CH₂)-R¹⁶, -(CHR¹⁵)-(CH₂)-(CH₂)-R¹⁶, -(CHR¹⁵)-O-R¹⁶, -(CHR¹⁵)-S-R¹⁶, -(CHR¹⁵)-NH-R¹⁶, -(CHR¹⁵)-N(CH₃)-R¹⁶, -(CHR¹⁵)-(CH₂)O-R¹⁶, -(CHR¹⁵)-(CH₂)-S-R¹⁶, -(CHR¹⁵)-NH-R¹⁶, -(CHR¹⁵)-N(CH₃)-R¹⁶,-(CHR¹⁵)-(CH₂)-(CH₂)-O-R¹⁶, -(CHR¹⁵)-(CH₂)-(CH₂)-S-R¹⁶, -(CHR¹⁵)-(CH₂)-(CH₂)-NH-R¹⁶, -(CHR¹⁵)-(CH₂)-(CH₂)-N(CH₃)-R¹⁶, -(CHR¹⁵)-a-(CH₂)-R¹⁶,-(CHR¹⁵)-S-(CH₂)-R¹⁶ oder-(CHR¹⁵)-NH-(CH₂)-R¹⁶ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, 1, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)C(CH₃)₃, -C(=O)-CF₃, - C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃,-C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=OFNH-C(CH₃)₃, -C(=O)-N(CH₃)₂, -C(=O)-N(C₂H₅)_{2,} -S(=O)₂-CH₃, -S(=O)₂-C₂H₅, -NH-S(=O)₂-CH₃, -S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃,-SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann.

6. Verbindungen gemäß einem oder mehreren der Ansprüche 1 bis 5, **dadurch gekennzeichnet, dass**
R⁵ für -(CHR¹⁷)-R¹⁸, -(CHR¹⁷)-(CH₂)-R¹⁸, -(CHR¹⁷)-(CH₂)-(CH₂)-R¹⁸, -(CHR¹⁷)-O-R¹⁸, -(CHR¹⁷)-S-R¹⁸, -(CHR¹⁷)-NH-R¹⁸, -(CHR¹⁷)-N(CH₃)-R¹⁸, -(CHR¹⁷)-(CH₂)-O-R¹⁸, -(CHR¹⁷)-(CH₂)-S-R¹⁸, -(CHR¹⁷)-NH-R¹⁸, -(CHR¹⁷)-N(CH₃)-R¹⁸, - (CHR¹⁷)-(CH₂)-(CH₂)-O-R¹⁸, -(CHR¹⁷)-(CH₂)-(CH₂)-S-R¹⁸, -(CHR¹⁷)-(CH₂)-(CH₂)-NH-R¹⁸, -(CHR¹⁷)-(CH₂)-(CH₂)-N(CH₃)-R¹⁸, -(CHR¹⁷)-O-(CH₂)-R¹⁸, - (CHR¹⁷)-S-(CH₂)-R¹⁸ oder-(CHR¹⁷)-NH-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃,-C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅ -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Ghromenyl, Thiophenyl, Furanyl, Pyrrolyl, Pyrazolyl, Pyrazinyl, Pyranyl, Triazolyl, Pyridinyl, Imidazotyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN,-CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H,-C(=O)-CH₃, -C(=O)-C₂H₅, -C(-O)-C(CH₃)₃, -C(-O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann.

7. Verbindungen gemäß einem oder mehreren der Ansprüche 1 bis 6, **dadurch gekennzeichnet, dass**
R⁶ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, n-Hexy, n-Heptyl, -C(=O)-CH₃, -C(=O)-C₂H₅, -C(=O)-C(CH₃)₃, -C(=O)-CF₃,-C(=O)-C₂F₅. -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ und -S(=O)₂-NH₂ substituiert sein kann;
oder für einen Rest ausgewählt aus der Gruppe bestehend aus Phenyl, Naphthyl, (1,3)-Bvenzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN,-CF₃, -SF₅, -OH, -O-CH₃, -O-C₂H₅, -O-CH(CH₃)₂, -O-CH₂-CH₂-CH₃, -O-G(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂Hs, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H,-C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H_{5,} -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyt, n-Pentyl, n-Hexyl, n-Heptyl, -O-CH₃, -O-C₂H₅, -O-CH(CH₃)₂, -O-CH₂-CH₂-CH₃,-O-C(CH₃)₃, -O-CH₂-CH₂-CH₂-CH₃, -O-CF₃, -S-CF₃, Phenyl und -O-Benzyl substituiert sein kann.

8. Verbindungen gemäß einem oder mehreren der Ansprüche 1 bis 7, **dadurch gekennzeichnet, dass**
R⁸ für-(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-S-R²⁰, -(CHR¹⁹)-NH-R²⁰, -(CHR¹⁹)-N(CH₃)-R²⁰, -(CHR¹⁹)-(CH₂)-O-R²⁰, -(CHR¹⁹)-(CH₂)-S-R²⁰, -(CHR¹⁹)-NH-R²⁰, -(CHR¹⁹)-N(CH₃)-R²⁰, - (CHR¹⁹)-(CH₂)-(CH₂)-O-R²⁰, -(CHR¹⁹)-(CH₂)-(CH₂)-S-R²⁰, -(CHR¹⁹)-(CH₂)-(CH₂)-NH-R²⁰, -(CHR¹⁹)-(CH₂)-(CH₂)-N(CH₃)-R²⁰, -(CHR¹⁹)-O-(CH₂)-R²⁰, - (CHR¹⁹)-S-(CH₂)-R²⁰, -(CHR¹⁹)-NH-(CH₂)-R²⁰, -(CHR¹⁹)-O-(CH₂)-(CH₂)-O-R²⁰, -(CHR¹⁹)-O-(CH₂)-(CH₂)-S-R²⁰ oder -(CHR¹⁹)-S-(CH₂)-(CH₂)-S-R²⁰ steht;
für -(CH=CH)-R²¹ steht;
für -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶, -(CR²²R²³)-O-(CR²⁴R²⁵)-C(=C)-C-R²⁶, - (CR²²R²³)-S-(CR²⁴R²⁵)-C(=O)-O-R²⁶ oder -(CR²²R²³)-NH-(CR²⁴R²⁵)-C(=O)-O-R²⁶ steht;
für -(CHR²⁷)-O-C(=O)-R²⁸ steht;
für-CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(GH₂)-(CH₂)-(C(CH₃)₃), n-Hexyl, 2-Hexyl, 3-Hexyl, n-Heptyl, 2-Heptyl, 3-Heptyl, 4-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht, wobei der Rest ggf. mit 1, 2, 3, 4 oder 5 Substituenten unabhängig voneinander ausgewählt aus der Gruppe bestehend aus F, Cl, Br, I, -CN,-NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl steht, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃, Phenyl und -S(=O)₂-NH₂ substituiert sein kann; wobei der Phenyl-Rest jeweils mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃ und -O-CH₂-CH₂-CH₂-CH₃ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂,-N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃,-S(=O)₂-C₂H₅, -NH-S(=O)₂-CH₃, -S(=O)₂-NH-CH₃, -S(=O)₂-NH₂, -S(=O)₂-NH-Phenyl, Phenyl, -O-Phenyl, -O-Benzyl und Benzyl substituiert sein kann, wobei jeweils der zyklische Teil der Reste -S(=O)₂-NH-Phenyl, Phenyl, -O-Phenyl,-O-Benzyl und Benzyl mit 1, 2, 3, 4 oder 5 Substituenten unabhängig voneinander ausgewählt aus der Gruppe bestehend aus F, Cl, Br, -OH, -CF₃,-SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann.

9. Verbindungen gemäß einem oder mehreren der Ansprüche 1 bis 8, **dadurch gekennzeichnet, dass**
R⁹ und R¹⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen.

10. Verbindungen gemäß einem oder mehreren der Ansprüche 1 bis 9, **dadurch gekennzeichnet, dass**
R¹¹, R¹³, R¹⁵, R¹⁷, R¹⁹, R²², R²³, R²⁴, R²⁵ und R²⁶, unabhängig voneinander, jeweils
für einen Wasserstoff-Rest oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen.

11. Verbindungen gemäß einem oder mehreren der Ansprüche 1 bis 10, **dadurch gekennzeichnet, dass**
R¹², R²⁸ und R³², unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen.

12. Verbindungen gemäß einem oder mehreren der Ansprüche 1 bis 11, **dadurch gekennzeichnet, dass**
R¹⁴, R¹⁶, R¹⁸ und R²⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl stehen, wobei der Rest jeweils ggf. mit 1, 2, 3, 4 oder 5 Substituenten unabhängig voneinander ausgewählt aus der Gruppe bestehend aus Oxo (=O), Thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃,-S(=O)₂-NH-CH₃ und -S(=O)₂-NH₂ substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃,-N(CH₃)₂, -N(C₂H₅)₂, -N(CH₃)(C₂H₅) und S(=O)₂-NH₂ substituiert sein kann.

13. Verbindungen gemäß einem oder mehreren der Ansprüche 1 bis 12, **dadurch gekennzeichnet, dass**
R²¹, R²⁷, R²⁹, R³⁰ und R³¹, unabhängig voneinander, jeweils für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I, -CN,-CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂,-SH, -S-CH₃, -S-C₂H₅, -S-CH(CH₃)₂, -S-CH₂-CH₂-CH₃, -S-C(CH₃)₃, Methyl, Ethyl, n-Propyl, Isopropyl, n-Butyl, sec-Butyl, Isobutyl, tert-Butyl, n-Pentyl,-C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) und S(=O)₂-NH₂ substituiert sein kann.

14. Verbindungen gemäß einem oder mehreren der Ansprüche 1 bis 13, **dadurch gekennzeichnet, dass**
n gleich 1, 2 oder 3 ist;
R¹ für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, Methyl, Ethyl, n-Propyl, Isopropyl, n-Butyl, sec-Butyl, Isobutyl, tert-Butyl, n-Pentyl, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl, tert-Butyl, n-Pentyl, n-Hexyl, n-Heptyl, -O-CH₃, -O-C₂H₅, -O-CH(CH₃)₂, -O-CH₂-CH₂-CH₃ und -O-C(CH₃)₃ substituiert sein kann;
R² für -C(=S)-NH-R³;
für -C(=O)-NH-R⁴;
für -S(=O)₂-R⁵;
für -(CH₂)-C(=O)-NH-R⁶;
für -(CH₂)-O-R⁷, für -(CH₂)-S-R⁷, für -(CH₂)-NH-R⁷, für -(CH₂)-N(CH₃)-R⁷, für -(CH₂)-(CH₂)-O-R⁷, für -(CH₂)-(CH₂)-S-R⁷, für -(CH₂)-NH-R⁷, für-(CH₂)-N(CH₃)-R⁷, für -(CH₂)-(CH₂)-(CH₂)-O-R⁷, für -(CH₂)-(CH₂)-(CH₂)-S-R⁷, für -(CH₂)-(CH₂)-(CH₂)-NH-R⁷, für -(CH₂)-(CH₂)-(CH₂)-N(CH₃)-R⁷, für -(CH₂)-O-(CH₂)-R⁷, für -(CH₂)-S-(CH₂)-R⁷; für -(CH₂)-NH-(CH₂)-R⁷;
für -C(=O)-R⁸
oder für -S(=O)₂-NR⁹R¹⁰ steht;
R³ für -(CHR¹¹)-C(=O)-O-R¹² oder -(CHR¹¹)-(CH₂)-C(=O)-O-R¹² steht;
für -(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-(CH₂)-O-R¹⁴, -(CHR¹³)-(CH₂)-(CH₂)-O-R¹⁴ oder -(CHR¹³)-O-(CH₂)-R¹⁴ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl, -(CH₂)-(CH)(C₂H₅)-(CH₂)-(CH₂)-(CH₂)-(CH₃), Vinyl, 1-Propenyl, 2-Propenyl, 1-Butenyl, 2-Butenyl und 3-Butenyl steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, -C(=O)-C(CH₃)₃, -C(=O)-CF₃ und -C(=O)-C₂F₅ substituiert sein kann;
R⁴ für -(CHR¹⁵)-R¹⁶, -(CHR¹⁵)-(CH₂)-R¹⁶ oder -(CHR¹⁵)-(CH₂)-(CH₂)-R¹⁶ steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -NO₂, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CH₃, -C(=O)-C₂H₅, -C(=O)-C(CH₃)₃,-C(=O)-CF₃ und -C(=O)-C₂F₅ substituiert sein kann;
R⁵ für -(CHR¹⁷)-R¹⁸, -(CHR¹⁷)-(CH₂)-R¹⁸ oder -(CHR¹⁷)-(CH₂)-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN,-CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ und-S(=O)₂-C₂H₅ substituiert sein kann;
R⁶ für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CF₃, -CN, - O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -S(=O)₂-NH-Phenyl, Phenyl und Benzyl substituiert sein kann, wobei jeweils der zyklische Teil der Reste -S(=O)₂-NH-Phenyl, Phenyl und Benzyl mit 1, 2, 3, 4 oder 5 Substituenten unabhängig voneinander ausgewählt aus der Gruppe bestehend aus F, Cl und Br substituiert sein kann;
R⁷ für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Imidazolyl, Indolyl, Isoindolyl, Benzo[b]furanyl, Benzo[b]thiophenyl, Thiazolyl, [1,2,4]-Oxadiazolyl, [1,2,3]-Thiadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃,-SF₅, -OH, -O-CH₃, -O-C₂H₅, -NO₂, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl,-C(CH₃)₂-C₂H₅, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃,-O-C(CH₃)₃ und -O-CH₂-CH₂-CH₂-CH₃ substituiert sein kann;
R⁸ für -(CHR¹⁹)-R²⁰, -(CHR¹⁹)-(CH₂)-R²⁰, -(CHR¹⁹)-(CH₂)-(CH₂)-R²⁰,-(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ oder -(CHR¹⁹)-O-(CH₂)-(CH₂)-O-R²⁰ steht;
für -(CH=CH)-R²¹ steht;
für -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ oder -(CR²²R²³)-O-(CR²⁴R²⁵)-C(=O)-O-R²⁶ steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und Bicyclo[2.2.1]heptyl steht, wobei der Rest jeweils ggf. mit 1, 2, 3, 4 oder 5 Substituenten unabhängig voneinander ausgewählt aus der Gruppe bestehend aus -C(=O)-CH₃, -C(=O)-C₂H₅, -C(=O)-C(CH₃)₃ und Phenyl substituiert sein kann;
oder für einen Rest ausgewählt aus der Gruppe bestehend aus Phenyl, Naphthyl, (1,3)-Benzodioxolyl, (1,4)-Benzodioxanyl, 2H-Chromenyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, [1,2,3]-Thiadiazolyl, [1,2,4]-Oxadiazolyl, Benzo[2,1,3]thiadiazolyl, [1,2,3]-Benzothiadiazolyl, [2,1,3]-Benzoxadiazolyl, [1,2,3]-Benzoxadiazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, Phenyl, -O-Phenyl, -O-Benzyl und Benzyl substituiert sein kann, wobei jeweils der zyklische Teil der Reste Phenyl, -O-Phenyl, -O-Benzyl und Benzyl mit 1, 2, 3, 4 oder 5 Substituenten unabhängig voneinander ausgewählt aus der Gruppe bestehend aus F, Cl, Br, -OH, -CF₃, -SF₅, -NO₂, Methyl, Ethyl, n-Propyl, Isopropyl, n-Butyl, sec-Butyl, Isobutyl, tert-Butyl, n-Pentyl, n-Hexyl, n-Heptyl, - O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, Phenyl und -O-Benzyl substituiert sein kann;
R⁹ und R¹⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl stehen;
R¹¹, R¹³, R¹⁵, R¹⁷, R¹⁹, R²², R²³, R²⁴, R²⁵ und R²⁶, unabhängig voneinander, jeweils für einen Wasserstoff-Rest oder für einen Rest ausgewählt aus der Gruppe bestehend aus Methyl, Ethyl, n-Propyl, Isopropyl, n-Butyl, sec-Butyl, Isobutyl und tert-Butyl stehen;
R¹², R²⁸ und R³², unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
R¹⁴, R¹⁶, R¹⁸ und R²⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
für einen Rest ausgewählt aus der Gruppe bestehend aus Cyclopropyl, Cyclobutyl, Cyclopentyl, Cyclohexyl, Cycloheptyl, Tetrahydrofuranyl, Tetrahydrothiophenyl, Tetrahydropyranyl und Bicyclo[2.2.1]heptyl stehen, wobei der Rest jeweils ggf. mit 1, 2, 3, 4 oder 5 Substituenten unabhängig voneinander ausgewählt aus der Gruppe bestehend aus Oxo (=O), Thioxo (=S), Methyl, Ethyl, n-Propyl, Isopropyl, n-Butyl, sec-Butyl, Isobutyl, tert-Butyl, n-Pentyl, n-Hexyl, n-Heptyl, -(CH₂)-C(=O)-OH und -C(=O)-OH substituiert sein kann;
oder für einen Rest ausgewählt aus der Gruppe bestehend aus Phenyl und Naphthyl stehen, wobei der Rest jeweils mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl und n-Heptyl substituiert sein kann;
und
R²¹, R²⁷, R²⁹, R³⁰ und R³¹, unabhängig voneinander, jeweils für einen Rest ausgewählt aus der Gruppe bestehend aus Phenyl, Naphthyl, Indolyl und Isoindolyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃,-O-CH₂-CH₂-CH₂-CH₃, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl, -C(CH₃)₂-C₂H₅, n-Hexyl und n-Heptyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

15. Verbindungen gemäß einem oder mehreren der Ansprüche 1 bis 14, **dadurch gekennzeichnet, dass**
n gleich 1 oder 2 ist;
R¹ für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, Methyl, Ethyl, n-Propyl, Isopropyl, n-Butyl, sec-Butyl, Isobutyl, tert-Butyl, n-Pentyl, n-Hexyl, n-Heptyl, Phenyl und Benzyl substituiert sein kann;
R² für -C(=S)-NH-R³;
für -C(=O)-NH-R⁴;
für -S(=O)₂-R⁵;
für -(CH₂)-C(=O)-NH-R⁶;
für -(CH₂)-O-R⁷, für -(CH₂)-(CH₂)-O-R⁷, für -(CH₂)-(CH₂)-(CH₂)-O-R⁷;
für -C(=O)-R⁸
oder für -S(=O)₂-NR⁹R¹⁰ steht;
R³ für -(CHR¹¹)-(CH₂)-C(=O)-O-R¹² steht;
für -(CHR¹³)-R¹⁴, -(CHR¹³)-(CH₂)-R¹⁴, -(CHR¹³)-(CH₂)-(CH₂)-R¹⁴ oder-(CHR¹³)-(CH₂)-O-R¹⁴ steht;
für einen Rest ausgewählt aus der Gruppe bestehend aus Vinyl, 1-Propenyl, 2-Propenyl, 1-Butenyl, 2-Butenyl und 3-Butenyl steht;
oder für einen Phenyl-Rest steht, der Rest mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃,-O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-CH₃, -C(=O)-C₂H₅, - C(=O)-C(CH₃)₃, -C(=O)-CF₃ und -C(=O)-C₂F₅ substituiert sein kann;
R⁴ für -(CHR¹⁵)-R¹⁶, -(CHR¹⁵)-(CH₂)-R¹⁶ oder -(CHR¹⁵)-(CH₂)-(CH₂)-R¹⁶ steht;
oder für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus F, Cl, Br, -NO₂, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, Methyl, Ethyl, n-Propyl, Isopropyl, n-Butyl, sec-Butyl, Isobutyl, tert-Butyl, n-Pentyl, n-Hexyl, n-Heptyl, -C(=O)-O-CH₃,-C(=O)-O-CH₂-CH₃, -C(=O)-O-CH(CH₃)₂, -C(=O)-O-C(CH₃)₃, -C(=O)-CH₃,-C(=O)-C₂H₅, -C(=O)-C(CH₃)₃, -C(=O)-CF₃ und -C(=O)-C₂F₅ substituiert sein kann;
R⁵ für -(CHR¹⁷)-R¹⁸, -(CHR¹⁷)-(CH₂)-R¹⁸ oder -(CHR¹⁷)-(CH₂)-(CH₂)-R¹⁸ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
oder für einen Rest ausgewählt aus der Gruppe bestehend aus Phenyl, Naphthyl, Pyrazolyl, Thiophenyl, [1,2,3,4]-Tetrahydrochinolinyl und [1,2,3,4]-Tetrahydroisochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ und - S(=O)₂-C₂H₅ substituiert sein kann;
R⁶ für einen Rest ausgewählt aus der Gruppe bestehend aus Phenyl, Naphthyl, Pyrazolyl, Thiophenyl und Thiazolyl steht, wobei der Rest jeweils ggf. mit 1, 2, 3, 4 oder 5 Substituenten unabhängig voneinander ausgewählt aus der Gruppe bestehend aus F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -S(=O)₂-NH-Phenyl, Phenyl und Benzyl substituiert sein kann, wobei jeweils der zyklische Teil der Reste-S(=O)₂-NH-Phenyl, Phenyl und Benzyl mit 1, 2, 3, 4 oder 5 Substituenten unabhängig voneinander ausgewählt aus der Gruppe bestehend aus F, Cl und Br substituiert sein kann;
R⁷ für einen Rest ausgewählt aus der Gruppe bestehend aus Phenyl, Naphthyl, Benzo[b]furanyl, Benzo[b]thiophenyl und [1,2,4]-Oxadiazolyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃,-SF₅, -OH, -O-CH₃, -O-C₂H₅, -NO₂, -O-CF₃, -S-CF₃, -S-CF₂H, -S-CFH₂, Methyl, Ethyl, n-Propyl, Isopropyl, n-Butyl, sec-Butyl, Isobutyl, tert-Butyl, n-Pentyl,-C(CH₃)₂-C₂H₅, n-Hexyl, n-Heptyl, Phenyl und Benzyl substituiert sein kann, wobei jeweils der zyklische Teil der Reste Phenyl und Benzyl mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃,-O-C(CH₃)₃ und -O-CH₂-CH₂-CH₂-CH₃ substituiert sein kann;
R⁸ für -(CHR¹⁹)-R²⁰, -(CHR¹⁹)-(CH₂)-R²⁰, -(CHR¹⁹)-(CH₂)-(CH₂)-R²⁰,-(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ oder -(CHR¹⁹)-O-(CH₂)-(CH₂)-O-R²⁰ steht;
für -(CH=CH)-R²¹ steht;
für -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ oder -(CR²²R²³)-O-(CR²⁴R²⁵)-C(=O)-O-R²⁶ steht;
für -(CHR²⁷)-O-C(=O)-R²⁸ steht;
für -CH[(CH₂)R²⁹][NH-S(=O)₂-R³⁰] steht;
für -CH[(CH₂)R³¹][NH-C(=O)-O-R³²] steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, 3-Pentyl, -(CH₂)-(CH₂)-(C(CH₃)₃), n-Hexyl, 2-Hexyl, 3-Hexyl, n-Heptyl, 2-Heptyl, 3-Heptyl, 4-Heptyl und n-Octyl steht;
für einen Rest ausgewählt aus der Gruppe bestehend aus Cyclopropyl, Cyclobutyl, Cyclopentyl, Cyclohexyl, Cycloheptyl, Pyrrolidinyl, Piperidinyl und Adamantyl steht, wobei der Rest jeweils ggf. mit 1, 2, 3, 4 oder 5 Substituenten unabhängig voneinander ausgewählt aus der Gruppe bestehend aus -C(=O)-CH₃, -C(=O)-C₂H₅, -C(=O)-C(CH₃)₃ und Phenyl substituiert sein kann;
oder für einen Rest ausgewählt aus der Gruppe bestehend aus Phenyl, Naphthyl, 2H-Chromenyl, Thiophenyl, Furanyl, Pyrazolyl, Triazolyl, Pyridinyl, [1,2,3]-Thiadiazolyl, [2,1,3]-Benzoxadiazolyl, [1,2,3]-Benzoxadiazolyl, Oxazolyl und Isoxazolyl steht, wobei der Rest jeweils ggf. mit 1, 2, 3, 4 oder 5 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, Methyl, Ethyl, n-Propyl, Isopropyl, n-Butyl, sec-Butyl, Isobutyl, tert-Butyl, n-Pentyl, -C(CH₃)₂-C₂H₅, n-Hexyl, n-Heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, Phenyl, -O-Phenyl, -O-Benzyl und Benzyl substituiert sein kann, wobei jeweils der zyklische Teil der Reste Phenyl, -O-Phenyl, -O-Benzyl und Benzyl mit 1, 2, 3, 4 oder 5 Substituenten unabhängig voneinander ausgewählt aus der Gruppe bestehend aus F, Cl und Br substituiert sein kann;
R⁹ und R¹⁰, unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
R¹¹, R¹³, R¹⁵, R¹⁷, R¹⁹, R²², R²³, R²⁴, R²⁵ und R²⁶, unabhängig voneinander, jeweils für einen Wasserstoff-Rest oder für einen Rest ausgewählt aus der Gruppe bestehend aus Methyl, Ethyl und n-Propyl stehen;
R¹², R²⁸ und R³², unabhängig voneinander, jeweils für einen Rest ausgewählt aus der Gruppe bestehend aus Methyl, Ethyl, n-Propyl, Isopropyl, n-Butyl, sec-Butyl, Isobutyl und tert-Butyl stehen;
R¹⁴ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Tetrahydrofuranyl, Tetrahydrothiophenyl und Tetrahydropyranyl steht;
oder für einen Phenyl-Rest steht, der mit 1, 2, 3, 4 oder 5 Substituenten ausgewählt aus der Gruppe bestehend aus F, Cl und Br substituiert sein kann;
R¹⁶ für einen Rest ausgewählt aus der Gruppe bestehend aus Phenyl und Naphthyl steht, wobei der Rest jeweils mit 1, 2, 3, 4 oder 5 Substituenten unabhängig voneinander ausgewählt aus der Gruppe bestehend aus F, Cl und Br substituiert sein kann;
R¹⁸ für einen 7,7-Dimethyl-2-oxo-bicyclo[2.2.1]hept-1-yl-Rest steht;
R²⁰ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl und Cycloheptyl steht; wobei der Rest jeweils mit einem Substituenten ausgewählt aus der Gruppe bestehend aus - (CH₂)-C(=O)-OH und -C(=O)-OH substituiert sein kann;
oder für Phenyl-Rest steht, der mit 1, 2, 3, 4 oder 5 Substituenten unabhängig voneinander ausgewählt aus der Gruppe bestehend aus F, Cl, Br, -O-CH₃, -O-C₂H₅, -O-CH(CH₃)₂, -O-CH₂-CH₂-CH₃, -O-C(CH₃)₃ und -O-CH₂-CH₂-CH₂-CH₃, substituiert sein kann;
R²¹ für einen Phenyl-Rest steht, der mit 1, 2, 3, 4 oder 5 Substituenten ausgewählt aus der Gruppe bestehend aus F, Cl und Br substituiert sein kann;
R²⁷ für einen Phenyl-Rest steht;
R²⁹ für einen Phenyl-Rest steht;
R³⁰ für einen Phenyl-Rest steht, der mit 1, 2, 3, 4 oder 5 Substituenten ausgewählt aus der Gruppe bestehend aus Methyl, Ethyl, n-Propyl, Isopropyl, n-Butyl, sec-Butyl und tert-Butyl substituiert sein kann;
und
R³¹ für einen Indolyl-Rest steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

16. Verbindungen gemäß einem oder mehreren der Ansprüche 1 bis 15 ausgewählt aus der Gruppe bestehend aus
[1] 3-(4-Methyl-benzyl)-2-oxo-1-oxa-3,8-diaza-spiro[4.5]decan-8-carbothiosäure-pentafluorphenyl-amid
[2] 3-(3-Brom-benzyl)-2-oxo-1-oxa-3,8-diaza-spiro[4.5]decan-8-carbothiosäure-(2,5-dichlor-phenyl)-amid
[3] 2-Oxo-3-(4-trifluormethyl-benzyl)-1-oxa-3,8-diaza-spiro[4.5]decan-8-carbothiosäurebenzylamid
[4] 3-(3,5-Dimethyl-benzyl)-2-oxo-1-oxa-3,8-diaza-spiro[4.5]decan-8-carbothiosäure-(tetrahydro-furan-2-ylmethyl)-amid
[5] 3-(3-Brom-benzyl)-2-oxo-1-oxa-3,8-diaza-spiro[4.5]decan-8-carbothiosäure-(2-methoxy-ethyl)-amid
[6] 3-(3-Brom-benzyl)-2-oxo-1-oxa-3,8-diaza-spiro[4.5]decan-8-carbothiosäure-(2-methoxy-phenyl)-amid
[7] 3-(3,5-Dimethyl-benzyl)-2-oxo-1-oxa-3,8-diaza-spiro[4.5]decan-8-carbothiosäure-(3-acetyl-phenyl)-amid
[8] 3-Benzyl-2-oxo-1-oxa-3,8-diaza-spiro[4.5]decan-8-carbothiosäure-o-tolylamid
[9] 3-{[3-(3-Brom-benzyl)-2-oxo-1-oxa-3,8-diaza-spiro[4.5]decan-8-carbothioyl]-amino}-butansäureethylester
[10] 3-Naphthalen-2-ylmethyl-2-oxo-1-oxa-3,8-diaza-spiro[4.5]decan-8-carbothiosäureallylamid
[11] 3-(3,4-Difluor-benzyl)-2-oxo-1-oxa-3,8-diaza-spiro[4.5]decan-8-carbothiosäurebenzylamid
[12] 3-Biphenyl-2-ylmethyl-2-oxo-1-oxa-3,8-diaza-spiro[4.5]decan-8-carbothiosäure-(1-phenyl-ethyl)-amid
[13] 3-(3,4-Difluor-benzyl)-2-oxo-1-oxa-3,8-diaza-spiro[4.5]decan-8-carbothiosäure-(4-ethoxy-phenyl)-amid
[14] 3-(3-Cyano-benzyl)-2-oxo-1-oxa-3,8-diaza-spiro[4.5]decan-8-carbothiosäure-(3,5-dichlor-phenyl)-amid
[15] 3-(3-Cyano-benzyl)-2-oxo-1-oxa-3,8-diaza-spiro[4.5]decan-8-carbothiosäure-(3-trifluormethyl-phenyl)-amid
[16] 3-(3-Brom-benzyl)-2-oxo-1-oxa-3,8-diaza-spiro[4.5]decan-8-carbothiosäure-benzylamid
[17] 3-(2-Methoxy-5-nitro-benzyl)-2-oxo-1-oxo-3,8-diaza-spiro[4.5]decan-8-carbothiosäure-(tetrahydro-furan-2-ylmethyl)-amid
[18] 3-Biphenyl-2-ylmethyl-2-oxo-1-oxa-3,8-diaza-spiro[4.5]decan-8-carbothiosäure-phenylamid
[19] 3-(3-Cyano-benzyl)-2-oxo-1-oxa-3,8-diaza-spiro[4.5]decan-8-carbothiosäure-cyclohexylmethyl-amid
[20] 3-(2-Fluor-benzyl)-2-oxo-1-oxa-3,8-diaza-spiro[4,5]decan-8-carbothiosäure-(3-acetyl-phenyl)-amid
[21] 3-(2-Methoxy-5-nitro-benzyl)-2-oxo-1-oxa-3,8-diaza-spiro[4.5]decan-8-carbothiosäure-cyclohexylmethyl-amid
[22] 3-(3-Cyano-benzyl)-2-oxo-1-oxa-3,8-diaza-spiro[4.5]decan-8-carbothiosäure-4-chlor-benzylamid
[23] 3-(2-Methoxy-5-nitro-benzyl)-2-oxo-1-oxa-3,8-diaza-spiro[4.5]decan-8-carbothiosäure-benzylamid
[24] 3-(2-Fluor-benzyl)-2-oxo-1-oxa-3,8-diaza-spiro[4.5]decan-8-carbonsäure-(4-ethoxy-phenyl)-amid
[25] 3-Biphenyl-2-ylmethyl-2-oxo-1-oxa-3,8-diaza-spiro[4.5]decan-8-carbonsäure-(2,5-dichlor-phenyl)-amid
[26] 3-(2-Methoxy-5-nitro-benzyl)-2-oxo-1-oxa-3,8-diaza-spiro[4.5]decan-8-carbonsäure-(2,5-difluor-phenyl)-amid
[27] 3-(4-Methyl-benzyl)-2-oxo-1-oxa-3,8-diaza-spiro[4.5]decan-8-carbonsäure-(4-methyl-3-nitro-phenyl)-amid
[28] 3-(3,4-Difluor-benzyl)-2-oxo-1-oxa-3,8-diaza-spiro[4.5]decan-8-carbonsäure-4-fluor-benzylamid
[29] 3-(3-Brom-benzyl)-2-oxo-1-oxa-3,8-diaza-spiro[4.5]decan-8-carbonsäure-(2,5-difluor-phenyl)-amid
[30] 4-[(3-Naphthalen-2-ylmethyl-2-oxo-1-oxa-3,8-diaza-spiro[4.5]decan-8-carbonyl)-amino]-benzoesäure-ethyl-ester
[31] 2-Oxo-3-(4-trifluormethyl-benzyl)-1-oxa-3,8-diaza-spiro[4.5]decan-8-carbonsäure-(4-chlor-3-trifluormethyl-phenyl)-amid
[32] 3-(2-Cyano-benzyl)-2-oxo-1-oxa-3,8-diaza-spiro[4.5]decan-8-carbonsäure-(2,5-difluor-phenyl)-amid
[33] 3-Benzyl-2-oxo-1-oxa-3,8-diaza-spiro[4.5]decan-8-carbonsäure-(3-cyano-phenyl)-amid
[34] 3-(3,4-Difluor-benzyl)-2-oxo-1-oxa-3,8-diaza-spiro[4.5]decan-8-carbonsäure-(4-methyl-3-nitro-phenyl)-amid
[35] 3-(3,4-Difluor-benzyl)-2-oxo-1-oxa-3,8-diaza-spiro[4,5]decan-8-carbonsäure-(3-methoxy-phenyl)-amid
[36] 3-(4-Methyl-benzyl)-2-oxo-1-oxa-3,8-diaza-spiro[4.5]decan-8-carbonsäure-3,4-dichlor-benzylamid
[37] 3-Benzyl-2-oxo-1-oxa-3,8-diaza-spiro[4.5]decan-8-carbonsäure-(4-chlor-3-trifluormethyl-phenyl)-amid
[38] 2-Oxo-3-(4-trifluormethylsulfanyl-benzyl)-oxa-3,8-diaza-spiro[4.5]decan-8-carbonsäure-(3-cyano-phenyl)-amid
[39] 3-Benzyl-2-oxo-1-oxa-3,8-diaza-spiro[4.5]decan-8-carbonsäure-(4-methoxy-phenyl)-amid
[40] 2-Oxo-3-(4-trifluormethylsulfanyl-benzyl)-oxa-3,8-diaza-spiro[4.5]decan-8-carbonsäure-(4-methyl-3-nitro-phenyl)-amid
[41] 3-(4-lod-benzyl)-2-oxo-1-oxa-3,8-diaza-spiro[4.5]decan-8-carbonsäure-(4-butoxy-phenyl)-amid
[42] 3-(3-Brom-benzyl)-2-oxo-1-oxa-3,8-diaza-spiro[4.5]decan-8-carbonsäure-3,4-dichlor-benzylamid
[43] 3-Benzyl-2-oxo-1-oxa-3,8-diaza-spiro[4.5]decan-8-carbonsäure-(1-phenyl-ethyl)-amid
[44] 3-Naphthalen-2-ylmethyl-2-oxo-1-oxa-3,8-diaza-spiro[4.5]decan-8-carbonsäure-3,4-dichlor-benzylamid
[45] 3-(4-Methyl-benzyl)-2-oxo-1-oxa-3,8-diaza-spiro[4.5]decan-8-carbonsäure-phenethyl-amid
[46] 2-Oxo-3-(2-trifluormethyl-benzyl)-1-oxa-3,8-diaza-spiro[4.5]decan-8-carbonsäure-(3-methoxy-phenyl)-amid
[47] 3-(4-Cyano-benzyl)-2-oxo-1-oxa-3,8-diaza-spiro[4.5]decan-8-carbonsäure-(4-methoxy-phenyl)-amid
[48] 2-Oxo-3-(4-trifluormethyl-benzyl)-1-oxa-3,8-diaza-spiro[4.5]decan-8-carbonsäure-(1-naphthalen-1-yl-ethyl)-amid
[49] 2-Oxo-3-(2-trifluormethyl-benzyl)-1-oxa-3,8-diaza-spiro[4.5]decan-8-carbonsäure-phenethyl-amid
[50] 3-(4-lod-benzyl)-2-oxo-1-oxa-3,8-diaza-spiro[4.5]decan-8-carbonsäure-(4-methyl-3-nitro-phenyl)-amid
[51] 3-(2-Methoxy-5-nitro-benzyl)-2-oxo-1-oxa-3,8-diaza-spiro[4.5]decan-8-carbonsäure-(3-cyano-phenyl)-amid
[52] 3-Naphthalen-2-ylmethyl-2-oxo-1-oxa-3,8-diaza-spiro[4.5]decan-8-c carbonsäure-(4-methoxy-phenyl)-amid
[53] 3-{[3-(4-Methyl-benzyl)-2-oxo-1-oxa-3,8-diaza-spiro[4.5]decan-8-carbonyl]-amino}-benzoesäure-ethyl-ester
[54] 2-Oxo-3-(2-trifluormethyl-benzyl)-1-oxa-3,8-diaza-spiro[4.5]decan-8-carbonsäure-(1-naphthalen-1-yl-ethyl)-amid
[55] 3-Naphthalen-2-ylmethyl-2-oxo-1-oxa-3,8-diaza-spiro[4.5]decan-8-carbonsäure-(2,5-dimethoxy-phenyl)-amid
[56] 3-(4-Cyano-benzyl)-2-oxo-1-oxa-3,8-diaza-spiro[4.5]decan-8-carbonsäure-(3-acetyl-phenyl)-amid
[57] 3-Biphenyl-2-ylmethyl-2-oxo-1-oxa-3,8-diaza-spiro[4.5]decan-8-carbonsäure-phenethyl-amid
[58] 3-(4-Cyano-benzyl)-2-oxo-1-oxa-3,8-diaza-spiro[4.5]decan-8-carbonsäure-(5-chlor-2-methoxy-phenyl)-amid
[59] 3-{[2-Oxo-3-(2-trifluormethyl-benzyl)-1-oxa-3,8-diaza-spiro[4.5]decan-8-carbonyl]-amino}-benzoesäure-ethyl-ester
[60] 3-Benzyl-2-oxo-1-oxa-3,8-diaza-spiro[4.5]decan-8-carbonsäure-(3-fluorphenyl)-amid
[61] 3-{[3-(3-Cyano-benzyl)-2-oxo-1-oxa-3,8-diaza-spiro[4.5]decan-8-carbonyl]-amino}-benzoesäure-ethyl-ester
[62] 3-Biphenyl-2-ylmethyl-2-oxo-1-oxa-3,8-diaza-spiro[4.5]decan-8-carbonsäure-(3-methoxy-phenyl)-amid
[63] 3-(2-Methoxy-5-nitro-benzyl)-2-oxo-1-oxa-3,8-diaza-spiro[4.5]decan-8-carbonsäure-(1-naphthalen-1-yl-ethyl)-amid
[64] {2-[3-(3,4-Difluor-benzyl)-2-oxo-1-oxa-3,8-diaza-spiro[4.5]dec-8-yl]-2-oxo-ethoxy}-essigsäure
[65] 4-[3-(3,4-Difluor-benzyl)-2-oxo-1-oxa-3,8-diaza-spiro[4.5]dec-8-yl]-3,3-dimethyl-4-oxo-butansäure
[66] [2-[3-(3-Brom-benzyl)-2-oxo-1-oxa-3,8-diaza-spiro[4.5]dec-8-yl]-1-(1H-indol-3-ylmethyl)-2-oxo-ethyl]-carbaminsäure-tert-butyl-ester
[67] 5-[3-(2-Fluor-benzyl)-2-oxo-1-oxa-3,8-diaza-spiro[4.5]dec-8-yl]-3-methyl-5-oxo-pentansäure
[68] 3,3-Dimethyl-5-[3-(4-methyl-benzyl)-2-oxo-1-oxa-3,8-diaza-spiro[4.5]dec-8-yl]-5-oxo-pentansäure
[69] 5-[3-(3,4-Difluor-benzyl)-2-oxo-1-oxa-3,8-diaza-spiro[4.5]dec-8-yl]-3-methyl-5-oxo-pentansäure
[70] {2-Oxo-2-[2-oxo-3-(4-trifluormethyl-benzyl)-1-oxa-3,8-diaza-spiro[4.5]dec-8-yl]-ethoxy}-essigsäure
[71] (1-{2-[3-(2-Cyano-benzyl)-2-oxo-1-oxa-3,8-diaza-spiro[4.5]dec-8-yl]-2-oxo-ethyl}-cyclopentyl)-essigsäure
[72] (1-{2-[3-(3,4-Difluor-benzyl)-2-oxo-1-oxa-3,8-diaza-spiro[4.5]dec-8-yl]-2-oxo-ethyl}-cyclopentyl)-essigsäure
[73] 5-(3-Biphenyl-2-ylmethyl-2-oxo-1-oxa-3,8-diaza-spiro[4.5]dec-8-yl)-3-methyl-5-oxo-pentansäure
[75] N-[4-(3,5-Dichlor-phenylsulfamoyl)-phenyl]-2-[3-(4-methyl-benzyl)-2-oxo-1-oxa-3,8-diaza-spiro[4.5]dec-8-yl]-acetamid
[76] N-(2,5-Dimethyl-2H-pyrazol-3-yl)-2-[3-(2-methoxy-5-nitro-benzyl)-2-oxo-1-oxa-3,8-diaza-spiro[4.5]dec-8-yl]-acetamid
[80] N-(3-Cyano-4-methyl-thiophen-2-yl)-2-[3-(3,4-difluor-benzyl)-2-oxo-1-oxa-3,8-diaza-spiro[4.5]dec-8-yl]-acetamid
[85] 2-[2-Oxo-3-(4-trifluormethylsulfanyl-benzyl)-1-oxa-3,8-diaza-spiro[4.5]dec-8-yl]-N-(4-phenyl-5-trifluomethyl-thiophen-3-yl)-acetamid
[86] 2-[3-(2-Fluor-benzyl)-2-oxo-1-oxa-3,8-diaza-spiro[4.5]dec-8-yl]-N-(4-phenyl-thiazol-2-yl)-acetamid
[87] 2-[2-Oxo-3-(2-trifluormethyl-benzyl)-1-oxa-3,8-diaza-spiro[4.5]dec-8-yl]-N-(4-trifluormethoxy-phenyl)-acetamid
[91] N-{4-[3-(3-Cyano-benzyl)-2-oxo-1-oxa-3,8-diaza-spiro[4.5]decan-8-carbonyl]-phenyl}-acetamid
[92] 8-(4-Ethoxy-benzoyl)-3-(4-methyl-benzyl)-1-oxa-3,8-diaza-spiro[4.5]decan-2-on
[93] 3-[3-(4-Cyano-benzyl)-2-oxo-1-oxa-3,8-diaza-spiro[4.5]dec-8-yl]-3-oxo-propionsäuremethylester
[94] 2-[8-(2,4-Difluor-benzoyl)-2-oxo-1-oxa-3,8-diaza-spiro[4.5]dec-3-ylmethyl]-benzonitril
[95] 8-(3-Dimethylamino-benzoyl)-3-(3,5-dimethyl-benzyl)-1-oxa-3,8-diaza-spiro[4.5]decan-2-on
[96] N-{4-[3-(3,4-Difluor-benzyl)-2-oxo-1-oxa-3,8-diaza-spiro[4.5]decan-8-carbonyl]-phenyl}-acetamid
[97] 8-(4-Brom-benzoyl)-3-(4-fluor-benzyl)-1-oxa-3,8-diaza-spiro[4.5]decan-2-on
[98] 4-[8-(Adamantan-1-carbonyl)-2-oxo-1-oxa-3,8-diaza-spiro[4.5]dec-3-ylmethyl]-benzonitril
[99] 3-(3,5-Dimethyl-benzyl)-8-(2-ethylsulfanyl-pyridin-3-carbonyl)-1-oxa-3,8-diaza-spiro[4.5]decan-2-on
[100] 3-(3-Brom-benzyl)-8-(4-chlor-benzoyl)-1-oxa-3,8-diaza-spiro[4.5]decan-2-on
[101]8-[3-(2-Chlorphenyl)-5-methyl-isoxazol-4-carbonyl]-3-(3,5-dimethyl-benzyl)-1-oxa-3,8-diaza-spiro[4.5]decan-2-on
[102] 3-(3-Brom-benzyl)-8-(4-ethyl-benzylsulfonyl)-1-oxa-3,8-diaza-spiro[4.5]decan-2-on
[103]8-(4-Butoxy-benzylsulfonyl)-3-(3,5-dimethyl-benzyl)-1-oxa-3,8-diaza-spiro[4.5]decan-2-on
[104]8-(Biphenyl-4-carbonyl)-3-(3-brom-benzyl)-1-oxa-3,8-diaza-spiro[4.5]decan-2-on
[105] Essigsäure-2-[3-(3,5-dimethyl-benzyl)-2-oxo-1-oxa-3,8-diaza-spiro[4.5]dec-8-yl]-2-oxo-1-phenyl-ethylester
[106]4-[2-Oxo-8-(2-phenyl-cyclopropancarbonyl)-1-oxa-3,8-diaza-spiro[4,5]dec-3-ylmethyl]-benzonitril
[107] 8-(2,5-Dimethoxy-benzylsulfonyl)-3-(3,5-dimethyl-benzyl)-1-oxa-3,8-diaza-spiro[4.5]decan-2-on
[108]8-(2-Chlor-benzoyl)-3-(3,5-dimethyl-benzyl)-1-oxa-3,8-diaza-spiro[4.5]decan-2-on
[109] 3-(3-Brom-benzyl)-8-(4-nitro-benzoyl)-1-oxa-3,8-diaza-spiro[4.5]decan-2-on
[110]N-{1-Benzyl-2-[3-(3-cyano-benzyl)-2-oxo-1-oxa-3,8-diaza-spiro[4.5]dec-8-yl]-2-oxo-ethyl}-4-methyl-benzylsulforlamid
[111]8-(2-Ethylsulfanyl-pyridin-3-carbonyl)-3-(3-methoxy-benzyl)-1-oxa-3,8-diaza-spiro[4.5]decan-2-on
[112]8-(2,4-Dimethoxy-benzoyl)-3-(3-methoxy-benzyl)-1-oxa-3,8-diaza-spiro[4.5]decan-2-on
[113]3-(3,5-Dimethyl-benzyl)-8-[2-(3-methoxy-phenyl)-acetyl]-1-oxa-3,8-diaza-spiro[4.5]decan-2-on
[114]3-(3,5-Dimethyl-benzyl)-8-(4-methoxy-2,3,6-trimethyl-benzylsulfonyl)-1-oxa-3,8-diaza-spiro[4.5]decan-2-on
[115]4-[8-(Biphenyl-4-carbonyl)-2-oxo-1-oxa-3,8-diaza-spiro[4.5]dec-3-ylmethyl]-benzonitril
[116]8-(2-Chlor-benzylsulfonyl)-3-(3,5-dimethyl-benzyl)-1-oxa-3,8-diaza-spiro[4.5]decan-2-on
[117]8-(Benzo[1,2,5]oxadiazol-5-carbonyl)-3-(3,5-dimethyl-benzyl)-1-oxa-3,8-diaza-spiro[4.5]decan-2-on
[118]8-(Biphenyl-4-carbonyl)-3-(4-iod-benzyl)-1-oxa-3,8-diaza-spiro[4.5]decan-2-on
[119]3-(3,5-Dimethyl-benzyl)-8-(pyridin-2-carbonyl)-1-oxa-3,8-diaza-spiro[4.5]decan-2-on
[120]8-(6-Chlor-pyridin-3-carbonyl)-3-naphthalen-2-ylmethyl-1-oxa-3,8-diaza-spiro[4.5]decan-2-on
[121]3-(3-Brom-benzyl)-8-(2-ethylsulfanyl-pyridin-3-carbonyl)-1-oxa-3,8-diaza-spiro[4.5]decan-2-on
[122]3-(3,5-Dimethyl-benzyl)-8-(5-methyl-isoxazol-3-carbonyl)-1-oxa-3,8-diaza-spiro[4.5]decan-2-on
[123]3-(2-Methoxy-5-nitro-benzyl)-8-pentanoyl-1-oxa-3,8-diaza-spiro[4.5]decan-2-on
[124]8-(4-Brom-benzoyl)-3-(3,5-dimethyl-benzyl)-1-oxa-3,8-diaza-spiro[4.5]decan-2-on
[125]8-(5-tert-Butyl-2-methyl-furan-3-carbonyl)-3-(2-methoxy-5-nitro-benzyl)-1-oxa-3,8-diaza-spiro[4.5]decan-2-on
[126]8-(3-Chlor-benzoyl)-3-(3,5-dimethyl-benzyl)-1-oxa-3,8-diaza-spiro[4.5]decan-2-on
[127]8-(2-Ethylsulfanyl-pyridin-3-carbonyl)-3-(2-trifluormethyl-benzyl)-1-oxa-3,8-diaza-spiro[4.5]decan-2-on
[128]3-(3,5-Dimethyl-benzyl)-8-(isoxazol-5-carbonyl)-1-oxa-3,8-diaza-spiro[4.5]decan-2-on
[129]3-(3-Brom-benzyl)-8-(pyridin-2-carbonyl)-1-oxa-3,8-diaza-spiro[4.5]decan-2-on
[130]3-(3,5-Dimethyl-benzyl)-8-(thiophen-2-carbonyl)-1-oxa-3,8-diaza-spiro[4.5]decan-2-on
[131]8-(3-Chlor-4-fluor-benzoyl)-3-(3,5-dimethyl-benzyl)-1-oxa-3,8-diaza-spiro[4.5]decan-2-on
[132]8-(2,6-Difluor-3-methyl-benzoyl)-3-(3,5-dimethyl-benzyl)-1-oxa-3,8-diaza-spiro[4.5]decan-2-on
[133]4-[3-(3-Brom-benzyl)-2-oxo-1-oxa-3,8-diaza-spiro[4.5]dec-8-yl]-4-oxo-butansäuremethylester
[134]3-[8-(2-Ethyl-butyryl)-2-oxo-1-oxa-3,8-diaza-spiro[4.5]dec-3-ylmethyl]-benzonitril
[135]3-[8-(3-Brom-benzylsulfonyl)-2-oxo-1-oxa-3,8-diaza-spiro[4.5]dec-3-ylmethyl]-benzonitril
[136]3-(3,4-Difluor-benzyl)-8-(4-methyl-benzoyl)-1-oxa-3,8-diaza-spiro[4.5]decan-2-on
[137]3-(3-Brom-benzyl)-8-(3,5-dimethoxy-benzoyl)-1-oxa-3,8-diaza-spiro[4.5]decan-2-on
[138]8-(3-Dimethylamino-benzoyl)-3-(2-methoxy-5-nitro-benzyl)-1-oxa-3,8-diaza-spiro[4.5]decan-2-on
[139]8-(2,6-Dichlor-benzoyl)-3-(2-fluor-benzyl)-1-oxa-3,8-diaza-spiro[4.5]decan-2-on
[140]3-(2-Mothoxy-5-nitro-benzyl)-8-(2-phenoxy-pyridin-3-carbonyl)-1-oxa-3,8-diaza-spiro[4.5]decan-2-on
[141]8-[2-(3-Methoxy-phenyl)-acetyl]-3-naphthalen-2-ylmethyl-1-oxa-3,8-diaza-spiro[4.5]decan-2-on
[142]4-{2-Oxo-8-[2-(2,2,2-trifluoro-acetyl)-1,2,3,4-tetrahyd ro-isochinolin-7-sulfonyl]-1-oxa-3,8-diaza-spiro[4.5]dec-3-ylmethyl}-benzonitril
[143]8-(3,5-Bis-trifluormethyl-benzoyl)-3-(4-trifluormethyl-benzyl)-1-oxa-3,8-diaza-spiro[4.5]decan-2-on
[144]3-(3-Brom-benzyl)-8-(2-phenoxy-pyridin-3-carbonyl)-1-oxa-3,8-d iaza-spiro[4.5]decan-2-on
[145]3-(2-Methoxy-5-nitro-benzyl)-8-[2-(3-methoxy-phenyl)-acetyl]-1-oxa-3,8-diaza-spiro[4.5]decan-2-on
[146]3-(3,4-Difluor-benzyl)-8-(2-phenoxy-pyridin-3-carbonyl)-1-oxa-3,8-diaza-spiro[4.5]decan-2-on
[147]3-[8-(4-Brom-3-methyl-benzoyl)-2-oxo-1-oxa-3,8-diaza-spiro[4.5]deo-3-ylmethyl]-benzonitril
[148]3-(3-Brom-benzyl)-8-(4-propyl-benzoyl)-1-oxa-3,8-diaza-spiro[4.5]decan-2-on
[149]3-Biphenyl-2-ylmethyl-8-(3-chlor-benzylsulfonyl)-1-oxa-3,8-diaza-spiro[4.5]decan-2-on
[150]8-[2-(3,4-Dimethoxy-phenyl)-acetyl]-3-(2-methoxy-5-nitro-benzyl)-1-oxa-3,8-diaza-spiro[4.5]decan-2-on
[151]2-[8-(2-Chlor-4-nitro-benzoyl)-2-oxo-1-oxa-3,8-diaza-spiro[4.5]dec-3-ylmethyl]-benzonitril
[152]3-[2-Oxo-8-(2,4,6-trimethyl-benzylsulfonyl)-1-oxa-3,8-diaza-spiro[4.5]dec-3-ylmethyl]-benzonitril
[153]3-Benzyl-8-(4-fluor-benzylsulfonyl)-1-oxa-3,8-diaza-spiro[4.5]decan-2-on
[154]3-(4-Fluor-benzyl)-8-(toluol-4-sulfonyl)-1-oxa-3,8-diaza-spiro[4.5]decan-2-on
[155]4-[2-Oxo-8-(toluol-4-sulfonyl)-1-oxa-3,8-diaza-spiro[4.5]dec-3-ylmethyl]-benzonitril
[156]3-(2-Fluor-benzyl)-8-(toluol-4-sulfonyl)-1-oxa-3,8-diaza-spiro[4.5]decan-2-on
[157]3-(3,4-Difluor-benzyl)-8-(toluol-4-sulfonyl)-1-oxa-3,8-diaza-spiro[4.5]decan-2-on
[158]3-(-4-tert-Butyl-benzyl)-8-(toluol-4-sulfonyl)-1-oxa-3,8-diaza spiro[4.5]decan-2-on
[159]8-(Toluol-4-sulfonyl)-3-(4-trifluormethylsulfanyl-benzyl)-1-oxa-3,8-diaza-spiro[4.5]decan-2-on
[160]2-[3-(4-Fluor-benzyl)-2-oxo-1-oxa-3,8-diaza-spiro[4.5]decan-8-sulfonyl]-benzoesäuremethylester
[161]2-[3-(3-Methoxy-benzyl)-2-oxo-1-oxa-3,8-diaza-spiro[4.5]decan-8-sulfonyl]-benzoesäuremethylester
[162]2-[3-(3-Brom-benzyl)-2-oxo-1-oxa-3,8-diaza-spiro[4.5]decan-8-sulfonyl]-benzoesäuremethylester
[163]3-Benzyl-8-(2-methansulfonyl-benzylsulfonyl)-1-oxa-3,8-diaza-spiro[4.5]decan-2-on
[164] 8-(2-Methansulfonyl-benzylsulfonyl)-3-(2-methoxy-5-nitro-benzyl)-1-oxa-3,8-diaza-spiro[4.5]decan-2-on
[165]3-(4-Methyl-benzyl)-2-oxo-1-oxa-3,8-diaza-spiro[4.5]decan-8-sulfonsäure-dimethylamid
[166]4-[8-(7,7-Dimethyl-2-oxo-bicyclo[2.2.1]hept-1-ylmethansulfonyl)-2-oxo-1-oxa-3,8-diaza-spiro[4.5]dec-3-ylmethyl]-benzonitril
[167]3-(4-Fluor-benzyl)-8-(4-methoxy-benzylsulfonyl)-1-oxa-3,8-diaza-spiro[4.5]decan-2-on
[168]3-(4-Methyl-benzyl)-8-(4-trifiuormethoxy-benzylsulfonyl)-1-oxa-3,8-diaza-spiro[4.5]decan-2-on
[169]8-(4-Trifluormethoxy-benzylsulfonyl)-3-(4-trifluormethylsulfanyl-benzyl)-1-oxa-3,8-diaza-spiro[4.5]decan-2-on
[170]8-(Propan-1-sulfonyl)-3-(4-trifluormethylsulfanyl-benzyl)-1-oxa-3,8-d iaza-spiro[4.5]decan-2-on
[171]3-(2-Methoxy-5-nitro-benzyl)-8-(4-propyl-benzylsulfonyl)-1-oxa-3,8-diaza-spiro[4.5]decan-2-on
[172]3-Biphenyl-2-ylmethyl-8-(4-propyl-benzylsulfonyl)-1-oxa-3,8-diaza-spiro[4.5]decan-2-on
[173]8-(3-Brom-benzylsulfonyl)-3-(2-fluor-benzyl)-1-oxa-3,8-diaza-spiro[4.5]decan-2-on
[174]8-(3-Brom-benzylsulfonyl)-3-(3-brom-benzyl)-1-oxa-3,8-diaza-spiro[4.5]decan-2-on
[175]2-{8-[4-(1,1-Dimethyl-propyl)-benzylsulfonyl]-2-oxo-1-oxa-3,8-diaza-spiro[4.5]dec-3-ylmethyl}-benzonitril
[176]3-Benzyl-8-[4-(1,1-dimethyl-propyl)-benzylsulfonyl]-1-oxa-3,8-diaza-spiro[4.5]decan-2-on
[177]8-[4-(1,1-Dimethyl-propyl)-benzylsulfonyl]-3-(4-iod-benzyl)-1-oxa-3,8-diaza-spiro[4.5]decan-2-on
[178]8-[4-(1,1-Dimethyl-propyl)-benzylsulfonyl]-3-(2-methoxy-5-nitro-benzyl)-1-oxa-3,8-diaza-spiro[4.5]decan-2-on
[179]8-[4-(1,1-Dimethyl-propyl)-benzylsulfonyl]-3-(4-trifluormethyl-benzyl)-1-oxa-3,8-diaza-spiro[4.5]decan-2-on
[180]3-Biphenyl-2-ylmethyl-8-[4-(1,1-dimethyl-propyl)-benzylsulfonyl]-1-oxa-3,8-diaza-spiro[4.5]decan-2-on
[181]8-(4,5-Dichlor-thiophen-2-sulfonyl)-3-(3-methoxy-benzy)-1-oxa-3,8-diaza-spiro[4.5]decan-2-on
[182]8-(4,5-Dichlor-thiophen-2-sulfonyl)-3-(4-trifluormethyl-benzyl)-1-oxa-3,8-diaza-spiro[4.5]decan-2-on
[183]3-(3-Brom-benzyl)-8-(4,5-dichlor-thiophen-2-sulfonyl)-1-oxa-3,8-diaza-spiro[4.5]decan-2-on
[184] 2-[8-(4-Methoxy-2,3,6-trimethyl-benzylsulfonyl)-2-oxo-1-oxa-3,8-diaza-spiro[4.5]dec-3-ylmethyl]-benzonitril
[185]3-(2-Fluor-benzyl)-8-(4-methoxy-2,3,6-trimethyl-berizylsulfonyl)-1-oxa-3,8-diaza-spiro[4.5]decan-2-on
[186]3-(4-tert-Butyl-benzyl)-8-[2-(2,2,2-trifluor-acetyl)-1,2,3,4-tetrahydroisochinolin-7-sulfonyl]-1-oxa-3,8-diaza-spiro[4.5]decan-2-on
[187]8-(Butan-1-sulfonyl)-3-(3-methoxy-benzyl)-1-oxa-3,8-diaza-spiro[4.5]decan-2-on
[188]3-(4-Methyl-benzyl)-8-(thiophen-2-sulfonyl)-1-oxa-3,8-diaza-spiro[4.5]decan-2-on
[189]8-(4-Chlor-2,5-dimethyl-benzylsulfonyl)-3-(4-methyl-benzyl)-1-oxa-3,8-diaza-spiro[4.5]decan-2-on
[190]3-Benzyl-8-(4-chlor-2,5-dimethyl-benzylsulfonyl)-1-oxa-3,8-diaza-spiro[4.5]decan-2-on
[191]8-(4-Chlor-2,5-dimethyl-benzylsulfonyl)-3-(4-trifluormethyl-benzyl)-1-oxa-3,8-diaza-spiro[4.5]decan-2-on
[192]3-Biphenyl-2-ylmethyl-8-(4-chlor-2,5-dimethyl-benzylsulfonyl)-1-oxa-3,8-diaza-spiro[4.5]decan-2-on
[193]8-(4-Chlor-2,5-dimethyl-benzylsulfonyl)-3-(4-trifluormethylsulfanyl-benzyl)-1-oxa-3, 8-d iaza-spiro[4.5]decan-2-on
[194]3-(4-Fluor-benzyl)-8-(2-trifluormethyl-benzylsulfonyl)-1-oxa-3,8-diaza-spiro[4.5]decan-2-on
[195]3-(3-Methoxy-benzyl)-8-(2-trifluormethyl-benzylsulfonyl)-1-oxa-3,8-diaza-spiro[4.5]decan-2-on
[196]3-Benzyl-8-(2-methyl-5-nitro-benzylsulfonyl)-1-oxa-3,8-diaza-spiro[4.5]decan-2-on
[197]3-(4-tert-Butyl-benzyl)-8-(2-methyl-5-nitro-benzylsulfonyl)-1-oxa-3,8-diaza-spiro[4.5]decan-2-on
[198]3-(4-tert-Butyl-benzyl)-8-(toluol-3-sulfonyl)-1-oxa-3,8-diaza-spiro[4.5]decan-2-on
[199]8-(Furan-2-carbonyl)-3-(4-trifluormethyl-benzyl)-1-oxa-3,8-diaza-spiro[4.5]decan-2-on
[200]3-(3-Brom-benzyl)-8-(furan-2-carbonyl)-1-oxa-3,8-diaza-spiro[4.5]decan-2-on
[201]4-[8-(Naphthalen-1-carbonyl)-2-oxo-1-oxa-3,8-diaza-spiro[4.5]dec-3-ylmethyl]-benzonitril
[202] 3-(3,4-Difluor-benzyl)-8-(naphthalen-1-carbonyl)-1-oxa-3,8-diaza-spiro[4.5]decan-2-on
[203]3-(3-Brom-benzyl)-8-(naphthalen-1-carbonyl)-1-oxa-3,8-diaza-spiro[4.5]decan-2-on
[204]2-[8-(3-Nitro-benzoyl)-2-oxo-1-oxa-3,8-diaza-spiro[4.5]dec-3-ylmethyl]-benzonitril
[205]4-[8-(3-Nitro-benzoyl)-2-oxo-1-oxa-3,8-diaza-spiro[4.5]dec-3-ylmethyl]-benzonitril
[206]3-(3-Brom-benzyl)-8-(3,5-difluor-benzoyl)-1-oxa-3,8-diaza-spiro[4.5]decan-2-on
[207]8-(2-Benzyloxy-acetyl)-3-(2-fluor-benzyl)-1-oxa-3,8-diaza-spiro[4.5]decan-2-on
[208] 8-(2-Chlor-benzoyl)-3-(2-trifluormethyl-benzyl)-1-oxa-3,8-diaza-spiro[4.5]decan-2-on
[209] 8-(2-Chlor-benzoyl)-3-(4-iod-benzyl)-1-oxa-3,8-diaza-spiro[4.5]decan-2-on
[210]8-(2-Chlor-benzoyl)-3-(2-methoxy-5-nitro-benzyl)-1-oxa-3,8-diaza-spiro[4.5]decan-2-on
[211]3-Biphenyl-2-ylmethyl-8-(2-chlor-benzoyl)-1-oxa-3,8-diaza-spiro[4.5]decan-2-on
[212]8-(2,6-Dichlor-benzoyl)-3-naphthalen-2-ylmethyl-1-oxa-3,8-diaza-spiro[4.5]decan-2-on
[213]3-(4-tert-Butyl-benzyl)-8-(2,6-dichlor-benzoyl)-1-oxa-3,8-diaza-spiro[4.5]decan-2-on
[214]3-(3-Brom-benzyl)-8-(2,6-dichlor-benzoyl)-1-oxa-3,8-diaza-spiro[4.5]decan-2-on
[215]8-(2,6-Dichlor-benzoyl)-3-(4-trifluormethylsulfanyl-benzyl)-1-oxa-3,8-diaza-spiro[4.5]decan-2-on
[216]2-[8-(2-Methyl-benzoyl)-2-oxo-1-oxa-3,8-iaza-spiro[4.5]dec-3-ylmethyl]-benzonitril
[217]8-(2-Methyl-benzoyl)-3-(4-methyl-benzyl)-1-oxa-3,8-diaza-spiro[4.5]decan-2-on
[218]8-(2-Methyl-benzoyl)-3-(2-trifluormethyl-benzyl)-1-oxa-3,8-diaza-spiro[4.5]decan-2-on
[219]3-(2-Methoxy-5-nitro-benzyl)-8-(2-methyl-benzoyl)-1-oxa-3,8-diaza-spiro[4.5]decan-2-on
[220]3-(3,4-Difluor-benzyl)-8-(2-methyl-benzoyl)-1-oxa-3,8-diaza-spiro[4.5]decan-2-on
[221]8-(2-Methyl-benzoyl)-3-(4-trifluormethyl-benzyl)-1-oxa-3,8-diaza-spiro[4.5]decan-2-on
[222]8-(2-Methyl-benzoyl)-3-(4-trifluormethylsulfanyl-benzyl)-1-oxa-3,8-diaza-spiro[4.5]decan-2-on
[223]8-(3-Brom-benzoyl)-3-(4-fluor-benzyl)-1-oxa-3,8-diaza-spiro[4.5]decan-2-on
[224]8-(3-Brom-benzoyl)-3-(2-trifluormethyl-benzyl)-1-oxa-3,8-diaza-spiro[4.5]decan-2-on
[225]8-(3-Brom-benzoyl)-3-(2-fluor-benzyl)-1-oxa-3,8-diaza-spiro[4.5]decan-2-on
[226]8-(3-Brom-benzoyl)-3-(3,4-difluor-benzyl)-1-oxa-3,8-diaza-spiro[4.5]decan-2-on
[227]8-(3-Brom-benzoyl)-3-naphthalen-2-ylmethyl-1-oxa-3,8-diaza-spiro[4.5]decan-2-on
[228]8-(3-Brom-benzoyl)-3-(4-tert-butyl-benzyl)-1-oxa-3,8-diaza-spiro[4.5]decan-2-on
[229]3-Benzyl-8-(3-fluor-benzoyl)-1-oxa-3,8-diaza-spiro[4.5]decan-2-on
[230]8-(3-Fluor-benzoyl)-3-(2-fluor-benzyl)-1-oxa-3,8-diaza-spiro[4.5]decan-2-on
[231]3-(3,4-Difluor-benzyl)-8-(3-fluor-benzoyl)-1-oxa-3,8-diaza-spiro[4.5]decan-2-on
[232]8-(3-Fluor-benzoyl)-3-(4-trifluormethyl-benzyl)-1-oxa-3,8-diaza-spiro[4.5]decan-2-on
[233]3-(3-Brom-benzyl)-8-(3-fluor-benzoyl)-1-oxa-3,8-diaza-spiro[4.5]decan-2-on
[234]8-(3-Chlor-benzoyl)-3-(3,5-dimethyl-benzyl)-1-oxa-3,8-diaza-spiro[4.5]decan-2-on
[235]3-Benzyl-8-(3-chlor-benzoyl)-1-oxa-3,8-diaza-spiro[4.5]decan-2-on
[236]4-[8-(3,4-Dichlor-benzoyl)-2-oxo-1-oxa-3,8-diaza-spiro[4.5]dec-3-ylmethyl]-benzonitril
[237]3-(4-lod-benzyl)-8-(3-methoxy-benzoyl)-1-oxa-3,8-diaza-spiro[4.5]decan-2-on
[238]3-(2-Fluorbenzyl)-8-(3-methoxy-benzoyl)-1-oxa-3,8-diaza-spiro[4.5]decan-2-on
[239]3-(4-tert-Butyl-benzyl)-8-(3-methoxy-benzoyl)-1-oxa-3,8-diaza-spiro[4.5]decan-2-on
[240]3-Benzyl-8-(3-methyl-benzoyl)-1-oxa-3,8-diaza-spiro[4.5]decan-2-on
[241]3-(2-Methoxy-5-nitro-benzyl)-8-(3-methyl-benzoyl)-1-oxa-3,8-diaza-spiro[4.5]decan-2-on
[242]3-(4-tert-Butyl-benzyl)-8-(3-methyl-benzoyl)-1-oxa-3,8-diaza-spiro[4.5]decan-2-on
[243]8-(3-Methyl-benzoyl)-3-(4-trifluormethyl-benzyl)-1-oxa-3,8-diaza-spiro[4.5]decan-2-on
[244]3-(3,4-Difluor-benzyl)-8-(2-phenyl-butyryl)-1-oxa-3,8-diaza-spiro[4.5]decan-2-on
[245]3-Benzyl-8-[3-(2-chlor-phenyl)-5-methyl-isoxazol-4-carbonyl]-1-oxa-3,8-diaza-spiro[4.5]decan-2-on
[246]4-[8-(2,3-Dichlor-benzoyl)-2-oxo-1-oxa-3,8-diaza-spiro[4.5]dec-3-ylmethyl]-benzonitril
[247]8-[2-(4-Chlor-phenyl)-acetyl]-3-(3,4-difluor-benzyl)-1-oxa-3,8-diaza-spiro[4.5]decan-2-on
[248]3-Biphenyl-2-ylmethyl-8-[2-(4-chlor-phenyl)-acetyl]-1-oxa-3,8-diaza-spiro[4.5]decan-2-on
[249]8-[2-(4-Chlor-phenyl)-acetyl]-3-(4-trifluormethylsulfanyl-benzyl)-1-oxa-3,8-diaza-spiro[4.5]decan-2-on
[250]3-(4-tert-Butyl-benzyl)-8-[3-(2-chlor-phenyl)-acryloyl]-1-oxa-3,8-diaza-spiro[4.5]decan-2-on
[251]3-Biphenyl-2-ylmethyl-8-[3-(2-chlor-phenyl)-acryloyl]-1-oxa-3,8-diaza-spiro[4.5]decan-2-on
[252]3-(3-Brom-benzyl)-8-[3-(2-chlor-phenyl)-acryloyl]-1-oxa-3,8-diaza-spiro[4.5]decan-2-on
[253]4-[8-(2,3-Difluor-benzoyl)-2-oxo-1-oxa-3,8-diaza-spiro[4.5]dec-3-ylmethyl]-benzonitril
[254]8-(2,3-Difluor-benzoyl)-3-(2-trifluormethyl-benzyl)-1-oxa-3,8-diaza-spiro[4.5]decan-2-on
[255]8-(2,3-Difluor-benzoyl)-3-(4-iod-benzyl)-1-oxa-3,8-diaza-spiro[4.5]decan-2-on
[256]2-[2-Oxo-8-(2-propyl-pentanoyl)-1-oxa-3,8-diaza-spiro[4.5]dec-3-ylmethyl]-benzonitril
[257]3-(3,4-Difluor-benzyl)-8-(2-propyl-pentanoyl)-1-oxa-3,8-diaza-spiro[4.5]decan-2-on
[258]8-(2-Chlor-4-nitro-benzoyl)-31-(4-methyl-benzyl)-1-oxa-3,8-diaza-spiro[4.5]decan-2-on
[259]8-(2-Chlor-4-nitro-benzoyl)-3I-(2-trifluormethyl-benzyl)-1-oxa-3,8-diaza-spiro[4.5]decan-2-on
[260]3-(4-tert-Butyl-benzyl)-8-(2-chlor-4-nitro-benzoyl)-1-oxa-3,8-diaza-spiro[4.5]decan-2-on
[261]8-(2-Chlor-4-nitro-benzoyl)-31-(4-trifluormethyl-benzyl)-1-oxa-3,8-diaza-spiro[4.5]decan-2-on
[262]3-Biphenyl-2-ylmethyl-8-(2-chlor-4-nitro-benzoyl)-1-oxa-3,8-diaza-spiro[4.5]decan-2-on
[263]3-(3-Brom-benzyl)-8-(2-chlor-4-nitro-benzoyl)-1-oxa-3,8-diaza-spiro[4.5]decan-2-on
[264]8-(2-Chlor-4-nitro-benzoyl)-3I-(4-trifluormethylsulfanyl-benzyl)-1-oxa-3,8-diaza-spiro[4.5]decan-2-on
[265]8-(2-Chlor-pyridin-3-carbonyl)-3-(4-fluor-benzyl)-1-oxa-3,8-diaza-spiro[4.5]decan-2-on
[266]2-[8-(2-Chlor-pyridin-3-carbonyl)-2-oxo-1-oxa-3,8-diaza-spiro[4.5]dec-3-ylmethyl]-benzonitril
[267]8-(2-Chlor-pyridin-3-carbonyl)-3-(4-methyl-benzyl)-1-oxa-3,8-diaza-spiro[4.5]decan-2-on
[288]8-(2-Chlor-pyridin-3-carbonyl)-3-(4-trifluormethyl-benzyl)-1-oxa-3,8-diaza-spiro[4.5]decan-2-on
[269]3-(3-Methoxy-benzyl)-8-(2-methylsulfanyl-pyridin-3-carbonyl)-1-oxa-3,8-diaza-spiro[4.5]decan-2-on
[270]3-(4-Methyl-benzyl)-8-(2-methylsulfanyl-pyridin-3-carbonyl)-1-oxa-3,8-diaza-spiro[4.5]decan-2-on
[271]3-(4-lod-benzyl)-8-(2-methylsulfanyl-pyridin-3-carbonyl)-1-oxa-3,8-diaza-spiro[4.5]decan-2-on
[272] 3-(3,4-Difluor-benzyl)-8-(2-methylsulfanyl-pyridin-3-carbonyl)-1-oxa-3,8-diaza-spiro[4.5]decan-2-on
[273]3-(4-tert-Butyl-benzyl)-8-(2-methylsulfanyl-pyridin-3-carbonyl)-1-oxa-3,8-diaza-spiro[4.5]decan-2-on
[274] 8-(2-Methylsulfanyl-pyridin-3-carbonyl)-3-(4-trifluormethyl-benzyl)-1-oxa-3,8-diaza-spiro[4.5]decan-2-on
[275]8-(2-Ethylsulfanyl-pyridin-3-carbonyl)-3-(4-fluor-benzyl)-1-oxa-3,8-diaza-spiro[4.5]decan-2-on
[276]3-[8-(2-Ethylsulfanyl-pyridin-3-carbonyl)-2-oxo-1-oxa-3,8-diaza-spiro[4.5]dec-3-ylmethyl]-benzonitril
[277]2-[8-(2-Ethylsulfanyl-pyridin-3-carbonyl)-2-oxo-1-oxa-3,8-diaza-spiro[4.5]dec-3-ylmethyl]-benzonitril
[278]8-(2-Ethylsulfanyl-pyridin-3-carbonyl)-3-(4-methyl-benzyl)-1-oxa-3,8-diaza-spiro[4.5]decan-2-on
[279]3-Benzyl-8-(2-ethylsulfanyl-pyridin-3-carbonyl)-1-oxa-3,8-diaza-spiro[4.5]decan-2-on
[280]8-(2-Ethylsulfanyl-pyridin-3-carbonyl)-3-(2-fluor-benzyl)-1-oxa-3,8-diaza-spiro[4.5]decan-2-on
[281]4-[8-(6-Chlor-pyridin-3-carbonyl)-2-oxo-1-oxa-3,8-diaza-spiro[4.5]dec-3-ylmethyl]-benzonitril
[282]8-(6-Chlor-pyridin-3-carbonyl)-3-(2-trifluormethyl-benzyl)-1-oxa-3,8-diaza-spiro[4.5]decan-2-on
[283]8-(6-Chlor-pyridin-3-carbonyl)-3-(4-iod-benzyl)-1-oxa-3,8-diaza-spiro[4.5]decan-2-on
[284]3-(4-tert-Butyl-benzyl)-8-(6-chlor-pyridin-3-carbonyl)-1-oxa-3,8-diaza-spiro[4.5]decan-2-on
[285]3-Biphenyl-2-ylmethyl-8-(6-chlor-pyridin-3-carbonyl)-1-oxa-3,8-diaza-spiro[4.5]decan-2-on
[286]3-(3-Methoxy-benzyl)-8-(4-trifluormethoxy-benzoyl)-1-oxa-3,8-diaza-spiro[4.5]decan-2-on
[287]3-(3,4-Difluor-benzyl)-8-(4-trifluormethoxy-benzoyl)-1-oxa-3,8-diaza-spiro[4.5]decan-2-on
[288]8-[3-(2-Chlor-6-fluor-phenyl)-5-methyl-isoxazol-4-carbonyl]-3-(4-fluorbenzyl)-1-oxa-3,8-diaza-spiro[4.5]decan-2-on
[289]8-[3-(2-Chlor-6-fluor-phenyl)-5-methyl-isoxazol-4-carbonyl]-3-(3-methoxybenzyl)-1-oxa-3,8-diaza-spiro[4.5]decan-2-on
[290]8-[3-(2-Chlor-6-fluor-phenyl)-5-methyl-isoxazol-4-carbonyl]-3-(4-trifluormethyl-benzyl)-1-oxa-3,8-diaza-spiro[4.5]decan-2-on
[291]8-[3-(2-Chlor-6-fluor-phenyl)-5-methyl-isoxazol-4-carbonyl]-3-(4-trifluormethylsulfanyl-benzyl)-1-oxa-3,8-diaza-spiro[4.5]decan-2-on
[292]3-(4-Methyl-benzyl)-8-(3-trifluormethoxy-benzoyl)-1-oxa-3,8-diaza-spiro[4.5]decan-2-on
[293]3-(4-lod-benzyl)-8-(3-trifluormethoxy-benzoyl)-1-oxa-3,8-diaza-spiro[4.5]decan-2-on
[294]3-(3,4-Difluor-benzyl)-8-(3-trifluormethoxy-benzoyl)-1-oxa-3,8-diaza-spiro[4.5]decan-2-on
[295]3-Naphthalen-2-ylmethyl-8-(3-trifluormethoxy-benzoyl)-1-oxa-3,8-diaza-spiro[4.5]decan-2-on
[296]4-[8-(Isoxazol-5-carbonyl)-2-oxo-1-oxa-3,8-diaza-spiro[4.5]dec-3-ylmethyl]-benzonitril
[297]8-(Isoxazol-5-carbonyl)-3-(2-trifluormethyl-benzyl)-1-oxa-3,8-diaza-spiro[4.5]decan-2-on
[298]8-(Isoxazol-5-carbonyl)-3-(2-methoxy-5-nitro-benzyl)-1-oxa-3,8-diaza-spiro[4.5]decan-2-on
[299]3-(3-Brom-benzyl)-8-(isoxazol-5-carbonyl)-1-oxa-3,8-diaza-spiro[4.5]decan-2-on
[300]2-[8-(2-Chlor-6-fluor-benzoyl)-2-oxo-1-oxa-3,8-diaza-spiro[4.5]dec-3-ylmethyl]-benzonitril
[301]4-[8-(2-Chlor-6-fluor-benzoyl)-2-oxo-1-oxa-3,8-diaza-spiro[4.5]dec-3-ylmethyl]-benzonitril
[302]8-(2-Chlor-6-fluor-benzoyl)-3-(4-methyl-benzyl)-1-oxa-3,8-diaza-spiro[4.5]decan-2-on
[303]3-Biphenyl-2-ylmethyl-8-(2-chlor-6-fluor-benzoyl)-1-oxa-3,8-diaza-spiro[4.5]decan-2-on
[304]8-(2,5-Dimethyl-furan-3-carbonyl)-3-(3-methoxy-benzyl)-1-oxa-3,8-diaza-spiro[4.5]decan-2-on
[305]4-[8-(2,5-Dimethyl-furan-3-carbonyl)-2-oxo-1-oxa-3,8-diaza-spiro[4.5]dec-3-ylmethyl]-benzonitril
[306]4-[8-(4-Brom-3-methyl-benzoyl)-2-oxo-1-oxa-3,8-diaza-spiro[4.5]dec-3-ylmethyl]-benzonitril
[307]8-(4-Brom-3-methyl-benzoyl)-3-(4-methyl-benzyl)-1-oxa-3,8-diaza-spiro[4.5]decan-2-on
[308]3-Benzyl-8-(4-brom-3-methyl-benzoyl)-1-oxa-3,8-diaza-spiro[4.5]decan-2-on
[309]8-(4-Brom-3-methyl-benzoyl)-3-(2-trifluormethyl-benzyl)-1-oxa-3,8-diaza-spiro[4.5]decan-2-on
[310]8-(4-Brom-3-methyl-benzoyl)-3-(3,4-difluor-benzyl)-1-oxa-3,8-diaza-spiro[4.5]decan-2-on
[311]8-(4-Brom-3-methyl-benzoyl)-3-(4-tert-butyl-benzyl)-1-oxa-3,8-diaza-spiro[4.5]decan-2-on
[312]3-Biphenyl-2-ylmethyl-8-(4-brom-3-methyl-benzoyl)-1-oxa-3,8-diaza-spiro[4.5]decan-2-on
[313]8-(4-Brom-3-methyl-benzoyl)-3-(4-trifluormethylsulfanyl-benzyl)-1-oxa-3,8-diaza-spiro[4.5]decan-2-on
[314]8-(2,6-Difluor-3-methyl-benzoyl)-3-(3-methoxy-benzyl)-1-oxa-3,8-diaza-spiro[4.5]decan-2-on
[315]2-[8-(2,6-Difluor-3-methyl-benzoyl)-2-oxo-1-oxa-3,8-diaza-spiro[4.5]dec-3-ylmethyl]-benzonitril
[316]4-[8-(2,6-Difluor-3-methyl-benzoyl)-2-xo-1-oxa-3,8-diaza-spiro[4.5]dec-3-ylmethyl]-benzonitril
[317]8-(2,6-Difluor-3-methyl-benzoyl)-3-(2-trifluormethyl-benzyl)-1-oxa-3,8-diaza-spiro[4.5]decan-2-on
[318]8-(2,6-Difluor-3-methyl-benzoyl)-3-(2-fluor-benzyl)-1-oxa-3,8-diaza-spiro[4.5]decan-2-on
[319]8-(2-Chlor-6-fluor-3-methyl-benzoyl)-3-(4-fluor-benzyl)-1-oxa-3,8-diaza-spiro[4.5]decan-2-on
[320]8-(2-Chlor-6-fluor-3-methyl-benzoyl)-3-(3-methoxy-benyl)-oxa-3,8-diaza-spiro[4.5]decan-2-on
[321]8-(2-Chlor-6-fluor-3-methyl-benzoyl)-3-(4-trifluormethylsulfanyl-benzyl)-1-oxa-3,8-diaza-spiro[4.5]decan-2-on
[322]3-Biphenyl-2-ylmethyl-8-(6-chlor-2-fluor-3-methyl-benzoyl)-1-oxa-3,8-diaza-spiro[4.5]decan-2-on
[323]3-Benzyl-8-(3-difluormethylsulfanyl-benzoyl)-1-oxa-3,8-diaza-spiro[4.5]decan-2-on
[324]8-(3-Difluormethylsulfanyl-benzoyl)-3-naphthalen-2-ylmethyl-1-oxa-3,8-diaza-spiro[4.5]decan-2-on
[325]8-(3-Difluormethylsulfanyl-benzoyl)-3-(4-trifluormethyl-benzyl)-1-oxa-3,8-diaza-spiro[4.5]decan-2-on
[326]3-(3-Brom-benzyl)-8-(3-difluormethylsulfanyl-benzoyl)-1-oxa-3,8-diaza-spiro[4.5]decan-2-on
[327]3-[8-(3-Chlor-2-fluor-benzoyl)-2-oxo-1-oxa-3,8-diaza-spiro[4.5]dec-3-ylmethyl]-benzonitril
[328]8-(3-Chlor-2-fluor-benzoyl)-3-(4-iod-benzyl)-1-oxa-3,8-diaza-spiro[4.5]decan-2-on
[329]8-(3-Chlor-2-fluor-benzoyl)-3-naphthalen-2-ylmethyl-1-oxa-3,8-diaza-spiro[4.5]decan-2-on
[330]3-(3-Methoxy-benzyl)-8-(4-trifluormethylsulfanyl-benzoyl)-1-oxa-3,8-diaza-spiro[4.5]decan-2-on
[331]2-[2-Oxo-8-(4-trifluormethylsulfanyl-benzoyl)-1-oxa-3,8-diaza-spiro[4.5]dec-3-ylmethyl]-benzonitril
[332]4-[2-Oxo-8-(4-trifluormethylsulfanyl-benzoyl)-1-oxa-3,8-diaza-spiro[4.5]dec-3-ylmethyl]-benzonitril
[333]3-(4-lod-benzyl)-8-(4-trifluormethylsulfanyl-benzoyl)-1-oxa-3,8-diaza-spiro[4.5]decan-2-on
[334]3-Naphthalen-2-ylmethyl-8-(4-trifluormethylsulfanyl-benzoyl)-1-oxa-3,8-diaza-spiro[4.5]decan-2-on
[335]3-(4-Trifluormethyl-benzyl)-8-(4-trifluomlethylsulfanyl-benzoyl)-1-oxa-3,8-diaza-spiro[4.5]decan-2-on
[336]8-(2-Chlor-5-trifluormethyl-benzoyl)-3-(2-thfluormethyl-benzyl)-1-oxa-3,8-diaza-spiro[4.5]decan-2-on
[337]8-(2-Chlor-5-trifluormethyl-benzoyl)-3-(2-fluor-benzyl)-1-oxa-3,8-diaza-spiro[4.5]decan-2-on
[338]8-(2-Chlor-5-trifluormethyl-benzoyl)-3-(3,4-difluor-benzyl)-1-oxa-3,8-diaza-spiro[4.5]decan-2-on
[339]8-(2,3-Difluor-4-methyl-benzoyl)-3-(3-methoxy-benzyl)-1-oxa-3,8-diaza-spiro[4.5]decan-2-on
[340]4-[8-(2,3-Difluor-4-methyl-benzoyl)-2-oxo-1-oxa-3,8-diaza-spiro[4.5]dec-3-ylmethyl]-benzonitril
[341]3-Benzyl-8-(2,3-difluor-4-methyl-benzoyl)-1-oxa-3,8-diaza-spiro[4.5]decan-2-on
[342]8-(2,3-Difluor-4-methyl-benzoyl)-3-(4-iod-benzyl)-1-oxa-3,8-diaza-spiro[4.5]decan-2-on
[343]3-(3,4-Difluor-benzyl)-8-(2,3-difluor-4-methyl-benzoyl)-1-oxa-3,8-diaza-spiro[4.5]decan-2-on
[344]8-(2,3-Difluor-4-methyl-benzoyl)-3-naphthalen-2-ylmethyl-1-oxa-3,8-diaza-spiro[4.5]decan-2-on
[345]3-Biphenyl-2-ylmethyl-8-(2,3-difluor-4-methyl-benzoyl)-1-oxa-3,8-diaza-spiro[4.5]decan-2-on
[346]3-(3-Brorn-benzyl)-8-(2,3-difluor-4-methyl-benzoyl)-1-oxa-3,8-diaza-spiro[4.5]decan-2-on
[347]3-Benzyl-8-[2-(2-methoxy-ethoxy)-acetyl]-1-oxa-3,8-diaza-spiro[4.5]decan-2-on
[348]3-(2-Fluor-benzyl)-8-[2-(2-methoxy-ethoxy)-acetyl]-1-oxa-3,8-diaza-spiro[4.5]decan-2-on
[349]3-(3,4-Difluor-benzyl)-8-[2-(2-methoxy-ethoxy)-acetyl]-1-oxa-3,8-diaza-spiro[4.5]decan-2-on
[350]3-(4-tert-Butyl-benzyl)-8-[2-(2-methoxy-ethoxy)-acetyl]-1-oxa-3,8-diaza-spiro[4.5]decan-2-on
[351]8-[2-(2-Methoxy-ethoxy)-acetyl]-3-(4-trifluormethylsulfanyl-benzyl)-1-oxa-3,8-diaza-spiro[4.5]decan-2-on
[352]8-(1-Acetyl-piperidin-4-carbonyl)-3-(3,4-difluor-benzyl)-1-oxa-3,8-diaza-spiro[4.5]decan-2-on
[353] 4-{8-[2-(3-Chlor-phenoxy)-acetyl]-2-oxo-1-oxa-3,8-diaza-spiro[4.5]dec-3-ylmethyl}-benzonitril
[354]8-[2-(3-Chlor-phenoxy)-acetyl]-3-(3,4-difluor-benzyl)-1-oxa-3,8-diaza-spiro[4.5]decan-2-on
[355]3-(3-Brom-benzyl)-8-[2-(3-chlor-phenoxy)-acetyl]-1-oxa-3,8-diaza-spiro[4.5]decan-2-on
[356] 8-[2-(3-Chlor-phenoxy)-acetyl]-3-(4-trifluormethylsulfanyl-benzyl)-1-oxa-3,8-diaza-spiro[4.5]decan-2-on
[357]4-[3-(3-Methoxy-benzyl)-2-oxo-1-oxa-3,8-diaza-spiro[4.5]decan-8-carbonyl]-benzylsulfonamid
[358]N-{4-[3-(3,4-Difluor-benzyl)-2-oxo-1-oxa-3,8-diaza-spiro[4.5]decan-8-carbonyl]-phenyl}-acetamid
[359]8-(3-Dimethylamino-benzoyl)-3-(3,5-dimethyl-benzyl)-1-oxa-3,8-diaza-spiro[4.5]decan-2-on
[360]3-(4-tert-Butyl-benzyl)-8-(3-dimethylamino-benzoyl)-1-oxa-3,8-diaza-spiro[4.5]decan-2-on
[361]3-(3-Brom-benzyl)-8-(3-dimethylamino-benzoyl)-1-oxa-3,8-diaza-spiro[4.5]decan-2-on
[362]3-(4-Methyl-benzyl)-8-(4-phenoxy-butyryl)-1-oxa-3,8-diaza-spiro[4.5]decan-2-on
[363]8-(4-Phenoxy-butyryl)-3-(2-trifluormethyl-benzyl)-1-oxa-3,8-diaza-spiro[4.5]decan-2-on
[364]3-(2-Fluor-benzyl)-8-(4-phenoxy-butyryl)-1-oxa-3,8-diaza-spiro[4.5]decan-2-on
[365]8-(2,3-Dimethyl-benzoyl)-3-(4-fluor-benzyl)-1-oxa-3,8-diaza-spiro[4.5]decan-2-on
[366]3-[8-(2,3-Dimethyl-benzoyl)-2-oxo-1-oxa-3,8-diaza-spiro[4.5]dec-3-ylmethyl]-benzonitril
[367]8-(2,3-Dimethyl-benzoyl)-3-(3,5-dimethyl-benzyl)-1-oxa-3,8-diaza-spiro[4.5]decan-2-on
[368]8-(2,3-Dimethyl-benzoyl)-3-(4-methyl-benzyl)-1-oxa-3,8-diaza-spiro[4.5]decan-2-on
[369]8-(2,3-Dimethyl-benzoyl)-3-(2-trifluormethyl-benzyl)-1-oxa-3,8-diaza-spiro[4.5]decan-2-on
[370]8-(2,3-Dimethyl-benzoyl)-3-(2-methoxy-5-nitro-benzyl)-1-oxa-3,8-diaza-spiro[4.5]decan-2-on
[371]8-(2,3-Dimethyl-benzoyl)-3-(4-trifluormethyl-benzyl)-1-oxa-3,8-diaza-spiro[4.5]decan-2-on
[372]3-Biphenyl-2-ylmethyl-8-(2,3-dimethyl-benzoyl)-1-oxa-3,8-diaza-spiro[4.5]decan-2-on
[373]4-[8-(5-Methyl-2-phenyl-2H-[1,2,3]triazol-4-carbonyl)-2-oxo-1-oxa-3,8-diaza-spiro[4.5]dec-3-ylmethyl]-benzonitril
[374]3-Benzyl-8-(5-methyl-2-phenyl-2H-[1,2,3]triazol-4-carbonyl)-1-oxa-3,8-diaza-spiro[4.5]decan-2-on
[375]3-(3,4-Difluor-benzyl)-8-(5-methyl-2-phenyl-2H-[1,2,3]triazol-4-carbonyl)-1-oxa-3,8-diaza-spiro[4.5]decan-2-on
[376]8-(5-Methyl-2-phenyl-2H-[1,2,3]triazol-4-carbonyl)-3-naphthalen-2-ylmethyl-1-oxa-3,8-diaza-spiro[4.5]decan-2-on
[377]3-Biphenyl-2-ylmethyl-8-(5-methyl-2-phenyl-2H-[1,2,3]triazol-4-carbonyl)-1-oxa-3,8-diaza-spiro[4.5]decan-2-on
[378]3-(4-tert-Butyl-benzyl)-8-[3-(2,5-dichlor-phenyl)-5-methyl-isoxazol-4-carbonyl]-1-oxa-3,8-diaza-spiro[4.5]decan-2-on
[379]3-[2-Oxo-8-(2-phenoxy-pyridin-3-carbonyl)-1-oxa-3,8-diaza-spiro[4.5]dec-3-ylmethyl]-benzonitril
[380]2-[2-Oxo-8-(2-phenoxy-pyridin-3-carbonyl)-1-oxa-3,8-diaza-spiro[4.5]dec-3-ylmethyl]-benzonitril
[381]3-(4-Fluor-benzyl)-8-(pyridin-2-carbonyl)-1-oxa-3,8-diaza-spiro[4.5]decan-2-on
[382]3-(3-Methoxy-benzyl)-8-(pyridin-2-carbonyl)-2-oxa-3,8-diaza-spiro[4.5]decan-2-on
[383]3-(3,5-Dimethyl-benzyl)-8-(pyridin-2-carbonyl)-1-oxa-3,8-diaza-spiro[4.5]decan-2-on
[384]2-[2-Oxo-8-(pyridin-2-carbonyl)-1-oxa-3,8-diaza-spiro[4.5]dec-3-ylmethyl]-benzonitril
[385]3-(2-Fluor-benzyl)-8-(pyridin-2-carbonyl)-1-oxa-3,8-diaza-spiro[4.5]decan-2-on
[386]3-(3,4-Difluor-benzyl)-8-(pyridin-2-carbonyl)-1-oxa-3,8-diaza-spiro[4.5]decan-2-on
[387]3-Biphenyl-2-ylmethyl-8-(pyridin-2-carbonyl)-1-oxa-3,8-diaza-spiro[4.5]decan-2-on
[388]4-[8-(3-Chlor-thiophen-2-carbonyl)-2-oxo-1-oxa-3,8-diaza-spiro[4.5]dec-3-ylmethyl]-benzonitril
[389]8-(3-Chlor-thiophen-2-Carbonyl)-3-(3,4-difluor-benzyl)-1-oxa-3,8-diaza-spiro[4.5]decan-2-on
[390]3-Biphenyl-2-ylmethyl-8-(3-chlor-thiophen-2-carbonyl)-1-oxa-3,8-diaza-spiro[4.5]decan-2-on
[391]8-[1-(4-Chlor-phenyl)-5-trifluormethyl-1H-pyrazol-4-carbonyl]-3-(2-fluor benzyl)-1-oxa-3,8-diaza-spiro[4.5]decan-2-on
[392]8-[1-(4-Chlor-phenyl)-5-trifluormethyl-1H-pyrazol-4-carbonyl]-3-(3,4-difluor-benzyl)-1-oxa-3,8-diaza-spiro[4.5]decan-2-on
[393]8-[1-(4-Chlor-phenyl)-5-trifluormethyl-1H-pyrazol-4-carbonyl]-3-(4-trifluormethylsulfanyl-benzyl)-1-oxa-3,8-diaza-spiro[4.5]decan-2-on
[394]3-(4-tert-Butyl-benzyl)-8-(5-tert-butyl-2-methyl-2H-pyrazol-3-carbonyl)-1-oxa-3,8-diaza-spiro[4.5]decan-2-on
[395]3-[8-(5-Methyl-isoxazol-3-carbonyl)-2-oxo-1-oxa-3,8-diaza-spiro[4.5]dec-3-ylmethyl]-benzonitril
[396]3-(3,5-Dimethyl-benzyl)-8-(5-methyl-isoxazol-3-carbonyl)-1-oxa-3,8-diaza-spiro[4.5]decan-2-on
[397]2-[8-(5-Methyl-isoxazol-3-carbonyl)-2-oxo-1-oxa-3,8-diaza-spiro[4.5]dec-3-ylmethyl]-benzonitril
[398]4-[8-(5-Methyl-isoxazol-3-carbonyl)-2-oxo-1-oxa-3,8-diaza-spiro[4.5]dec-3-ylmethyl]-benzonitril
[399]3-Benzyl-8-(5-methyl-isoxazol-3-carbonyl)-1-oxa-3,8-diaza-spiro[4.5]decan-2-on
[400]3-(3,4-Difluor-benzyl)-8-(5-methyl-isoxazol-3-carbonyl)-1-oxa-3,8-diaza-spiro[4.5]decan-2-on
[401]3-Biphenyl-2-ylmethyl-8-(5-methyl-isoxazol-3-carbonyl)-1-oxa-3,8-diaza-spiro[4.5]decan-2-on
[402]8-(4-Methyl-[1,2,3]thiadiazol-5-carbonyl)-3-(2-trifluormethyl-benzyl)-1-oxa-3,8-diaza-spiro[4.5]decan-2-on
[403]3-(4-tert-Butyl-benzyl)-8-(4-methyl-[1,2,3]thiadiazol-5-carbonyl)-1-oxa-3,8-diaza-spiro[4.5]decan-2-on
[404]8-(4-Methyl-[1,2,3]thiadiazol-5-carbonyl)-3-(4-trifluormethylsulfanyl-benzyl)-1-oxa-3,8-diaza-spiro[4.5]decan-2-on
[405]4-[2-Oxo-8-(1-phenyl-5-propyl-1H-pyrazol-4-carbonyl)-1-oxa-3,8-diaza-spiro[4.5]dec-3-ylmethyl]-benzonitri
[406]3-(3,4-Difluor-benzyl)-8-(1-phenyl-5-propyl-1H-pyrazol-4-carbonyl)-1-oxa-3,8-diaza-spiro[4.5]decan-2-on
[407]3-Naphthalen-2-ylmethyl-8-(1-phenyl-5-propyl-1H-pyrazol-4-carbonyl)-1-oxa-3,8-diaza-spiro[4.5]decan-2-on
[408]8-(1-Phenyl-5-propyl-1 H-pyrazol-4-carbonyl)-3-(4-trifluormethylsulfanyl-benzyl)-1-oxa-3,8-diaza-spiro[4.5]decan-2-on
[409]3-[8-(2-Chlor-pyridin-4-carbonyl)-2-oxo-1-oxa-3,8-diaza-spiro[4.5]dec-3-ylmethyl]-benzonitril
[410]8-(2-Chlor-pyridin-4-carbonyl)-3-(3-methoxy-benzyl)-1-oxa-3,8-diaza-spiro[4.5]decan-2-on
[411]8-(5-tert-Butyl-2-methyl-furan-3-carbonyl)-3-(4-fluor-benzyl)-1-oxa-3,8-diaza-spiro[4.5]decan-2-on
[412]3-[8-(5-tert-Butyl-2-methyl-furan-3-carbonyl)-2-oxo-1-oxa-3,8-diaza-spiro[4.5]dec-3-ylmethyl]-benzonitril
[413]2-[8-(5-tert-Butyl-2-methyl-furan-3-carbonyl)-2-oxo-1-oxa-3,8-diaza-spiro[4.5]dec-3-ylmethyl]-benzonitril
[414]4-[8-(5-tert-Butyl-2-methyl-furan-3-carbonyl)-2-oxo-1-oxa-3,8-diaza-spiro[4.5]dec-3-ylmethyl]-benzonitril
[415] 8-(5-tert-Butyl-2-methyl-furan-3-carbonyl)-3-(2-trifluormethyl-benzyl)-1-oxa-3,8-diaza-spiro[4.5]decan-2-on
[416]8-(5-tert-Butyl-2-methyl-furan-3-carbonyl)-3-naphthalen-2-ylmethyl-1-oxa-3,8-diaza-spiro[4.5]decan-2-on
[417]3-Biphenyl-2-ylmethyl-8-(5-tert-butyl-2-methyl-furan-3-carbonyl)-1-oxa-3,8-diaza-spiro[4.5]decan-2-on
[418]3-(3-Brom-benzyl)-8-(5-tert-butyl-2-methyl-furan-3-carbonyl)-1-oxa-3,8-diaza-spiro[4.5]decan-2-on
[419]8-(Benzo[1,2,5]oxadiazol-5-carbonyl)-3-(3,4-difluor-benzyl)-1-oxa-3,8-diaza-spiro[4.5]decan-2-on
[420] 8-(Benzo[1,2,5]oxadiazol-5-carbonyl)-3-(4-trifluormethyl-benzyl)-1-oxa-3,8-diaza-spiro[4.5]decan-2-on
[421]3-[8-(2-Methyl-6-trifluormethyl-pyridin-3-carbonyl)-2-oxo-1-oxa-3,8-diaza-spiro[4.5]dec-3-ylmethyl]-benzonitril
[422]2-[8-(2-Methyl-6-trifluormethyl-pyridin-3-carbonyl)-2-oxo-1-oxa-3,8-diaza-spiro[4.5]dec-3-ylmethyl]-benzonitril
[423]4-[8-(2-Methyl-6-trifluormethyl-pyridin-3-carbonyl)-2-oxo-1-oxa-3,8-diaza-spiro[4.5]dec-3-ylmethyl]-benzonitril
[424]8-(2-Methyl-6-trifluormethyl-pyridin-3-carbonyl)-3-(2-trifluormethyl-benzyl)-1-oxa-3,8-diaza-spiro[4.5]decan-2-on
[425]3-(3,4-Difluor-benzyl)-8-(2-methyl-6-trifluormethyl-pyridin-3-carbonyl)-1-oxa-3,8-diaza-spiro[4.5]decan-2-on
[426]3-(3-Brom-benzyl)-8-(2-methyl-6-trifluormethyl-pyridin-3-carbonyl)-1-oxa-3,8-diaza-spiro[4.5]decan-2-on
[427]8-(6-Chlor-2H-chromen-3-carbonyl)-3-(3,4-difluor-benzyl)-1-oxa-3,8-diaza-spiro[4.5]decan-2-on
[428]8-(2-Chlor-pyridin-4-carbonyl)-3-(2-fluor-benzyl)-1-oxa-3,8-diaza-spiro[4.5]decan-2-on
[429]8-(2-Chlor-pyridin-4-carbonyl)-3-naphthalen-2-ylmethyl-1-oxa-3,8-diaza-spiro[4.5]decan-2-on
[430]8-Acetyl-3-(2-fluor-benzyl)-1-oxa-3,8-diaza-spiro[4.5]decan-2-on
[431]8-Acetyl-3-(3-brom-benzyl)-1-oxa-3,8-diaza-spiro[4.5]decan-2-on
und
[432] 3-(2-Methoxy-5-nitro-benzyl)-8-(3-phenoxy-propyl)-1-oxa-3,8-diaza-spiro[4.5]decan-2-on;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

17. Verfahren zur Herstellung substituierter 1-Oxa-3,8-diazaspiro[4.5]-decan-2-on-Verbindungen der allgemeinen Formel I gemäß einem oder mehreren der Ansprüche 1 bis 16, **dadurch gekennzeichnet, dass** wenigstens eine Verbindung der allgemeinen Formel II, 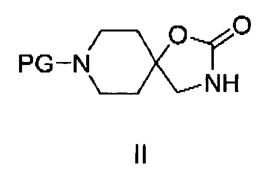 worin PG für eine Schutzgruppe, bevorzugt eine tert-Butyloxy-carbonyl- oder Benzyloxycarbonyl-Gruppe steht, in einem Reaktionsmedium, in Gegenwart wenigstens einer Base, bevorzugt in Gegenwart wenigstens eines Metallhydridsalzes, besonders bevorzugt in Gegenwart von Kalium- und/oder Natriumhydrid, mit wenigstens einer Verbindung der allgemeinen Formel R¹-(CH₂)ₙ-X. worin R¹ und n die Bedeutung gemäß einem oder mehreren der Ansprüche 1 bis 16 haben und X für eine Abgangsgruppe, bevorzugt ein Halogenatom, besonders bevorzugt ein Chlor oder Bromatom steht, zu wenigstens einer Verbindung der allgemeinen Formel III, 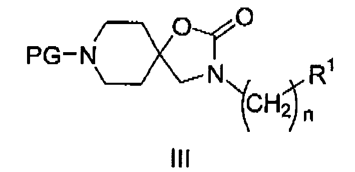 worin R¹, PG und n die vorstehend genannte Bedeutung haben, umgesetzt wird, und diese ggf. gereinigt und/oder isoliert wird,
und wenigstens eine Verbindung der allgemeinen Formel III durch Abspaltung der Schutzgruppe, vorzugsweise der tert-Butoxycarbonyl-Gruppe in einem Reaktionsmedium, vorzugsweise in Gegenwart wenigstens einer Säure oder in Gegenwart wenigstens einer Base oder durch Abspaltung der Benzyloxycarbonyl-Gruppe in einem Reaktionsmedium, vorzugsweise in Gegenwart von Wasserstoff und Katalysator, bevorzugt Palladium auf Kohle, zu wenigstens einer Verbindung der allgemeinen Formel IV, ggf. in Form eines entsprechenden Salzes, bevorzugt in Form eines entsprechenden Hydrochlorids, 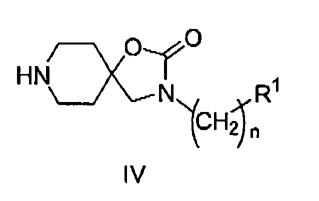 worin R¹ und n die vorstehend genannte Bedeutung haben, umgesetzt wird, und diese ggf. gereinigt und/oder isoliert wird,
und wenigstens eine Verbindung der allgemeinen Formel IV in einem Reaktionsmedium mit wenigstens einem Isothiocyanat der allgemeinen Formel R³-N=C=S, worin R³ die Bedeutung gemäß einem oder mehreren der Ansprüche 1 bis 16 hat, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, zu wenigstens einer Verbindung der allgemeinen Formel I umgesetzt wird, worin R¹ und n die vorstehend genannte Bedeutung haben und R² für-C(=S)-NH-R³, worin R³ die vorstehend genannte Bedeutung hat, steht, und diese ggf. gereinigt und/oder isoliert wird;
oder
wenigstens eine Verbindung der allgemeinen Formel IV in einem Reaktionsmedium mit wenigstens einem Isocyanat der allgemeinen Formel R⁴-N=C=O, worin R⁴ die Bedeutung gemäß einem oder mehreren der Ansprüche 1 bis 16 hat, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, zu wenigstens einer Verbindung der allgemeinen Formel umgesetzt wird, worin R¹ und n die vorstehend genannte Bedeutung haben und R² für -C(=O)-NH-R⁴, worin R⁴ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Sulfonsäure-Derivat der allgemeinen Formel R⁵-S(=O)₂-X, worin R⁵ die Bedeutung gemäß einem oder mehreren der Ansprüche 1 bis 16 hat und X für eine Abgangsgruppe, bevorzugt ein Halogenatom, besonders bevorzugt ein Chlor oder Bromatom steht, zu wenigstens einer Verbindung der allgemeinen Formel I umgesetzt wird, worin R¹ und n die vorstehend genannte Bedeutung haben und R² für -S(=O)₂-R⁵, worin R⁵ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Verbindung der allgemeinen Formel X-(CH₂)-C(=O)-NHR⁶, worin R⁶ die Bedeutung gemäß einem oder mehreren der Ansprüche 1 bis 16 hat und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -(CH₂)-C(=O)-NH-R⁶, worin R⁶ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Verbindung der allgemeinen Formel X-(CH₂)-Dₐₐ-(CH₂)_{bb}-E_{cc}-(CH₂)_{dd}-R⁷, worin R⁷, D, E, aa, bb, cc und dd die Bedeutung gemäß einem oder mehreren der Ansprüche 1 bis 16 haben und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -(CH₂)-Dₐₐ-(CH₂)_{bb}-E_{cc}-(CH₂)_{dd}-R⁷, worin R⁷, D, E. aa, bb, cc und dd die vorstehend genannte Bedeutung haben, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Carbonsäure-Derivat der allgemeinen Formel R⁸-C(=O)-X, worin R⁸ die Bedeutung gemäß einem oder mehreren der Ansprüche 1 bis 16 hat und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für -C(=O)-R⁸, worin R⁸ die vorstehend genannte Bedeutung hat, steht, und diese ggf. gereinigt und/oder isoliert wird;
oder
wenigstens eine Verbindung der allgemeinen Formel IV in einem Reaktionsmedium, in Gegenwart wenigstens eines Kupplungsreagenzes, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einer Carbonsäure der allgemeinen Formel R⁸-C(=O)-OH, worin R⁸ die Bedeutung gemäß einem oder mehreren der Ansprüche 1 bis 16 hat, zu wenigstens einer Verbindung der allgemeinen Formel I umgesetzt wird, worin R¹ und n die vorstehend genannte Bedeutung haben und R² für -C(=O)-R⁸, worin R⁸ die vorstehend genannte Bedeutung hat, steht, und diese ggf. gereinigt und/oder isoliert wird;
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Triethylamin, (4,4)-Dimethylaminopyridin, Pyridin, Diisopropylethylamin und N-Methylmorpholin, mit wenigstens einem Sulfonsäure-Derivat der allgemeinen Formel X-S(=O)₂-NR⁹R¹⁰, worin R⁹ und R¹⁰ die Bedeutung gemäß einem oder mehreren der Ansprüche 1 bis 16 haben und X für eine Abgangsgruppe, bevorzugt ein Halogenatom, besonders bevorzugt ein Chlor- oder Bromatom steht, zu wenigstens einer Verbindung der allgemeinen Formel I umgesetzt wird, worin R¹ und n die vorstehend genannte Bedeutung haben und R² für-S(=O)₂-NR⁹R¹⁰, worin R⁹ und R¹⁰ die vorstehend genannte Bedeutung haben, steht, und diese ggf. gereinigt und/oder isoliert wird.

18. Arzneimittel enthaltend wenigstens eine Verbindung gemäß einem oder mehreren der Ansprüche 1 bis 16 sowie ggf. einen oder mehrere physiologisch verträgliche Hilfsstoffe.

19. Arzneimittel gemäß Anspruch 18 zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz.

20. Arzneimittel gemäß Anspruch 18 zur Prophylaxe und/oder Behandlung von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Wasserspeicher-Krankheiten; Migräne; chronische paroxysmale Hemikranie; Depressionen; Haminkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Katalepsie; Narkolepsie; Diarrhoe; Gastritis; Magengeschwür; Pruritus; Angstzuständen; Panikattacken; Schizophrenie; cerebralen Ischämien; Muskeispasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Unterdrückung des Miktionsreflexes; zur Regulation des kardiovaskulären Systems, bevorzugt zur Vasodilatation der Arterien; zur Lokalanästhesie; zur Vigilanzsteigerung; zur Libidosteigerung; zur Diurese und/oder zur Antinatriurese.

21. Verwendung wenigstens einer Verbindung gemäß einem oder mehreren der Ansprüche 1 bis 16 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz.

22. Verwendung wenigstens einer Verbindung gemäß einem oder mehreren der Ansprüche 1 bis 16 zur Herstellung eines Arzneimittels zur Prophylaxe und/oder Behandlung von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Wasserspeicher-Krankheiten; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Katalepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Unterdrückung des Miktionsreflexes; zur Regulation des kardiovaskulären Systems, bevorzugt zur Vasodilatation der Arterien; zur Lokalanästhesie; zur Vigilanzsteigerung; zur Libidosteigerung; zur Diurese und/oder zur Antinatriurese.

## Claims

1. Substituted 1-oxa-3,8-diazaspiro[4.5]-decan-2-one compounds of the general formula I, 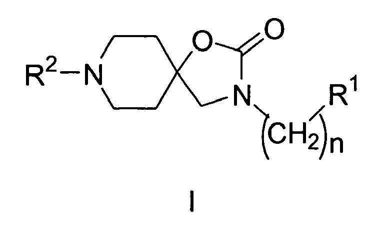 in which
n is equal to 1, 2, 3, 4 or 5;
R¹ denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R² denotes -C(=S)-NH-R³;
-C(=O)-NH-R⁴;
-S(=O)₂-R⁵;
-(CH₂)-C(=O)-NH-R⁶;
denotes -(CH₂)-Dₐₐ-(CH₂)_{bb}-E_{cc}-(CH₂)_{dd}-R⁷ with aa = 0 or 1; bb = 0, 1 or 2; cc = 0 or 1 and dd = 0 or 1; in which D and E mutually independently denote O, S, NH, N(CH₃), N(C₂H₅) or N[CH(CH₃)₂] and wherein the sum of aa and cc does not equal 0;
denotes -C(=O)-R⁸
or denotes -S(=O)₂-NR⁹R¹⁰;
R³ denotes -(CHR¹¹)-(CH₂)_{w}-C(=O)-O-R¹² with w = 0 or 1;
denotes -(CHR¹³)-(CH₂)ₐ-K_{b}-(CH₂)_{c}-L_{d}-R¹⁴ with a = 0, 1 or 2; b = 0 or 1; c = 0, 1 or 2 and d = 0 or 1; in which K and L mutually independently denote O, S, NH, N(CH₃), N(C₂H₅) or N[CH(CH₃)₂];
denotes a linear or branched, saturated or unsaturated, optionally substituted C₁₋₁₀ aliphatic residue;
denotes an unsaturated or saturated, optionally substituted cycloaliphatic residue which may be bridged with 1 or 2 linear or branched, optionally substituted C₁₋₅ alkylene groups;
or denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R⁴ denotes -(CHR¹⁵)-(CH₂)ₑ-M_{f}-(CH₂)_{g}-Pₕ-R¹⁶ with e = 0, 1 or 2; f = 0 or 1; g = 0, 1 or 2 and h = 0 or 1; in which M and P mutually independently denote O, S, NH, N(CH₃), N(C₂H₅) or N[CH(CH₃)₂]
denotes a linear or branched, saturated or unsaturated, optionally substituted C₁₋₁₀ aliphatic residue;
denotes an unsaturated or saturated, optionally substituted cycloaliphatic residue which may be bridged with 1 or 2 linear or branched, optionally substituted C₁₋₅ alkylene groups;
or denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R⁵ denotes -(CHR¹⁷)-(CH₂)ₖQₗ-(CH₂)ₘ-Tₒ-R¹⁸ with k = 0, 1 or 2; I = 0 or 1; m = 0, 1 or 2 and o = 0 or 1; in which Q and T mutually independently denote O, S, NH, N(CH₃), N(C₂H₅) or N[CH(CH₃)₂];
denotes a linear or branched, saturated or unsaturated, optionally substituted C₁₋₁₀ aliphatic residue;
denotes an unsaturated or saturated, optionally substituted cycloaliphatic residue which may be bridged with 1 or 2 linear or branched, optionally substituted C₁₋₅ alkylene groups;
or denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R⁶ denotes a linear or branched, saturated or unsaturated, optionally substituted C₁₋₁₀ aliphatic residue;
denotes an unsaturated or saturated, optionally substituted cycloaliphatic residue which may be bridged with 1 or 2 linear or branched, optionally substituted C₁₋₅ alkylene groups;
or denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R⁷ denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the residue in each case may optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, imidazolyl, indolyl, isoindolyl, benzo[b]furanyl, benzo[b]thiophenyl, thiazolyl, [1,2,3]-thiadiazolyl, [1,2,4]-oxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-phenyl, phenyl and benzyl, wherein in each case the cyclic moiety of the residues -S(=O)₂-NH-phenyl, phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl;
R⁸ denotes (CHR¹⁹)-Vₚ-(CH₂)_{q}-(CH₂)ᵣ-Wₛ-R²⁰ with p = 0 or 1; q = 0, 1 or 2; r = 0, 1 or 2 and s = 0 or 1; in which V and W mutually independently in each case denote O, S, NH, N(CH₃), N(C₂H₅) or N[CH(CH₃)₂];
denotes -(CH=CH)-R²¹;
denotes -(CR²²R²³)-Yₜ-(CR²⁴R²⁵)ᵤ-(CH₂)ᵥ-C(=O)-OR²⁶ with t = 0 or 1, u = 0 or 1 and v = 0 or 1, in which Y denotes O, S, NH, N(CH₃), N(C₂H₅) or N[CH(CH₃)₂];
denotes -(CHR²⁷)-O-C(=O)-R²⁸;
denotes -CH[(CH₂)R²⁹][NH-S(=O)₂-R³⁰];
denotes -CH[(CH₂)R³¹][NH-C(=O)-O-R³²];
denotes a linear or branched, saturated or unsaturated, optionally substituted C₁₋₁₀ aliphatic residue;
denotes an unsaturated or saturated, optionally substituted cycloaliphatic residue which may be bridged with 1 or 2 linear or branched, optionally substituted C₁₋₅ alkylene groups;
or denotes an optionally substituted aryl residue or denotes an optionally substituted heteroaryl residue, wherein the aryl and/or heteroaryl residue may in each case be fused with a saturated or unsaturated, optionally substituted mono- or bicyclic ring system;
R⁹ and R¹⁰, mutually independently, in each case
denote a linear or branched, saturated or unsaturated, optionally substituted C₁₋₁₀ aliphatic residue;
R¹¹, R¹³, R¹⁵, R¹⁷, R¹⁹, R²², R²³, R²⁴, R²⁵ and R²⁶, mutually independently in each case
denote a hydrogen residue
or denote a linear or branched, saturated or unsaturated, optionally substituted C₁₋₁₀ aliphatic residue;
R¹², R²⁸ and R³², mutually independently, in each case
denote a linear or branched, saturated or unsaturated, optionally substituted C₁₋₁₀ aliphatic residue;
R¹⁴, R¹⁶, R¹⁸ and R²⁰, mutually independently, in each case
denote a linear or branched, saturated or unsaturated, optionally substituted C₁₋₁₀ aliphatic residue;
denote an unsaturated or saturated, optionally substituted cycloaliphatic residue which may be bridged with 1 or 2 linear or branched, optionally substituted C₁₋₅ alkylene groups;
or denote an optionally substituted aryl residue or an optionally substituted heteroaryl residue, wherein the aryl and/or heteroaryl residue may in each case be fused with a saturated or unsaturated, optionally substituted mono- or bicyclic ring system;
and
R²¹, R²⁷, R²⁹, R³⁰ and R³¹, mutually independently, in each case
denote an unsaturated or saturated, optionally substituted cycloaliphatic residue which may be bridged with 1 or 2 linear or branched, optionally substituted C₁₋₅ alkylene groups;
or denote an optionally substituted aryl residue or an optionally substituted heteroaryl residue, wherein the aryl and/or heteroaryl residue may in each case be fused with a saturated or unsaturated, optionally substituted mono- or bicyclic ring system;
wherein
the above-stated C₁₋₁₀ aliphatic residues may in each case optionally be substituted with 1, 2, 3, 4 or 5 substituents mutually independently selected from the group consisting of F, Cl, Br, I, -CN, -NO₂, -OH, -SH and -NH₂;
the above-stated cycloaliphatic residues may in each case optionally be substituted with 1, 2, 3, 4 or 5 substituents mutually independently selected from the group consisting of oxo (=O), thioxo (=S), F, Cl, Br, I, -CN, -CF₃, -SF₅, -OH, -O-C₁₋₅₋alkyl, -NH₂, -NO₂, -O-CF₃, -S-CF₃, -S-CF₂H, -S-CFH₂, -SH, -S-C₁₋₅₋alkyl, -C₁₋₅₋alkyl, -C(=O)-OH, -(CH₂)-C(=O)-OH, -C(=O)-O-C₁₋₅₋alkyl, -(CH₂)-C(=O)-O-C₁₋₅₋alkyl, -O-C(=O)-C₁₋₅₋alkyl, -NH-C₁₋₅₋alkyl, -N(-C₁₋₅₋alkyl)₂, -NH-phenyl, -NH-pyridinyl, -N(-C₁₋₅₋alkyl)-phenyl, -N(-C₁₋₅₋alkyl)-pyridinyl, -NH-C(=O)-O-C₁₋₅₋alkyl, -C(=O)-H, -C(=O)-C₁₋₅₋alkyl, -C(=O)-C₁₋₅₋perfluoroalkyl, -C(=O)-NH₂, -C(=O)-NH-C₁₋₅₋alkyl, C(=O)-N-(-C₁₋₅₋alkyl)₂, -S(=O)₂-C₁₋₅₋alkyl, -S(=O)₂₋phenyl, -NH-S(=O)₂-C₁₋₅₋alkyl, -S(=O)₂-NH-C₁₋₅₋alkyl, -S(=O)₂-NH₂, -S(=O)₂-NH-phenyl, cyclohexyl, cyclopentyl, pyridinyl, [1,2,5]-thiadiazolyl, pyridazinyl, -(CH₂)-benzo[b]furanyl, -O-phenyl, -O-benzyl, phenyl and benzyl, wherein in each case the cyclic moiety of the residues -S(=O)₂-NH-phenyl, -NH-phenyl, -NH-pyridinyl, -N(-C₁₋₅₋alkyl)-phenyl, -N(-C₁₋₅₋alkyl)-pyridinyl, pyridinyl, cyclopentyl, [1,2,5]-thiadiazolyl, cyclohexyl, pyridazinyl, -S(=O)₂₋phenyl, -O-phenyl, -O-benzyl, phenyl, -(CH₂)-benzo[b]furanyl and benzyl may be substituted with 1, 2, 3, 4 or 5 substituents mutually independently selected from the group consisting of F, Cl, Br, -OH, -CF₃, -SF₅, -CN, -NO₂, -C₁₋₅₋alkyl, -O-C₁₋₅₋alkyl, -O-CF₃, -S-CF₃, phenyl and -O-benzyl,
the above-stated C₁₋₅₋alkylene groups may in each case optionally be substituted with 1, 2, 3, 4 or 5 substituents mutually independently selected from the group consisting of F, Cl, Br, I, -CN, -NO₂, -OH, -SH and -NH₂,
the rings of the above-stated mono- or polycyclic ring systems may in each case optionally be substituted with 1, 2, 3, 4 or 5 substituents mutually independently selected from the group consisting of oxo (=O), thioxo (=S), F, Cl, Br, I, -CN, -CF₃, -SF₅, -OH, -O-C₁₋₅₋alkyl, -NH₂, -NO₂, -O-CF₃, -S-CF₃, -S-CF₂H, -S-CFH₂, -SH, -S-C₁₋₅₋alkyl, -C₁₋₅₋alkyl, -C(=O)-OH, -(CH₂)-C(=O)-OH; -C(=O)-O-C₁₋₅₋alkyl, -(CH₂)-C(=O)-O-C₁₋₅₋alkyl, -O-C(=O)-C₁₋₅₋alkyl, -NH-C₁₋₅₋alkyl, -N(-C₁₋₅₋alkyl)₂, -NH-phenyl, -NH-pyridinyl, -N(-C₁₋₅₋alkyl)-phenyl, -N(-C₁₋₅₋alkyl)-pyridinyl, -NH-C(=O)-O-C₁₋₅₋alkyl, -C(=O)-H, -C(=O)-C₁₋₅₋alkyl, -C(=O)-C₁₋₅₋perfluoroalkyl, -C(=O)-NH₂, -C(=O)-NH-C₁₋₅₋alkyl, C(=O)-N-(-C₁₋₅₋alkyl)₂, -S(=O)₂-C₁₋₅₋alkyl, -S(=O)₂₋phenyl, -NH-S(=O)₂-C₁₋₅₋alkyl, -S(=O)₂-NH-C₁₋₅₋alkyl, -S(=O)₂-NH₂, -S(=O)₂-NH-phenyl, cyclohexyl, cyclopentyl, pyridinyl, [1 ,2,5]-thiadiazolyl, pyridazinyl, -(CH₂)-benzo[b]furanyl, -O-phenyl, -O-benzyl, phenyl and benzyl, wherein in each case the cyclic moiety of the residues -S(=O)₂-NH-phenyl, -NH-phenyl, -NH-pyridinyl, -N(-C₁₋₅₋alkyl)-phenyl, -N(-C₁₋₅₋alkyl)-pyridinyl, pyridinyl, cyclopentyl, [1,2,5]-thiadiazolyl, cyclohexyl, pyridazinyl, -S(=O)₂₋phenyl, -O-phenyl, -O-benzyl, phenyl, -(CH₂)-benzo[b]furanyl and benzyl may be substituted with 1, 2, 3, 4 or 5 substituents mutually independently selected from the group consisting of F, Cl, Br, -OH, -CF₃, -SF₅, -CN, -NO₂, -C₁₋₅₋alkyl, -O-C₁₋₅₋alkyl, -O-CF₃, -S-CF₃, phenyl and -O-benzyl,
the above-stated aryl or heteroaryl residues may in each case optionally be substituted with 1, 2, 3, 4 or 5 substituents mutually independently selected from the group consisting of F, Cl, Br, I, -CN, -CF₃, -SF₅, -OH, -O-C₁₋₅₋alkyl, -NH₂, -NO₂, -O-CF₃, -S-CF₃, -S-CF₂H, -S-CFH₂, SH, -S-C₁₋₅₋alkyl, -C₁₋₁₀₋alkyl, -C(=O)-OH, -(CH₂)-C(=O)-OH, -C(=O)-O-C₁₋₅₋alkyl, -(CH₂)-C(=O)-O-C₁₋₅₋alkyl, -O-C(=O)-C₁₋₅₋alkyl, -NH-C₁₋₅₋alkyl, -N(-C₁₋₅₋alkyl)₂, -NH-C(=O)-O-C₁₋₅₋alkyl, -NH-C(=O)-C₁₋₅₋alkyl, -C(=O)-H, -C(=O)-C₁₋₅₋alkyl, -C(=O)-C₁₋₅₋perfluoroalkyl, -C(=O)-NH₂, -C(=O)-NH-C₁₋₅₋alkyl, -C(=O)-N-(-C₁₋₅₋alkyl)₂, -S(=O)₂-C₁₋₅₋alkyl, -S(=O)₂₋phenyl, -NH-S(=O)₂-C₁₋₅₋alkyl, -S(=O)₂-NH-C₁₋₅₋alkyl, -S(=O)₂-NH₂, -S(=O)₂-NH-phenyl, cyclohexyl, cyclopentyl, pyridinyl, pyridazinyl, -(CH₂)-benzo[b]furanyl, -O-phenyl, -O-benzyl, phenyl and benzyl, wherein in each case the cyclic moiety of the residues pyridinyl, cyclopentyl, cyclohexyl, pyridazinyl, -S(=O)₂₋phenyl, -S(=O)₂-NH-phenyl, -O-phenyl, -O-benzyl, phenyl, -(CH₂)-benzo[b]furanyl and benzyl may be substituted with 1, 2, 3, 4 or 5 substituents mutually independently selected from the group consisting of F, Cl, Br, -OH, -CF₃, -SF₅, -NO₂, -C₁₋₅₋alkyl, -O-C₁₋₅₋alkyl, -O-CF₃, -S-CF₃, phenyl and -O-benzyl,
the above-stated cycloaliphatic residues are selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and bicyclo[2.2.1]heptyl,
the above-stated heteroaryl residues are selected from the group consisting of 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, oxazolyl, isoxazolyl, pyridazinyl, pyrazinyl, pyrimidinyl, indazolyl, quinazolinyl, quinolinyl and isoquinolinyl,
the above-stated aryl residues are selected from the group consisting of phenyl and naphthyl, and
the aryl residues which are fused with a mono- or polycyclic ring system are selected from the group consisting of [1,3]-benzodioxolyl, [1,4]-benzodioxanyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl and [3,4]-dihydro-2H-1,4-benzoxazinyl,
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

2. Compounds according to claim 1, **characterised in that**
R¹ denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, [3,4]-dihydro-2H-1,4-benzoxazinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, n-hexyl, n-heptyl, -NH-C(=O)-O-CH₃, -NH-C(=O)-O-C₂H₅, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phenyl and benzyl, wherein in each case the cyclic moiety of the residues phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

3. Compounds according to claim 1 or claim 2, **characterised in that**
R² denotes -C(=S)-NH-R³; -C(=O)-NH-R⁴; -S(=O)₂-R⁵; -(CH₂)-C(=O)-NH-R⁶; -(CH₂)-O-R⁷, -(CH₂)-S-R⁷, -(CH₂)-NH-R⁷, -(CH₂)-N(CH₃)-R⁷, -(CH₂)-(CH₂)-O-R⁷, -(CH₂)-(CH₂)-S-R⁷, -(CH₂)-NH-R⁷, -(CH₂)-N(CH₃)-R⁷, -(CH₂)-(CH₂)-(CH₂)-O-R⁷, -(CH₂)-(CH₂)-(CH₂)-S-R⁷, -(CH₂)-(CH₂)-(CH₂)-NH-R⁷, -(CH₂)-(CH₂)-(CH₂)-N(CH₃)-R⁷, -(CH₂)-O-(CH₂)-R⁷, -(CH₂)-S-(CH₂)-R⁷; -(CH₂)-NH-(CH₂)-R⁷; -C(=O)-R⁸ or -S(=O)₂-NR⁹R¹⁰.

4. Compounds according to one or more of claims 1 to 3, **characterised in that**
R³ denotes -(CHR¹¹)-C(=O)-O-R¹² or -(CHR¹¹)-(CH₂)-C(=O)-O-R¹²;
-(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-S-R¹⁴, -(CHR¹³)-NH-R¹⁴, -(CHR¹³)-N(CH₃)-R¹⁴, -(CHR¹³)-(CH₂)-O-R¹⁴, -(CHR¹³)-(CH₂)-S-R¹⁴, -(CHR¹³)-NH-R¹⁴, -(CHR¹³)-N(CH₃)-R¹⁴, -(CHR¹³)-(CH₂)-(CH₂)-O-R¹⁴, -(CHR¹³)-(CH₂)-(CH₂)-S-R¹⁴, -(CHR¹³)-(CH₂)-(CH₂)-NH-R¹⁴, -(CHR¹³)-(CH₂)-(CH₂)-N(CH₃)-R¹⁴, -(CHR¹³)-O-(CH₂)-R¹⁴, -(CHR¹³)-S-(CH₂)-R¹⁴ or -(CHR¹³)-NH-(CH₂)-R¹⁴;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl, wherein the residue may optionally be substituted with 1, 2, 3, 4 or 5 substituents mutually independently selected from the group consisting of F, Cl, Br, I, -CN, -NO₂, -OH, -SH and -NH₂;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the residue may in each case optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-CF₃, -C(=O)-C₂F₅, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phenyl and benzyl, wherein in each case the cyclic moiety of the residues phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

5. Compounds according to one or more of claims 1 to 4, **characterised in that**
R⁴ denotes -(CHR¹⁵)-R¹⁶, -(CHR¹⁵)-(CH₂)-R¹⁶, -(CHR¹⁵)-(CH₂)-(CH₂)-R¹⁶, -(CHR¹⁵)-O-R¹⁶, -(CHR¹⁵)-S-R¹⁶, -(CHR¹⁵)-NH-R¹⁶, -(CHR¹⁵)-N(CH₃)-R¹⁶, -(CHR¹⁵)-(CH₂)-O-R¹⁵, -(CHR¹⁵)-(CH₂)-S-R¹⁶, -(CHR¹⁵)-NH-R¹⁶, -(CHR¹⁵)-N(CH₃)-R¹⁶, -(CHR¹⁵)-(CH₂)-(CH₂)-O-R¹⁶, -(CHR¹⁵)-(CH₂)-(CH₂)-S-R¹⁶, -(CHR¹⁵)-(CH₂)-(CH₂)-NH-R¹⁶, -(CHR¹⁵)-(CH₂-(CH₂)-N(CH₃)-R¹⁶, -(CHR¹⁵)-O-(CH₂)-R¹⁶, -(CHR¹⁵)-S-(CH₂)-R¹⁶ or -(CHR¹⁵)-NH-(CH₂)-R¹⁶;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl, wherein the residue may optionally be substituted with 1, 2, 3, 4 or 5 substituents mutually independently selected from the group consisting of F, Cl, Br, I, -CN, -NO₂, -OH, -SH and -NH₂; denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the residue may in each case optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phenyl and benzyl, wherein in each case the cyclic moiety of the residues phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

6. Compounds according to one or more of claims 1 to 5, **characterised in that**
R⁵ denotes -(CHR¹⁷)-R¹⁸, -(CHR¹⁷)-(CH₂)-R¹⁸, -(CHR¹⁷)-(CH₂)-(CH₂)-R¹⁸, -(CHR¹⁷)-O-R¹⁸, -(CHR¹⁷)-S-R¹⁸, -(CHR¹⁷)-NH-R¹⁸, -(CHR¹⁷)-N(CH₃)-R¹⁸, -(CHR¹⁷)-(CH₂)-O-R¹⁸, -(CHR¹⁷)-(CH₂)-S-R¹⁸, -(CHR¹⁷)-NH-R¹⁸, -(CHR¹⁷)-N(CH₃)-R¹⁸, -(CHR¹⁷)-(CH₂)-(CH₂)-O-R¹⁸, -(CHR¹⁷)-(CH₂)-(CH₂)-S-R¹⁸, -(CHR¹⁷)-(CH₂)-(CH₂)-NH-R¹⁸, -(CHR¹⁷)-(CH₂)-(CH₂)-N(CH₃)-R¹⁸, -(CHR¹⁷)-O-(CH₂)-R¹⁸, -(CHR¹⁷)-S-(CH₂)-R¹⁸ or -(CHR¹⁷)-NH-(CH₂)-R¹⁸;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl, wherein the residue may optionally be substituted with 1, 2, 3, 4 or 5 substituents mutually independently selected from the group consisting of F, Cl, Br, I, -CN, -NO₂, -OH, -SH and -NH₂;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the residue may in each case optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phenyl and benzyl, wherein in each case the cyclic moiety of the residues phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

7. Compounds according to one or more of claims 1 to 6, **characterised in that**
R⁶ denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl, wherein the residue may optionally be substituted with 1, 2, 3, 4 or 5 substituents mutually independently selected from the group consisting of F, Cl, Br, I, -CN, -NO₂, -OH, -SH and -NH₂;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the residue may in each case optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(₌O)₂-C₂H₅, -NH-S(=O)₂-CH₃, -S(=O)₂-NH-CH₃, -S(=O)₂-NH₂, -S(=O)₂-NH-phenyl, phenyl and benzyl, wherein in each case the cyclic moiety of the residues -S(=O)₂-NH-phenyl, phenyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

8. Compounds according to one or more of claims 1 to 7, **characterised in that**
R⁸ denotes -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-S-R²⁰, -(CHR¹⁹)-NH-R²⁰, -(CHR¹⁹)-N(CH₃)-R²⁰, -(CHR¹⁹)-(CH₂)-O-R²⁰, -(CHR¹⁹)-(CH₂)-S-R²⁰, -(CHR¹⁹)-NH-R²⁰, -(CHR¹⁹)-N(CH₃)-R²⁰, -(CHR¹⁹)-(CH₂)-(CH₂)-O-R²⁰, -(CHR¹⁹)-(CH₂)-(CH₂)-S-R²⁰, -(CHR¹⁹)-(CH₂)-(CH₂)-NH-R²⁰, -(CHR¹⁹)-(CH₂)-(CH₂)-N(CH₃)-R²⁰, -(CHR¹⁹)-O-(CH₂)-R²⁰, -(CHR¹⁹)-S-(CH₂)-R²⁰, -(CHR¹⁹)-NH-(CH₂)-R²⁰, -(CHR¹⁹)-O-(CH₂)-(CH₂)-O-R²⁰, -(CHR¹⁹)-O-(CH₂)-(CH₂)-S-R²⁰ or -(CHR¹⁹)-S-(CH₂)-(CH₂)-S-R²⁰;
denotes -(CH=CH)-R²¹;
denotes -(CR²²R²³)-C(=O)-O-R²⁵, -(CR²²R²³)-(CR²⁴R²⁵)-C(=O)-O-R²⁶, -(CR²²R²³)-(CR²⁴R²⁵)-(CH₂)-C(=O)-O-R²⁶, ₋(CR²²R²³)-O-(CR²⁴R²⁵)-C(=O)-O-R²⁶, -(CR²²R²³)-S-(CR²⁴R²⁵)-C(=O)-O-R²⁶ or -(CR²²R²³)-NH-(CR²⁴R²⁵)-C(=O)-O-R²⁶;
denotes -(CHR²⁷)-O-C(=O)-R²⁸;
denotes -CH[(CH₂)R²⁹][NH-S(=O)₂-R³⁰];
denotes -CH[(CH₂)R³¹][NH-C(=O)-O-R³²];
denotes a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl, wherein the residue may optionally be substituted with 1, 2, 3, 4 or 5 substituents mutually independently selected from the group consisting of F, Cl, Br, I, -CN, -NO₂, -OH, -SH and -NH₂;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and bicyclo[2.2.1]heptyl, wherein the residue in each case may optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, phenyl and -S(=O)₂-NH₂; wherein the phenyl residue may in each case be substituted with 1, 2, 3, 4 or 5 substituents mutually independently selected from the group consisting of F, Cl, Br, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃ and -O-CH₂-CH₂-CH₂-CH₃;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-phenyl, phenyl, -O-phenyl, -O-benzyl and benzyl, wherein in each case the cyclic moiety of the residues -S(=O)₂-NH-phenyl, phenyl, -O-phenyl, -O-benzyl and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl.

9. Compounds according to one or more of claims 1 to 8, **characterised in that**
R⁹ and R¹⁰, mutually independently, in each case denote a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl.

10. Compounds according to one or more of claims 1 to 9, **characterised in that**
R¹¹, R¹³, R¹⁵ R¹⁷, R¹⁹, R²², R²³, R²⁴, R²⁵ and R²⁶, mutually independently, in each case
denote a hydrogen residue or a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl.

11. Compounds according to one or more of claims 1 to 10, **characterised in that**
R¹², R²⁸ and R³², mutually independently, in each case denote a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl.

12. Compounds according to one or more of claims 1 to 11, **characterised in that**
R¹⁴, R¹⁶, R¹⁸ and R²⁰, mutually independently, in each case denote a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and bicyclo[2.2.1]heptyl, wherein the residue may in each case optionally be substituted with 1, 2, 3, 4 or 5 substituents mutually independently selected from the group consisting of oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ and -S(=O)₂-NH₂;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) and S(=O)₂-NH₂.

13. Compounds according to one or more of claims 1 to 12, **characterised in that**
R²¹, R²⁷, R²⁹, R³⁰ and R³¹, mutually independently, in each case denote a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) and S(=O)₂-NH₂.

14. Compounds according to one or more of claims 1 to 13, **characterised in that**
n is equal to 1, 2 or 3;
R¹ denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, methyl, ethyl, n-propyl, isopropyl, n-butyl, sec-butyl, isobutyl, tert-butyl, n-pentyl, n-hexyl, n-heptyl, phenyl and benzyl, wherein in each case the cyclic moiety of the residues phenyl and benzyl may be substituted with 1, 2, 3, 4 or 5 substituents mutually independently selected from the group consisting of F, Cl, Br, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃ and -O-C(CH₃)₃;
R² denotes -C(=S)-NH-R³;
-C(=O)-NH-R⁴;
-S(=O)₂-R⁵;
-(CH₂)-C(=O)-NH-R⁶;
-(CH₂)-O-R⁷, -(CH₂)-S-R⁷, -(CH₂)-NH-R⁷, -(CH₂)-N(CH₃)-R⁷,
-(CH₂)-(CH₂)-O-R⁷, -(CH₂)-(CH₂)-S-R⁷, -(CH₂)-NH-R⁷, -(CH₂)-N(CH₃)-R⁷,
-(CH₂)-(CH₂)-(CH₂)-O-R⁷, -(CH₂)-(CH₂)-(CH₂)-S-R⁷,
-(CH₂)-(CH₂)-(CH₂)-NH-R⁷, -(CH₂)-(CH₂)-(CH₂)-N(CH₃)-R⁷,
-(CH₂)-O-(CH₂)-R⁷, -(CH₂)-S-(CH₂)-R⁷; -(CH₂)-NH-(CH₂)-R⁷; -C(=O)-R⁸
or denotes -S(=O)₂-NR⁹R¹⁰;
R³ denotes -(CHR¹¹)-C(=O)-O-R¹² or -(CHR¹¹)-(CH₂)-C(=O)-O-R¹²;
denotes -(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-(CH₂)-O-R¹⁴, -(CHR¹³)-(CH₂)-(CH₂)-O-R¹⁴ or -(CHR¹³)-O-(CH₂)-R¹⁴;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl and 3-butenyl;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃ and -C(=O)-C₂F₅;
R⁴ denotes -(CHR¹⁵)-R¹⁶, -(CHR¹⁵)-(CH₂)-R¹⁶ or -(CHR¹⁵)-(CH₂)-(CH₂)-R¹⁶;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -NO₂, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, methyl, ethyl, n-propyl, isopropyl, n-butyl, sec-butyl, isobutyl, tert-butyl, n-pentyl, n-hexyl, n-heptyl, -C(=O)-O-CH₃, -C(=O)-O-CH₂-CH₃, -C(=O)-O-CH(CH₃)₂, -C(-O)-O-C(CH₃)₃, -C(=O)-CH₃, -C(=O)-C₂H₅, -C(=O)-C(CH₃)₃, -C(=O)-CF₃ and -C(=O)-C₂F₅;
R⁵ denotes -(CHR¹⁷)-R¹⁸, -(CHR¹⁷)-(CH₂)-R¹⁸ or -(CHR¹⁷)-(CH₂)-(CH₂)-R¹⁸;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃);
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ and -S(=O)₂-C₂H₅;
R⁶ denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -S(=O)₂-NH-phenyl, phenyl and benzyl, wherein in each case the cyclic moiety of the residues -S(=O)₂-NH-phenyl, phenyl and benzyl may be substituted with 1, 2, 3, 4 or 5 substituents mutually independently selected from the group consisting of F, Cl and Br;
R⁷ denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, imidazolyl, indolyl, isoindolyl, benzo[b]furanyl, benzo[b]thiophenyl, thiazolyl, [1,2,4]-oxadiazolyl, [1,2,3]-thiadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, -CN, -CF₃, -SF₅, -OH, -O-CH₃, -O-C₂H₅, -NO₂, -O-CF₃, -S-CF₃, -S-CF₂H, -S-CFH₂, methyl, ethyl, n-propyl, isopropyl, n-butyl, sec-butyl, isobutyl, tert-butyl, n-pentyl, -C(CH₃)₂-C₂H₅, n-hexyl, n-heptyl, phenyl and benzyl, wherein in each case the cyclic moiety of the residues phenyl and benzyl may be substituted with 1, 2, 3, 4 or 5 substituents mutually independently selected from the group consisting of F, Cl, Br, -O-CH₃, -O-C₂H₅, -O-CH(CH₃)₂, -O-CH₂-CH₂-CH₃, -O-C(CH₃)₃ and -O-CH₂-CH₂-CH₂-CH₃;
R⁸ denotes -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ or -(CHR¹⁹)-O-(CH₂)-(CH₂)-O-R²⁰;
denotes -(CH=CH)-R²¹;
denotes -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ or -(CR²²R²³)-O-(CR²⁴R²⁵)-C(=O)-O-R²⁶;
denotes -(CHR²⁷)-O-C(=O)-R²⁸;
denotes -CH[(CH₂)R²⁹][NH-S(=O)₂-R³⁰];
denotes -CH[(CH₂)R³¹][NH-C(=O)-O-R³²];
denotes a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl;
denotes a residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl and bicyclo[2.2.1]heptyl, wherein the residue may in each case optionally be substituted with 1, 2, 3, 4 or 5 substituents mutually independently selected from the group consisting of -C(=O)-CH₃, -C(=O)-C₂H₅, -C(=O)-C(CH₃)₃ and phenyl;
or denotes a residue selected from the group consisting of phenyl, naphthyl, (1,3)-benzodioxolyl, (1,4)-benzodioxanyl, 2H-chromenyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, [1,2,3]-thiadiazolyl, [1,2,4]-oxadiazolyl, benzo[2,1,3]thiadiazolyl, [1,2,3]-benzothiadiazolyl, [2,1,3]-benzoxadiazolyl, [1,2,3]-benzoxadiazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, oxazolyl, isoxazolyl, pyridazinyl, pyrazinyl, pyrimidinyl, indazolyl, quinazolinyl, quinolinyl and isoquinolinyl, wherein the residue may in each case optionally be substituted with 1, 2, 3, 4 or 5 substituents mutually independently selected from the group consisting of F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, phenyl, -O-phenyl, -O-benzyl and benzyl, wherein in each case the cyclic moiety of the residues phenyl, -O-phenyl, -O-benzyl, and benzyl may be substituted with 1, 2, 3, 4 or 5 substituents mutually independently selected from the group consisting of F, Cl, Br, -OH, -CF₃, -SF₅, -NO₂, methyl, ethyl, n-propyl, isopropyl, n-butyl, sec-butyl, isobutyl, tert-butyl, n-pentyl, n-hexyl, n-heptyl, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phenyl and -O-benzyl;
R⁹ and R¹⁰, mutually independently, in each case denote a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl;
R¹¹, R¹³, R¹⁵, R¹⁷, R¹⁹, R²², R²³, R²⁴, R²⁵ and R²⁶, mutually independently, in each case denote a hydrogen residue or a residue selected from the group consisting of methyl, ethyl, n-propyl, isopropyl, n-butyl, sec-butyl, isobutyl and tert-butyl;
R¹², R²⁸ and R³², mutually independently, in each case denote a residue selected from the group consisting of methyl, ethyl, n-propyl, isopropyl, n-butyl, sec-butyl, isobutyl and tert-butyl;
R¹⁴, R¹⁶, R¹⁸ and R²⁰, mutually independently, in each case denote a residue selected from the group consisting of methyl, ethyl, n-propyl, isopropyl, n-butyl, sec-butyl, isobutyl and tert-butyl;
denote a residue selected from the group consisting of cyclopropyl, cyclobutyl, cyclopentyl, cyclohexyl, cycloheptyl, tetrahydrofuranyl, tetrahydrothiophenyl, tetrahydropyranyl and bicyclo[2.2.1]heptyl, wherein the residue may in each case optionally be substituted with 1, 2, 3, 4 or 5 substituents mutually independently selected from the group consisting of oxo (=O), thioxo (=S), methyl, ethyl, n-propyl, isopropyl, n-butyl, sec-butyl, isobutyl, tert-butyl, n-pentyl, n-hexyl, n-heptyl, -(CH₂)-C(=O)-OH and -C(=O)-OH;
or denote a residue selected from the group consisting of phenyl and naphthyl, wherein the residue may in each case be substituted with 1, 2, 3, 4 or 5 substituents mutually independently selected from the group consisting of F, Cl, Br, -O-CH₃, -OC₂H₅, -O-CH(CH₃)₂, -O-CH₂-CH₂-CH₃, -O-C(CH₃)₃, -O-CH₂-CH₂-CH₂-CH₃, methyl, ethyl, n-propyl, isopropyl, n-butyl, sec-butyl, isobutyl, tert-butyl, n-pentyl, -C(CH₃)₂-C₂H₅, n-hexyl and n-heptyl;
and
R²¹, R²⁷, R²⁹, R³⁰ and R³¹, mutually independently, in each case denote a residue selected from the group consisting of phenyl, naphthyl, indolyl and isoindolyl, wherein the residue may in each case be substituted with 1, 2, 3, 4 or 5 substituents mutually independently selected from the group consisting of F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, methyl, ethyl, n-propyl, isopropyl, n-butyl, sec-butyl, isobutyl, tert-butyl, n-pentyl, -C(CH₃)₂-C₂H₅, n-hexyl and n-heptyl;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

15. Compounds according to one or more of claims 1 to 14, **characterised in that**
n is equal to 1 or 2;
R¹ denotes a residue selected from the group consisting of phenyl and naphthyl, wherein the residue may in each case optionally be substituted with 1, 2, 3, 4 or 5 substituents mutually independently selected from the group consisting of F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, methyl, ethyl, n-propyl, isopropyl, n-butyl, sec-butyl, isobutyl, tert-butyl, n-pentyl, n-hexyl, n-heptyl, phenyl and benzyl;
R² denotes -C(=S)-NH-R³;
-C(=O)-NH-R⁴; -S(=O)₂-R⁵;
-(CH₂)-C(=O)-NH-R⁶;
-(CH₂)-O-R⁷, -(CH₂)-(CH₂)-O-R⁷, -(CH₂)-(CH₂)-(CH₂)-O-R⁷;
-C(=O)-R⁸
or -S(=O)₂-NR⁹R¹⁰;
R³ denotes -(CHR¹¹)-(CH₂)-C(=O)-O-R¹²;
denotes -(CHR¹³)-R¹⁴, -(CHR¹³)-(CH₂)-R¹⁴, -(CHR¹³)-(CH₂)-(CH₂)-R¹⁴ or -(CHR¹³)-(CH₂)-O-R¹⁴;
denotes a residue selected from the group consisting of vinyl, 1-propenyl, 2-propenyl, 1-butenyl, 2-butenyl and 3-butenyl;
or denotes a phenyl residue, [wherein] the residue may be substituted with 1, 2, 3, 4 or 5 substituents mutually independently selected from the group consisting of F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, methyl, ethyl, n-propyl, isopropyl, n-butyl, sec-butyl, isobutyl, tert-butyl, n-pentyl, n-hexyl, n-heptyl, -C(=O)-CH₃, -C(=O)-C₂H₅, -C(=O)-C(CH₃)₃, -C(=O)-CF₃ and -C(=O)-C₂F₅;
R⁴ denotes -(CHR¹⁵)-R¹⁶, -(CHR¹⁵)-(CH₂)-R¹⁶ or -(CHR¹⁵)-(CH₂)-(CH₂)-R¹⁶;
or denotes a residue selected from the group consisting of phenyl and naphthyl, wherein the residue may in each case optionally be substituted with 1, 2, 3, 4 or 5 substituents mutually independently selected from the group consisting of F, Cl, Br, -NO₂, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, methyl, ethyl, n-propyl, isopropyl, n-butyl, sec-butyl, isobutyl, tert-butyl, n-pentyl, n-hexyl, n-heptyl, -C(=O)-O-CH₃, -C(=O)-O-CH₂-CH₃, -C(=O)-O-CH(CH₃)₂, -C(=O)-O-C(CH₃)₃, -C(=O)-CH₃, -C(=O)-C₂H₅, -C(=O)-C(CH₃)₃, -C(=O)-CF₃ and -C(=O)-C₂F₅;
R⁵ denotes -(CHR¹⁷)-R¹⁸, -(CHR¹⁷)-(CH₂)-R¹⁸ or -(CHR¹⁷)-(CH₂)-(CH₂)-R¹⁸;
denotes a residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃);
or denotes a residue selected from the group consisting of phenyl, naphthyl, pyrazolyl, thiophenyl, [1,2,3,4]-tetrahydroquinolinyl and [1,2,3,4]-tetrahydroisoquinolinyl, wherein the residue may in each case optionally be substituted with 1, 2, 3, 4 or 5 substituents mutually independently selected from the group consisting of F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ and -S(=O)₂-C₂H₅;
R⁶ denotes a residue selected from the group consisting of phenyl, naphthyl, pyrazolyl, thiophenyl and thiazolyl, wherein the residue may in each case optionally be substituted with 1, 2, 3, 4 or 5 substituents mutually independently selected from the group consisting of F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, methyl, ethyl, n-propyl, isopropyl, n-butyl, sec-butyl, isobutyl, tert-butyl, n-pentyl, -C(CH₃)₂-C₂H₅, n-hexyl, n-heptyl, -S(=O)₂-NH-phenyl, phenyl and benzyl, wherein in each case the cyclic moiety of the residues -S(=O)₂ -NH-phenyl, phenyl and benzyl may be substituted with 1, 2, 3, 4 or 5 substituents mutually independently selected from the group consisting of F, Cl and Br;
R⁷ denotes a residue selected from the group consisting of phenyl, naphthyl, benzo[b]furanyl, benzo[b]thiophenyl and [1,2,4]-oxadiazolyl, wherein the residue may in each case optionally be substituted with 1, 2, 3, 4 or 5 substituents mutually independently selected from the group consisting of F, Cl, Br, -CN, -CF₃, -SF₅, -OH, -O-CH₃, -O-C₂H₅, -NO₂, -O-CF₃, -S-CF₃, -S-CF₂H, -S-CFH₂, methyl, ethyl, n-propyl, isopropyl, n-butyl, sec-butyl, isobutyl, tert-butyl, n-pentyl, -C(CH₃)₂-C₂H₅, n-hexyl, n-heptyl, phenyl and benzyl, wherein in each case the cyclic moiety of the residues phenyl and benzyl may be substituted with 1, 2, 3, 4 or 5 substituents mutually independently selected from the group consisting of F, Cl, Br, -O-CH₃, -O-C₂H₅, -O-CH(CH₃)₂, -O-CH₂-CH₂-CH₃, -O-C(CH₃)₃ and -O-CH₂-CH₂-CH₂-CH₃;
R⁸ denotes -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ or -(CHR¹⁹)-O-(CH₂)-(CH₂)-O-R²⁰;
denotes -(CH=CH)-R²¹;
denotes -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ or -(CR²²R²³)-O-(CR²⁴R²⁵)-C(=O)-O-R²⁶;
denotes -(CHR²⁷)-O-C(=O)-R²⁸;
denotes -CH[(CH₂)R²⁹][NH-S(=O)₂-R³⁰]; denotes -CH[(CH₂)R³¹][NH-C(=O)-O-R³²];
denotes a residue selected from the group consisting of methyl, ethyl, n-propyl, isopropyl, n-butyl, sec-butyl, isobutyl, tert-butyl, n-pentyl, sec-pentyl, 3-pentyl, -(CH₂)-(CH₂)-(C(CH₃)₃), n-hexyl, 2-hexyl, 3-hexyl, n-heptyl, 2-heptyl, 3-heptyl, 4-heptyl and n-octyl;
denotes a residue selected from the group consisting of cyclopropyl, cyclobutyl, cyclopentyl, cyclohexyl, cycloheptyl, pyrrolidinyl, piperidinyl and adamantyl, wherein the residue may in each case optionally be substituted with 1, 2, 3, 4 or 5 substituents mutually independently selected from the group consisting of -C(=O)-CH₃, -C(=O)-C₂H₅, -C(=O)-C(CH₃)₃ and phenyl;
or denotes a residue selected from the group consisting of phenyl, naphthyl, 2H-chromenyl, thiophenyl, furanyl, pyrazolyl, triazolyl, pyridinyl, [1,2,3]-thiadiazolyl, [2,1,3]-benzoxadiazolyl, [1,2,3]-benzoxadiazolyl, oxazolyl and isoxazolyl, wherein the residue may in each case optionally be substituted with 1, 2, 3, 4 or 5 substituents mutually independently selected from the group consisting of F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, methyl, ethyl, n-propyl, isopropyl, n-butyl, sec-butyl, isobutyl, tert-butyl, n-pentyl, -C(CH₃)₂-C₂H₅, n-hexyl, n-heptyl, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, phenyl, -O-phenyl, -O-benzyl and benzyl, wherein in each case the cyclic moiety of the residues phenyl, -O-phenyl, -O-benzyl and benzyl may be substituted with 1, 2, 3, 4 or 5 substituents mutually independently selected from the group consisting of F, Cl and Br;
R⁹ and R¹⁰, mutually independently, in each case denote a residue selected from the group consisting of methyl, ethyl, n-propyl, isopropyl, n-butyl, sec-butyl, isobutyl and tert-butyl;
R¹¹, R¹³, R¹⁵, R¹⁷, R¹⁹, R²², R²³, R²⁴, R²⁵ and R²⁶, mutually independently, in each case denote a hydrogen residue or a residue selected from the group consisting of methyl, ethyl and n-propyl;
R¹², R²⁸ and R³², mutually independently, in each case denote a residue selected from the group consisting of methyl, ethyl, n-propyl, isopropyl, n-butyl, sec-butyl, isobutyl and tert-butyl;
R¹⁴ denotes a residue selected from the group consisting of methyl, ethyl, n-propyl, isopropyl, n-butyl, sec-butyl, isobutyl and tert-butyl;
denotes a residue selected from the group consisting of cyclopropyl, cyclobutyl, cyclopentyl, cyclohexyl, cycloheptyl, tetrahydrofuranyl, tetrahydrothiophenyl and tetrahydropyranyl;
or denotes a phenyl residue which may be substituted with 1, 2, 3, 4 or 5 substituents selected from the group consisting of F, Cl and Br;
R¹⁶ denotes a residue selected from the group consisting of phenyl and naphthyl, wherein the residue may in each case be substituted with 1, 2, 3, 4 or 5 substituents mutually independently selected from the group consisting of F, Cl and Br;
R¹⁸ denotes a 7,7-dimethyl-2-oxobicyclo[2.2.1]hept-1-yl residue;
R²⁰ denotes a residue selected from the group consisting of methyl, ethyl, n-propyl, isopropyl, n-butyl, sec-butyl, isobutyl and tert-butyl;
denotes a residue selected from the group consisting of cyclopropyl, cyclobutyl, cyclopentyl, cyclohexyl and cycloheptyl; wherein the residue may in each case be substituted with a substituent selected from the group consisting of -(CH₂)-C(=O)-OH and -C(=O)-OH;
or denotes a phenyl residue which may be substituted with 1, 2, 3, 4 or 5 substituents mutually independently selected from the group consisting of F, Cl, Br, -O-CH₃, -O-C₂H₅, -O-CH(CH₃)₂, -O-CH₂-CH₂-CH₃, -O-C(CH₃)₃ and -O-CH₂-CH₂-CH₂-CH₃;
R²¹ denotes a phenyl residue which may be substituted with 1, 2, 3, 4 or 5 substituents selected from the group consisting of F, Cl, and Br;
R²⁷ denotes a phenyl residue;
R²⁹ denotes a phenyl residue;
R³⁰ denotes a phenyl residue which may be substituted with 1, 2, 3, 4 or 5 substituents selected from the group consisting of methyl, ethyl, n-propyl, isopropyl, n-butyl, sec-butyl, and tert-butyl;
and
R³¹ denotes an indolyl residue;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

16. Compounds according to one or more of claims 1 to 15 selected from the group consisting of
[1] 3-(4-methylbenzyl)-2-oxo-1-oxa-3,8-diazaspiro[4.5]decane-8-carbothioic acid pentafluorophenylamide
[2] 3-(3-bromobenzyl)-2-oxo-1-oxa-3,8-diazaspiro[4.5]decane-8-carbothioic acid (2,5-dichlorophenyl)amide
[3] 2-oxo-3-(4-trifluoromethylbenzyl)-1-oxa-3,8-diazaspiro[4.5]decane-8-carbothioic acid benzylamide
[4] 3-(3,5-dimethylbenzyl)-2-oxo-1-oxa-3,8-diazaspiro[4.5]decane-8-carbothioic acid (tetrahydrofuran-2-ylmethyl)amide
[5] 3-(3-bromobenzyl)-2-oxo-1-oxa-3,8-diazaspiro[4.5]decane-8-carbothioic acid (2-methoxyethyl)amide
[6] 3-(3-bromobenzyl)-2-oxo-1-oxa-3,8-diazaspiro[4.5]decane-8-carbothioic acid (2-methoxyphenyl)amide
[7] 3-(3,5-dimethylbenzyl)-2-oxo-1-oxa-3,8-diazaspiro[4.5]decane-8-carbothioic acid (3-acetylphenyl)amide
[8] 3-benzyl-2-oxo-1-oxa-3,8-diazaspiro[4.5]decane-8-carbothioic acid o-tolylamide
[9] 3-{[3-(3-bromobenzyl)-2-oxo-1-oxa-3,8-diazaspiro[4.5]decane-8-carbothioyl]amino}butanoic acid ethyl ester
[10] 3-naphthalen-2-ylmethyl-2-oxo-1-oxa-3,8-diazaspiro[4.5]decane-8-carbothioic acid allylamide
[11] 3-(3,4-difluorobenzyl)-2-oxo-1-oxa-3,8-diazaspiro[4.5]decane-8-carbothioic acid benzylamide
[12] 3-biphenyl-2-ylmethyl-2-oxo-1-oxa-3,8-diazaspiro[4.5]decane-8-carbothioic acid (1-phenylethyl)amide
[13] 3-(3,4-difluorobenzyl)-2-oxo-1-oxa-3,8-diazaspiro[4.5]decane-8-carbothioic acid (4-ethoxyphenyl)amide
[14] 3-(3-cyanobenzyl)-2-oxo-1-oxa-3,8-diazaspiro[4.5]decane-8-carbothioic acid (3,5-dichlorophenyl)amide
[15] 3-(3-cyanobenzyl)-2-oxo-1-oxa-3,8-diazaspiro[4.5]decane-8-carbothioic acid (3-trifluoromethylphenyl)amide
[16] 3-(3-bromobenzyl)-2-oxo-1-oxa-3,8-diazaspiro[4.5]decane-8-carbothioic acid benzylamide
[17] 3-(2-methoxy-5-nitrobenzyl)-2-oxo-1-oxa-3,8-diazaspiro[4.5]decane-8-carbothioic acid (tetrahydrofuran-2-ylmethyl)amide
[18] 3-biphenyl-2-ylmethyl-2-oxo-1-oxa-3,8-diazaspiro[4.5]decan-8-carbothioic acid phenylamide
[19] 3-(3-cyanobenzyl)-2-oxo-1-oxa-3,8-diazaspiro[4.5]decane-8-carbothioic acid cyclohexylmethylamide
[20] 3-(2-fluorobenzyl)-2-oxo-1-oxa-3,8-diazaspiro[4.5]decane-8-carbothioic acid (3-acetylphenyl)amide
[21] 3-(2-methoxy-5-nitrobenzyl)-2-oxo-1-oxa-3,8-diazaspiro[4.5]decane-8-carbothioic acid cyclohexylmethylamide
[22] 3-(3-cyanobenzyl)-2-oxo-1-oxa-3,8-diazaspiro[4.5]decane-8-carbothioic acid 4-chlorobenzylamide
[23] 3-(2-methoxy-5-nitrobenzyl)-2-oxo-1-oxa-3,8-diazaspiro[4.5]decane-8-carbothioic acid benzylamide
[24] 3-(2-fluorobenzyl)-2-oxo-1-oxa-3,8-diazaspiro[4.5]decane-8-carboxylic acid (4-ethoxyphenyl)amide
[25] 3-biphenyl-2-ylmethyl-2-oxo-1-oxa-3,8-diazaspiro[4.5]decane-8-carboxylic acid (2,5-dichlorophenyl)amide
[26] 3-(2-methoxy-5-nitrobenzyl)-2-oxo-1-oxa-3,8-diazaspiro[4.5]decane-8-carboxylic acid (2,5-difluorophenyl)amide
[27] 3-(4-methylbenzyl)-2-oxo-1-oxa-3,8-diazaspiro[4.5]decane-8-carboxylic acid (4-methyl-3-nitrophenyl)amide
[28] 3-(3,4-difluorobenzyl)-2-oxo-1-oxa-3,8-diazaspiro[4.5]decane-8-carboxylic acid 4-fluorobenzylamide
[29] 3-(3-bromobenzyl)-2-oxo-1-oxa-3,8-diazaspiro[4.5]decane-8-carboxylic acid (2,5-difluorophenyl)amide
[30] 4-[(3-naphthalen-2-ylmethyl-2-oxo-1-oxa-3,8-diazaspiro[4.5]decane-8-carbonyl)amino]benzoic acid ethyl ester
[31] 2-oxo-3-(4-trifluoromethylbenzyl)-1-oxa-3,8-diazaspiro[4.5]decane-8-carboxylic acid (4-chloro-3-trifluoromethylphenyl)amide
[32] 3-(2-cyanobenzyl)-2-oxo-1-oxa-3,8-diazaspiro[4.5]decane-8-carboxylic acid (2,5-difluorophenyl)amide
[33] 3-benzyl-2-oxo-1-oxa-3,8-diazaspiro[4.5]decane-8-carboxylic acid (3-cyanophenyl)amide
[34] 3-(3,4-difluorobenzyl)-2-oxo-1-oxa-3,8-diazaspiro[4.5]decane-8-carboxylic acid (4-methyl-3-nitrophenyl)amide
[35] 3-(3,4-difluorobenzyl)-2-oxo-1-oxa-3,8-diazaspiro[4.5]decane-8-carboxylic acid (3-methoxyphenyl)amide
[36] 3-(4-methylbenzyl)-2-oxo-1-oxa-3,8-diazaspiro[4.5]decane-8-carboxylic acid 3,4-dichlorobenzylamide
[37] 3-benzyl-2-oxo-1-oxa-3,8-diazaspiro[4.5]decane-8-carboxylic acid (4-chloro-3-trifluoromethylphenyl)amide
[38] 2-oxo-3-(4-trifluoromethylsulfanylbenzyl)-1-oxa-3,8-diazaspiro[4.5]decane-8-carboxylic acid (3-cyanophenyl)amide
[39] 3-benzyl-2-oxo-1-oxa-3,8-diazaspiro[4.5]decane-8-carboxylic acid (4-methoxyphenyl)amide
[40] 2-oxo-3-(4-trifluoromethylsulfanylbenzyl)-1-oxa-3,8-diazaspiro[4.5]decane-8-carboxylic acid (4-methyl-3-nitrophenyl)amide
[41] 3-(4-iodobenzyl)-2-oxo-1-oxa-3,8-diazaspiro[4.5]decane-8-carboxylic acid (4-butoxyphenyl)amide
[42] 3-(3-bromobenzyl)-2-oxo-1-oxa-3,8-diazaspiro[4.5]decane-8-carboxylic acid 3,4-dichlorobenzylamide
[43] 3-benzyl-2-oxo-1-oxa-3,8-diazaspiro[4.5]decane-8-carboxylic acid (1-phenylethyl)amide
[44] 3-naphthalen-2-ylmethyl-2-oxo-1-oxa-3,8-diazaspiro[4.5]decane-8-carboxylic acid 3,4-dichlorobenzylamide
[45] 3-(4-methylbenzyl)-2-oxo-1-oxa-3,8-diazaspiro[4.5]decane-8-carboxylic acid phenethylamide
[46] 2-oxo-3-(2-trifluoromethylbenzyl)-1-oxa-3,8-diazaspiro[4.5]decane-8-carboxylic acid (3-methoxyphenyl)amide
[47] 3-(4-cyanobenzyl)-2-oxo-1-oxa-3,8-diazaspiro[4.5]decane-8-carboxylic acid (4-methoxyphenyl)amide
[48] 2-oxo-3-(4-trifluoromethylbenzyl)-1-oxa-3,8-diazaspiro[4.5]decane-8-carboxylic acid (1-naphthalen-1-yl-ethyl)amide
[49] 2-oxo-3-(2-trifluoromethylbenzyl)-1-oxa-3,8-diazaspiro[4.5]decane-8-carboxylic acid phenethylamide
[50] 3-(4-iodobenzyl)-2-oxo-1-oxa-3,8-diazaspiro[4.5]decane-8-carboxylic acid (4-methyl-3-nitrophenyl)amide
[51] 3-(2-methoxy-5-nitrobenzyl)-2-oxo-1-oxa-3,8-diazaspiro[4.5]decane-8-carboxylic acid (3-cyanophenyl)amide
[52] 3-naphthalen-2-ylmethyl-2-oxo-1-oxa-3,8-diazaspiro[4.5]decane-8-carboxylic acid (4-methoxyphenyl)amide
[53] 3-{[3-(4-methylbenzyl)-2-oxo-1-oxa-3,8-diazaspiro[4.5]decane-8-carbonyl]amino}benzoic acid ethyl ester
[54] 2-oxo-3-(2-trifluoromethylbenzyl)-1-oxa-3,8-diazaspiro[4.5]decane-8-carboxylic acid (1-naphthalen-1-yl-ethyl)amide
[55] 3-naphthalen-2-ylmethyl-2-oxo-1-oxa-3,8-diazaspiro[4.5]decane-8-carboxylic acid (2,5-dimethoxyphenyl)amide
[56] 3-(4-cyanobenzyl)-2-oxo-1-oxa-3,8-diazaspiro[4.5]decane-8-carboxylic acid (3-acetylphenyl)amide
[57] 3-biphenyl-2-ylmethyl-2-oxo-1-oxa-3,8-diazaspiro[4.5]decane-8-carboxylic acid phenethylamide
[58] 3-(4-cyanobenzyl)-2-oxo-1-oxa-3,8-diazaspiro[4.5]decane-8-carboxylic acid (5-chloro-2-methoxyphenyl)amide
[59] 3-{[2-oxo-3-(2-trifluoromethylbenzyl)-1-oxa-3,8-diazaspiro[4.5]decane-8-carbonyl]amino}benzoic acid ethyl ester
[60] 3-benzyl-2-oxo-1-oxa-3,8-diazaspiro[4.5]decane-8-carboxylic acid (3-fluorophenyl)amide
[61] 3-{[3-(3-cyanobenzyl)-2-oxo-1-oxa-3,8-diazaspiro[4.5]decane-8-carbonyl]amino}benzoic acid ethyl ester
[62] 3-biphenyl-2-ylmethyl-2-oxo-1-oxa-3,8-diazaspiro[4.5]decane-8-carboxylic acid (3-methoxyphenyl)amide
[63] 3-(2-methoxy-5-nitrobenzyl)-2-oxo-1-oxa-3,8-diazaspiro[4.5]decane-8-carboxylic acid (1-naphthalen-1-yl-ethyl)amide
[64] {2-[3-(3,4-difluorobenzyl)-2-oxo-1-oxa-3,8-diazaspiro[4.5]dec-8-yl]-2-oxoethoxy}acetic acid
[65] 4-[3-(3,4-difluorobenzyl)-2-oxo-1-oxa-3,8-diazaspiro[4.5]dec-8-yl]-3,3-dimethyl-4-oxobutanoic acid
[66] [2-[3-(3-bromobenzyl)-2-oxo-1-oxa-3,8-diazaspiro[4.5]dec-8-yl]-1-(1H-indol-3-ylmethyl)-2-oxoethyl]carbamic acid tert-butyl ester
[67] 5-[3-(2-fluorobenzyl)-2-oxo-1-oxa-3,8-diazaspiro[4.5]dec-8-yl]-3-methyl-5-oxopentanoic acid
[68] 3,3-dimethyl-5-[3-(4-methylbenzyl)-2-oxo-1-oxa-3,8-diazaspiro[4.5]dec-8-yl]-5-oxopentanoic acid
[69] 5-[3-(3,4-difluorobenzyl)-2-oxo-1-oxa-3,8-diazaspiro[4.5]dec-8-yl]-3-methyl-5-oxopentanoic acid
[70] {2-oxo-2-[2-oxo-3-(4-trifluoromethylbenzyl)-1-oxa-3,8-diazaspiro[4.5]dec-8-yl]ethoxy}acetic acid
[71] (1-{2-[3-(2-cyanobenzyl)-2-oxo-1-oxa-3,8-diazaspiro[4.5]dec-8-yl]-2-oxoethyl}cyclopentyl)acetic acid
[72] (1-{2-[3-(3,4-difluorobenzyl)-2-oxo-1-oxa-3,8-diazaspiro[4.5]dec-8-yl]-2-oxoethyl}cyclopentyl)acetic acid
[73] 5-(3-biphenyl-2-ylmethyl-2-oxo-1-oxa-3,8-diazaspiro[4.5]dec-8-yl)-3-methyl-5-oxopentanoic acid
[75] N-[4-(3,5-dichlorophenylsulfamoyl)phenyl]-2-[3-(4-methylbenzyl)-2-oxo-1-oxa-3,8-diazaspiro[4.5]dec-8-yl]acetamide
[76] N-(2,5-dimethyl-2H-pyrazol-3-yl)-2-[3-(2-methoxy-5-nitrobenzyl)-2-oxo-1-oxa-3,8-diazaspiro[4.5]dec-8-yl]acetamide
[80] N-(3-cyano-4-methylthiophen-2-yl,)-2-[3-(3,4-difluorobenzyl)-2-oxo-1-oxa-3,8-diazaspiro[4.5]dec-8-yl]acetamide
[85] 2-[2-oxo-3-(4-trifluoromethylsulfanylbenzyl)-1-oxa-3,8-diazaspiro[4.5]dec-8-yl]-N-(4-phenyl-5-trifluoromethylthiophen-3-yl)acetamide
[86] 2-[3-(2-fluorobenzyl)-2-oxo-1-oxa-3,8-diazaspiro[4.5]dec-8-yl]-N-(4-phenyl-thiazolyl-2-yl)acetamide
[87] 2-[2-oxo-3-(2-trifluoromethylbenzyl)-1-oxa-3,8-diazaspiro[4.5]dec-8-yl]-N-(4-trifluoromethoxyphenyl)acetamide
[91] N-{4-[3-(3-cyanobenzyl)-2-oxo-1-oxa-3,8-diazaspiro[4.5]decane-8-carbonyl]phenyl}acetamide
[92] 8-(4-ethoxybenzoyl)-3-(4-methylbenzyl)-1-oxa-3,8-diazaspiro[4.5]decan-2-one
[93] 3-[3-(4-cyanobenzyl)-2-oxo-1-oxa-3,8-diazaspiro[4.5]dec-8-yl]-3-oxopropionic acid methyl ester
[94] 2-[8-(2,4-difluorobenzoyl)-2-oxo-1-oxa-3,8-diazaspiro[4.5]dec-3-ylmethyl]benzonitrile
[95] 8-(3-dimethylaminobenzoyl)-3-(3,5-dimethylbenzyl)-1-oxa-3,8-diazaspiro[4.5]decan-2-one
[96] N-{4-[3-(3,4-difluorobenzyl)-2-oxo-1-oxa-3,8-diazaspiro[4.5]decane-8-carbonyl]phenyl}acetamide
[97] 8-(4-bromobenzoyl)-3-(4-fluorobenzyl)-1-oxa-3,8-diazaspiro[4.5]decan-2-one
[98] 4-[8-(adamantane-1-carbonyl)-2-oxo-1-oxa-3,8-diazaspiro[4.5]dec-3-ylmethyl]benzonitrile
[99] 3-(3,5-dimethylbenzyl)-8-(2-ethylsulfanylpyridine-3-carbonyl)-1-oxa-3,8-diazaspiro[4.5]decan-2-one
[100]3-(3-bromobenzyl)-8-(4-chlorobenzoyl)-1-oxa-3,8-diazaspiro[4.5]decan-2-one
[101] 8-[3-(2-chlorophenyl)-5-methylisoxazole-4-carbonyl]-3-(3,5-dimethylbenzyl)-1-oxa-3,8-diazaspiro[4.5]decan-2-one
[102]3-(3-bromobenzyl)-8-(4-ethylbenzylsulfonyl)-1-oxa-3,8-diazaspiro[4.5]decan-2-one
[103]8-(4-butoxybenzylsulfonyl)-3-(3,5-dimethylbenzyl)-1-oxa-3,8-diazaspiro[4.5]decan-2-one
[104]8-(biphenyl-4-carbonyl)-3-(3-bromobenzyl)-1-oxa-3,8-diazaspiro[4.5]decan-2-one
[105]acetic acid 2-[3-(3,5-dimethylbenzyl)-2-oxo-1-oxa-3,8-diazaspiro[4.5]dec-8-yl]-2-oxo-1-phenylethyl ester
[106]4-[2-oxo-8-(2-phenylcyclopropanecarbonyl)-1-oxa-3,8-diazaspiro[4.5]dec-3-ylmethyl]benzonitrile
[107]8-(2,5-dimethoxybenzylsulfonyl)-3-(3,5-dimethylbenzyl)-1-oxa-3,8-diazaspiro[4.5]decan-2-one
[108]8-(2-chlorobenzoyl)-3-(3,5-dimethylbenzyl)-1-oxa-3,8-diazaspiro[4.5]decan-2-one
[109]3-(3-bromobenzyl)-8-(4-nitrobenzoyl)-1-oxa-3,8-diazaspiro[4.5]decan-2-one
[110] N-{1-benzyl-2-[3-(3-cyanobenzyl)-2-oxo-1-oxa-3,8-diazaspiro[4.5]dec-8-yl]-2-oxoethyl}-4-methylbenzylsulfonamide
[111]8-(2-ethylsulfanylpyridine-3-carbonyl)-3-(3-methoxybenzyl)-1-oxa-3,8-diazaspiro[4.5]decan-2-one
[112]8-(2,4-dimethoxybenzoyl)-3-(3-methoxybenzyl)-1-oxa-3,8-diazaspiro[4.5]decan-2-one
[113]3-(3,5-dimethylbenzyl)-8-[2-(3-methoxyphenyl)acetyl]-1-oxa-3,8-diazaspiro[4.5]decan-2-one
[114]3-(3,5-dimethylbenzyl)-8-(4-methoxy-2,3,6-trimethylbenzylsulfonyl)-1-oxa-3,8-diazaspiro[4.5]decan-2-one
[115]4-[8-(biphenyl-4-carbonyl)-2-oxo-1-oxa-3,8-diazaspiro[4.5]dec-3-ylmethyl]benzonitrile
[116]8-(2-chlorobenzylsulfonyl)-3-(3,5-dimethylbenzyl)-1-oxa-3,8-diazaspiro[4.5]decan-2-one
[117]8-(benzo[1,2,5]oxadiazole-5-carbonyl)-3-(3,5-dimethylbenzyl)-1-oxa-3,8-diazaspiro[4.5]decan-2-one
[118]8-(biphenyl-4-carbonyl)-3-(4-iodobenzyl)-1-oxa-3,8-diazaspiro[4.5]decan-2-one
[119]3-(3,5-dimethylbenzyl)-8-(pyridine-2-carbonyl)-1-oxa-3,8-diazaspiro[4.5]decan-2-one
[120] 8-(6-chloropyrid ine-3-carbonyl)-3-naphthalen-2-ylmethyl-1-oxa-3,8-diazaspiro[4.5]decan-2-one
[121]3-(3-bromobenzyl)-8-(2-ethylsulfanylpyridine-3-carbonyl)-1-oxa-3,8-diazaspiro[4.5]decan-2-one
[122]3-(3,5-dimethylbenzyl)-8-(5-methylisoxazole-3-carbonyl)-1-oxa-3,8-diazaspiro[4.5]decan-2-one
[123]3-(2-methoxy-5-nitrobenzyl)-8-pentanoyl-1-oxa-3,8-diazaspiro[4.5]decan-2-one
[124]8-(4-bromobenzoyl)-3-(3,5-dimethylbenzyl)-1-oxa-3,8-diazaspiro[4.5]decan-2-one
[125]8-(5-tert-butyl-2-methylfuran-3-carbonyl)-3-(2-methoxy-5-nitrobenzyl)-1-oxa-3,8-diazaspiro[4.5]decan-2-one
[126]8-(3-chlorobenzoyl)-3-(3,5-dimethylbenzyl)-1-oxa-3,8-diazaspiro[4.5]decan-2-one
[127]8-(2-ethylsulfanylpyridine-3-carbonyl)-3-(2-trifluoromethylbenzyl)-1-oxa-3,8-diazaspiro[4.5]decan-2-one
[128] 3-(3, 5-dimethylbenzyl)-8-(isoxazole-5-carbonyl)-1-oxa-3,8-diazaspiro[4.5]decan-2-one
[129]3-(3-bromobenzyl)-8-(pyridine-2-carbonyl)-1-oxa-3,8-diazaspiro[4.5]decan-2-one
[130]3-(3,5-dimethylbenzyl)-8-(thiophene-2-carbonyl)-1-oxa-3,8-diazaspiro[4.5]decan-2-one
[131]8-(3-chloro-4-fluorobenzoyl)-3-(3,5-dimethylbenzyl)-1-oxa-3,8-diazaspiro[4.5]decan-2-one
[132]8-(2,6-difluoro-3-methylbenzoyl)-3-(3,5-dimethylbenzyl)-1-oxa-3,8-diazaspiro[4.5]decan-2-one
[133]4-[3-(3-bromobenzyl)-2-oxo-1-oxa-3,8-diazaspiro[4.5]dec-8-yl]-4-oxobutanoic acid methyl ester [134]3-[8-(2-ethylbutyryl)-2-oxo-1-oxa-3,8-diazaspiro[4.5]dec-3-ylmethyl]benzonitrile
[135]3-[8-(3-bromobenzylsulfonyl)-2-oxo-1-oxa-3,8-diazaspiro[4.5]dec-3-ylmethyl]benzonitrile
[136]3-(3,4-difluorobenzyl)-8-(4-methylbenzoyl)-1-oxa-3,8-diazaspiro[4.5]decan-2-one
[137]3-(3-bromobenzyl)-8-(3,5-dimethoxybenzoyl)-1-oxa-3,8-diazaspiro[4.5]decan-2-one
[138] 8-(3-d imethylaminobenzoyl)-3-(2-methoxy-5-nitrobenzyl)-1-oxa-3,8-diazaspiro[4.5]decan-2-one
[139]8-(2,6-dichlorobenzoyl)-3-(2-fluorobenzyl)-1-oxa-3,8-diazaspiro[4.5]decan-2-one
[140]3-(2-methoxy-5-nitrobenzyl)-8-(2-phenoxypyridine-3-carbonyl)-1-oxa-3,8-diazaspiro[4.5]decan-2-one
[141]8-[2-(3-methoxyphenyl)acetyl]-3-naphthalen-2-ylmethyl-1-oxa-3,8-diazaspiro[4.5]decan-2-one
[142]4-{2-oxo-8-[2-(2,2,2-trifluoroacetyl)-1,2,3,4-tetrahydroisoquinoline-7-sulfonyl]-1-oxa-3,8-diazaspiro[4.5]dec-3-ylmethyl}benzonitrile
[143]8-(3,5-bis-trifluoromethylbenzoyl)-3-(4-trifluoromethylbenzyl)-1-oxa-3,8-diazaspiro[4.5]decan-2-one
[144]3-(3-bromobenzyl)-8-(2-phenoxypyridine-3-carbonyl)-1-oxa-3,8-diazaspiro[4.5]decan-2-one
[145]3-(2-methoxy-5-nitrobenzyl)-8-[2-(3-methoxyphenyl)acetyl]-1-oxa-3,8-diazaspiro[4.5]decan-2-one
[146]3-(3,4-difluorobenzyl)-8-(2-phenoxypyridine-3-carbonyl)-1-oxa-3,8-diazaspiro[4.5]decan-2-one
[147]3-[8-(4-bromo-3-methylbenzoyl)-2-oxo-1-oxa-3,8-diazaspiro[4.5]dec-3-ylmethyl]benzonitrile
[148]3-(3-bromobenzyl)-8-(4-propylbenzoyl)-1-oxa-3,8-diazaspiro[4.5]decan-2-one
[149]3-biphenyl-2-ylmethyl-8-(3-chlorobenzylsulfonyl)-1-oxa-3,8-diazaspiro[4.5]decan-2-one
[150]8-[2-(3,4-dimethoxyphenyl)acetyl]-3-(2-methoxy-5-nitrobenzyl)-1-oxa-3,8-diazaspiro[4.5]decan-2-one
[151]2-[8-(2-chloro-4-nitrobenzoyl)-2-oxo-1-oxa-3,8-diazaspiro[4.5]dec-3-ylmethyl]benzonitrile
[152]3-[2-oxo-8-(2,4,6-trimethylbenzylsulfonyl)-1-oxa-3,8-diazaspiro[4.5]dec-3-ylmethyl]benzonitrile
[153]3-benzyl-8-(4-fluorobenzylsulfonyl)-1-oxa-3,8-diazaspiro[4.5]decan-2-one
[154]3-(4-fluorobenzyl)-8-(toluene-4-sulfonyl)-1-oxa-3,8-diazaspiro[4.5]decan-2-one
[155]4-[2-oxo-8-(toluene-4-sulfonyl)-1-oxa-3,8-diazaspiro[4.5]dec-3-ylmethyl]benzonitrile
[156]3-(2-fluorobenzyl)-8-(toluene-4-sulfonyl)-1-oxa-3,8-diazaspiro[4.5]decan-2-one
[157]3-(3,4-difluorobenzyl)-8-(toluene-4-sulfonyl)-1-oxa-3,8-diazaspiro[4.5]decan-2-one
[158]3-(4-tert-butylbenzyl)-8-(toluene-4-sulfonyl)-1-oxa-3,8-diazaspiro[4.5]decan-2-one
[159]8-(toluene-4-sulfonyl)-3-(4-trifluoromethylsulfanylbenzyl)-1-oxa-3,8-diazaspiro[4.5]decan-2-one
[160]2-[3-(4-fluorobenzyl)-2-oxo-1-oxa-3,8-diazaspiro[4.5]decan-8-sulfonyl]benzoic acid methyl ester
[161]2-[3-(3-methoxybenzyl)-2-oxo-1-oxa-3,8-diazaspiro[4.5]decan-8-sulfonyl]benzoic acid methyl ester
[162]2-[3-(3-bromobenzyl)-2-oxo-1-oxa-3,8-diazaspiro[4.5]decan-8-sulfonyl]benzoic acid methyl ester
[163]3-benzyl-8-(2-methanesulfonylbenzylsulfonyl)-1-oxa-3,8-diazaspiro[4.5]decan-2-one
[164]8-(2-methanesulfonylbenzylsulfonyl)-3-(2-methoxy-5-nitrobenzyl)-1-oxa-3,8-diazaspiro[4.5]decan-2-one
[165]3-(4-methylbenzyl)-2-oxo-1-oxa-3,8-diazaspiro[4.5]decane-8-sulfonic acid dimethylamide
[166]4-[8-(7,7-dimethyl-2-oxobicyclo[2.2.1]hept-1-ylmethanesulfonyl)-2-oxo-1-oxa-3,8-diazaspiro[4.5]dec-3-ylmethyl]benzonitrile
[167]3-(4-fluorobenzyl)-8-(4-methoxybenzylsulfonyl)-1-oxa-3,8-diazaspiro[4.5]decan-2-one
[168]3-(4-methylbenzyl)-8-(4-trifluoromethoxybenzylsulfonyl)-1-oxa-3,8-diazaspiro[4.5]decan-2-one
[169] 8-(4-trifluoromethoxybenzylsulfonyl)-3-(4-trifluoromethylsulfanylbenzyl)-1-oxa-3,8-diazaspiro[4.5]decan-2-one
[170]8-(propane-1-sulfonyl)-3-(4-trifluoromethylsulfanylbenzyl)-1-oxa-3,8-diazaspiro[4.5]decan-2-one
[171]3-(2-methoxy-5-nitrobenzyl)-8-(4-propylbenzylsulfonyl)-1-oxa-3,8-diazaspiro[4.5]decan-2-one
[172]3-biphenyl-2-ylmethyl-8-(4-propylbenzylsulfonyl)-1-oxa-3,8-diazaspiro[4.5]decan-2-one
[173]8-(3-bromobenzylsulfonyl)-3-(2-fluorobenzyl)-1-oxa-3,8-diazaspiro[4.5]decan-2-one
[174]8-(3-bromobenzylsulfonyl)-3-(3-bromobenzyl)-1-oxa-3,8-diazaspiro[4.5]decan-2-one
[175]2-{8-[4-(1,1-dimethylpropyl)benzylsulfonyl]-2-oxo-1-oxa-3,8-diazaspiro[4.5]dec-3-ylmethyl}benzonitrile
[176]3-benzyl-8-[4-(1,1-dimethylpropyl)benzylsulfonyl]-1-oxa-3,8-diazaspiro[4.5]decan-2-one
[177]8-[4-(1,1-dimethylpropyl)benzylsulfonyl]-3-(4-iodobenzyl)-1-oxa-3,8-diazaspiro[4.5]decan-2-one
[178]8-[4-(1,1-dimethylpropyl)benzylsulfonyl]-3-(2-methoxy-5-nitrobenzyl)-1-oxa-3,8-diazaspiro[4.5]decan-2-one
[179]8-[4-(1,1-dimethylpropyl)benzylsulfonyl]-3-(4-trifluoromethylbenzyl)-1-oxa-3,8-diazaspiro[4.5]decan-2-one
[180]3-biphenyl-2-ylmethyl-8-[4-(1,1-dimethylpropyl)benzylsulfonyl]-1-oxa-3,8-diazaspiro[4.5]decan-2-one
[181]8-(4,5-dichlorothiophene-2-sulfonyl)-3-(3-methoxybenzyl)-1-oxa-3,8-diazaspiro[4.5]decan-2-one
[182]8-(4,5-dichlorothiophene-2-sulfonyl)-3-(4-trifluoromethylbenzyl)-1-oxa-3,8-diazaspiro[4.5]decan-2-one
[183]3-(3-bromobenzyl)-8-(4,5-dichlorothiophene-2-sulfonyl)-1-oxa-3,8-diazaspiro[4.5]decan-2-one
[184]2-[8-(4-methoxy-2,3,6-trimethylbenzylsulfonyl)-2-oxo-1-oxa-3,8-diazaspiro[4.5]dec-3-ylmethyl]benzonitrile
[185]3-(2-fluorobenzyl)-8-(4-methoxy-2,3,6-trimethylbenzylsulfonyl)-1-oxa-3,8-diazaspiro[4.5]decan-2-one
[186]3-(4-tert-butylbenzyl)-8-[2-(2,2,2-trifluoroacetyl)-1,2,3,4-tetrahydroisoquinoline-7-sulfonyl]-1-oxa-3,8-diazaspiro[4.5]decan-2-one
[187]8-(butane-1-sulfonyl)-3-(3-methoxybenzyl)-1-oxa-3,8-diazaspiro[4.5]decan-2-one
[188]3-(4-methylbenzyl)-8-(thiophene-2-sulfonyl)-1-oxa-3,8-diazaspiro[4.5]decan-2-one
[189]8-(4-chloro-2,5-dimethylbenzylsulfonyl)-3-(4-methylbenzyl)-1-oxa-3,8-diazaspiro[4.5]decan-2-one
[190]3-benzyl-8-(4-chloro-2,5-dimethylbenzylsulfonyl)-1-oxa-3,8-diazaspiro[4.5]decan-2-one
[191]8-(4-chloro-2,5-dimethylbenzylsulfonyl)-3-(4-trifluoromethylbenzyl)-1-oxa-3,8-diazaspiro[4.5]decan-2-one
[192]3-biphenyl-2-ylmethyl-8-(4-chloro-2,5-dimethylbenzylsulfonyl)-1-oxa-3,8-diazaspiro[4.5]decan-2-one
[193]8-(4-chloro-2,5-dimethylbenzylsulfonyl)-3-(4-trifluoromethylsulfanylbenzyl)-1-oxa-3,8-diazaspiro[4.5]decan-2-one
[194]3-(4-fluorobenzyl)-8-(2-trifluoromethylbenzylsulfonyl)-1-oxa-3,8-diazaspiro[4.5]decan-2-one
[195]3-(3-methoxybenzyl)-8-(2-trifluoromethylbenzylsulfonyl)-1-oxa-3,8-diazaspiro[4.5]decan-2-one
[196]3-benzyl-8-(2-methyl-5-nitrobenzylsulfonyl)-1-oxa-3,8-diazaspiro[4.5]decan-2-one
[197]3-(4-tert-butylbenzyl)-8-(2-methyl-5-nitrobenzylsulfonyl)-1-oxa-3,8-diazaspiro[4.5]decan-2-one
[198]3-(4-tert-butylbenzyl)-8-(toluene-3-sulfonyl)-1-oxa-3,8-diazaspiro[4.5]decan-2-one
[199]8-(furan-2-carbonyl)-3-(4-trifluoromethylbenzyl)-1-oxa-3,8-diazaspiro[4.5]decan-2-one
[200]3-(3-bromobenzyl)-8-(furan-2-carbonyl)-1-oxa-3,8-diazaspiro[4.5]decan-2-one
[201]4-[8-(naphthalene-1-carbonyl)-2-oxo-1-oxa-3,8-diazaspiro[4.5]dec-3-ylmethyl]benzonitrile
[202]3-(3,4-difluorobenzyl)-8-(naphthalene-1-carbonyl)-1-oxa-3,8-diazaspiro[4.5]decan-2-one
[203]3-(3-bromobenzyl)-8-(naphthalene-1-carbonyl)-1-oxa-3,8-diazaspiro[4.5]decan-2-one
[204]2-[8-(3-nitrobenzoyl)-2-oxo-1-oxa-3,8-diazaspiro[4.5]dec-3-ylmethyl]benzonitrile
[205]4-[8-(3-nitrobenzoyl)-2-oxo-1-oxa-3,8-diazaspiro[4.5]dec-3-ylmethyl]benzonitrile
[206]3-(3-bromobenzyl)-8-(3,5-difluorobenzoyl)-1-oxa-3,8-diazaspiro[4.5]decan-2-one
[207]8-(2-benzyloxy-acetyl)-3-(2-fluorobenzyl)-1-oxa-3,8-diazaspiro[4.5]decan-2-one
[208]8-(2-chlorobenzoyl)-3-(2-trifluoromethylbenzyl)-1-oxa-3,8-diazaspiro[4.5]decan-2-one
[209]8-(2-chlorobenzoyl)-3-(4-iodobenzyl)-1-oxa-3,8-diazaspiro[4.5]decan-2-one
[210]8-(2-chlorobenzoyl)-3-(2-methoxy-5-nitrobenzyl)-1-oxa-3,8-diazaspiro[4.5]decan-2-one
[211]3-biphenyl-2-ylmethyl-8-(2-chlorobenzoyl)-1-oxa-3,8-diazaspiro[4.5]decan-2-one
[212]8-(2,6-dichlorobenzoyl)-3-naphthalen-2-ylmethyl-1-oxa-3,8-diazaspiro[4.5]decan-2-one
[213]3-(4-tert-butylbenzyl)-8-(2,6-dichlorobenzoyl)-1-oxa-3,8-diazaspiro[4.5]decan-2-one
[214]3-(3-bromobenzyl)-8-(2,6-dichlorobenzoyl)-1-oxa-3,8-diazaspiro[4.5]decan-2-one
[215]8-(2,6-dichlorobenzoyl)-3-(4-trifluoromethylsulfanylbenzyl)-1-oxa-3,8-diazaspiro[4.5]decan-2-one
[216]2-[8-(2-methylbenzoyl)-2-oxo-1-oxa-3,8-diazaspiro[4.5]dec-3-ylmethyl]benzonitrile
[217]8-(2-methylbenzoyl)-3-(4-methylbenzyl)-1-oxa-3,8-diazaspiro[4.5]decan-2-one
[218]8-(2-methylbenzoyl)-3-(2-trifluoromethylbenzyl)-1-oxa-3,8-diazaspiro[4.5]decan-2-one
[219]3-(2-methoxy-5-nitrobenzyl)-8-(2-methylbenzoyl)-1-oxa-3,8-diazaspiro[4.5]decan-2-one
[220] 3-(3,4-d ifluorobenzyl)-8-(2-methylbenzoyl)-1-oxa-3,8-diazaspiro[4.5]decan-2-one
[221]8-(2-methylbenzoyl)-3-(4-trifluoromethylbenzyl)-1-oxa-3,8-diazaspiro[4.5]decan-2-one
[222]8-(2-methylbenzoyl)-3-(4-trifluoromethylsulfanylbenzyl)-1-oxa-3,8-diazaspiro[4.5]decan-2-one
[223]8-(3-bromobenzoyl)-3-(4-fluorobenzyl)-1-oxa-3,8-diazaspiro[4.5]decan-2-one
[224]8-(3-bromobenzoyl)-3-(2-trifluoromethylbenzyl)-1-oxa-3,8-diazaspiro[4.5]decan-2-one
[225]8-(3-bromobenzoyl)-3-(2-fluorobenzyl)-1-oxa-3,8-diazaspiro[4.5]decan-2-one
[226]8-(3-bromobenzoyl)-3-(3,4-difluorobenzyl)-1-oxa-3,8-diazaspiro[4.5]decan-2-one
[227]8-(3-bromobenzoyl)-3-naphthalen-2-ylmethyl-1-oxa-3,8-diazaspiro[4.5]decan-2-one
[228]8-(3-bromobenzoyl)-3-(4-tert-butylbenzyl)-1-oxa-3,8-diazaspiro[4.5]decan-2-one
[229]3-benzyl-8-(3-fluorobenzoyl)-1-oxa-3,8-diazaspiro[4.5]decan-2-one
[230]8-(3-fluorobenzoyl)-3-(2-fluorobenzyl)-1-oxa-3,8-diazaspiro[4.5]decan-2-one
[231]3-(3,4-difluorobenzyl)-8-(3-fluorobenzoyl)-1-oxa-3,8-diazaspiro[4.5]decan-2-one
[232]8-(3-fluorobenzoyl)-3-(4-trifluoromethylbenzyl)-1-oxa-3,8-diazaspiro[4.5]decan-2-one
[233]3-(3-bromobenzyl)-8-(3-fluorobenzoyl)-1-oxa-3,8-diazaspiro[4.5]decan-2-one
[234]8-(3-chlorobenzoyl)-3-(3,5-dimethylbenzyl)-1-oxa-3,8-diazaspiro[4.5]decan-2-one
[235]3-benzyl-8-(3-chlorobenzoyl)-1-oxa-3,8-diazaspiro[4.5]decan-2-one
[236]4-[8-(3,4-dichlorobenzoyl)-2-oxo-1-oxa-3,8-diazaspiro[4.5]dec-3-ylmethyl]benzonitrile
[237]3-(4-iodobenzyl)-8-(3-methoxybenzoyl)-1-oxa-3,8-diazaspiro[4.5]decan-2-one
[238]3-(2-fluorobenzyl)-8-(3-methoxybenzoyl)-1-oxa-3,8-diazaspiro[4.5]decan-2-one
[239]3-(4-tert-butylbenzyl)-8-(3-methoxybenzoyl)-1-oxa-3,8-diazaspiro[4.5]decan-2-one
[240]3-benzyl-8-(3-methylbenzoyl)-1-oxa-3,8-diazaspiro[4.5]decan-2-one
[241]3-(2-methoxy-5-nitrobenzyl)-8-(3-methylbenzoyl)-1-oxa-3,8-diazaspiro[4.5]decan-2-one
[242]3-(4-tert-butylbenzyl)-8-(3-methylbenzoyl)-1-oxa-3,8-diazaspiro[4.5]decan-2-one
[243]8-(3-methylbenzoyl)-3-(4-trifluoromethylbenzyl)-1-oxa-3,8-diazaspiro[4.5]decan-2-one
[244]3-(3,4-d ifluorobenzyl)-8-(2-phenyl-butyryl)-1-oxa-3,8-diazaspiro[4.5]decan-2-one
[245]3-benzyl-8-[3-(2-chlorophenyl)-5-methylisoxazole-4-carbonyl]-1-oxa-3,8-diazaspiro[4.5]decan-2-one
[246]4-[8-(2,3-dichlorobenzoyl)-2-oxo-1-oxa-3,8-diazaspiro[4.5]dec-3-ylmethyl]benzonitrile
[247]8-[2-(4-chlorophenyl)acetyl]-3-(3,4-difluorobenzyl)-1-oxa-3,8-diazaspiro[4.5]decan-2-one
[248]3-biphenyl-2-ylmethyl-8-[2-(4-chlorophenyl)acetyl]-1-oxa-3,8-diazaspiro[4.5]decan-2-one
[249]8-[2-(4-chlorophenyl)acetyl]-3-(4-trifluoromethylsulfanylbenzyl)-1-oxa-3,8-diazaspiro[4.5]decan-2-one
[250]3-(4-tert-butylbenzyl)-8-[3-(2-chlorophenyl)acryloyl]-1-oxa-3,8-diazaspiro[4.5]decan-2-one
[251]3-biphenyl-2-ylmethyl-8-[3-(2-chlorophenyl)acryloyl]-1-oxa-3,8-diazaspiro[4.5]decan-2-one
[252]3-(3-bromobenzyl)-8-[3-(2-chlorophenyl)acryloyl]-1-oxa-3,8-diazaspiro[4.5]decan-2-one
[253]4-[8-(2,3-difluorobenzoyl)-2-oxo-1-oxa-3,8-diazaspiro[4.5]dec-3-ylmethyl]benzonitrile
[254]8-(2,3-difluorobenzoyl)-3-(2-trifluoromethylbenzyl)-1-oxa-3,8-diazaspiro[4.5]decan-2-one
[255]8-(2,3-difluorobenzoyl)-3-(4-iodobenzyl)-1-oxa-3,8-diazaspiro[4.5]decan-2-one
[256]2-[2-oxo-8-(2-propylpentanoyl)-1-oxa-3,8-diazaspiro[4.5]dec-3-ylmethyl]benzonitrile
[257]3-(3,4-difluorobenzyl)-8-(2-propylpentanoyl)-1-oxa-3,8-diazaspiro[4.5]decan-2-one
[258]8-(2-chloro-4-nitrobenzoyl)-31-(4-methylbenzyl)-1-oxa-3,8-diazaspiro[4.5]decan-2-one
[259]8-(2-chloro-4-nitrobenzoyl)-31-(2-trifluoromethylbenzyl)-1-oxa-3,8-diazaspiro[4.5]decan-2-one
[260]3-(4-tert-butylbenzyl)-8-(2-chloro-4-nitrobenzoyl)-1-oxa-3,8-diazaspiro[4.5]decan-2-one
[261]8-(2-chloro-4-nitrobenzoyl)-31-(4-trifluoromethylbenzyl)-1-oxa-3,8-diazaspiro[4.5]decan-2-one
[262]3-biphenyl-2-ylmethyl-8-(2-chloro-4-nitrobenzoyl)-1-oxa-3,8-diazaspiro[4.5]decan-2-one
[263]3-(3-bromobenzyl)-8-(2-chloro-4-nitrobenzoyl)-1-oxa-3,8-diazaspiro[4.5]decan-2-one
[264]8-(2-chloro-4-nitrobenzoyl)-31-(4-trifluoromethylsulfanylbenzyl)-1-oxa-3,8-diazaspiro[4.5]decan-2-one
[265]8-(2-chloropyridine-3-carbonyl)-3-(4-fluorobenzyl)-1-oxa-3,8-diazaspiro[4.5]decan-2-one
[266]2-[8-(2-chloropyridine-3-carbonyl)-2-oxo-1-oxa-3,8-diazaspiro[4.5]dec-3-ylmethyl]benzonitrile
[267]8-(2-chloropyridine-3-carbonyl)-3-(4-methylbenzyl)-1-oxa-3,8-diazaspiro[4.5]decan-2-one
[268]8-(2-chloropyridine-3-carbonyl)-3-(4-trifluoromethylbenzyl)-1-oxa-3,8-diazaspiro[4.5]decan-2-one
[269]3-(3-methoxybenzyl)-8-(2-methylsulfanylpyridine-3-carbonyl)-1-oxa-3,8-diazaspiro[4.5]decan-2-one
[270]3-(4-methylbenzyl)-8-(2-methylsulfanylpyridine-3-carbonyl)-1-oxa-3,8-diazaspiro[4.5]decan-2-one
[271]3-(4-iodobenzyl)-8-(2-methylsulfanylpyridine-3-carbonyl)-1-oxa-3,8-diazaspiro[4.5]decan-2-one
[272]3-(3,4-difluorobenzyl)-8-(2-methylsulfanylpyridine-3-carbonyl)-1-oxa-3,8-diazaspiro[4.5]decan-2-one
[273]3-(4-tert-butylbenzyl)-8-(2-methylsulfanylpyridine-3-carbonyl)-1-oxa-3,8-diazaspiro[4.5]decan-2-one
[274]8-(2-methylsulfanylpyridine-3-carbonyl)-3-(4-trifluoromethylbenzyl)-1-oxa-3,8-diazaspiro[4.5]decan-2-one
[275]8-(2-ethylsulfanylpyridine-3-carbonyl)-3-(4-fluorobenzyl)-1-oxa-3,8-diazaspiro[4.5]decan-2-one
[276]3-[8-(2-ethylsulfanylpyridine-3-carbonyl)-2-oxo-1-oxa-3,8-diazaspiro[4.5]dec-3-ylmethyl]benzonitrile
[277]2-[8-(2-ethylsulfanylpyridine-3-carbonyl)-2-oxo-1-oxa-3,8-diazaspiro[4.5]dec-3-ylmethyl]benzonitrile
[278]8-(2-ethylsulfanylpyridine-3-carbonyl)-3-(4-methylbenzyl)-1-oxa-3,8-diazaspiro[4.5]decan-2-one
[279]3-benzyl-8-(2-ethylsulfanylpyridine-3-carbonyl)-1-oxa-3,8-diazaspiro[4.5]decan-2-one
[280]8-(2-ethylsulfanylpyridine-3-carbonyl)-3-(2-fluorobenzyl)-1-oxa-3,8-diazaspiro[4.5]decan-2-one
[281]4-[8-(6-chloropyridine-3-carbonyl)-2-oxo-1-oxa-3,8-diazaspiro[4.5]dec-3-ylmethyl]benzonitrile
[282]8-(6-chloropyridine-3-carbonyl)-3-(2-trifluoromethylbenzyl)-1-oxa-3,8-diazaspiro[4.5]decan-2-one
[283]8-(6-chloropyridine-3-carbonyl)-3-(4-iodobenzyl)-1-oxa-3,8-diazaspiro[4.5]decan-2-one
[284]3-(4-tert-butylbenzyl)-8-(6-chloropyridine-3-carbonyl)-1-oxa-3,8-diazaspiro[4.5]decan-2-one
[285]3-biphenyl-2-ylmethyl-8-(6-chloropyridine-3-carbonyl)-1-oxa-3,8-diazaspiro[4.5]decan-2-one
[286]3-(3-methoxybenzyl)-8-(4-trifluoromethoxybenzoyl)-1-oxa-3,8-diazaspiro[4.5]decan-2-one
[287]3-(3,4-difluorobenzyl)-8-(4-trifluoromethoxybenzoyl)-1-oxa-3,8-diazaspiro[4.5]decan-2-one
[288]8-[3-(2-chloro-6-fluorophenyl)-5-methylisoxazole-4-carbonyl]-3-(4-fluorobenzyl)-1-oxa-3,8-diazaspiro[4.5]decan-2-one
[289]8-[3-(2-chloro-6-fluorophenyl)-5-methylisoxazole-4-carbonyl]-3-(3-methoxybenzyl)-1-oxa-3,8-diazaspiro[4.5]decan-2-one
[290]8-[3-(2-chloro-6-fluorophenyl)-5-methylisoxazole-4-carbonyl]-3-(4-trifluoromethylbenzyl)-1-oxa-3,8-diazaspiro[4.5]decan-2-one
[291]8-[3-(2-chloro-6-fluorophenyl)-5-methylisoxazole-4-carbonyl]-3-(4-trifluoromethylsulfanylbenzyl)-1-oxa-3,8-diazaspiro[4.5]decan-2-one
[292]3-(4-methylbenzyl)-8-(3-trifluoromethoxybenzoyl)-1-oxa-3,8-diazaspiro[4.5]decan-2-one
[293]3-(4-iodobenzyl)-8-(3-trifluoromethoxybenzoyl)-1-oxa-3,8-diazaspiro[4.5]decan-2-one
[294]3-(3,4-difluorobenzyl)-8-(3-trifluoromethoxybenzoyl)-1-oxa-3,8-diazaspiro[4.5]decan-2-one
[295]3-naphthalen-2-ylmethyl-8-(3-trifluoromethoxybenzoyl)-1-oxa-3,8-diazaspiro[4.5]decan-2-one
[296]4-[8-(isoxazole-5-carbonyl)-2-oxo-1-oxa-3,8-diazaspiro[4.5]dec-3-ylmethyl]benzonitrile
[297]8-(isoxazole-5-carbonyl)-3-(2-trifluoromethylbenzyl)-1-oxa-3,8-diazaspiro[4.5]decan-2-one
[298]8-(isoxazole-5-carbonyl)-3-(2-methoxy-5-nitrobenzyl)-1-oxa-3,8-diazaspiro[4.5]decan-2-one
[299]3-(3-bromobenzyl)-8-(isoxazole-5-carbonyl)-1-oxa-3,8-diazaspiro[4.5]decan-2-one
[300]2-[8-(2-chloro-6-fluorobenzoyl)-2-oxo-1-oxa-3,8-diazaspiro[4.5]dec-3-ylmethyl]benzonitrile
[301]4-[8-(2-chloro-6-fluorobenzoyl)-2-oxo-1-oxa-3,8-diazaspiro[4.5]dec-3-ylmethyl]benzonitrile
[302]8-(2-chloro-6-fluorobenzoyl)-3-(4-methylbenzyl)-1-oxa-3,8-diazaspiro[4.5]decan-2-one
[303]3-biphenyl-2-ylmethyl-8-(2-chloro-6-fluorobenzoyl)-1-oxa-3,8-diazaspiro[4.5]decan-2-one
[304]8-(2,5-dimethylfuran-3-carbonyl)-3-(3-methoxybenzyl)-1-oxa-3,8-diazaspiro[4.5]decan-2-one
[305] 4-[8-(2, 5-d imethylfuran-3-carbonyl)-2-oxo-1-oxa-3,8-d iazaspiro[4.5]dec-3-ylmethyl]benzonitrile
[306] 4-[8-(4-bromo-3-methylbenzoyl)-2-oxo-1-oxa-3,8-d iazaspiro[4.5]dec-3-ylmethyl]benzonitrile
[307]8-(4-bromo-3-methylbenzoyl)-3-(4-methylbenzyl)-1-oxa-3,8-diazaspiro[4.5]decan-2-one
[308]3-benzyl-8-(4-bromo-3-methylbenzoyl)-1-oxa-3,8-diazaspiro[4.5]decan-2-one
[309]8-(4-bromo-3-methylbenzoyl)-3-(2-trifluoromethylbenzyl)-1-oxa-3,8-diazaspiro[4.5]decan-2-one
[310]8-(4-bromo-3-methylbenzoyl)-3-(3,4-difluorobenzyl)-1-oxa-3,8-diazaspiro[4.5]decan-2-one
[311]8-(4-bromo-3-methylbenzoyl)-3-(4-tert-butylbenzyl)-1-oxa-3,8-diazaspiro[4.5]decan-2-one
[312]3-biphenyl-2-ylmethyl-8-(4-bromo-3-methylbenzoyl)-1-oxa-3,8-diazaspiro[4.5]decan-2-one
[313]8-(4-bromo-3-methylbenzoyl)-3-(4-trifluoromethylsulfanylbenzyl)-1-oxa-3,8-diazaspiro[4.5]decan-2-one
[314]8-(2,6-difluoro-3-methylbenzoyl)-3-(3-methoxybenzyl)-1-oxa-3,8-diazaspiro[4.5]decan-2-one
[315]2-[8-(2,6-difluoro-3-methylbenzoyl)-2-oxo-1-oxa-3,8-diazaspiro[4.5]dec-3-ylmethyl]benzonitrile
[316]4-[8-(2,6-difluoro-3-methylbenzoyl)-2-oxo-1-oxa-3,8-diazaspiro[4.5]dec-3-ylmethyl]benzonitrile
[317]8-(2,6-difluoro-3-methylbenzoyl)-3-(2-trifluoromethylbenzyl)-1-oxa-3,8-diazaspiro[4.5]decan-2-one
[318]8-(2,6-difluoro-3-methylbenzoyl)-3-(2-fluorobenzyl)-1-oxa-3,8-diazaspiro[4.5]decan-2-one
[319]8-(2-chloro-6-fluoro-3-methylbenzoyl)-3-(4-fluorobenzyl)-1-oxa-3,8-diazaspiro[4.5]decan-2-one
[320]8-(2-chloro-6-fluoro-3-methylbenzoyl)-3-(3-methoxybenzyl)-1-oxa-3,8-diazaspiro[4.5]decan-2-one
[321]8-(2-chloro-6-fluoro-3-methylbenzoyl)-3-(4-trifluoromethylsulfanylbenzyl)-1-oxa-3,8-diazaspiro[4.5]decan-2-one
[322]3-biphenyl-2-ylmethyl-8-(6-chloro-2-fluoro-3-methylbenzoyl)-1-oxa-3,8-diazaspiro[4.5]decan-2-one
[323]3-benzyl-8-(3-difluoromethylsulfanylbenzoyl)-1-oxa-3,8-diazaspiro[4.5]decan-2-one
[324]8-(3-difluoromethylsulfanylbenzoyl)-3-naphthalen-2-ylmethyl-1-oxa-3,8-diazaspiro[4.5]decan-2-one
[325]8-(3-difluoromethylsulfanylbenzoyl)-3-(4-trifluoromethylbenzyl)-1-oxa-3,8-diazaspiro[4.5]decan-2-one
[326]3-(3-bromobenzyl)-8-(3-difluoromethylsulfanylbenzoyl)-1-oxa-3,8-diazaspiro[4.5]decan-2-one
[327]3-[8-(3-chloro-2-fluorobenzoyl)-2-oxo-1-oxa-3,8-diazaspiro[4.5]dec-3-ylmethyl]benzonitrile
[328]8-(3-chloro-2-fluorobenzoyl)-3-(4-iodobenzyl)-1-oxa-3,8-diazaspiro[4.5]decan-2-one
[329]8-(3-chloro-2-fluorobenzoyl)-3-naphthalen-2-ylmethyl-1-oxa-3,8-diazaspiro[4.5]decan-2-one
[330]3-(3-methoxybenzyl)-8-(4-trifluoromethylsulfanylbenzoyl)-1-oxa-3,8-diazaspiro[4.5]decan-2-one
[331]2-[2-oxo-8-(4-trifluoromethylsulfanylbenzoyl)-1-oxa-3,8-diazaspiro[4.5]dec-3-ylmethyl]benzonitrile
[332]4-[2-oxo-8-(4-trifluoromethylsulfanylbenzoyl)-1-oxa-3,8-diazaspiro[4.5]dec-3-ylmethyl]benzonitrile
[333]3-(4-iodobenzyl)-8-(4-trifluoromethylsulfanylbenzoyl)-1-oxa-3,8-diazaspiro[4.5]decan-2-one
[334]3-naphthalen-2-ylmethyl-8-(4-trifluoromethylsulfanylbenzoyl)-1-oxa-3,8-diazaspiro[4.5]decan-2-one
[335]3-(4-trifluoromethylbenzyl)-8-(4-trifluoromethylsulfanylbenzoyl)-1-oxa-3,8-diazaspiro[4.5]decan-2-one
[336]8-(2-chloro-5-trifluoromethylbenzoyl)-3-(2-trifluoromethylbenzyl)-1-oxa-3,8-diazaspiro[4.5]decan-2-one
[337]8-(2-chloro-5-trifluoromethylbenzoyl)-3-(2-fluorobenzyl)-1-oxa-3,8-diazaspiro[4.5]decan-2-one
[338]8-(2-chloro-5-trifluoromethylbenzoyl)-3-(3,4-difluorobenzyl)-1-oxa-3,8-diazaspiro[4.5]decan-2-one
[339]8-(2,3-difluoro-4-methylbenzoyl)-3-(3-methoxybenzyl)-1-oxa-3,8-diazaspiro[4.5]decan-2-one
[340]4-[8-(2,3-difluoro-4-methylbenzoyl)-2-oxo-1-oxa-3,8-diazaspiro[4.5]dec-3-ylmethyl]benzonitrile
[341]3-benzyl-8-(2,3-difluoro-4-methylbenzoyl)-1-oxa-3,8-diazaspiro[4.5]decan-2-one
[342]8-(2,3-difluoro-4-methylbenzoyl)-3-(4-iodobenzyl)-1-oxa-3,8-diazaspiro[4.5]decan-2-one
[343]3-(3,4-difluorobenzyl)-8-(2,3-difluoro-4-methylbenzoyl)-1-oxa-3,8-diazaspiro[4.5]decan-2-one
[344]8-(2,3-difluoro-4-methylbenzoyl)-3-naphthalen-2-ylmethyl-1-oxa-3,8-diazaspiro[4.5]decan-2-one
[345]3-biphenyl-2-ylmethyl-8-(2,3-difluoro-4-methylbenzoyl)-1-oxa-3,8-diazaspiro[4.5]decan-2-one
[346]3-(3-bromobenzyl)-8-(2,3-difluoro-4-methylbenzoyl)-1-oxa-3,8-diazaspiro[4.5]decan-2-one
[347]3-benzyl-8-[2-(2-methoxyethoxy)acetyl]-1-oxa-3,8-diazaspiro[4.5]decan-2-one
[348]3-(2-fluorobenzyl)-8-[2-(2-methoxyethoxy)acetyl]-1-oxa-3,8-diazaspiro[4.5]decan-2-one
[349]3-(3,4-difluorobenzyl)-8-[2-(2-methoxyethoxy)acetyl]-1-oxa-3,8-diazaspiro[4.5]decan-2-one
[350]3-(4-tert-butylbenzyl)-8-[2-(2-methoxyethoxy)acetyl]-1-oxa-3,8-diazaspiro[4.5]decan-2-one
[351]8-[2-(2-methoxyethoxy)acetyl]-3-(4-trifluoromethylsulfanylbenzyl)-1-oxa-3,8-diazaspiro[4.5]decan-2-one
[352]8-(1-acetylpiperidine-4-carbonyl)-3-(3,4-difluorobenzyl)-1-oxa-3,8-diazaspiro[4.5]decan-2-one
[353]4-{8-[2-(3-chlorophenoxy)acetyl]-2-oxo-1-oxa-3,8-diazaspiro[4.5]dec-3-ylmethyl}benzonitrile
[354]8-[2-(3-chlorophenoxy)acetyl]-3-(3,4-difluorobenzyl)-1-oxa-3,8-diazaspiro[4.5]decan-2-one
[355]3-(3-bromobenzyl)-8-[2-(3-chlorophenoxy)acetyl]-1-oxa-3,8-diazaspiro[4.5]decan-2-one
[356]8-[2-(3-chlorophenoxy)acetyl]-3-(4-trifluoromethylsulfanylbenzyl)-1-oxa-3,8-diazaspiro[4.5]decan-2-one
[357]4-[3-(3-methoxybenzyl)-2-oxo-1-oxa-3,8-diazaspiro[4.5]decane-8-carbonyl]benzylsulfonamide
[358]N-{4-[3-(3,4-difluorobenzyl)-2-oxo-1-oxa-3,8-diazaspiro[4.5]decane-8-carbonyl]phenyl}acetamide
[359]8-(3-dimethylaminobenzoyl)-3-(3,5-dimethylbenzyl)-1-oxa-3,8-diazaspiro[4.5]decan-2-one
[360]3-(4-tert-butylbenzyl)-8-(3-dimethylaminobenzoyl)-1-oxa-3,8-diazaspiro[4.5]decan-2-one
[361]3-(3-bromobenzyl)-8-(3-dimethylaminobenzoyl)-1-oxa-3,8-diazaspiro[4.5]decan-2-one
[362]3-(4-methylbenzyl)-8-(4-phenoxybutyryl)-1-oxa-3,8-diazaspiro[4.5]decan-2-one
[363]8-(4-phenoxybutyryl)-3-(2-trifluoromethylbenzyl)-1-oxa-3,8-diazaspiro[4.5]decan-2-one
[364]3-(2-fluorobenzyl)-8-(4-phenoxybutyryl)-1-oxa-3,8-diazaspiro[4.5]decan-2-one
[365]8-(2,3-dimethylbenzoyl)-3-(4-fluorobenzyl)-1-oxa-3,8-diazaspiro[4.5]decan-2-one
[366]3-[8-(2,3-dimethylbenzoyl)-2-oxo-1-oxa-3,8-diazaspiro[4.5]dec-3-ylmethyl]benzonitrile
[367]8-(2,3-dimethylbenzoyl)-3-(3,5-dimethylbenzyl)-1-oxa-3,8-diazaspiro[4.5]decan-2-one
[368]8-(2,3-dimethylbenzoyl)-3-(4-methylbenzyl)-1-oxa-3,8-diazaspiro[4.5]decan-2-one
[369]8-(2,3-dimethylbenzoyl)-3-(2-trifluoromethylbenzyl)-1-oxa-3,8-diazaspiro[4.5]decan-2-one
[370]8-(2,3-dimethylbenzoyl)-3-(2-methoxy-5-nitrobenzyl)-1-oxa-3,8-diazaspiro[4.5]decan-2-one
[371]8-(2,3-dimethylbenzoyl)-3-(4-trifluoromethylbenzyl)-1-oxa-3,8-diazaspiro[4.5]decan-2-one
[372]3-biphenyl-2-ylmethyl-8-(2,3-dimethylbenzoyl)-1-oxa-3,8-diazaspiro[4.5]decan-2-one
[373]4-[8-(5-methyl-2-phenyl-2H-[1,2,3]triazole-4-carbonyl)-2-oxo-1-oxa-3,8-diazaspiro[4.5]dec-3-ylmethyl]benzonitrile
[374]3-benzyl-8-(5-methyl-2-phenyl-2H-[1,2,3]triazole-4-carbonyl)-1-oxa-3,8-diazaspiro[4.5]decan-2-one
[375]3-(3,4-difluorobenzyl)-8-(5-methyl-2-phenyl-2H-[1,2,3]triazole-4-carbonyl)-1-oxa-3,8-diazaspiro[4.5]decan-2-one
[376]8-(5-methyl-2-phenyl-2H-[1,2,3]triazole-4-carbonyl)-3-naphthalen-2-ylmethyl-1-oxa-3,8-diazaspiro[4.5]decan-2-one
[377] 3-biphenyl-2-ylmethyl-8-(5-methyl-2-phenyl-2H-[1,2,3]triazole-4-carbonyl)-1-oxa-3,8-diazaspiro[4.5]decan-2-one
[378]3-(4-tert-butylbenzyl)-8-[3-(2,6-dichlorophenyl)-5-methylisoxazole-4-carbonyl]-1-oxa-3,8-diazaspiro[4.5]decan-2-one
[379]3-[2-oxo-8-(2-phenoxypyridine-3-carbonyl)-1-oxa-3,8-diazaspiro[4.5]dec-3-ylmethyl]benzonitrile
[380]2-[2-oxo-8-(2-phenoxypyridine-3-carbonyl)-1-oxa-3,8-diazaspiro[4.5]dec-3-ylmethyl]benzonitrile
[381]3-(4-fluorobenzyl)-8-(pyridine-2-carbonyl)-1-oxa-3,8-diazaspiro[4.5]decan-2-one
[382]3-(3-methoxybenzyl)-8-(pyridine-2-carbonyl)-1-oxa-3,8-diazaspiro[4.5]decan-2-one
[383]3-(3,5-dimethylbenzyl)-8-(pyridine-2-carbonyl)-1-oxa-3,8-diazaspiro[4.5]decan-2-one
[384]2-[2-oxo-8-(pyridine-2-carbonyl)-1-oxa-3,8-diazaspiro[4.5]dec-3-ylmethyl]benzonitrile
[385]3-(2-fluorobenzyl)-8-(pyridine-2-carbonyl)-1-oxa-3,8-diazaspiro[4.5]decan-2-one
[386]3-(3,4-difluorobenzyl)-8-(pyridine-2-carbonyl)-1-oxa-3,8-diazaspiro[4.5]decan-2-one
[387]3-biphenyl-2-ylmethyl-8-(pyridine-2-carbonyl)-1-oxa-3,8-diazaspiro[4.5]decan-2-one
[388]4-[8-(3-chlorothiophene-2-carbonyl)-2-oxo-1-oxa-3,8-diazaspiro[4.5]dec-3-ylmethyl]benzonitrile
[389]8-(3-chlorothiophene-2-carbonyl)-3-(3,4-difluorobenzyl)-1-oxa-3,8-diazaspiro[4.5]decan-2-one
[390]3-biphenyl-2-ylmethyl-8-(3-chlorothiophene-2-carbonyl)-1-oxa-3,8-diazaspiro[4.5]decan-2-one
[391]8-[1-(4-chlorophenyl)-5-trifluoromethyl-1H-pyrazole-4-carbonyl]-3-(2-fluorobenzyl)-1-oxa-3,8-diazaspiro[4.5]decan-2-one
[392]8-[1-(4-chlorophenyl)-5-trifluoromethyl-1H-pyrazole-4-carbonyl]-3-(3,4-difluorobenzyl)-1-oxa-3,8-diazaspiro[4.5]decan-2-one
[393]8-[1-(4-chlorophenyl)-5-trifluoromethyl-1H-pyrazole-4-carbonyl]-3-(4-trifluoromethylsulfanylbenzyl)-1-oxa-3,8-diazaspiro[4.5]decan-2-one
[394]3-(4-tert-butylbenzyl)-8-(5-tert-butyl-2-methyl-2H-pyrazole-3-carbonyl)-1-oxa-3,8-diazaspiro[4.5]decan-2-one
[395]3-[8-(5-methylisoxazole-3-carbonyl)-2-oxo-1-oxa-3,8-d iazaspiro[4.5]dec-3-ylmethyl]benzonitrile
[396]3-(3,5-dimethylbenzyl)-8-(5-methylisoxazole-3-carbonyl)-1-oxa-3,8-diazaspiro[4.5]decan-2-one
[397]2-[8-(5-methylisoxazole-3-carbonyl)-2-oxo-1-oxa-3,8-diazaspiro[4.5]dec-3-ylmethyl]benzonitrile
[398]4-[8-(5-methylisoxazole-3-carbonyl)-2-oxo-1-oxa-3,8-diazaspiro[4.5]dec-3-ylmethyl]benzonitrile
[399]3-benzyl-8-(5-methylisoxazole-3-carbonyl)-1-oxa-3,8-diazaspiro[4.5]decan-2-one
[400]3-(3,4-difluorobenzyl)-8-(5-methylisoxazole-3-carbonyl)-1-oxa-3,8-diazaspiro[4.5]decan-2-one
[401]3-biphenyl-2-ylmethyl-8-(5-methylisoxazole-3-carbonyl)-1-oxa-3,8-diazaspiro[4.5]decan-2-one
[402]8-(4-methyl[1,2,3]thiadiazole-5-carbonyl)-3-(2-trifluoromethylbenzyl)-1-oxa-3,8-diazaspiro[4.5]decan-2-one
[403]3-(4-tert-butylbenzyl)-8-(4-methyl[1,2,3]thiadiazole-5-carbonyl)-1-oxa-3,8-diazaspiro[4.5]decan-2-one
[404]8-(4-methyl[1,2,3]thiadiazole-5-carbonyl)-3-(4-trifluoromethylsulfanylbenzyl)-1-oxa-3,8-diazaspiro[4.5]decan-2-one
[405]4-[2-oxo-8-(1-phenyl-5-propyl-1H-pyrazole-4-carbonyl)-1-oxa-3,8-diazaspiro[4.5]dec-3-ylmethyl]benzonitrile
[406]3-(3,4-difluorobenzyl)-8-(1-phenyl-5-propyl-1H-pyrazole-4-carbonyl)-1-oxa-3,8-diazaspiro[4.5]decan-2-one
[407]3-naphthalen-2-ylmethyl-8-(1-phenyl-5-propyl-1H-pyrazole-4-carbonyl)-1-oxa-3,8-diazaspiro[4.5]decan-2-one
[408]8-(1-phenyl-5-propyl-1H-pyrazole-4-carbonyl)-3-(4-trifluoromethylsulfanylbenzyl)-1-oxa-3,8-diazaspiro[4.5]decan-2-one
[409]3-[8-(2-chloropyridine-4-carbonyl)-2-oxo-1-oxa-3,8-diazaspiro[4.5]dec-3-ylmethyl]benzonitrile
[410]8-(2-chloropyridine-4-carbonyl)-3-(3-methoxybenzyl)-1-oxa-3,8-diazaspiro[4.5]decan-2-one
[411]8-(5-tert-butyl-2-methylfuran-3-carbonyl)-3-(4-fluorobenzyl)-1-oxa-3,8-diazaspiro[4.5]decan-2-one
[412]3-[8-(5-tert-butyl-2-methylfuran-3-carbonyl)-2-oxo-1-oxa-3,8-diazaspiro[4.5]dec-3-ylmethyl]benzonitrile
[413]2-[8-(5-tert-butyl-2-methylfuran-3-carbonyl)-2-oxo-1-oxa-3,8-diazaspiro[4.5]dec-3-ylmethyl]benzonitrile
[414]4-[8-(5-tert-butyl-2-methylfuran-3-carbonyl)-2-oxo-1-oxa-3,8-diazaspiro[4.5]dec-3-ylmethyl]benzonitrile
[415]8-(5-tert-butyl-2-methylfuran-3-carbonyl)-3-(2-trifluoromethylbenzyl)-1-oxa-3,8-diazaspiro[4.5]decan-2-one
[416]8-(5-tert-butyl-2-methylfuran-3-carbonyl)-3-naphthalen-2-ylmethyl-1-oxa-3,8-diazaspiro[4.5]decan-2-one
[417]3-biphenyl-2-ylmethyl-8-(5-tert-butyl-2-methylfuran-3-carbonyl)-1-oxa-3,8-diazaspiro[4.5]decan-2-one
[418]3-(3-bromobenzyl)-8-(5-tert-butyl-2-methylfuran-3-carbonyl)-1-oxa-3,8-diazaspiro[4.5]decan-2-one
[419]8-(benzo[1,2,5]oxadiazole-5-carbonyl)-3-(3,4-difluorobenzyl)-1-oxa-3,8-diazaspiro[4.5]decan-2-one
[420]8-(benzo[1,2,5]oxadiazole-5-carbonyl)-3-(4-trifluoromethylbenzyl)-1-oxa-3,8-diazaspiro[4.5]decan-2-one
[421]3-[8-(2-methyl-6-trifluoromethylpyridine-3-carbonyl)-2-oxo-1-oxa-3,8-diazaspiro[4.5]dec-3-ylmethyl]benzonitrile
[422]2-[8-(2-methyl-6-trifluoromethylpyridine-3-carbonyl)-2-oxo-1-oxa-3,8-diazaspiro[4.5]dec-3-ylmethyl]benzonitrile
[423]4-[8-(2-methyl-6-trifluoromethylpyridine-3-carbonyl)-2-oxo-1-oxa-3,8-diazaspiro[4.5]dec-3-ylmethyl]benzonitrile
[424]8-(2-methyl-6-trifluoromethylpyridine-3-carbonyl)-3-(2-trifluoromethylbenzyl)-1-oxa-3,8-diazaspiro[4.5]decan-2-one
[425]3-(3,4-difluorobenzyl)-8-(2-methyl-6-trifluoromethylpyridine-3-carbonyl)-1-oxa-3,8-diazaspiro[4.5]decan-2-one
[426]3-(3-bromobenzyl)-8-(2-methyl-6-trifluoromethylpyridine-3-carbonyl)-1-oxa-3,8-diazaspiro[4.5]decan-2-one
[427]8-(6-chloro-2H-chromene-3-carbonyl)-3-(3,4-difluorobenzyl)-1-oxa-3,8-diazaspiro[4.5]decan-2-one
[428]8-(2-chloropyridine-4-carbonyl)-3-(2-fluorobenzyl)-1-oxa-3,8-diazaspiro[4.5]decan-2-one
[429]8-(2-chloropyridine-4-carbonyl)-3-naphthalen-2-ylmethyl-1-oxa-3,8-diazaspiro[4.5]decan-2-one
[430]8-acetyl-3-(2-fluorobenzyl)-1-oxa-3,8-diazaspiro[4.5]decan-2-one
[431] 8-acetyl-3-(3-bromobenzyl)-1-oxa-3,8-diazaspiro[4.5]decan-2-one
and
[432] 3-(2-methoxy-5-nitrobenzyl)-8-(3-phenoxypropyl)-1-oxa-3,8-diazaspiro[4.5]decan-2-one;
in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

17. A method for producing substituted 1-oxa-3,8-diazaspiro[4.5]-decan-2-one compounds of the general formula I according to one or more of claims 1 to 16, **characterised in that** at least one compound of the general formula II, 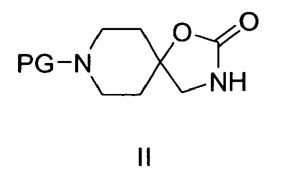 in which PG denotes a protective group, preferably a tert-butyloxycarbonyl or benzyloxycarbonyl group, is reacted in a reaction medium, in the presence of at least one base, preferably in the presence of at least one metal hydride salt, particularly preferably in the presence of potassium hydride and/or sodium hydride, with at least one compound of the general formula R¹-(CH₂)ₙ-X, in which R¹ and n have the meaning according to one or more of claims 1 to 16 and X denotes a leaving group, preferably a halogen atom, particularly preferably a chlorine or bromine atom, to yield at least one compound of the general formula III, 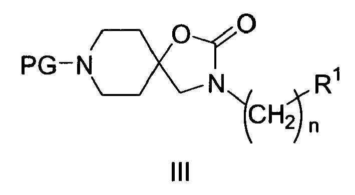 in which R¹, PG and n have the above-stated meaning, and this is optionally purified and/or isolated,
and at least one compound of the general formula III is reacted by elimination of the protective group, preferably the tert-butoxycarbonyl residue-group in a reaction medium, preferably in the presence of at least one acid or in the presence of at least one base or by elimination of benzyloxycarbonyl group in a reaction medium, preferably in the presence of hydrogen and catalyst, preferably palladium on carbon, to yield at least one compound of the general formula IV, optionally in the form of a corresponding salt, preferably in the form of a corresponding hydrochloride, 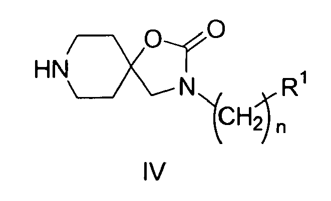 in which R¹ and n have the above-stated meaning, and this is optionally purified and/or isolated,
and at least one compound of the general formula IV is reacted in a reaction medium with at least one isothiocyanate of the general formula R³-N=C=S, in which R³ has the meaning according to one or more of claims 1 to 16, optionally in the presence of at least one base, preferably in the presence of at least one base selected from the group consisting of triethylamine, (4,4)-dimethylaminopyridine, pyridine, diisopropylethylamine and
N-methylmorpholine, to yield at least one compound of the general formula I, in which R¹ and n have the above-stated meaning and R² denotes -C(=S)-NH-R³ in which R³ has the above-stated meaning, and this is optionally purified and/or isolated;
or
at least one compound of the general formula IV is reacted in a reaction medium with at least one isocyanate of the general formula R⁴-N=C=O, in which R⁴ has the meaning according to one or more of claims 1 to 16, optionally in the presence of at least one base, preferably in the presence of at least one base selected from the group consisting of triethylamine, (4,4)-dimethylaminopyridine, pyridine, diisopropylethylamine and N-methylmorpholine, to yield at least one compound of the general formula I, in which R¹ and n have the above-stated meaning and R² denotes -C(=O)-NH-R⁴, in which R⁴ has the above-stated meaning, and this is optionally purified and/or isolated;
or
at least one compound of the general formula IV is reacted in a reaction medium, optionally in the presence of at least one base, preferably in the presence of at least one base selected from the group consisting of triethylamine, (4,4)-dimethylaminopyridine, pyridine, diisopropylethylamine and N-methylmorpholine, with at least one sulfonic acid derivative of the general formula R⁵-S(=O)₂-X, in which R⁵ has the meaning according to one or more of claims 1 to 16 and X denotes a leaving group, preferably a halogen atom, particularly preferably a chlorine or bromine atom, to yield at least one compound of the general formula I, in which R¹ and n have the above-stated meaning and R² denotes -S(=O)₂-R⁵, in which R⁵ has the above-stated meaning, and this is optionally purified and/or isolated;
or
at least one compound of the general formula IV is reacted in a reaction medium, optionally in the presence of at least one base, preferably in the presence of at least one base selected from the group consisting of triethylamine, (4,4)-dimethylaminopyridine, pyridine, diisopropylethylamine and N-methylmorpholine, with at least one compound of the general formula X-(CH₂)-C(=O)-NHR⁶, in which R⁶ has the meaning according to one or more of claims 1 to 16 and X denotes a leaving group, preferably a halogen atom, particularly preferably a chlorine or bromine atom, to yield at least one compound of the general formula I, in which R¹ and n have the above-stated meaning and R² denotes -(CH₂)-C(=O)-NH-R⁶ in which R⁶ has the above-stated meaning, and this is optionally purified and/or isolated;
or
at least one compound of the general formula IV is reacted in a reaction medium, optionally in the presence of at least one base, preferably in the presence of at least one base selected from the group consisting of triethylamine, (4,4)-dimethylaminopyridine, pyridine, diisopropylethylamine and N-methylmorpholine, with at least one compound of the general formula X-(CH₂)-Dₐₐ-(CH₂)_{bb}-E_{cc}-(CH₂)_{dd}-R⁷, in which R⁷, D, E, aa, bb, cc and dd have the meaning according to one or more of claims 1 to 16 and X denotes a leaving group, preferably a halogen atom, particularly preferably a chlorine or bromine atom, to yield at least one compound of the general formula I, in which R¹ and n have the above-stated meaning and R² denotes -(CH₂)-Dₐₐ-(CH₂)_{bb}-E_{cc}-(CH₂)_{dd}-R⁷, in which R⁷, D, E, aa, bb, cc and dd have the above-stated meaning, and this is optionally purified and/or isolated;
or
at least one compound of the general formula IV is reacted in a reaction medium, optionally in the presence of at least one base, preferably in the presence of at least one base selected from the group consisting of triethylamine, (4,4)-dimethylaminopyridine, pyridine, diisopropylethylamine and N-methylmorpholine, with at least one carboxylic acid derivative of the general formula R⁸-C(=O)-X, in which R⁸ has the meaning according to one or more of claims 1 to 16 and X denotes a leaving group, preferably a halogen atom, particularly preferably a chlorine or bromine atom, to yield at least one compound of the general formula I, in which R¹ and n have the above-stated meaning and R² denotes -C(=O)-R⁸, in which R⁸ has the above-stated meaning, and this is optionally purified and/or isolated;
or
at least one compound of the general formula IV is reacted in a reaction medium, in the presence of at least one coupling reagent, optionally in the presence of at least one base, preferably in the presence of at least one base selected from the group consisting of triethylamine, (4,4)-dimethylaminopyridine, pyridine, diisopropylethylamine and N-methylmorpholine, with at least one carboxylic acid of the general formula R⁸-C(=O)-OH, in which R⁸ has the meaning according to one or more of claims 1 to 16, to yield at least one compound of the general formula I, in which R¹ and n have the above-stated meaning and R² denotes -C(=O)-R⁸, in which R⁸ has the above-stated meaning, and this is optionally purified and/or isolated;
or
at least one compound of the general formula IV is reacted in a reaction medium, optionally in the presence of at least one base, preferably in the presence of at least one base selected from the group consisting of triethylamine, (4,4)-dimethylaminopyridine, pyridine, diisopropylethylamine and N-methylmorpholine, with at least one sulfonic acid derivative of the general formula X-S(=O)₂-NR⁹R¹⁰, in which R⁹ and R¹⁰ have the meaning according to one or more of claims 1 to 16 and X denotes a leaving group, preferably a halogen atom, particularly preferably a chlorine or bromine atom, to yield at least one compound of the general formula I, in which R¹ and n have the above-stated meaning and R² denotes -S(=O)₂-NR⁹R¹⁰, in which R⁹ and R¹⁰ have the above-stated meaning, and this is optionally purified and/or isolated.

18. A medicament containing at least one compound according to one or more of claims 1 to 16 and optionally one or more physiologically acceptable auxiliary substances.

19. A medicament according to claim 18 for the prevention and/or treatment of pain, preferably of pain selected from the group consisting of acute pain, chronic pain, visceral pain and neuropathic pain.

20. A medicament according to claim 18 for the prevention and/or treatment of one or more diseases selected from the group consisting of disorders of food intake, preferably selected from the group consisting of bulimia, anorexia, obesity and cachexia; water retention conditions; migraine; chronic paroxysmal hemicrania; depression; urinary incontinence; coughing; asthma; glaucoma; tinnitus; inflammation; neurodegenerative diseases, preferably selected from the group consisting of Parkinson's disease, Huntington's chorea, Alzheimer's disease and multiple sclerosis; cognitive dysfunction, preferably memory disorders; cognitive deficiency states (attention deficit syndrome, ADS); epilepsy; catalepsy; narcolepsy; diarrhoea; gastritis; stomach ulcer; pruritus; anxiety states; panic attacks; schizophrenia; cerebral ischaemic episodes; muscle spasms; cramps; gastro-oesophageal reflux syndrome; alcohol and/or drug abuse, preferably nicotine and/or cocaine abuse, and/or abuse of medicines; alcohol and/or drug dependency, preferably nicotine and/or cocaine dependency, and/or dependency on medicines, preferably for the prevention and/or reduction of withdrawal symptoms associated with alcohol and/or drug dependency, preferably nicotine and/or cocaine dependency and/or dependency on medicines; for the prevention and/or reduction of the development of tolerance to medicines and/or drugs, in particular medicines based on opioids; for regulating food intake; for modulating locomotor activity, for suppression of the urinary reflex; for regulating the cardiovascular system, preferably for vasodilating the arteries; for local anaesthesia; for increasing vigilance; for increasing libido; for diuresis, and/or for antinatriuresis.

21. Use of at least one compound according to one or more of claims 1 to 16 for the production of a medicament for the prevention and/or treatment of pain, preferably of pain selected from the group consisting of acute pain, chronic pain, visceral pain and neuropathic pain.

22. Use of at least one compound according to one or more of claims 1 to 16 for the production of a medicament for the prevention and/or treatment of one or more diseases selected from the group consisting of disorders of food intake, preferably selected from the group consisting of bulimia, anorexia, obesity and cachexia; water retention conditions; migraine; chronic paroxysmal hemicrania; depression; urinary incontinence; coughing; asthma; glaucoma; tinnitus; inflammation; neurodegenerative diseases, preferably selected from the group consisting of Parkinson's disease, Huntington's chorea, Alzheimer's disease and multiple sclerosis; cognitive dysfunction, preferably memory disorders; cognitive deficiency states (attention deficit syndrome, ADS); epilepsy; catalepsy; narcolepsy; diarrhoea; gastritis; stomach ulcer; pruritus; anxiety states; panic attacks; schizophrenia; cerebral ischaemic episodes; muscle spasms; cramps; gastro-oesophageal reflux syndrome; alcohol and/or drug abuse, preferably nicotine and/or cocaine abuse, and/or abuse of medicines; alcohol and/or drug dependency, preferably nicotine and/or cocaine dependency, and/or dependency on medicines, preferably for prevention and/or reduction of withdrawal symptoms associated with alcohol and/or drug dependency, preferably nicotine and/or cocaine dependency and/or dependency on medicines; for the prevention and/or reduction of the development of tolerance to medicines and/or drugs, in particular medicines based on opioids; for regulating food intake; for modulating locomotor activity, for suppression of the urinary reflex; for regulating the cardiovascular system, preferably for vasodilating the arteries; for local anaesthesia; for increasing vigilance; for increasing libido; for diuresis, and/or for antinatriuresis.

## Revendications

1. Composés substitués de 1-oxa-3,8-diazaspiro[4,5]-décan-2-one de formule générale I, 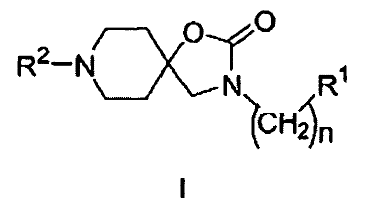 où
n vaut 1, 2, 3, 4 ou 5 ;
R¹ représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R² représente -C(=S)-NH-R³ ;
représente -C(=O)-NH-R⁴ ;
représente -S(=O)₂-R⁵ ;
représente -(CH₂)-C(=O)-NH-R⁶ ;
représente -(CH₂)-Dₐₐ-(CH₂)_{bb}-E_{cc}-(CH₂)_{dd}-R⁷ avec aa = 0 ou 1 ; bb = 0, 1 ou 2 ; cc = 0 ou 1 et dd = 0 ou 1 ; où D et E représentent, indépendamment l'un de l'autre, O, S, NH, N(CH₃), N (C₂H₅) ou N[CH(CH₃)₂] et la somme de aa et cc étant différente de 0 ;
représente -C(=O)-R⁸
ou représente -S(=O)₂-NR⁹R¹⁰ ;
R³ représente -(CHR¹¹)-(CH₂)_{w}-C(=O)-O-R¹² avec w = 0 ou 1 ;
représente -(CHR¹³)-(CH₂)ₐ-K_{b}-(CH₂)_{c}-L_{d}-R¹⁴ avec a = 0, 1 ou 2 ; b = 0 ou 1 ; c = 0, 1 ou 2 et d = 0 ou 1 ; où K et L représentent, indépendamment l'un de l'autre, O, S, NH, N(CH₃), N(C₂H₅) ou N[CH(CH₃)₂] ;
représente un radical aliphatique en C₁₋₁₀ linéaire ou ramifié, saturé ou insaturé, le cas échéant substitué ;
représente un radical cycloaliphatique le cas échéant substitué, qui peut être ponté avec 1 ou 2 groupes C₁₋₅-alkylène linéaires ou ramifiés, le cas échéant substitués ;
ou représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R⁴ représente -(CHR¹⁵)-(CH₂)ₑ-M_{f}(CH₂)_{g}-Pₕ-R¹⁶ avec e = 0, 1 ou 2 ; f = 0 ou 1 ; g = 0, 1 ou 2 et h = 0 ou 1 ; où M et P représentent, indépendamment l'un de l'autre, O, S, NH, N(CH₃) , N(C₂H₅) ou N[CH(CH₃)₂] ;
représente un radical aliphatique en C₁₋₁₀ linéaire ou ramifié, saturé ou insaturé, le cas échéant substitué ;
représente un radical cycloaliphatique le cas échéant substitué, qui peut être ponté avec 1 ou 2 groupes C₁₋₅-alkylène linéaires ou ramifiés, le cas échéant substitués ;
ou représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R⁵ représente -(CHR¹⁷)-(CH₂)ₖ-Q₁-(CH₂)ₘ-Tₒ-R¹⁸ avec k = 0, 1 ou 2 ; 1 = 0 ou 1 ; m = 0, 1 ou 2 et o = 0 ou 1 ; où Q et T représentent, indépendamment l'un de l'autre, O, S, NH, N(CH₃), N(C₂H₅) ou N[CH(CH₃)₂] ;
représente un radical aliphatique en C₁₋₁₀ linéaire ou ramifié, saturé ou insaturé, le cas échéant substitué ;
représente un radical cycloaliphatique le cas échéant substitué, qui peut être ponté avec 1 ou 2 groupes C₁₋₅-alkylène linéaires ou ramifiés, le cas échéant substitués ;
ou représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R⁶ représente un radical aliphatique en C₁₋₁₀ linéaire ou ramifié, saturé ou insaturé, le cas échéant substitué ;
représente un radical cycloaliphatique le cas échéant substitué, qui peut être ponté avec 1 ou 2 groupes C₁₋₅-alkylène linéaires ou ramifiés, le cas échéant substitués ;
ou représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R⁷ représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical pouvant le cas échéant à chaque fois être substitué par 1, 2, 3, 4 ou 5 substituants, choisis indépendamment l'un de l'autre dans le groupe constitué par oxo (=O), thioxo (= S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)_{3,} -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)₂-NH₂ ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, imidazolyle, indolyle, iso-indolyle, benzo[b]furannyle, benzo[b]thiophényle, thiazolyle, [1,2,3]-thiadiazolyle, [1,2,4]-oxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment dans le groupe constitué par F, Cl, Br, I, -CN, -CF₃, -SF₅, -OH, -O-CH₃, -O-C₂H₅, -O-CH(CH₃)₂, -O-CH₂-CH₂-CH3, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C (=O) -C (CH₃)₃, -C(=O)-OH, -(CH₂)-C(-O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃) (C₂H₅), -C(=O)-H, -C(=O)-CH₃, -C(=O)-C₂H_{5,} -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH3)3, -C(=O)-N(CH₃)_{2,} -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-phényle, phényle et benzyle, la partie cyclique des radicaux -S(=O)₂-NH-phényle, phényle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle ;
R⁸ représente -(CHR¹⁹)-Vₚ-(CH₂)_{q}-(CH₂)ᵣ-Wₛ-R²⁰ avec p = 0 ou 1 ; q = 0, 1 ou 2 ; r = 0, 1 ou 2 et s = 0 ou 1 ; où V et W représentent à chaque fois indépendamment l'un de l'autre O, S, NH, N(CH₃), N(C₂H₅) ou N[CH(CH₃)₂] ;
représente -(CH=CH)-R₂₁ ;
représente -(CR²²R²³)-Yₜ(CR²⁴R²⁵)ᵤ-(CH₂)ᵥ-C(=O)-OR²⁶ avec t = 0 ou 1, u = 0 ou 1 et v = 0 ou 1, où Y représente 0, S, NH, N(CH₃), N(C₂H₅) ou N[CH(CH₃)₂] ;
représente-(CHR²⁷)-O-C(=O)-R²⁸ ;
représente -CH[(CH₂)R²⁹][NH-S(=O)₂-R³⁰] ;
représente -CH[(CH₂)R³¹][NH-C(=O)-O-R³²] ;
représente un radical aliphatique en C₁₋₁₀ linéaire ou ramifié, saturé ou insaturé, le cas échéant substitué ;
représente un radical cycloaliphatique le cas échéant substitué, qui peut être ponté avec 1 ou 2 groupes C₁₋₅-alkylène linéaires ou ramifiés, le cas échéant substitués ; ou représente un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
R⁹ et R¹⁰ représentent, indépendamment l'un de l'autre, à chaque fois
un radical aliphatique en C₁₋₁₀ linéaire ou ramifié, saturé ou insaturé, le cas échéant substitué ;
R¹¹, R¹³, R¹⁵, R¹⁷, R¹⁹, R²², R²³, R²⁴, R²⁵ et R²⁶, représentent, indépendamment l'un de l'autre, à chaque fois
un radical hydrogène
ou un radical aliphatique en C₁₋₁₀ linéaire ou ramifié, saturé ou insaturé, le cas échéant substitué ;
R¹², R²⁸ et R³², représentent, indépendamment l'un de l'autre, à chaque fois
un radical aliphatique en C₁₋₁₀ linéaire ou ramifié, saturé ou insaturé, le cas échéant substitué ;
R¹⁴, R¹⁶, R¹⁸ et R²⁰, représentent, indépendamment l'un de l'autre, à chaque fois
un radical aliphatique en C₁₋₁₀ linéaire ou ramifié, saturé ou insaturé, le cas échéant substitué ;
un radical cycloaliphatique le cas échéant substitué, qui peut être ponté avec 1 ou 2 groupes C₁₋₅-alkylène linéaires ou ramifiés, le cas échéant substitués ;
ou un radical aryle le cas échéant substitué ou un radical hétéroaryle le cas échéant substitué, le radical aryle pouvant être condensé avec un système cyclique monocyclique ou bicyclique, saturé ou insaturé, le cas échéant substitué ;
et
R²¹, R²⁷, R²⁹, R³⁰ et R³¹, représentent, indépendamment l'un de l'autre, à chaque fois un radical cycloaliphatique le cas échéant substitué, qui peut être ponté avec 1 ou 2 groupes C₁₋₅-alkylène linéaires ou ramifiés, le cas échéant substitués ;
ou un radical aryle le cas échéant substitué ou un radical hétéroaryle le cas échéant substitué, le radical aryle pouvant à chaque fois être condensé avec un système cyclique monocyclique ou bicyclique, saturé ou insaturé, le cas échéant substitué ;
où
- les radicaux aliphatiques en C₁₋₁₀ susmentionnés peuvent le cas échéant à chaque fois être substitués par 1, 2, 3, 4 ou 5 substituants, choisis indépendamment l'un de l'autre dans le groupe constitué par F, Cl, Br, I, -CN, -NO₂, -OH,
- SH et -NH₂ ;
- les radicaux cycloaliphatiques susmentionnés peuvent le cas échéant à chaque fois être substitués par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -S-CF₂H, -S-CFH₂, -SH, -S-C₁₋₅-alkyle, -C₁₋₅-alkyle, -C(=O)-OH, -(CH₂)-C(=O)-OH ; -C(=O)-O-C₁₋₅-alkyle, -(CH₂)-C(=O)-O-C₁₋₅-alkyle, -OC(=O)-C₁₋₅-alkyle, -NH-C₁₋₅-alkyle, -N(C₁₋₅-alkyle)₂, -NH-phényle, -NH-pyridinyle, -N(C₁₋₅-alkyl)-phényle, -N(C₁₋₅-alkyl)-pyridinyle, -NH-C(=O)-OC₁₋₅-alkyle, -C(=O)-H, -C (=O)-C₁₋₅-alkyle, -C(=O)-C₁₋₅-perfluoroalkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, -S(=O)₂-NH₂, -S(=O)₂-NH-phényle, cyclohexyle, cyclopentyle, pyridinyle, [1,2,5]-thiadiazolyle, pyridazinyle, -(CH₂)-benzo[b]furannyle, -O-phényle, -O-benzyle, phényle et benzyle, la partie cyclique des radicaux -S(=O)₂-NH-phényle, -NH-phényle, -NH-pyridinyle, -N(C₁₋₅-alkyl)-phényle, -N(C₁₋₅-alkyl)-pyridinyle, pyridinyle, cyclopentyle, [1,2,5]-thiadiazolyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
- les groupes C₁₋₅-alkylène susmentionnés peuvent le cas échéant à chaque fois être substitués par 1, 2, 3, 4 ou 5 substituants, choisis indépendamment l'un de l'autre dans le groupe constitué par F, Cl, Br, I, -CN, -NO₂, -OH, -SH et -NH₂ ;
- les cycles des systèmes monocycliques ou polycycliques susmentionnés peuvent le cas échéant à chaque fois être substitués par 1, 2, 3, 4 ou 5 substituants choisis indépendamment l'un de l'autre dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -S-CF₂H, -S-CFH₂, -SH, -S-C₁₋₅-alkyle, -C₁₋₅-alkyle, -C(=O)-OH, -(CH₂)-C(=O)-OH, -C(=O)-O-C₁₋₅-alkyle, -(CH₂)-C(=O)-O-C₁₋₅-alkyle, -O-C(=O)-C₁₋₅-alkyle, -NH-C₁₋₅-alkyle, -N(C₁₋₅-alkyle)₂, -NH-phényle, -NH-pyridinyle, -N(C₁₋₅-alkyl)-phényle, -N(C₁₋₅-alkyl)-pyridinyle, -NH-C(=O)-O-C₁₋₅-alkyle, -C(=O)-H, -C (=O) -C₁₋₅-alkyle, -C(=O)-C₁₋₅-perfluoroalkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, -S(=O)₂-NH₂, -S(=O)₂-NH-phényle, cyclohexyle, cyclopentyle, pyridinyle, [1,2,5]-thiadiazolyle, pyridazinyle, -(CH₂)-benzo[b]furannyle, -O-phényle, -O-benzyle, phényle et benzyle, la partie cyclique des radicaux -S(=O)₂-NH-phényle, -NH-phényle, -NH-pyridinyle, -N(C₁₋₅-alkyl)-phényle, -N(C₁₋₅-alkyl)-pyridinyle, pyridinyle, cyclopentyle, [1,2,5]-thiadiazolyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
- les radicaux aryle ou hétéroaryle susmentionnés peuvent le cas échéant à chaque fois être substitués par 1, 2, 3, 4 ou 5 substituants, choisis indépendamment dans le groupe constitué par F, Cl, Br, I, -CN, -CF₃, -SF₅, -OH, -O-C₁₋₅-alkyle, -NH₂, -NO₂, -O-CF₃, -S-CF₃, -S-CF₂H, -S-CFH₂, -SH, -S-C₁₋₅-alkyle, -C₁₋₁₀-alkyle, -C(=O)-OH, -(CH₂)-C(=O)-OH, -C(=O)-O-C₁₋₅-alkyle, -(CH₂)-C(=O)-O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -NH-C(=O)-C₁₋₅-alkyle, -C(=O)-H, -C(=O)-C₁₋₅-alkyle, -C(=O)-C₁₋₅-perfluoroalkyle, -C(=O)-NH₂, -C(=O)-NH-C₁₋₅-alkyle, -C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, -S(=O)₂-NH₂, -S(=O)₂-NH-phényle, cyclohexyle, cyclopentyle, pyridinyle, pyridazinyle, -(CH₂)-benzo[b]furannyle, -O-phényle, -O-benzyle, phényle et benzyle, la partie cyclique des radicaux pyridinyle, cyclopentyle, cyclohexyle, pyridazinyle, -S(=O)₂-phényle, -S(=O)₂-NH-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, -C₁₋₅-alkyle, -O-C₁₋₅-alkyle, -O-CF₃, -S-CF₃, phényle et -O-benzyle,
- les radicaux cycloaliphatiques susmentionnés sont choisis dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et bicyclo[2,2,1]heptyle,
- les radicaux hétéroaryle susmentionnés sont choisis dans le groupe constitué par 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, oxazolyle, isoxazolyle, pyridazinyle, pyrazinyle, pyrimidinyle, indazolyle, quinazolinyle, quinoléinyle et isoquinoléinyle,
- les radicaux aryle susmentionnés sont choisis dans le groupe constitué par phényle et naphtyle, et
- les radicaux aryle qui sont condensés avec un système monocyclique ou polycyclique sont choisis dans le groupe constitué par [1,3]-benzodioxolyle, [1,4]-benzodioxanyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle et [3,4]-dihydro-2H-1,4-benzoxazinyle,
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1, **caractérisés en ce que** R¹ représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, [3,4]-dihydro-2H-1,4-benzoxazinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants, choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, n-hexyle, n-heptyle, -NH-C(=O)-O-CH₃, -NH-C(=O)-O-C₂H₅, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phényle et benzyle, la partie cyclique des radicaux phényle et benzyle pouvant chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

3. Composés selon la revendication 1 ou 2, **caractérisés en ce que** R² représente -C(=S)-NH-R³ ; représente -C(=O)-NH-R⁴ ; représente -S(=O)₂-R⁵ ; représente -(CH₂)-C(=O)-NH-R⁶ ; représente -(CH₂)-O-R⁷, représente - (CH₂)-S-R⁷, représente - (CH₂)-NH-R⁷, représente -(CH₂)-N(CH₃)-R⁷, représente -(CH₂)-(CH₂)-OR⁷, représente -(CH₂)-(CH₂)-S-R⁷, représente - (CH₂)-NH-R⁷, représente -(CH₂)-N(CH₃)-R⁷, représente -(CH₂)-(CH₂)-(CH₂)-O-R⁷, représente -(CH₂)-(CH₂)-(CH₂)-S-R⁷, représente -(CH₂)-(CH₂)-(CH₂)-NH-R⁷, représente -(CH₂)-(CH₂)-(CH₂)-N(CH₃)-R⁷, représente -(CH₂)-O-(CH₂)-R⁷, représente -(CH₂)-S-(CH₂)-R⁷ ; représente -(CH₂)-NH-(CH₂)-R⁷ ; représente -C(=O)-R⁸ ou représente -S(=O)₂-NR⁹R¹⁰.

4. Composés selon l'une ou plusieurs des revendications 1 à 3, **caractérisés en ce que**
R³ représente -(CHR¹¹)-C(=O)-O-R¹² ou -(CHR¹¹)-(CH₂)-C(=O)-O-R¹² ;
représente -(CHR¹³)-R¹⁴, -(CHR¹³)-(CH₂)-R¹⁴, -(CHR¹³)-(CH₂)-(CH₂)-R¹⁴, -(CHR¹³)-O-R¹⁴, -(CHR¹³)-S-R¹⁴, -(CHR¹³)-NH-R¹⁴, -(CHR¹³)-N(CH₃)-R¹⁴, -(CHR¹³)-(CH₂)-O-R¹⁴, -(CHR¹³)-(CH₂)-S-R¹⁴, -(CHR¹³)-NH-R¹⁴, -(CHR¹³)-N(CH₃)-R¹⁴, -(CHR¹³)-(CH₂)-(CH₂)-O-R¹⁴, -(CHR¹³)-(CH₂)-(CH₂)-S-R¹⁴, -(CHR¹³)-(CH₂)-(CH₂)-NH-R¹⁴, -(CHR¹³)-(CH₂)-(CH₂)-N(CH₃)-R¹⁴, -(CHR¹³)-O-(CH₂)-R¹⁴, -(CHR¹³)-S-(CH₂)-R¹⁴ ou -(CHR¹³)-NH-(CH₂₎-R¹⁴ ;
représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle, où le radical peut le cas échéant être substitué par 1, 2, 3, 4 ou 5 substituants choisis indépendamment l'un de l'autre dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical pouvant le cas échéant à chaque fois être substitué par 1, 2, 3, 4 ou 5 substituants, choisis indépendamment l'un de l'autre dans le groupe constitué par oxo (=O), thioxo (= S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C (CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)₂-NH₂ ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-CF₃, -C(=O)-C₂F₅, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

5. Composés selon l'une ou plusieurs des revendications 1 à 4, **caractérisés en ce que**
R⁴ représente -(CHR¹⁵)-R¹⁶, -(CHR¹⁵)-(CH₂)-R¹⁶, -(CHR¹⁵)-(CH₂)-(CH₂)-R¹⁶, -(CHR¹⁵)-O-R¹⁶, -(CHR¹⁵)-S-R¹⁶, -(CHR¹⁵)-NH-R¹⁶, -(CHR¹⁵)-N(CH₃)-R¹⁶, -(CHR¹⁵)-(CH₂)-O-R¹⁶, -(CHR¹⁵)-(CH₂)-S-R¹⁶, -(CHR¹⁵)-NH-R¹⁶, -(CHR¹⁵)-N(CH₃)-R¹⁶, -(CHR¹⁵)-(CH₂)-(CH₂)-O-R¹⁶, -(CHR¹⁵)-(CH₂)-(CH₂)-S-R¹⁶, -(CHR¹⁵)-(CH₂)-(CH₂)-NH-R¹⁶, -(CHR¹⁵)-(CH₂)-(CH₂)-N(CH₃)-R¹⁶, -(CHR¹⁵)-O-(CH₂)-R¹⁶, -(CHR¹⁵)-S-(CH₂)-R¹⁶ ou -(CHR¹⁵)-NH-(CH₂)-R¹⁶ ; représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle, où le radical peut le cas échéant être substitué par 1, 2, 3, 4 ou 5 substituants choisis indépendamment l'un de l'autre dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical pouvant le cas échéant à chaque fois être substitué par 1, 2, 3, 4 ou 5 substituants, choisis indépendamment l'un de l'autre dans le groupe constitué par oxo (=O), thioxo (= S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N (CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)-NH₂ ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, C(=O)-NH₂, -C(=O)-NH-CH₃, -C(=O)-NH-C₂H₅, -C(=O)NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

6. Composés selon l'une ou plusieurs des revendications 1 à 5, **caractérisés en ce que**
R⁵ représente -(CHR¹⁷)-R¹⁸, -(CHR¹⁷)-(CH₂)-R¹⁸, -(CHR¹⁷)-(CH₂)-(CH₂)-R¹⁸, -(CHR¹⁷)-O-R¹⁸, -(CHR¹⁷)-S-R¹⁸, -(CHR¹⁷)-NH-R¹⁸, -(CHR¹⁷)-N(CH₃)-R¹⁸, -(CHR¹⁷)-(CH₂)-O-R¹⁸, -(CHR¹⁷)-(CH₂)-S-R¹⁸, -(CHR¹⁷)-NH-R¹⁸, -(CHR¹⁷)-N(CH₃)-R¹⁸, -(CHR¹⁷)-(CH₂)-(CH₂)-O-R¹⁸, -(CHR¹⁷)-(CH₂)-(CH₂)-S-R¹⁸, -(CHR¹⁷)-(CH₂)-(CH₂)-NH-R¹⁸, -(CHR¹⁷)-(CH₂)-(CH₂)-N(CH₃)-R¹⁸, -(CHR¹⁷)-O-(CH₂)-R¹⁸, -(CHR¹⁷)-S-(CH₂)-R¹⁸ ou -(CHR¹⁷)-NH-(CH₂)-R¹⁸ ;
représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle, où le radical peut le cas échéant être substitué par 1, 2, 3, 4 ou 5 substituants choisis indépendamment l'un de l'autre dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical pouvant le cas échéant à chaque fois être substitué par 1, 2, 3, 4 ou 5 substituants, choisis indépendamment l'un de l'autre dans le groupe constitué par oxo (=O), thioxo (= S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)₂-NH₂ ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C (CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

7. Composés selon l'une ou plusieurs des revendications 1 à 6, **caractérisés en ce que**
R⁶ représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle, où le radical peut le cas échéant être substitué par 1, 2, 3, 4 ou 5 substituants choisis indépendamment l'un de l'autre dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical pouvant le cas échéant à chaque fois être substitué par 1, 2, 3, 4 ou 5 substituants, choisis indépendamment l'un de l'autre dans le groupe constitué par oxo (=O), thioxo (= S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)₂-NH₂ ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH(CH₃)₂, -C(=O)-O-C(CH₃)₃, -NH-CH₃, -NH-C₂H₅, -NH-CH (CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-phényle, phényle et benzyle, la partie cyclique des radicaux -S(=O)₂-NH-phényle, phényle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

8. Composés selon l'une ou plusieurs des revendications 1 à 7, **caractérisés en ce que**
R⁸ représente -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-S-R²⁰, -(CHR¹⁹)-NH-R²⁰, -(CHR¹⁹)-N(CH₃)-R²⁰, -(CHR¹⁹)-(CH₂)-O-R²⁰, -(CHR¹⁹)-(CH₂)-S-R²⁰, -(CHR¹⁹)-NH-R²⁰, -(CHR¹⁹)-N(CH₃)-R²⁰, -(CHR¹⁹)-(CH₂)-(CH₂)-O-R²⁰, -(CHR¹⁹)-(CH₂)-(CH₂)-S-R²⁰, -(CHR¹⁹)-(CH₂)-(CH₂)-NH-R²⁰, -(CHR¹⁹)-(CH₂)-(CH₂)-N(CH₃)-R²⁰, -(CHR¹⁹)-O-(CH₂)-R²⁰, -(CHR¹⁹)-S-(CH₂)-R²⁰, -(CHR¹⁹)-NH-(CH₂)-R²⁰, -(CHR¹⁹)-O-(CH₂)-(CH₂)-O-R²⁰, -(CHR¹⁹)-O-(CH₂)-(CH₂)-S-R²⁰ ou -(CHR¹⁹)-S-(CH₂)-(CH₂)-S-R²⁰ ;
représente -(CH=CH)-R²¹ ;
représente -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶, -(CR²²R²³)-O-(CR²⁴R²⁵)-C(=O)-O-R²⁶, -(CR²²R²³)-S-(CR²⁴R²⁵)-C(=O)-O-R²⁶ ou -(CR²²R²³)-NH-(CR²⁴R²⁵)-C-(=O)-O-R²⁶ ;
représente -(CHR²⁷)-O-C(=O)-R²⁸ ;
représente -CH[(CH₂)R²⁹][NH-S(=O)₂-R³⁰] ;
représente -CH[(CH₂)R³¹][NH-C(=O)-O-R³²] ;
représente un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle, le radical pouvant le cas échéant être substitué par 1, 2, 3, 4 ou 5 substituants choisis indépendamment l'un de l'autre dans le groupe constitué par F, Cl, Br, I, -CN, -NO₂, -OH, -SH et -NH₂;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et bicyclo[2,2,1]heptyle, le radical pouvant le cas échéant à chaque fois être substitué par 1, 2, 3, 4 ou 5 substituants choisis indépendamment l'un de l'autre dans le groupe constitué par oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S (=O)₂-NH-CH₃, phényle et -S(=O)₂-NH₂; le radical phényle pouvant à chaque fois être substitué par 1, 2, 3, 4 ou 5 substituants choisis indépendamment l'un de l'autre dans le groupe constitué par F, Cl, Br, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃) et -O-CH₂-CH₂-CH₂-CH₃;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-O-CH₃, -NH-C(=O)-O-C₂H₅, -NH-C(=O)-O-C(CH₃)₃, -O-CH₂-CH₂-CH₂-CH₃, -NH-C(=O)-CH₃, -NH-C(=O)-C₂H₅, -NH-C(=O)-C(CH₃)₃, -C(=O)-OH, -(CH₂)-C(=O)-OH, -C(=O)-O-CH₃, -C(=O)-O-CH₂-CH₃, -C(=O)-O-CH (CH₃)₂, -C(=O)-O-C(CH₃)₃, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -C(=O)-H, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃, -S(=O)₂-NH₂, -S(=O)₂-NH-phényle, phényle, -O-phényle, -O-benzyle et benzyle, la partie cyclique des radicaux -S(=O)₂-NH-phényle, phényle, -O-phényle, -O-benzyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle.

9. Composés selon l'une ou plusieurs des revendications 1 à 8, **caractérisés en ce que**
R⁹ et R¹⁰, indépendamment l'un de l'autre, représentent à chaque fois un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle.

10. Composés selon l'une ou plusieurs des revendications 1 à 9, **caractérisés en ce que**
R¹¹, R¹³, R¹⁵, R¹⁷, R¹⁹, R²², R²³, R²⁴, R²⁵ et R²⁶, représentent, indépendamment l'un de l'autre, à chaque fois un radical hydrogène ou un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle.

11. Composés selon l'une ou plusieurs des revendications 1 à 10, **caractérisés en ce que**
R¹², R²⁸ et R³², indépendamment l'un de l'autre, représentent à chaque fois un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle.

12. Composés selon l'une ou plusieurs des revendications 1 à 11, **caractérisés en ce que**
R¹⁴, R¹⁶, R¹⁸ et R²⁰, indépendamment l'un de l'autre, représentent à chaque fois un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle ; représentent un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et bicyclo[2,2,1]heptyle, le radical pouvant le cas échéant à chaque fois être substitué par 1, 2, 3, 4 ou 5 substituants choisis indépendamment l'un de l'autre dans le groupe constitué par oxo (=O), thioxo (=S), -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, -(CH₂)-C(=O)-OH, -C(=O)-OH, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃, -C(=O)-C₂F₅, -C(=O)-NH₂, -C(=O)-NH-CH₃, -C(=O)-NH-C₂H₅, -C(-O)-NH-C(CH₃)₃, -C(=O)-N(CH₃)₂, -C(=O)-N(C₂H₅)₂, -S(=O)₂-CH₃, -S(=O)₂-C₂H₅, -NH-S(=O)₂-CH₃, -S(=O)₂-NH-CH₃ et -S(=O)₂-NH₂;
ou représentent un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) et S(=O)₂-NH₂.

13. Composés selon l'une ou plusieurs des revendications 1 à 12, **caractérisés en ce que**
R²¹, R²⁷, R²⁹, R³⁰ et R³¹, indépendamment l'un de l'autre, représentent à chaque fois un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -OH, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -SH, -S-CH₃, -S-C₂H₅, -S-CH(CH₃)₂, -S-CH₂-CH₂-CH₃, -S-C(CH₃)₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅) et S(=O)₂-NH₂.

14. Composés selon l'une ou plusieurs des revendications 1 à 13, **caractérisés en ce que**
n vaut 1, 2 ou 3 ;
R¹ représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, méthyle, éthyle, n-propyle, isopropyle, n-butyle, sec-butyle, isobutyle, tert-butyle, n-pentyle, n-hexyle, n-heptyle, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃ et -O-C(CH₃)₃ ;
R² représente -C(=S)-NH-R³ ;
représente -C(=O)-NH-R⁴ ;
représente -S(=O)₂-R⁵ ;
représente -(CH₂)-C(=O)-NH-R⁶ ;
représente -(CH₂)-O-R⁷, représente -(CH₂)-S-R⁷,
représente -(CH₂)-NH-R⁷, représente -(CH₂)-N(CH₃)-R⁷, représente -(CH₂)-(CH₂)-O-R⁷, représente -(CH₂)-(CH₂)-S-R⁷, représente -(CH₂)-NH-R⁷, représente -(CH₂)-N(CH₃)-R⁷, représente -(CH₂)-(CH₂)-(CH₂)-O-R⁷, représente - (CH₂)-(CH₂)-(CH₂)-S-R⁷, représente - (CH₂)-(CH₂)-(CH₂)-NH-R⁷, représente -(CH₂)-(CH₂)-(CH₂)-N(CH₃)-R⁷, représente - (CH₂)-O-(CH₂)-R⁷, représente -(CH₂)-S-(CH₂)-R⁷ ; représente -(CH₂)-NH-(CH₂)-R⁷; représente -C(=O)-R⁸
ou représente -S(=O)₂-NR⁹R¹⁰;
R³ représente -(CHR¹¹)-C(=O)-O-R¹² ou -(CHR¹¹)-(CH₂)-C(=O)-O-R¹²;
représente -(CHR¹³)-R¹⁴, -(CHR¹³)-(CH₂)R¹⁴, -(CHR¹³)-(CH₂)-(CH₂)-R¹⁴, -(CHR¹³)-O-R¹⁴, -(CHR¹³)-(CH₂)-O-R¹⁴, -(CHR¹³)-(CH₂)-(CH₂)-O-R¹⁴ ou -(CHR¹³)-O-(CH₂)-R¹⁴; représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle et 3-butényle ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃ et -C(=O)-C₂F5 ;
R⁴ représente -(CHR¹⁵)-R¹⁶, -(CHR¹⁵)-(CH₂)-R¹⁶ ou -(CHR¹⁵)-(CH₂)-(CH₂)-R¹⁶;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -NO₂, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, méthyle, éthyle, n-propyle, isopropyle, n-butyle, sec-butyle, isobutyle, tert-butyle, n-pentyle, n-hexyle, n-heptyle, -C(=O)-O-CH₃, -C(=O)-O-CH₂-CH₃, -C(=O)-O-CH(CH₃)₂, -C(=O)-O-C(CH₃)₃, -C(=O)-CH₃, -C(=O)-C₂H₅, -C(=O)-C(CH₃)₃, -C(=O)-CF₃ et -C(=O)-C₂F₅ ;
R⁵ représente -(CHR¹⁷)-R¹⁸, -(CHR¹⁷)-(CH₂)-R¹⁸ ou -(CHR¹⁷)-(CH₂)-(CH₂)-R¹⁸;
représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃);
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ et -S(=O)₂-C₂H₅ ;
R⁶ représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -S(=O)₂-NH-phényle, phényle et benzyle, la partie cyclique des radicaux -S(=O)₂-NH-phényle, phényle et benzyle pouvant à chaque fois être substituée par 1, 2, 3, 4 ou 5 substituants choisis indépendamment l'un de l'autre dans le groupe constitué par F, Cl et Br ;
R⁷ représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, imidazolyle, indolyle, iso-indolyle, benzo[b]furannyle, benzo[b]thiophényle, thiazolyle, [1,2,4]-oxadiazolyle, [1,2,3]-thiadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, -CN, -CF₃, -SF₅, -OH, -O-CH₃, -O-C₂H₅, -NO₂, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, -C(CH₃)₂-C₂H₅, n-hexyle, n-heptyle, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, -O-CH₃, -O-C₂H₅, -O-CH(CH₃)₂, -O-CH₂-CH₂-CH₃, -O-C(CH₃)₃ et -O-CH₂-CH₂-CH₂-CH₃ ;
R⁸ représente -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²¹-(CHR¹⁹)-(CH₂)-O-R²⁰, -(CHR¹⁹)-(CH₂)-(CH₂)-O-R²⁰, -(CHR¹⁹)-O-(CH₂)-R²⁰ ou -(CHR¹⁹)-O-(CH²)-(CH²)-O-R²⁰;
représente -(CH=CH)-R²¹;
représente -(CR²²R²³)-C(=O)-O-R²⁶, -(CR²²R²³)-(CR²⁴R^{z5})-C(=O)-O-R²⁶ , -(CR²²R²³)-(CR²⁴R²⁵)-(CH₂)-C(=O)-O-R²⁶ ou -(CR²²R²³)-O-(CR²⁴R²⁵)-C(=O)-O-R²⁶;
représente -(CHR²⁷)-O-C(=O)-R²⁸ ;
représente CH[(CH₂)R²⁹][NH-S(=O)₂-R³⁰];
représente -CH[(CH₂)R³¹][NH-C(=O)-O-R³²]; représente un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et bicyclo[2,2,1]heptyle, le radical pouvant le cas échéant à chaque fois être substitué par 1, 2, 3, 4 ou 5 substituants choisis indépendamment l'un de l'autre dans le groupe constitué par -C(=O)-CH₃, -C(=O)-C₂H₅, -C(=O)-C(CH₃)₃ et phényle ;
ou représente un radical choisi dans le groupe constitué par phényle, naphtyle, (1,3)-benzodioxolyle, (1,4)-benzodioxanyle, 2H-chroményle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, [1,2,3]-thiadiazolyle, [1,2,4]-oxadiazolyle, benzo[2,1,3]thiadiazolyle, [1,2,3]-benzothiadiazolyle, [2,1,3]-benzoxadiazolyle, [1,2,3]-benzoxadiazolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle, oxazolyle, isoxazolyle, pyridazinyle, pyrazinyle, pyrimidinyle, indazolyle, quinazolinyle, quinoléinyle et isoquinoléinyle, le radical pouvant le cas échéant à chaque fois être substitué par 1, 2, 3, 4 ou 5 substituants choisis indépendamment l'un de l'autre dans le groupe constitué par F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, phényle, -O-phényle, -O-benzyle et benzyle, la partie cyclique des radicaux phényle, -O-phényle, -O-benzyle et benzyle pouvant à chaque fois être substituée par 1, 2, 3, 4 ou 5 substituants choisis indépendamment l'un de l'autre dans le groupe constitué par F, Cl, Br, -OH, -CF₃, -SF₅, -NO₂, méthyle, éthyle, n-propyle, isopropyle, n-butyle, sec-butyle, isobutyle, tert-butyle, n-pentyle, n-hexyle, n-heptyle, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, phényle et -O-benzyle ;
R⁹ et R¹⁰, indépendamment l'un de l'autre, représentent à chaque fois un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle ;
R¹¹, R¹³, R¹⁵, R¹⁷, R¹⁹, R²², R²³, R²⁴, R²⁵ et R²⁶, indépendamment l'un de l'autre, représentent à chaque fois un radical hydrogène ou un radical choisi dans le groupe constitué par méthyle, éthyle, n-propyle, isopropyle, n-butyle, sec-butyle, isobutyle et tert-butyle ;
R¹⁴, R²⁸ et R³², indépendamment l'un de l'autre, représentent à chaque fois un radical choisi dans le groupe constitué par méthyle, éthyle, n-propyle, isopropyle, n-butyle, sec-butyle, isobutyle et tert-butyle ;
R¹⁴, R¹⁶, R¹⁸ et R²⁰, indépendamment l'un de l'autre, représentent à chaque fois un radical choisi dans le groupe constitué par méthyle, éthyle, n-propyle, isopropyle, n-butyle, sec-butyle, isobutyle et tert-butyle ; représentent un radical choisi dans le groupe constitué par cyclopropyle, cyclobutyle, cyclopentyle, cyclohexyle, cycloheptyle, tétrahydrofurannyle, tétrahydrothiophényle, tétrahydropyrannyle et bicyclo[2,2,1]heptyle, le radical pouvant le cas échéant à chaque fois être substitué par 1, 2, 3, 4 ou 5 substituants choisis indépendamment l'un de l'autre dans le groupe constitué par oxo (=O), thioxo (=S), méthyle, éthyle, n-propyle, isopropyle, n-butyle, sec-butyle, isobutyle, tert-butyle, n-pentyle, n-hexyle, n-heptyle, -(CH₂)-C(=O)-OH et -C(=O)-OH ; ou représentent un radical choisi dans le groupe constitué par phényle et naphtyle, le radical pouvant à chaque fois être substitué par 1, 2, 3, 4 ou 5 substituants choisis indépendamment l'un de l'autre dans le groupe constitué par F, Cl, Br, -O-CH₃, -O-C₂H₅, -O-CH(CH₃)₂, -O-CH₂-CH₂-CH₃, -O-C(CH₃)₃, -O-CH₂-CH₂-CH₂-CH₃, méthyle, éthyle, n-propyle, isopropyle, n-butyle, sec-butyle, isobutyle, tert-butyle, n-pentyle, -C(CH₃)₂-C₂H₅, n-hexyle et n-heptyle ;
et
R²¹, R²⁷, R²⁹, R³⁰ et R³¹, indépendamment l'un de l'autre, représentent à chaque fois un radical choisi dans le groupe constitué par phényle, naphtyle, indolyle et iso-indolyle, le radical pouvant à chaque fois être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, -C(CH₃)₂-C₂H₅, n-hexyle et n-heptyle ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

15. Composés selon l'une ou plusieurs des revendications 1 à 14, **caractérisés en ce que**
n vaut 1 ou 2 ;
R¹ représente un radical choisi dans le groupe constitué par phényle et naphtyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -SF₅, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, méthyle, éthyle, n-propyle, isopropyle, n-butyle, sec-butyle, isobutyle, tert-butyle, n-pentyle, n-hexyle, n-heptyle, phényle et benzyle ;
R² représente -C(=S)-NH-R³;
représente -C(=O)-NH-R⁴;
représente -S(=O)₂-R⁵;
représente -(CH₂)-C(=O)-NH-R⁶;
représente -(CH₂)-O-R⁷, -(CH₂)-(CH₂)-O-R⁷, -(CH₂)-(CH₂)-(CH₂)-O-R;
représente -C(=O)-R⁸
ou représente -S(=O)₂-NR⁹R¹⁰;
R³ représente -(CHR¹¹)-(CH₂)-C(=O)-O-R¹²;
représente -(CHR¹³)-R¹⁴, -(CHR¹³)-(CH₂)-R¹⁴, -(CHR¹³)-(CH₂)-(CH₂)-R¹⁴ ou -(CHR¹³)-(CH₂)-O-R¹⁹;
représente un radical choisi dans le groupe constitué par vinyle, 1-propényle, 2-propényle, 1-butényle, 2-butényle et 3-butényle ;
ou représente un radical phényle, le radical pouvant être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, méthyle, éthyle, n-propyle, isopropyle, n-butyle, sec-butyle, isobutyle, tert-butyle, n-pentyle, n-hexyle, n-heptyle, -C(=O)-CH₃, -C(=O)-C₂H₅, -C(=O)-C(CH₃)₃, -C(=O)-CF₃ et -C(=O)-C₂F₅;
R⁴ représente -(CHR¹⁵)-R¹⁶, -(CHR¹⁵)-(CH₂)-R¹⁶ ou -(CHR¹⁵)-(CH₂)-(CH₂)-R¹⁶;
ou représente un radical choisi dans le groupe constitué par phényle et naphtyle, le radical pouvant le cas échéant à chaque fois être substitué par 1, 2, 3, 4 ou 5 substituants choisis indépendamment l'un de l'autre dans le groupe constitué par F, Cl, Br, -NO₂, I, -CN, -CF₃, -O-C₂H₅, -O-CH(CH₃)₂, -O-CH₂-CH₂-CH₃, -O-C(CH₃)₃, -O-CH₂-CH₂-CH₂-CH₃, méthyle, éthyle, n-propyle, isopropyle, n-butyle, sec-butyle, isobutyle, tert-butyle, n-pentyle, n-hexyle, n-heptyle, -C(=O)-O-CH₃, -C(=O)-O-CH₂-CH₃, -C(=O)-O-CH(CH₃)₂, -C(=O)-O-C(CH₃)₃, -C(=O)-CH₃, -C(=O)-C₂H₅, -C(=O)-C(CH₃)₃, -C(=O)-CF₃ et -C(=O)-C₂F₅;
R⁵ représente -(CHR¹⁷)-R¹⁸, -(CHR¹⁷)-(CH₂)-R¹⁸ ou -(CHR¹⁷)-(CH₂)-(CH₂)-R¹⁸;
représente un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃);
ou représente un radical choisi dans le groupe constitué par phényle, naphtyle, pyrazolyle, thiophényle, [1,2,3,4]-tétrahydroquinoléinyle et [1,2,3,4]-tétrahydro-isoquinoléinyle, le radical pouvant le cas échéant à chaque fois être substitué par 1, 2, 3, 4 ou 5 substituants choisis indépendamment l'un de l'autre dans le groupe constitué par F, Cl, Br, I, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -C(=O)-O-CH₃, -C(=O)-O-CH₂-CH₃, -C(=O)-O-CH(CH₃)₂, -C(=O)-O-C(CH₃)₃, -C(=O)-CF₃, -C(=O)-C₂F₅, -S(=O)₂-CH₃ et -S(=O)₂-C₂H₅ ;
R⁶ représente un radical choisi dans le groupe constitué par phényle, naphtyle, pyrazolyle, thiophényle et thiazolyle, le radical pouvant le cas échéant à chaque fois être substitué par 1, 2, 3, 4 ou 5 substituants choisis indépendamment l'un de l'autre dans le groupe constitué par F, Cl, Br, -CF₃, -CN, -O-CH₃, -O-C₂H₅, -O-CH(CH₃)₂, -O-CH₂-CH₂-CH₃, -O-C(CH₃)₃, -O-CH₂-CH₂-CH₂-CH₃, -O-CF₃, méthyle, éthyle, n-propyle, isopropyle, n-butyle, sec-butyle, isobutyle, tert-butyle, n-pentyle, -C(CH₃)₂-C₂H₅, n-hexyle, n-heptyle, -S(=O)₂-NH-phényle, phényle et benzyle, la partie cyclique des radicaux -S(=O)₂-NH-phényle, phényle et benzyle pouvant à chaque fois être substituée par 1, 2, 3, 4 ou 5 substituants choisis indépendamment l'un de l'autre dans le groupe constitué par F, Cl et Br ;
R⁷ représente un radical choisi dans le groupe constitué par phényle, naphtyle, benzo[b]furannyle, benzo[b]thiophényle et [1,2,4]-oxadiazolyle, le radical pouvant le cas échéant à chaque fois être substitué par 1, 2, 3, 4 ou 5 substituants choisis indépendamment l'un de l'autre dans le groupe constitué par F, Cl, Br, -CN, -CF₃, -SF₅, -OH, -O-CH₃, -O-C₂H₅, -NO₂, -O-CF₃, -S-CF₃, -S-CF₂H, -S-CFH₂, méthyle, éthyle, n-propyle, isopropyle, n-butyle, sec-butyle, isobutyle, tert-butyle, n-pentyle, -C(CH₃)₂-C₂H₅, n-hexyle, n-heptyle, phényle et benzyle, la partie cyclique des radicaux phényle et benzyle pouvant à chaque fois être substituée par 1, 2, 3, 4 ou 5 substituants choisis indépendamment l'un de l'autre dans le groupe constitué par F, Cl, Br, -O-CH₃, -O-C₂H₅, -O-CH(CH₃)₂, -O-CH₂-CH₂-CH₃, -O-C(CH₃)₃ et -O-CH₂-CH₂-CH₂-CH₃;
R⁸ représente -(CHR¹⁹)-R²⁰, -(CHR¹⁹)-(CH₂)-R²⁰, -(CHR¹⁹)-(CH₂)-(CH₂)-R²⁰, -(CHR¹⁹)-O-R²⁰, -(CHR¹⁹)-(CH₂)-O-R²⁰, -(CHR¹⁹)-(CH₂)-(CH₂)-O-R²⁰, -(CHR¹⁹)-O-(CH₂)-R²⁰ ou -(CHR¹⁹)-O-(CH₂)-(CH₂)-O-R²⁰ représente -(CH=CH)-R²¹; représente -(CR²²R²³)-C(=O)-O-R²⁶, -(CR²²R²³)-(CR²⁴R²⁵)-C(=O)-O-R²⁶, -(CR²²R²³)-(CR²⁴R²⁵)-(CH₂)-C(=O)-O-R²⁶ ou -(CR²²R²³)-O-(CR²⁴R²⁵)-C(=O)-O-R²⁶;
représente -(CHR²⁷)-O-C(=O)-R²⁸;
représente -CH[(CH₂)R²⁹][NH-S(=O)₂-R³⁰];
représente -CH[(CH₂)R³¹][NH-C(=O)-O-R³²];
représente un radical choisi dans le groupe constitué par méthyle, éthyle, n-propyle, isopropyle, n-butyle, sec-butyle, isobutyle, tert-butyle, n-pentyle, sec-pentyle, 3-pentyle, -(CH₂)-(CH₂)-(C(CH₃)₃), n-hexyle, 2-hexyle, 3-hexyle, n-heptyle, 2-heptyle, 3-heptyle, 4-heptyle et n-octyle ;
représente un radical choisi dans le groupe constitué par cyclopropyle, cyclobutyle, cyclopentyle, cyclohexyle, cycloheptyle, pyrrolidinyle, pipéridinyle et adamantyle, le radical pouvant le cas échéant à chaque fois être substitué par 1, 2, 3, 4 ou 5 substituants choisis indépendamment l'un de l'autre dans le groupe constitué par -C(=O)-CH₃, -C(=O)-C₂H₅, -C(=O)-C(CH₃) et phényle ;
ou représente un radical choisi dans le groupe constitué par phényle, naphtyle, 2H-chroményle, thiophényle, furannyle, pyrazolyle, triazolyle, pyridinyle, [1,2,3]-thiadiazolyle, [2,1,3]-benzoxadiazolyle, [1,2,3]-benzoxadiazolyle, oxazolyle et isoxazolyle, le radical pouvant le cas échéant à chaque fois être substitué par 1, 2, 3, 4 ou 5 substituants choisis indépendamment l'un de l'autre dans le groupe constitué par F, Cl, Br, -CN, -CF₃, -O-CH₃, -O-C₂H₅, -O-CH(CH₃)₂, -O-CH₂-CH₂-CH₃, -O-C(CH₃)₃, -O-CH₂-CH₂-CH₂-CH₃, -NO₂, -O-CF₃, -S-CF₃, -S-CF₂H, -S-CFH₂, -S-CH₃, -S-C₂H₅, méthyle, éthyle, n-propyle, isopropyle, n-butyle, sec-butyle, isobutyle, tert-butyle, n-pentyle, -C(CH₃)₂-C₂H₅, n-hexyle, n-heptyle, -NH-C(=O)-CH₃, -NH-C(=O)-C₂H₅, -NH-CH₃, -NH-C₂H₅, -NH-CH(CH₃)₂, -NH-C(CH₃)₃, -N(CH₃)₂, -N(C₂H₅)₂, -N(CH₃)(C₂H₅), -S(=O)₂-NH₂, phényle, -O-phényle, -O-benzyle et benzyle, la partie cyclique des radicaux phényle, -O-phényle, -O-benzyle et benzyle pouvant à chaque fois être substituée par 1, 2, 3, 4 ou 5 substituants choisis indépendamment l'un de l'autre dans le groupe constitué par F, Cl et Br ;
R⁹ et R¹⁰, indépendamment l'un de l'autre, représentent à chaque fois un radical choisi dans le groupe constitué par méthyle, éthyle, n-propyle, isopropyle, n-butyle, sec-butyle, isobutyle et tert-butyle ;
R¹¹, R¹³, R¹⁵, R¹⁷, R¹⁹, R²², R²³, R²⁴, R²⁵ et R²⁶, indépendamment l'un de l'autre, représentent à chaque fois un radical hydrogène ou un radical choisi dans le groupe constitué par méthyle, éthyle et n-propyle ;
R¹², R²⁸ et R³², indépendamment l'un de l'autre, représentent à chaque fois un radical choisi dans le groupe constitué par méthyle, éthyle, n-propyle, isopropyle, n-butyle, sec-butyle, isobutyle et tert-butyle ;
R¹⁴ représente un radical choisi dans le groupe constitué par méthyle, éthyle, n-propyle, isopropyle, n-butyle, sec-butyle, isobutyle et tert-butyle ;
représente un radical choisi dans le groupe constitué par cyclopropyle, cyclobutyle, cyclopentyle, cyclohexyle, cycloheptyle, tétrahydrofurannyle, tétrahydrothiophényle et tétrahydropyrannyle ;
ou représente un radical phényle, qui peut être substitué par 1, 2, 3, 4 ou 5 substituants choisis dans le groupe constitué par F, Cl et Br ;
R¹⁶ représente un radical choisi dans le groupe constitué par phényle et naphtyle, le radical pouvant à chaque fois être substitué par 1, 2, 3, 4 ou 5 substituants choisis indépendamment l'un de l'autre dans le groupe constitué par F, Cl et Br ;
R¹⁸ représente un radical 7,7-diméthyl-2-oxobicyclo[2,2,1]hept-1-yle ;
R²⁰ représente un radical choisi dans le groupe constitué par méthyle, éthyle, n-propyle, isopropyle, n-butyle, sec-butyle, isobutyle et tert-butyle ; représente un radical choisi dans le groupe constitué par cyclopropyle, cyclobutyle, cyclopentyle, cyclohexyle et cycloheptyle ; le radical pouvant à chaque fois être substitué par un substituant choisi dans le groupe constitué par -(CH₂)-C(=O)-OH et -C(=O)-OH ;
ou représente un radical phényle, qui peut être substitué par 1, 2, 3, 4 ou 5 substituants choisis indépendamment l'un de l'autre dans le groupe constitué par F, Cl, Br, -O-CH₃, -O-C₂H₅, -O-CH(CH₃)₂, -O-CH₂-CH₂-CH₃, -O-C(CH₃)₃ et -O-CH₂-CH₂-CH₂-CH₃ ;
R²¹ représente un radical phényle, qui peut être substitué par 1, 2, 3, 4 ou 5 substituants choisis dans le groupe constitué par F, Cl et Br ;
R²⁷ représente un radical phényle ;
R²⁹ représente un radical phényle ;
R³⁰ représente un radical phényle, qui peut être substitué par 1, 2, 3, 4 ou 5 substituants choisis dans le groupe constitué par méthyle, éthyle, n-propyle, isopropyle, n-butyle, sec-butyle et tert-butyle ;
et
R³¹ représente un radical indolyle ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

16. Composés selon l'une ou plusieurs des revendications 1 à 15 choisis dans le groupe constitué par
1. le pentafluorophénylamide de l'acide 3-(4-méthylbenzyl)-2-oxo-1-oxa-3,8-diazaspiro[4,5]décane-8-carbothioïque
2. le (2,5-dichlorophényl)-amide de l'acide 3-(3-bromobenzyl)-2-oxo-1-oxa-3,8-diazaspiro[4,5]décane-8-carbothioïque
3. le benzylamide de l'acide 2-oxo-3-(4-trifluorométhylbenzyl)-1-oxa-3,8-diazaspiro[4,5]décane-8-carbothioïque
4. le (tétrahydrofurann-2-ylméthyl)-amide de l'acide 3-(3,5-diméthylbenzyl)-2-oxo-1-oxa-3,8-diazaspiro[4,5]décane-8-carbothioïque
5. le (2-méthoxyéthyl)-amide de l'acide 3-(3-bromobenzyl)-2-oxo-1-oxa-3,8-diazaspiro[4,5]décane-8-carbothioïque
6. le (2-méthoxyphényl)-amide de l'acide 3-(3-bromobenzyl)-2-oxo-1-oxa-3,8-diazaspiro[4,5]décane-8-carbothioïque
7. le (3-acétylphényl)-amide de l'acide 3-(3,5-diméthylbenzyl)-2-oxo-1-oxa-3,8-diazaspiro[4,5]décane-8-carbothioïque
8. l'o-toluylamide de l'acide 3-benzyl-2-oxo-1-oxa-3,8-diazaspiro[4,5]décane-8-carbothioïque
9. l'ester éthylique de l'acide 3-{[3-(3-bromobenzyl)-2-oxo-1-oxa-3,8-diazaspiro[4,5]décane-8-carbothioyl]-amino}-butanoïque
10. l'allylamide de l'acide 3-naphtalén-2-ylméthyl-2-oxo-1-oxa-3,8-diazaspiro[4,5]décane-8-carbothioïque
11. le benzylamide de l'acide 3-(3,4-difluorobenzyl)-2-oxo-1-oxa-3,8-diazaspiro[4,5]décane-8-carbothioïque
12. le (1-phényléthyl)-amide de l'acide 3-biphényl-2-ylméthyl-2-oxo-1-oxa-3,8-diazaspiro[4,5]décane-8-carbothioïque
13. le (4-éthoxyphényl)-amide de l'acide 3-(3,4-difluorobenzyl)-2-oxo-1-oxa-3,8-diazaspiro[4,5]décane-8-carbothioïque
14. le (3,5-dichlorophényl)-amide de l'acide 3-(3-cyanobenzyl)-2-oxo-1-oxa-3,8-diazaspiro[4,5]décane-8-carbothioïque
15. le (3-trifluorométhylphényl)-amide de l'acide 3-(3-cyanobenzyl)-2-oxo-1-oxa-3,8-diazaspiro[4,5]décane-8-carbothioïque
16. le benzylamide de l'acide 3-(3-bromobenzyl)-2-oxo-1-oxa-3,8-diazaspiro[4,5]décane-8-carbothioïque
17. le (tétrahydrofurann-2-ylméthyl)-amide de l'acide 3-(2-méthoxy-5-nitrobenzyl)-2-oxo-1-oxa-3,8-diazaspiro[4,5]décane-8-carbothioïque
18. le phénylamide de l'acide 3-biphényl-2-ylméthyl-2-oxo-1-oxa-3,8-diazaspiro[4,5]décane-8-carbothioïque
19. le cyclohexylméthylamide de l'acide 3-(3-cyanobenzyl)-2-oxo-1-oxa-3,8-diazaspiro[4,5]décane-8-carbothioïque
20. le (3-acétylphényl)-amide de l'acide 3-(2-fluorobenzyl)-2-oxo-1-oxa-3,8-diazaspiro[4,5]décane-8-carbothioïque
21. le cyclohexylméthylamide de l'acide 3-(2-méthoxy-5-nitrobenzyl)-2-oxo-1-oxa-3,8-diazaspiro[4,5]décane-8-carbothioïque
22. le 4-chlorobenzylamide de l'acide 3-(3-cyanobenzyl)-2-oxo-1-oxa-3,8-diazaspiro[4,5]décane-8-carbothioïque
23. le benzylamide de l'acide 3-(2-méthoxy-5-nitrobenzyl)-2-oxo-1-oxa-3,8-diazaspiro[4,5]décane-8-carbothioïque
24. le (4-éthoxyphényl)-amide de l'acide 3-(2-fluorobenzyl)-2-oxo-1-oxa-3,8-diazaspiro[4,5]décane-8-carboxylique
25. le (2,5-dichlorophényl)-amide de l'acide 3-biphényl-2-ylméthyl-2-oxo-1-oxa-3,8-diazaspiro[4,5]décane-8-carboxylique
26. le (2,5-difluorophényl)-amide de l'acide 3-(2-méthoxy-5-nitrobenzyl)-2-oxo-1-oxa-3,8-diazaspiro[4,5]décane-8-carboxylique
27. (4-méthyl-3-nitrophényl)-amide de l'acide 3-(4-méthylbenzyl)-2-oxo-1-oxa-3,8-diazaspiro[4,5]décane-8-carboxylique
28. le 4-fluorobenzylamide de l'acide 3-(3,4-difluorobenzyl)-2-oxo-1-oxa-3,8-diazaspiro[4,5]décane-8-carboxylique
29. le (2,5-difluorophényl)-amide de l'acide 3-(3-bromobenzyl)-2-oxo-1-oxa-3,8-diazaspiro[4,5]décane-8-carboxylique
30. l'ester éthylique de l'acide 4-[(3-naphtalén-2-ylméthyl-2-oxo-1-oxa-3,8-diazaspiro[4,5]décane-8-carbonyl)-amino]-benzoïque
31. le (4-chloro-3-trifluorométhylphényl)-amide de l'acide 2-oxo-3-(4-trifluorométhylbenzyl)-1-oxa-3,8-diazaspiro[4,5]décane-8-carboxylique
32. le (2,5-difluorophényl)-amide de l'acide 3-(2-cyanobenzyl)-2-oxo-1-oxa-3,8-diazaspiro[4,5]décane-8-carboxylique
33. le (3-cyanophényl)-amide de l'acide 3-benzyl-2-oxo-1-oxa-3,8-diazaspiro[4,5]décane-8-carboxylique
34. le (4-méthyl-3-nitrophényl)-amide de l'acide 3-(3,4-difluorobenzyl)-2-oxo-1-oxa-3,8-diazaspiro[4,5]décane-8-carboxylique
35. le (3-méthoxyphényl)-amide de l'acide 3-(3,4-difluorobenzyl)-2-oxo-1-oxa-3,8-diazaspiro[4,5]décane-8-carboxylique
36. le 3,4-dichlorobenzylamide de l'acide 3-(4-méthylbenzyl)-2-oxo-1-oxa-3,8-diazaspiro[4,5]décane-8-carboxylique
37. le (4-chloro-3-trifluorométhylphényl)-amide de l'acide 3-benzyl-2-oxo-1-oxa-3,8-diazaspiro[4,5]décane-8-carboxylique
38. le (3-cyanophényl)-amide de l'acide 2-oxo-3-(4-trifluorométhylsulfanylbenzyl)-1-oxa-3,8-diazaspiro[4,5]décane-8-carboxylique
39. le (4-méthoxyphényl)-amide de l'acide 3-benzyl-2-oxo-1-oxa-3,8-diazaspiro[4,5]décane-8-carboxylique
40. le (4-méthyl-3-nitrophényl)-amide de l'acide 2-oxo-3-(4-trifluorométhylsulfanylbenzyl)-1-oxa-3,8-diazaspiro[4,5]décane-8-carboxylique
41. le (4-butoxyphényl)-amide de l'acide 3-(4-iodobenzyl)-2-oxo-1-oxa-3,8-diazaspiro[4,5]décane-8-carboxylique
42. le 3,4-dichlorobenzylamide de l'acide 3-(3-bromobenzyl)-2-oxo-1-oxa-3,8-diazaspiro[4,5]décane-8-carboxylique
43. le (1-phényléthyl)-amide de l'acide 3-benzyl-2-oxo-1-oxa-3,8-diazaspiro[4,5]décane-8-carboxylique
44. le 3,4-dichlorobenzylamide de l'acide 3-naphtalén-2-ylméthyl-2-oxo-1-oxa-3,8-diazaspiro[4,5]décane-8-carboxylique
45. le phénéthylamide de l'acide 3-(4-méthylbenzyl)-2-oxo-1-oxa-3,8-diazaspiro[4,5]décane-8-carboxylique
46. le (3-méthoxyphényl)-amide de l'acide 2-oxo-3-(2-trifluorométhylbenzyl)-1-oxa-3,8-diazaspiro[4,5]décane-8-carboxylique
47. le (4-méthoxyphényl)-amide de l'acide 3-(4-cyanobenzyl)-2-oxo-1-oxa-3,8-diazaspiro[4,5]décane-8-carboxylique
48. le (1-naphtalén-1-yl-éthyl)-amide de l'acide 2-oxo-3-(4-trifluorométhylbenzyl)-1-oxa-3,8-diazaspiro[4,5]décane-8-carboxylique
49. le phénéthylamide de l'acide 2-oxo-3-(2-trifluorométhylbenzyl)-1-oxa-3,8-diazaspiro[4,5]décane-8-carboxylique
50. le (4-méthyl-3-nitrophényl)-amide de l'acide 3-(4-iodobenzyl)-2-oxo-1-oxa-3,8-diazaspiro[4,5]décane-8-carboxylique
51. le (3-cyanophényl)-amide de l'acide 3-(2-méthoxy-5-nitrobenzyl)-2-oxo-1-oxa-3,8-diazaspiro[4,5]décane-8-carboxylique
52. le (4-méthoxyphényl)-amide de l'acide 3-naphtalén-2-ylméthyl-2-oxo-1-oxa-3,8-diazaspiro[4,5]décane-8-carboxylique
53. l'ester éthylique de l'acide 3-{[3-(4-méthylbenzyl)-2-oxo-1-oxa-3,8-diazaspiro[4,5]décane-8-carbonyl]-amino}-benzoïque
54. le (1-naphtalén-1-yl-éthyl)-amide de l'acide 2-oxo-3-(2-trifluorométhylbenzyl)-1-oxa-3,8-diazaspiro[4,5]décane-8-carboxylique
55. (2,5-diméthoxyphényl)-amide de l'acide 3-naphtalén-2-ylméthyl-2-oxo-1-oxa-3,8-diazaspiro[4,5]décane-8-carboxylique
56. le (3-acétylphényl)-amide de l'acide 3-(4-cyanobenzyl)-2-oxo-1-oxa-3,8-diazaspiro[4,5]décane-8-carboxylique
57. le phénéthylamide de l'acide 3-biphényl-2-ylméthyl-2-oxo-1-oxa-3,8-diazaspiro[4,5]décane-8-carboxylique
58. le (5-chloro-2-méthoxyphényl)-amide de l'acide 3-(4-cyanobenzyl)-2-oxo-1-oxa-3,8-diazaspiro[4,5]décane-8-carboxylique
59. l'ester éthylique de l'acide 3-{[2-oxo-3-(2-trifluorométhylbenzyl)-1-oxa-3,8-diazaspiro[4,5]décane-8-carbonyl]-amino}-benzoïque
60. le (3-fluorophényl)-amide de l'acide 3-benzyl-2-oxo-1-oxa-3,8-diazaspiro[4,5]décane-8-carboxylique
61. l'ester éthylique de l'acide 3-{[3-(3-cyanobenzyl)-2-oxo-1-oxa-3,8-diazaspiro[4,5]décane-8-carbonyl]-amino}-benzoïque
62. le (3-méthoxyphényl)-amide de l'acide 3-biphényl-2-ylméthyl-2-oxo-1-oxa-3,8-diazaspiro[4,5]décane-8-carboxylique
63. le (1-naphtalén-1-yl-éthyl)-amide de l'acide 3-(2-méthoxy-5-nitrobenzyl)-2-oxo-1-oxa-3,8-diazaspiro[4,5]décane-8-carboxylique
64. l'acide {2-[3-(3,4-difluorobenzyl)-2-oxo-1-oxa-3,8-diazaspiro[4,5]déc-8-yl]-2-oxoéthoxy}-acétique
65. l'acide 4-[3-(3,4-difluorobenzyl)-2-oxo-1-oxa-3,8-diazaspiro[4,5]déc-8-yl]-3,3-diméthyl-4-oxobutanoïque
66. l'ester tert-butylique de l'acide [2-[3-(3-bromobenzyl)-2-oxo-1-oxa-3,8-diazaspiro[4,5]déc-8-yl]-1-(1H-indol-3-ylméthyl)-2-oxoéthyl]-carbamique
67. l'acide 5-[3-(2-fluorobenzyl)-2-oxo-1-oxa-3,8-diazaspiro[4,5]déc-8-yl]-3-méthyl-5-oxopentanoïque
68. l'acide 3,3-diméthyl-5-[3-(4-méthylbenzyl)-2-oxo-1-oxa-3,8-diazaspiro[4,5]déc-8-yl]-5-oxopentanoïque
69. l'acide 5-[3-(3,4-difluorobenzyl)-2-oxo-1-oxa-3,8-diazaspiro[4,5]déc-8-yl]-3-méthyl-5-oxopentanoïque
70. l'acide {2-oxo-2-[2-oxo-3-(4-trifluorométhylbenzyl)-1-oxa-3,8-diazaspiro[4,5]déc-8-yl]-éthoxy}-acétique
71. l'acide (1-{2-[3-(2-cyanobenzyl)-2-oxo-1-oxa-3,8-diazaspiro[4,5]déc-8-yl]-2-oxoéthyl}-cyclopentyl)-acétique
72. l'acide (1-{2-[3-(3,4-difluorobenzyl)-2-oxo-1-oxa-3,8-diazaspiro[4,5]déc-8-yl]-2-oxoéthyl}-cyclopentyl)-acétique
73. l'acide 5-(3-diphényl-2-ylméthyl-2-oxo-1-oxa-3,8-diazaspiro[4,5]déc-8-yl)-3-méthyl-5-oxopentanoïque
75. le N-[4-(3,5-dichlorophénylsulfamoyl)-phényl]-2-[3-(4-méthylbenzyl)-2-oxo-1-oxa-3,8-diazaspiro[4,5]déc-8-yl]-acétamide
76. le N-(2,5-diméthyl-2H-pyrazol-3-yl)-2-[3-(2-méthoxy-5-nitrobenzyl)-2-oxo-1-oxa-3,8-diazaspiro[4,5]déc-8-yl]-acétamide
80. le N-(3-cyano-4-méthylthiophén-2-yl)-2-[3-(3,4-difluorobenzyl)-2-oxo-1-oxa-3,8-diazaspiro[4,5]déc-8-yl]-acétamide
85. le 2-[2-oxo-3-(4-trifluorométhylsulfanylbenzyl)-1-oxa-3,8-diazaspiro[4,5]déc-8-yl]-N-(4-phényl-5-trifluorométhylthiophén-3-yl)-acétamide
86. le 2-[3-(2-fluorobenzyl)-2-oxo-1-oxa-3,8-diazaspiro[4,5]déc-8-yl]-N-(4-phénylthiazol-2-yl)-acétamide
87. le 2-[2-oxo-3-(2-trifluorométhylbenzyl)-1-oxa-3,8-diazaspiro[4,5]déc-8-yl]-N-(4-trifluorométhoxyphényl)-acétamide
91. le N-{4-[3-(3-cyanobenzyl)-2-oxo-1-oxa-3,8-diazaspiro[4,5]décane-8-carbonyl]-phényl}-acétamide
92. la 8-(4-éthoxybenzoyl)-3-(4-méthylbenzyl)-1-oxa-3,8-diazaspiro[4,5]décan-2-one
93. l'ester méthylique de l'acide 3-[3-(4-cyanobenzyl)-2-oxo-1-oxa-3,8-diazaspiro[4,5]déc-8-yl]-3-oxopropionique
94. le 2-[8-(2,4-difluorobenzoyl)-2-oxo-1-oxa-3,8-diazaspiro[4,5]déc-3-ylméthyl]-benzonitrile
95. la 8-(3-diméthylaminobenzoyl)-3-(3,5-diméthylbenzyl)-1-oxa-3,8-diazaspiro[4,5]décan-2-one
96. le N-{4-[3-(3,4-difluorobenzyl)-2-oxo-1-oxa-3,8-diazaspiro[4,5]décane-8-carbonyl]-phényl}-acétamide
97. la 8-(4-bromobenzoyl)-3-(4-fluorobenzyl)-1-oxa-3,8-diazaspiro[4,5]décan-2-one
98. le 4-[8-(adamantane-1-carbonyl)-2-oxo-1-oxa-3,8-diazaspiro[4,5]déc-3-ylméthyl]-benzonitrile
99. la 3-(3,5-diméthylbenzyl)-8-(2-éthylsulfanylpyridine-3-carbonyl)-1-oxa-3,8-diazaspiro[4,5]décan-2-one
100. la 3-(3-bromobenzyl)-8-(4-chlorobenzoyl)-1-oxa-3,8-diazaspiro[4,5]décan-2-one
101. la 8-[3-(2-chlorophényl)-5-méthylisoxazole-4-carbonyl]-3-(3,5-diméthylbenzyl)-1-oxa-3,8-diazaspiro[4,5]décan-2-one
102. la 3-(3-bromobenzyl)-8-(4-éthylbenzylsulfonyl)-1-oxa-3,8-diazaspiro[4,5]décan-2-one
103. la 8-(4-butoxybenzylsulfonyl)-3-(3,5-diméthylbenzyl)-1-oxa-3,8-diazaspiro[4,5]décan-2-one
104. la 8-(biphényl-4-carbonyl)-3-(3-bromobenzyl)-1-oxa-3,8-diazaspiro[4,5]décan-2-one
105. l'ester 2-[3-(3,5-diméthylbenzyl)-2-oxo-1-oxa-3,8-diazaspiro[4,5]déc-8-yl]-2-oxo-1-phényléthylique de l'acide acétique
106. le 4-[2-oxo-8-(2-phénylcyclopropanecarbonyl)-1-oxa-3,8-diazaspiro[4,5]déc-3-ylméthyl]-benzonitrile
107. la 8-(2,5-diméthoxybenzylsulfonyl)-3-(3,5-diméthylbenzyl)-1-oxa-3,8-diazaspiro[4,5]décan-2-one
108. la 8-(2-chlorobenzoyl)-3-(3,5-diméthylbenzyl)-1-oxa-3,8-diazaspiro[4,5]décan-2-one
109. la 3-(3-bromobenzyl)-8-(4-nitrobenzoyl)-1-oxa-3,8-diazaspiro[4,5]décan-2-one
110. le N-{1-benzyl-2-[3-(3-cyanobenzyl)-2-oxo-1-oxa-3,8-diazaspiro[4,5]déc-8-yl]-2-oxoéthyl}-4-méthylbenzylsulfonamide
111. la 8-(2-éthylsulfanylpyridine-3-carbonyl)-3-(3-méthoxybenzyl)-1-oxa-3,8-diazaspiro[4,5]décan-2-one
112. la 8-(2,4-diméthoxybenzoyl)-3-(3-méthoxybenzyl)-1-oxa-3,8-diazaspiro[4,5]décan-2-one
113. la 3-(3,5-diméthylbenzyl)-8-[2-(3-méthoxyphényl)-acétyl]-1-oxa-3,8-diazaspiro[4,5]décan-2-one
114. la 3-(3,5-diméthylbenzyl)-8-(4-méthoxy-2,3,6-triméthylbenzylsulfonyl)-1-oxa-3,8-diazaspiro[4,5]décan-2-one
115. le 4-[8-(biphényl-4-carbonyl)-2-oxo-1-oxa-3,8-diazaspiro[4,5]déc-3-ylméthyl]-benzonitrile
116. la 8-(2-chlorobenzylsulfonyl)-3-(3,5-diméthylbenzyl)-1-oxa-3,8-diazaspiro[4,5]décan-2-one
117. la 8-(benzo[1,2,5]oxadiazole-5-carbonyl)-3-(3,5-diméthylbenzyl)-1-oxa-3,8-diazaspiro[4,5]décan-2-one
118. la 8-(biphényl-4-carbonyl)-3-(4-iodobenzyl)-1-oxa-3,8-diazaspiro[4,5]décan-2-one
119. la 3-(3,5-diméthylbenzyl)-8-(pyridine-2-carbonyl)-1-oxa-3,8-diazaspiro[4,5]décan-2-one
120. la 8-(6-chloropyridine-3-carbonyl)-3-naphtalén-2-ylméthyl-1-oxa-3,8-diazaspiro[4,5]décan-2-one
121. la 3-(3-bromobenzyl)-8-(2-éthylsulfanylpyridine-3-carbonyl)-1-oxa-3,8-diazaspiro[4,5]décan-2-one
122. la 3-(3,5-diméthylbenzyl)-8-(5-méthylisoxazole-3-carbonyl)-1-oxa-3,8-diazaspiro[4,5]décan-2-one
123. la 3-(2-méthoxy-5-nitrobenzyl)-8-pentanoyl-1-oxa-3,8-diazaspiro[4,5]décan-2-one
124. la 8-(4-bromobenzoyl)-3-(3,5-diméthylbenzyl)-1-oxa-3,8-diazaspiro[4,5]décan-2-one
125. la 8-(5-tert-butyl-2-méthylfuranne-3-carbonyl)-3-(2-méthoxy-5-nitrobenzyl)-1-oxa-3,8-diazaspiro[4,5]décan-2-one
126. la 8-(3-chlorobenzoyl)-3-(3,5-diméthylbenzyl)-1-oxa-3,8-diazaspiro[4,5]décan-2-one
127. la 8-(2-éthylsulfanylpyridine-3-carbonyl)-3-(2-trifluorométhylbenzyl)-1-oxa-3,8-diazaspiro[4,5]décan-2-one
128. la 3-(3,5-diméthylbenzyl)-8-(isoxazole-5-carbonyl)-1-oxa-3,8-diazaspiro[4,5]décan-2-one
129. la 3-(3-bromobenzyl)-8-(pyridine-2-carbonyl)-1-oxa-3,8-diazaspiro[4,5]décan-2-one
130. la 3-(3,5-diméthylbenzyl)-8-(thiophène-2-carbonyl)-1-oxa-3,8-diazaspiro[4,5]décan-2-one
131. la 8-(3-chloro-4-fluorobenzoyl)-3-(3,5-diméthylbenzyl)-1-oxa-3,8-diazaspiro[4,5]décan-2-one
132. la 8-(2,6-difluoro-3-méthylbenzoyl)-3-(3,5-diméthylbenzyl)-1-oxa-3,8-diazaspiro[4,5]décan-2-one
133. l'ester méthylique de l'acide 4-[3-(3-bromobenzyl)-2-oxo-1-oxa-3,8-diazaspiro[4,5]déc-8-yl]-4-oxobutanoïque
134. le 3-[8-(2-éthylbutyryl)-2-oxo-1-oxa-3,8-diazaspiro[4,5]déc-3-ylméthyl]-benzonitrile
135. le 3-[8-(3-bromobenzylsulfonyl)-2-oxo-1-oxa-3,8-diazaspiro[4,5]déc-3-ylméthyl]-benzonitrile
136. la 3-(3,4-difluorobenzyl)-8-(4-méthylbenzoyl)-1-oxa-3,8-diazaspiro[4,5]décan-2-one
137. la 3-(3-bromobenzyl)-8-(3,5-diméthoxybenzoyl)-1-oxa-3,8-diazaspiro[4,5]décan-2-one
138. la 8-(3-diméthylaminobenzoyl)-3-(2-méthoxy-5-nitrobenzyl)-1-oxa-3,8-diazaspiro[4,5]décan-2-one
139. la 8-(2,6-dichlorobenzoyl)-3-(2-fluorobenzyl)-1-oxa-3,8-diazaspiro[4,5]décan-2-one
140. la 3-(2-méthoxy-5-nitrobenzyl)-8-(2-phénoxypyridine-3-carbonyl)-1-oxa-3,8-diazaspiro[4,5]décan-2-one
141. la 8-[2-(3-méthoxyphényl)-acétyl]-3-naphtalén-2-ylméthyl-1-oxa-3,8-diazaspiro[4,5]décan-2-one
142. le 4-{2-oxo-8-[2-(2,2,2-trifluoroacétyl)-1,2,3,4-tétrahydro-isoquinoléin-7-sulfonyl]-1-oxa-3,8-diazaspiro[4,5]déc-3-ylméthyl}-benzonitrile
143. la 8-(3,5-bistrifluorométhylbenzoyl)-3-(4-trifluorométhylbenzyl)-1-oxa-3,8-diazaspiro[4,5]décan-2-one
144. la 3-(3-bromobenzyl)-8-(2-phénoxypyridine-3-carbonyl)-1-oxa-3,8-diazaspiro[4,5]décan-2-one
145. la 3-(2-méthoxy-5-nitrobenzyl)-8-[2-(3-méthoxyphényl)-acétyl]-1-oxa-3,8-diazaspiro[4,5]décan-2-one
146. la 3-(3,4-difluorobenzyl)-8-(2-phénoxypyridine-3-carbonyl)-1-oxa-3,8-diazaspiro[4,5]décan-2-one
147. le 3-[8-(4-bromo-3-méthylbenzoyl)-2-oxo-1-oxa-3,8-diazaspiro[4,5]déc-3-ylméthyl]-benzonitrile
148. la 3-(3-bromobenzyl)-8-(4-propylbenzoyl)-1-oxa-3,8-diazaspiro[4,5]décan-2-one
149. la 3-biphényl-2-ylméthyl-8-(3-chlorobenzylsulfonyl)-1-oxa-3,8-diazaspiro[4,5]décan-2-one
150. la 8-[2-(3,4-diméthoxyphényl)-acétyl]-3-(2-méthoxy-5-nitrobenzyl)-1-oxa-3,8-diazaspiro[4,5]décan-2-one
151. le 2-[8-(2-chloro-4-nitrobenzoyl)-2-oxo-1-oxa-3,8-diazaspiro[4,5]déc-3-ylméthyl]-benzonitrile
152. le 3-[2-oxo-8-(2,4,6-triméthylbenzylsulfonyl)-1-oxa-3,8-diazaspiro[4,5]déc-3-ylméthyl]-benzonitrile
153. la 3-benzyl-8-(4-fluorobenzylsulfonyl)-1-oxa-3,8-diazaspiro[4,5]décan-2-one
154. la 3-(4-fluorobenzyl)-8-(toluène-4-sulfonyl)-1-oxa-3,8-diazaspiro[4,5]décan-2-one
155. le 4-[2-oxo-8-(toluène-4-sulfonyl)-1-oxa-3,8-diazaspiro[4,5]déc-3-ylméthyl]-benzonitrile
156. la 3-(2-fluorobenzyl)-8-(toluène-4-sulfonyl)-1-oxa-3,8-diazaspiro[4,5]décan-2-one
157. la 3-(3,4-difluorobenzyl)-8-(toluène-4-sulfonyl)-1-oxa-3,8-diazaspiro[4,5]décan-2-one
158. la 3-(4-tert-butylbenzyl)-8-(toluène-4-sulfonyl)-1-oxa-3,8-diazaspiro[4,5]décan-2-one
159. la 8-(toluène-4-sulfonyl)-3-(4-trifluorométhylsulfanylbenzyl)-1-oxa-3,8-diazaspiro[4,5]décan-2-one
160. l'ester méthylique de l'acide 2-[3-(4-fluorobenzyl)-2-oxo-1-oxa-3,8-diazaspiro[4,5]décane-8-sulfonyl]-benzoïque
161. l'ester méthylique de l'acide 2-[3-(3-méthoxybenzyl)-2-oxo-1-oxa-3,8-diazaspiro[4,5]décane-8-sulfonyl]-benzoïque
162. l'ester méthylique de l'acide 2-[3-(3-bromobenzyl)-2-oxo-1-oxa-3,8-diazaspiro[4,5]décane-8-sulfonyl]-benzoïque
163. la 3-benzyl-8-(2-méthanesulfonylbenzylsulfonyl)-1-oxa-3,8-diazaspiro[4,5]décan-2-one
164. la 8-(2-méthanesulfonylbenzylsulfonyl)-3-(2-méthoxy-5-nitrobenzyl)-1-oxa-3,8-diazaspiro[4,5]décan-2-one
165. le diméthylamide de l'acide 3-(4-méthylbenzyl)-2-oxo-1-oxa-3,8-diazaspiro[4,5]décane-8-sulfonique
166. le 4-[8-(7,7-diméthyl-2-oxobicyclo[2,2,1]hept-1-ylméthanesulfonyl)-2-oxo-1-oxa-3,8-diazaspiro[4,5]déc-3-ylméthyl]-benzonitrile
167. la 3-(4-fluorobenzyl)-8-(4-méthoxybenzylsulfonyl)-1-oxa-3,8-diazaspiro[4,5]décan-2-one
168. la 3-(4-méthylbenzyl)-8-(4-trifluorométhoxybenzylsulfonyl)-1-oxa-3,8-diazaspiro[4,5]décan-2-one
169. la 8-(4-trifluorométhoxybenzylsulfonyl)-3-(4-trifluorométhylsulfanylbenzyl)-1-oxa-3,8-diazaspiro[4,5]décan-2-one
170. la 8-(propane-1-sulfonyl)-3-(4-trifluorométhylsulfanylbenzyl)-1-oxa-3,8-diazaspiro[4,5]décan-2-one
171. la 3-(2-méthoxy-5-nitrobenzyl)-8-(4-propylbenzylsulfonyl)-1-oxa-3,8-diazaspiro[4,5]décan-2-one
172. la 3-biphényl-2-ylméthyl-8-(4-propylbenzylsulfonyl)-1-oxa-3,8-diazaspiro[4,5]décan-2-one
173. la 8-(3-bromobenzylsulfonyl)-3-(2-fluorobenzyl)-1-oxa-3,8-diazaspiro[4,5]décan-2-one
174. la 8-(3-bromobenzylsulfonyl)-3-(3-bromobenzyl)-1-oxa-3,8-diazaspiro[4,5]décan-2-one
175. le 2-{8-[4-(1,1-diméthylpropyl)-benzylsulfonyl]-2-oxo-1-oxa-3,8-diazaspiro[4,5]déc-3-ylméthyl}-benzonitrile
176. la 3-benzyl-8-[4-(1,1-diméthylpropyl)-benzylsulfonyl]-1-oxa-3,8-diazaspiro[4,5]décan-2-one
177. la 8-[4-(1,1-diméthylpropyl)-benzylsulfonyl]-3-(4-iodobenzyl)-1-oxa-3,8-diazaspiro[4,5]décan-2-one
178. la 8-[4-(1,1-diméthylpropyl)-benzylsulfonyl]-3-(2-méthoxy-5-nitrobenzyl)-1-oxa-3,8-diazaspiro[4,5]décan-2-one
179. la 8-[4-(1,1-diméthylpropyl)-benzylsulfonyl]-3-(4-trifluorométhylbenzyl)-1-oxa-3,8-diazaspiro[4,5]décan-2-one
180. la 3-biphényl-2-ylméthyl-8-[4-(1,1-diméthylpropyl)-benzylsulfonyl]-1-oxa-3,8-diazaspiro[4,5]décan-2-one
181. la 8-(4,5-dichlorothiophène-2-sulfonyl)-3-(3-méthoxybenzyl)-1-oxa-3,8-diazaspiro[4,5]décan-2-one
182. la 8-(4,5-dichlorothiophène-2-sulfonyl)-3-(4-trifluorométhylbenzyl)-1-oxa-3,8-diazaspiro[4,5]décan-2-one
183. la 3-(3-bromobenzyl)-8-(4,5-dichlorothiophène-2-sulfonyl)-1-oxa-3,8-diazaspiro[4,5]décan-2-one
184. le 2-[8-(4-méthoxy-2,3,6-triméthylbenzylsulfonyl)-2-oxo-1-oxa-3,8-diazaspiro[4,5]déc-3-ylméthyl]-benzonitrile
185. la 3-(2-fluorobenzyl)-8-(4-méthoxy-2,3,6-triméthylbenzylsulfonyl)-1-oxa-3,8-diazaspiro[4,5]décan-2-one
186. la 3-(4-tert-butylbenzyl)-8-[2-(2,2,2-trifluoroacétyl)-1,2,3,4-tétrahydro-isoquinoléine-7-sulfonyl]-1-oxa-3,8-diazaspiro[4,5]décan-2-one
187. la 8-(butane-1-sulfonyl)-3-(3-méthoxybenzyl)-1-oxa-3,8-diazaspiro[4,5]décan-2-one
188. la 3-(4-méthylbenzyl)-8-(thiophène-2-sulfonyl)-1-oxa-3,8-diazaspiro[4,5]décan-2-one
189. la 8-(4-chloro-2,5-diméthylbenzylsulfonyl)-3-(4-méthylbenzyl)-1-oxa-3,8-diazaspiro[4,5]décan-2-one
190. la 3-benzyl-8-(4-chloro-2,5-diméthylbenzylsulfonyl)-1-oxa-3,8-diazaspiro[4,5]décan-2-one
191. la 8-(4-chloro-2,5-diméthylbenzylsulfonyl)-3-(4-trifluorométhylbenzyl)-1-oxa-3,8-diazaspiro[4,5]décan-2-one
192. la 3-biphényl-2-ylméthyl-8-(4-chloro-2,5-diméthylbenzylsulfonyl)-1-oxa-3,8-diazaspiro[4,5]décan-2-one
193. la 8-(4-chloro-2,5-diméthylbenzylsulfonyl)-3-(4-trifluorométhylsulfanylbenzyl)-1-oxa-3,8-diazaspiro[4,5]décan-2-one
194. la 3-(4-fluorobenzyl)-8-(2-trifluorométhylbenzylsulfonyl)-1-oxa-3,8-diazaspiro[4,5]décan-2-one
195. la 3-(3-méthoxybenzyl)-8-(2-trifluorométhylbenzylsulfonyl)-1-oxa-3,8-diazaspiro[4,5]décan-2-one
196. la 3-benzyl-8-(2-méthyl-5-nitrobenzylsulfonyl)-1-oxa-3,8-diazaspiro[4,5]décan-2-one
197. la 3-(4-tert-butylbenzyl)-8-(2-méthyl-5-nitrobenzylsulfonyl)-1-oxa-3,8-diazaspiro[4,5]décan-2-one
198. la 3-(4-tert-butylbenzyl)-8-(toluène-3-sulfonyl)-1-oxa-3,8-diazaspiro[4,5]décan-2-one
199. la 8-(furanne-2-carbonyl)-3-(4-trifluorométhylbenzyl)-1-oxa-3,8-diazaspiro[4,5]décan-2-one
200. la 3-(3-bromobenzyl)-8-(furanne-2-carbonyl)-1-oxa-3,8-diazaspiro[4,5]décan-2-one
201. le 4-[8-(naphtalène-1-carbonyl)-2-oxo-1-oxa-3,8-diazaspiro[4,5]déc-3-ylméthyl]-benzonitrile
202. la 3-(3,4-difluorobenzyl)-8-(naphtalène-1-carbonyl)-1-oxa-3,8-diazaspiro[4,5]décan-2-one
203. la 3-(3-bromobenzyl)-8-(naphtalène-1-carbonyl)-1-oxa-3,8-diazaspiro[4,5]décan-2-one
204. le 2-[8-(3-nitrobenzoyl)-2-oxo-1-oxa-3,8-diazaspiro[4,5]déc-3-ylméthyl]-benzonitrile
205. le 4-[8-(3-nitrobenzoyl)-2-oxo-1-oxa-3,8-diazaspiro[4,5]déc-3-ylméthyl]-benzonitrile
206. la 3-(3-bromobenzyl)-8-(3,5-difluorobenzoyl)-1-oxa-3,8-diazaspiro[4,5]décan-2-one
207. la 8-(2-benzyloxyacétyl)-3-(2-fluorobenzyl)-1-oxa-3,8-diazaspiro[4,5]décan-2-one
208. la 8-(2-chlorobenzoyl)-3-(2-trifluorométhylbenzyl)-1-oxa-3,8-diazaspiro[4,5]décan-2-one
209. la 8-(2-chlorobenzoyl)-3-(4-iodobenzyl)-1-oxa-3,8-diazaspiro[4,5]décan-2-one
210. la 8-(2-chlorobenzoyl)-3-(2-méthoxy-5-nitrobenzyl)-1-oxa-3,8-diazaspiro[4,5]décan-2-one
211. la 3-biphényl-2-ylméthyl-8-(2-chlorobenzoyl)-1-oxa-3,8-diazaspiro[4,5]décan-2-one
212. la 8-(2,6-dichlorobenzoyl)-3-naphtalén-2-ylméthyl-1-oxa-3,8-diazaspiro[4,5]décan-2-one
213. la 3-(4-tert-butylbenzyl)-8-(2,6-dichlorobenzoyl)-1-oxa-3,8-diazaspiro[4,5]décan-2-one
214. la 3-(3-bromobenzyl)-8-(2,6-dichlorobenzoyl)-1-oxa-3,8-diazaspiro[4,5]décan-2-one
215. la 8-(2,6-dichlorobenzoyl)-3-(4-trifluorométhylsulfanylbenzyl)-1-oxa-3,8-diazaspiro[4,5]décan-2-one
216. le 2-[8-(2-méthylbenzoyl)-2-oxo-1-oxa-3,8-diazaspiro[4,5]déc-3-ylméthyl]-benzonitrile
217. la 8-(2-méthylbenzoyl)-3-(4-méthylbenzyl)-1-oxa-3,8-diazaspiro[4,5]décan-2-one
218. la 8-(2-méthylbenzoyl)-3-(2-trifluorométhylbenzyl)-1-oxa-3,8-diazaspiro[4,5]décan-2-one
219. la 3-(2-méthoxy-5-nitrobenzyl)-8-(2-méthylbenzoyl)-1-oxa-3,8-diazaspiro[4,5]décan-2-one
220. la 3-(3,4-difluorobenzyl)-8-(2-méthylbenzoyl)-1-oxa-3,8-diazaspiro[4,5]décan-2-one
221. la 8-(2-méthylbenzoyl)-3-(4-trifluorométhylbenzyl)-1-oxa-3,8-diazaspiro[4,5]décan-2-one
222. la 8-(2-méthylbenzoyl)-3-(4-trifluorométhylsulfanylbenzyl)-1-oxa-3,8-diazaspiro[4,5]décan-2-one
223. la 8-(3-bromobenzoyl)-3-(4-fluorobenzyl)-1-oxa-3,8-diazaspiro[4,5]décan-2-one
224. la 8-(3-bromobenzoyl)-3-(2-trifluorométhylbenzyl)-1-oxa-3,8-diazaspiro[4,5]décan-2-one
225. la 8-(3-bromobenzoyl)-3-(2-fluorobenzyl)-1-oxa-3,8-diazaspiro[4,5]décan-2-one
226. la 8-(3-bromobenzoyl)-3-(3,4-difluorobenzyl)-1-oxa-3,8-diazaspiro[4,5]décan-2-one
227. la 8-(3-bromobenzoyl)-3-naphtalén-2-ylméthyl-1-oxa-3,8-diazaspiro[4,5]décan-2-one
228. la 8-(3-bromobenzoyl)-3-(4-tert-butylbenzyl)-1-oxa-3,8-diazaspiro[4,5]décan-2-one
229. la 3-benzyl-8-(3-fluorobenzoyl)-1-oxa-3,8-diazaspiro[4,5]décan-2-one
230. la 8-(3-fluorobenzoyl)-3-(2-fluorobenzyl)-1-oxa-3,8-diazaspiro[4,5]décan-2-one
231. la 3-(3,4-difluorobenzyl)-8-(3-fluorobenzoyl)-1-oxa-3,8-diazaspiro[4,5]décan-2-one
232. la 8-(3-fluorobenzoyl)-3-(4-trifluorométhylbenzyl)-1-oxa-3,8-diazaspiro[4,5]décan-2-one
233. la 3-(3-bromobenzyl)-8-(3-fluorobenzoyl)-1-oxa-3,8-diazaspiro[4,5]décan-2-one
234. la 8-(3-chlorobenzoyl)-3-(3,5-diméthylbenzyl)-1-oxa-3,8-diazaspiro[4,5]décan-2-one
235. la 3-benzyl-8-(3-chlorobenzoyl)-1-oxa-3,8-diazaspiro[4,5]décan-2-one
236. le 4-[8-(3,4-dichlorobenzoyl)-2-oxo-1-oxa-3,8-diazaspiro[4,5]déc-3-ylméthyl]-benzonitrile
237. la 3-(4-iodobenzyl)-8-(3-méthoxybenzoyl)-1-oxa-3,8-diazaspiro[4,5]décan-2-one
238. la 3-(2-fluorobenzyl)-8-(3-méthoxybenzoyl)-1-oxa-3,8-diazaspiro[4,5]décan-2-one
239. la 3-(4-tert-butylbenzyl)-8-(3-méthoxybenzoyl)-1-oxa-3,8-diazaspiro[4,5]décan-2-one
240. la 3-benzyl-8-(3-méthylbenzoyl)-1-oxa-3,8-diazaspiro[4,5]décan-2-one
241. la 3-(2-méthoxy-5-nitrobenzyl)-8-(3-méthylbenzoyl)-1-oxa-3,8-diazaspiro[4,5]décan-2-one
242. la 3-(4-tert-butylbenzyl)-8-(3-méthylbenzoyl)-1-oxa-3,8-diazaspiro[4,5]décan-2-one
243. la 8-(3-méthylbenzoyl)-3-(4-trifluorométhylbenzyl)-1-oxa-3,8-diazaspiro[4,5]décan-2-one
244. la 3-(3,4-difluorobenzyl)-8-(2-phénylbutyryl)-1-oxa-3,8-diazaspiro[4,5]décan-2-one
245. la 3-benzyl-8-[3-(2-chlorophényl)-5-méthylisoxazole-4-carbonyl]-1-oxa-3,8-diazaspiro[4,5]décan-2-one
246. le 4-[8-(2,3-dichlorobenzoyl)-2-oxo-1-oxa-3,8-diazaspiro[4,5]déc-3-ylméthyl]-benzonitrile
247. la 8-[2-(4-chlorophényl)-acétyl]-3-(3,4-difluorobenzyl)-1-oxa-3,8-diazaspiro[4,5]décan-2-one
248. la 3-biphényl-2-ylméthyl-8-[2-(4-chlorophényl)-acétyl]-1-oxa-3,8-diazaspiro[4,5]décan-2-one
249. la 8-[2-(4-chlorophényl)-acétyl]-3-(4-trifluorométhylsulfanylbenzyl)-1-oxa-3,8-diazaspiro[4,5]décan-2-one
250. la 3-(4-tert-butylbenzyl)-8-[3-(2-chlorophényl)-acryloyl]-1-oxa-3,8-diazaspiro[4,5]décan-2-one
251. la 3-biphényl-2-ylméthyl-8-[3-(2-chlorophényl)-acryloyl]-1-oxa-3,8-diazaspiro[4,5]décan-2-one
252. la 3-(3-bromobenzyl)-8-[3-(2-chlorophényl)-acryloyl]-1-oxa-3,8-diazaspiro[4,5]décan-2-one
253. le 4-[8-(2,3-difluorobenzoyl)-2-oxo-1-oxa-3,8-diazaspiro[4,5]déc-3-ylméthyl]-benzonitrile
254. la 8-(2,3-difluorobenzoyl)-3-(2-trifluorométhylbenzyl)-1-oxa-3,8-diazaspiro[4,5]décan-2-one
255. la 8-(2,3-difluorobenzoyl)-3-(4-iodobenzyl)-1-oxa-3,8-diazaspiro[4,5]décan-2-one
256. le 2-[2-oxo-8-(2-propylpentanoyl)-1-oxa-3,8-diazaspiro[4,5]déc-3-ylméthyl]-benzonitrile
257. la 3-(3,4-difluorobenzyl)-8-(2-propylpentanoyl)-1-oxa-3,8-diazaspiro[4,5]décan-2-one
258. la 8-(2-chloro-4-nitrobenzoyl)-3I-(4-méthylbenzyl)-1-oxa-3,8-diazaspiro[4,5]décan-2-one
259. la 8-(2-chloro-4-nitrobenzoyl)-3I-(2-trifluorométhylbenzyl)-1-oxa-3,8-diazaspiro[4,5]décan-2-one
260. la 3-(4-tert-butylbenzyl)-8-(2-chloro-4-nitrobenzoyl)-1-oxa-3,8-diazaspiro[4,5]décan-2-one
261. la 8-(2-chloro-4-nitrobenzoyl)-3I-(4-trifluorométhylbenzyl)-1-oxa-3,8-diazaspiro[4,5]décan-2-one
262. la 3-biphényl-2-ylméthyl-8-(2-chloro-4-nitrobenzoyl)-1-oxa-3,8-diazaspiro[4,5]décan-2-one
263. la 3-(3-bromobenzyl)-8-(2-chloro-4-nitrobenzoyl)-1-oxa-3,8-diazaspiro[4,5]décan-2-one
264. la 8-(2-chloro-4-nitrobenzoyl)-3I-(4-trifluorométhylsulfanylbenzyl)-1-oxa-3,8-diazaspiro[4,5]décan-2-one
265. la 8-(2-chloropyridine-3-carbonyl)-3-(4-fluorobenzyl)-1-oxa-3,8-diazaspiro[4,5]décan-2-one
266. le 2-[8-(2-chloropyridine-3-carbonyl)-2-oxo-1-oxa-3,8-diazaspiro[4,5]déc-3-ylméthyl]-benzonitrile
267. la 8-(2-chloropyridine-3-carbonyl)-3-(4-méthylbenzyl)-1-oxa-3,8-diazaspiro[4,5]décan-2-one
268. la 8-(2-chloropyridine-3-carbonyl)-3-(4-trifluorométhylbenzyl)-1-oxa-3,8-diazaspiro[4,5]décan-2-one
269. la 3-(3-méthoxybenzyl)-8-(2-méthylsulfanylpyridine-3-carbonyl)-1-oxa-3,8-diazaspiro[4,5]décan-2-one
270. la 3-(4-méthylbenzyl)-8-(2-méthylsulfanylpyridine-3-carbonyl)-1-oxa-3,8-diazaspiro[4,5]décan-2-one
271. la 3-(4-iodobenzyl)-8-(2-méthylsulfanylpyridine-3-carbonyl)-1-oxa-3,8-diazaspiro[4,5]décan-2-one
272. la 3-(3,4-difluorobenzyl)-8-(2-méthylsulfanylpyridine-3-carbonyl)-1-oxa-3,8-diazaspiro[4,5]décan-2-one
273. la 3-(4-tert-butylbenzyl)-8-(2-méthylsulfanylpyridine-3-carbonyl)-1-oxa-3,8-diazaspiro[4,5]décan-2-one
274. la 8-(2-méthylsulfanylpyridine-3-carbonyl)-3-(4-trifluorométhylbenzyl)-1-oxa-3,8-diazaspiro[4,5]décan-2-one
275. la 8-(2-éthylsulfanylpyridine-3-carbonyl)-3-(4-fluorobenzyl)-1-oxa-3,8-diazaspiro[4,5]décan-2-one
276. le 3-[8-(2-éthylsulfanylpyridine-3-carbonyl)-2-oxo-1-oxa-3,8-diazaspiro[4,5]déc-3-ylméthyl]-benzonitrile
277. le 2-[8-(2-éthylsulfanylpyridine-3-carbonyl)-2-oxo-1-oxa-3,8-diazaspiro[4,5]déc-3-ylméthyl]-benzonitrile
278. la 8-(2-éthylsulfanylpyridine-3-carbonyl)-3-(4-méthylbenzyl)-1-oxa-3,8-diazaspiro[4,5]décan-2-one
279. la 3-benzyl-8-(2-éthylsulfanylpyridine-3-carbonyl)-1-oxa-3,8-diazaspiro[4,5]décan-2-one
280. la 8-(2-éthylsulfanylpyridine-3-carbonyl)-3-(2-fluorobenzyl)-1-oxa-3,8-diazaspiro[4,5]décan-2-one
281. le 4-[8-(6-chloropyridine-3-carbonyl)-2-oxo-1-oxa-3,8-diazaspiro[4,5]déc-3-ylméthyl]-benzonitrile
282. la 8-(6-chloropyridine-3-carbonyl)-3-(2-trifluorométhylbenzyl)-1-oxa-3,8-diazaspiro[4,5]décan-2-one
283. la 8-(6-chloropyridine-3-carbonyl)-3-(4-iodobenzyl)-1-oxa-3,8-diazaspiro[4,5]décan-2-one
284. la 3-(4-tert-butylbenzyl)-8-(6-chloropyridine-3-carbonyl)-1-oxa-3,8-diazaspiro[4,5]décan-2-one
285. la 3-biphényl-2-ylméthyl-8-(6-chloropyridine-3-carbonyl)-1-oxa-3,8-diazaspiro[4,5]décan-2-one
286. la 3-(3-méthoxybenzyl)-8-(4-trifluorométhoxybenzoyl)-1-oxa-3,8-diazaspiro[4,5]décan-2-one
287. la 3-(3,4-difluorobenzyl)-8-(4-trifluorométhoxybenzoyl)-1-oxa-3,8-diazaspiro[4,5]décan-2-one
288. la 8-[3-(2-chloro-6-fluorophényl)-5-méthylisoxazole-4-carbonyl]-3-(4-fluorobenzyl)-1-oxa-3,8-diazaspiro[4,5]décan-2-one
289. la 8-[3-(2-chloro-6-fluorophényl)-5-méthylisoxazole-4-carbonyl]-3-(3-méthoxybenzyl)-1-oxa-3,8-diazaspiro[4,5]décan-2-one
290. la 8-[3-(2-chloro-6-fluorophényl)-5-méthylisoxazole-4-carbonyl]-3-(4-trifluorométhylbenzyl)-1-oxa-3,8-diazaspiro[4,5]décan-2-one
291. la 8-[3-(2-chloro-6-fluorophényl)-5-méthylisoxazole-4-carbonyl]-3-(4-trifluorométhylsulfanylbenzyl)-1-oxa-3,8-diazaspiro[4,5]décan-2-one
292. la 3-(4-méthylbenzyl)-8-(3-trifluorométhoxybenzoyl)-1-oxa-3,8-diazaspiro[4,5]décan-2-one
293. la 3-(4-iodobenzyl)-8-(3-trifluorométhoxybenzoyl)-1-oxa-3,8-diazaspiro[4,5]décan-2-one
294. la 3-(3,4-difluorobenzyl)-8-(3-trifluorométhoxybenzoyl)-1-oxa-3,8-diazaspiro[4,5]décan-2-one
295. la 3-naphtalén-2-ylméthyl-8-(3-trifluorométhoxybenzoyl)-1-oxa-3,8-diazaspiro[4,5]décan-2-one
296. le 4-[8-(isoxazole-5-carbonyl)-2-oxo-1-oxa-3,8-diazaspiro[4,5]déc-3-ylméthyl]-benzonitrile
297. la 8-(isoxazole-5-carbonyl)-3-(2-trifluorométhylbenzyl)-1-oxa-3,8-diazaspiro[4,5]décan-2-one
298. la 8-(isoxazole-5-carbonyl)-3-(2-méthoxy-5-nitrobenzyl)-1-oxa-3,8-diazaspiro[4,5]décan-2-one
299. la 3-(3-bromobenzyl)-8-(isoxazole-5-carbonyl)-1-oxa-3,8-diazaspiro[4,5]décan-2-one
300. le 2-[8-(2-chloro-6-fluorobenzoyl)-2-oxo-1-oxa-3,8-diazaspiro[4,5]déc-3-ylméthyl]-benzonitrile
301. le 4-[8-(2-chloro-6-fluorobenzoyl)-2-oxo-1-oxa-3,8-diazaspiro[4,5]déc-3-ylméthyl]-benzonitrile
302. la 8-(2-chloro-6-fluorobenzoyl)-3-(4-méthylbenzyl)-1-oxa-3,8-diazaspiro[4,5]décan-2-one
303. la 3-biphényl-2-ylméthyl-8-(2-chloro-6-fluorobenzoyl)-1-oxa-3,8-diazaspiro[4,5]décan-2-one
304. la 8-(2,5-diméthylfuranne-3-carbonyl)-3-(3-méthoxybenzyl)-1-oxa-3,8-diazaspiro[4,5]décan-2-one
305. le 4-[8-(2,5-diméthylfuranne-3-carbonyl)-2-oxo-1-oxa-3,8-diazaspiro[4,5]déc-3-ylméthyl]-benzonitrile
306. le 4-[8-(4-bromo-3-méthylbenzoyl)-2-oxo-1-oxa-3,8-diazaspiro[4,5]déc-3-ylméthyl]-benzonitrile
307. la 8-(4-bromo-3-méthylbenzoyl)-3-(4-méthylbenzyl)-1-oxa-3,8-diazaspiro[4,5]décan-2-one
308. la 3-benzyl-8-(4-bromo-3-méthylbenzoyl)-1-oxa-3,8-diazaspiro[4,5]décan-2-one
309. la 8-(4-bromo-3-méthylbenzoyl)-3-(2-trifluorométhylbenzyl)-1-oxa-3,8-diazaspiro[4,5]décan-2-one
310. la 8-(4-bromo-3-méthylbenzoyl)-3-(3,4-difluorobenzyl)-1-oxa-3,8-diazaspiro[4,5]décan-2-one
311. la 8-(4-bromo-3-méthylbenzoyl)-3-(4-tert-butylbenzyl)-1-oxa-3,8-diazaspiro[4,5]décan-2-one
312. la 3-biphényl-2-ylméthyl-8-(4-bromo-3-méthylbenzoyl)-1-oxa-3,8-diazaspiro[4,5]décan-2-one
313. la 8-(4-bromo-3-méthylbenzoyl)-3-(4-trifluorométhylsulfanylbenzyl)-1-oxa-3,8-diazaspiro[4,5]décan-2-one
314. la 8-(2,6-difluoro-3-méthylbenzoyl)-3-(3-méthoxybenzyl)-1-oxa-3,8-diazaspiro[4,5]décan-2-one
315. le 2-[8-(2,6-difluoro-3-méthylbenzoyl)-2-oxo-1-oxa-3,8-diazaspiro[4,5]déc-3-ylméthyl]-benzonitrile
316. le 4-[8-(2,6-difluoro-3-méthylbenzoyl)-2-oxo-1-oxa-3,8-diazaspiro[4,5]déc-3-ylméthyl]-benzonitrile
317. la 8-(2,6-difluoro-3-méthylbenzoyl)-3-(2-trifluorométhylbenzyl)-1-oxa-3,8-diazaspiro[4,5]décan-2-one
318. la 8-(2,6-difluoro-3-méthylbenzoyl)-3-(2-fluorobenzyl)-1-oxa-3,8-diazaspiro[4,5]décan-2-one
319. la 8-(2-chloro-6-fluoro-3-méthylbenzoyl)-3-(4-fluorobenzyl)-1-oxa-3,8-diazaspiro[4,5]décan-2-one
320. la 8-(2-chloro-6-fluoro-3-méthylbenzoyl)-3-(3-méthoxybenzyl)-1-oxa-3,8-diazaspiro[4,5]décan-2-one
321. la 8-(2-chloro-6-fluoro-3-méthylbenzoyl)-3-(4-trifluorométhylsulfanylbenzyl)-1-oxa-3,8-diazaspiro[4,5]décan-2-one
322. la 3-biphényl-2-ylméthyl-8-(6-chloro-2-fluoro-3-méthylbenzoyl)-1-oxa-3,8-diazaspiro[4,5]décan-2-one
323. la 3-benzyl-8-(3-difluorométhylsulfanylbenzoyl)-1-oxa-3,8-diazaspiro[4,5]décan-2-one
324. la 8-(3-difluorométhylsulfanylbenzoyl)-3-naphtalén-2-ylméthyl-1-oxa-3,8-diazaspiro[4,5]décan-2-one
325. la 8-(3-difluorométhylsulfanylbenzoyl)-3-(4-trifluorométhylbenzyl)-1-oxa-3,8-diazaspiro[4,5]décan-2-one
326. la 3-(3-bromobenzyl)-8-(3-difluorométhylsulfanylbenzoyl)-1-oxa-3,8-diazaspiro[4,5]décan-2-one
327. le 3-[8-(3-chloro-2-fluorobenzoyl)-2-oxo-1-oxa-3,8-diazaspiro[4,5]déc-3-ylméthyl]-benzonitrile
328. la 8-(3-chloro-2-fluorobenzoyl)-3-(4-iodobenzyl)-1-oxa-3,8-diazaspiro[4,5]décan-2-one
329. la 8-(3-chloro-2-fluorobenzoyl)-3-naphtalén-2-ylméthyl-1-oxa-3,8-diazaspiro[4,5]décan-2-one
330. la 3-(3-méthoxybenzyl)-8-(4-trifluorométhylsulfanylbenzoyl)-1-oxa-3,8-diazaspiro[4,5]décan-2-one
331. le 2-[2-oxo-8-(4-trifluorométhylsulfanylbenzoyl)-1-oxa-3,8-diazaspiro[4,5]déc-3-ylméthyl]-benzonitrile
332. le 4-[2-oxo-8-(4-trifluorométhylsulfanylbenzoyl)-1-oxa-3,8-diazaspiro[4,5]déc-3-ylméthyl]-benzonitrile
333. la 3-(4-iodobenzyl)-8-(4-trifluorométhylsulfanylbenzoyl)-1-oxa-3,8-diazaspiro[4,5]décan-2-one
334. la 3-naphtalén-2-ylméthyl-8-(4-trifluorométhylsulfanylbenzoyl)-1-oxa-3,8-diazaspiro[4,5]décan-2-one
335. la 3-(4-trifluorométhylbenzyl)-8-(4-trifluorométhylsulfanylbenzoyl)-1-oxa-3,8-diazaspiro[4,5]décan-2-one
336. la 8-(2-chloro-5-trifluorométhylbenzoyl)-3-(2-trifluorométhylbenzyl)-1-oxa-3,8-diazaspiro[4,5]décan-2-one
337. la 8-(2-chloro-5-trifluorométhylbenzoyl)-3-(2-fluorobenzyl)-1-oxa-3,8-diazaspiro[4,5]décan-2-one
338. la 8-(2-chloro-5-trifluorométhylbenzoyl)-3-(3,4-difluorobenzyl)-1-oxa-3,8-diazaspiro[4,5]décan-2-one
339. la 8-(2,3-difluoro-4-méthylbenzoyl)-3-(3-méthoxybenzyl)-1-oxa-3,8-diazaspiro[4,5]décan-2-one
340. le 4-[8-(2,3-difluoro-4-méthylbenzoyl)-2-oxo-1-oxa-3,8-diazaspiro[4,5]déc-3-ylméthyl]-benzonitrile
341. la 3-benzyl-8-(2,3-difluoro-4-méthylbenzoyl)-1-oxa-3,8-diazaspiro[4,5]décan-2-one
342. la 8-(2,3-difluoro-4-méthylbenzoyl)-3-(4-iodo-benzyl)-1-oxa-3,8-diazaspiro[4,5]décan-2-one
343. la 3-(3,4-difluorobenzyl)-8-(2,3-difluoro-4-méthylbenzoyl)-1-oxa-3,8-diazaspiro[4,5]décan-2-one
344. la 8-(2,3-difluoro-4-méthylbenzoyl)-3-naphtalén-2-ylméthyl-1-oxa-3,8-diazaspiro[4,5]décan-2-one
345. la 3-biphényl-2-ylméthyl-8-(2,3-difluoro-4-méthylbenzoyl)-1-oxa-3,8-diazaspiro[4,5]décan-2-one
346. la 3-(3-bromobenzyl)-8-(2,3-difluoro-4-méthylbenzoyl)-1-oxa-3,8-diazaspiro[4,5]décan-2-one
347. la 3-benzyl-8-[2-(2-méthoxyéthoxy)-acétyl]-1-oxa-3,8-diazaspiro[4,5]décan-2-one
348. la 3-(2-fluorobenzyl)-8-[2-(2-méthoxyéthoxy)-acétyl]-1-oxa-3,8-diazaspiro[4,5]décan-2-one
349. la 3-(3,4-difluorobenzyl)-8-[2-(2-méthoxyéthoxy)-acétyl]-1-oxa-3,8-diazaspiro[4,5]décan-2-one
350. la 3-(4-tert-butylbenzyl)-8-[2-(2-méthoxyéthoxy)-acétyl]-1-oxa-3,8-diazaspiro[4,5]décan-2-one
351. la 8-[2-(2-méthoxyéthoxy)-acétyl]-3-(4-trifluorométhylsulfanylbenzyl)-1-oxa-3,8-diazaspiro[4,5]décan-2-one
352. la 8-(1-acétylpipéridine-4-carbonyl)-3-(3,4-difluorobenzyl)-1-oxa-3,8-diazaspiro[4,5]décan-2-one
353. le 4-{8-[2-(3-chlorophénoxy)-acétyl]-2-oxo-1-oxa-3,8-diazaspiro[4,5]déc-3-ylméthyl}-benzonitrile
354. la 8-[2-(3-chlorophénoxy)-acétyl]-3-(3,4-difluorobenzyl)-1-oxa-3,8-diazaspiro[4,5]décan-2-one
355. la 3-(3-bromobenzyl)-8-[2-(3-chlorophénoxy)-acétyl]-1-oxa-3,8-diazaspiro[4,5]décan-2-one
356. la 8-[2-(3-chlorophénoxy)-acétyl]-3-(4-trifluorométhylsulfanylbenzyl)-1-oxa-3,8-diazaspiro[4,5]décan-2-one
357. le 4-[3-(3-méthoxybenzyl)-2-oxo-1-oxa-3,8-diazaspiro[4,5]décane-8-carbonyl]-benzylsulfonamide
358. le N-{4-[3-(3,4-difluorobenzyl)-2-oxo-1-oxa-3,8-diazaspiro[4,5]décane-8-carbonyl]-phényl}-acétamide
359. la 8-(3-diméthylaminobenzoyl)-3-(3,5-diméthylbenzyl)-1-oxa-3,8-diazaspiro[4,5]décan-2-one
360. la 3-(4-tert-butylbenzyl)-8-(3-diméthylaminobenzoyl)-1-oxa-3,8-diazaspiro[4,5]décan-2-one
361. la 3-(3-bromobenzyl)-8-(3-diméthylaminobenzoyl)-1-oxa-3,8-diazaspiro[4,5]décan-2-one
362. la 3-(4-méthylbenzyl)-8-(4-phénoxybutyryl)-1-oxa-3,8-diazaspiro[4,5]décan-2-one
363. la 8-(4-phénoxybutyryl)-3-(2-trifluorométhylbenzyl)-1-oxa-3,8-diazaspiro[4,5]décan-2-one
364. la 3-(2-fluorobenzyl)-8-(4-phénoxybutyryl)-1-oxa-3,8-diazaspiro[4,5]décan-2-one
365. la 8-(2,3-diméthylbenzoyl)-3-(4-fluorobenzyl)-1-oxa-3,8-diazaspiro[4,5]décan-2-one
366. le 3-[8-(2,3-diméthylbenzoyl)-2-oxo-1-oxa-3,8-diazaspiro[4,5]déc-3-ylméthyl]-benzonitrile
367. la 8-(2,3-diméthylbenzoyl)-3-(3,5-diméthylbenzyl)-1-oxa-3,8-diazaspiro[4,5]décan-2-one
368. la 8-(2,3-diméthylbenzoyl)-3-(4-méthylbenzyl)-1-oxa-3,8-diazaspiro[4,5]décan-2-one
369. la 8-(2,3-diméthylbenzoyl)-3-(2-trifluorométhylbenzyl)-1-oxa-3,8-diazaspiro[4,5]décan-2-one
370. la 8-(2,3-diméthylbenzoyl)-3-(2-méthoxy-5-nitrobenzyl)-1-oxa-3,8-diazaspiro[4,5]décan-2-one
371. la 8-(2,3-diméthylbenzoyl)-3-(4-trifluorométhylbenzyl)-1-oxa-3,8-diazaspiro[4,5]décan-2-one
372. la 3-biphényl-2-ylméthyl-8-(2,3-diméthylbenzoyl)-1-oxa-3,8-diazaspiro[4,5]décan-2-one
373. le 4-[8-(5-méthyl-2-phényl-2H-[1,2,3]triazole-4-carbonyl)-2-oxo-1-oxa-3,8-diazaspiro[4,5]déc-3-ylméthyl]-benzonitrile
374. la 3-benzyl-8-(5-méthyl-2-phényl-2H-[1,2,3]triazole-4-carbonyl)-1-oxa-3,8-diazaspiro[4,5]décan-2-one
375. la 3-(3,4-difluorobenzyl)-8-(5-méthyl-2-phényl-2H-[1,2,3]triazole-4-carbonyl)-1-oxa-3,8-diazaspiro[4,5]décan-2-one
376. la 8-(5-méthyl-2-phényl-2H-[1,2,3]triazole-4-carbonyl)-3-naphtalén-2-ylméthyl-1-oxa-3,8-diazaspiro[4,5]décan-2-one
377. la 3-biphényl-2-ylméthyl-8-(5-méthyl-2-phényl-2H-[1,2,3]triazole-4-carbonyl)-1-oxa-3,8-diazaspiro[4,5]décan-2-one
378. la 3-(4-tert-butylbenzyl)-8-[3-(2,6-dichlorophényl)-5-méthylisoxazole-4-carbonyl]-1-oxa-3,8-diazaspiro[4,5]décan-2-one
379. le 3-[2-oxo-8-(2-phénoxypyridine-3-carbonyl)-1-oxa-3,8-diazaspiro[4,5]déc-3-ylméthyl]-benzonitrile
380. le 2-[2-oxo-8-(2-phénoxypyridine-3-carbonyl)-1-oxa-3,8-diazaspiro[4,5]déc-3-ylméthyl]-benzonitrile
381. la 3-(4-fluorobenzyl)-8-(pyridine-2-carbonyl)-1-oxa-3,8-diazaspiro[4,5]décan-2-one
382. la 3-(3-méthoxybenzyl)-8-(pyridine-2-carbonyl)-1-oxa-3,8-diazaspiro[4,5]décan-2-one
383. la 3-(3,5-diméthylbenzyl)-8-(pyridine-2-carbonyl)-1-oxa-3,8-diazaspiro[4,5]décan-2-one
384. le 2-[2-oxo-8-(pyridine-2-carbonyl)-1-oxa-3,8-diazaspiro[4,5]déc-3-ylméthyl]-benzonitrile
385. la 3-(2-fluorobenzyl)-8-(pyridine-2-carbonyl)-1-oxa-3,8-diazaspiro[4,5]décan-2-one
386. la 3-(3,4-difluorobenzyl)-8-(pyridine-2-carbonyl)-1-oxa-3,8-diazaspiro[4,5]décan-2-one
387. la 3-biphényl-2-ylméthyl-8-(pyridine-2-carbonyl)-1-oxa-3,8-diazaspiro[4,5]décan-2-one
388. le 4-[8-(3-chlorothiophène-2-carbonyl)-2-oxo-1-oxa-3,8-diazaspiro[4,5]déc-3-ylméthyl]-benzonitrile
389. la 8-(3-chlorothiophène-2-carbonyl)-3-(3,4-difluorobenzyl)-1-oxa-3,8-diazaspiro[4,5]décan-2-one
390. la 3-biphényl-2-ylméthyl-8-(3-chlorothiophène-2-carbonyl)-1-oxa-3,8-diazaspiro[4,5]décan-2-one
391. la 8-[1-(4-chlorophényl)-5-trifluorométhyl-1H-pyrazole-4-carbonyl]-3-(2-fluorobenzyl)-1-oxa-3,8-diazaspiro[4,5]décan-2-one
392. la 8-[1-(4-chlorophényl)-5-trifluorométhyl-1H-pyrazole-4-carbonyl]-3-(3,4-difluorobenzyl)-1-oxa-3,8-diazaspiro[4,5]décan-2-one
393. la 8-[1-(4-chlorophényl)-5-trifluorométhyl-1H-pyrazole-4-carbonyl]-3-(4-trifluorométhylsulfanylbenzyl)-1-oxa-3,8-diazaspiro[4,5]décan-2-one
394. la 3-(4-tert-butylbenzyl)-8-(5-tert-butyl-2-méthyl-2H-pyrazole-3-carbonyl)-1-oxa-3,8-diazaspiro[4,5]décan-2-one
395. le 3-[8-(5-méthylisoxazole-3-carbonyl)-2-oxo-1-oxa-3,8-diazaspiro[4,5]déc-3-ylméthyl]-benzonitrile
396. la 3-(3,5-diméthylbenzyl)-8-(5-méthylisoxazole-3-carbonyl)-1-oxa-3,8-diazaspiro[4,5]décan-2-one
397. le 2-[8-(5-méthylisoxazole-3-carbonyl)-2-oxo-1-oxa-3,8-diazaspiro[4,5]déc-3-ylméthyl]-benzonitrile
398. le 4-[8-(5-méthylisoxazole-3-carbonyl)-2-oxo-1-oxa-3,8-diazaspiro[4,5]déc-3-ylméthyl]-benzonitrile
399. la 3-benzyl-8-(5-méthylisoxazole-3-carbonyl)-1-oxa-3,8-diazaspiro[4,5]décan-2-one
400. la 3-(3,4-difluorobenzyl)-8-(5-méthylisoxazole-3-carbonyl)-1-oxa-3,8-diazaspiro[4,5]décan-2-one
401. la 3-biphényl-2-ylméthyl-8-(5-méthylisoxazole-3-carbonyl)-1-oxa-3,8-diazaspiro[4,5]décan-2-one
402. la 8-(4-méthyl-[1,2,3]thiadiazole-5-carbonyl)-3-(2-trifluorométhylbenzyl)-1-oxa-3,8-diazaspiro[4,5]décan-2-one
403. la 3-(4-tert-butylbenzyl)-8-(4-méthyl-[1,2,3]thiadiazole-5-carbonyl)-1-oxa-3,8-diazaspiro[4,5]décan-2-one
404. la 8-(4-méthyl-[1,2,3]thiadiazole-5-carbonyl)-3-(4-trifluorométhylsulfanylbenzyl)-1-oxa-3,8-diazaspiro[4,5]décan-2-one
405. le 4-[2-oxo-8-(1-phényl-5-propyl-1H-pyrazole-4-carbonyl)-1-oxa-3,8-diazaspiro[4,5]déc-3-ylméthyl]-benzonitrile
406. la 3-(3,4-difluorobenzyl)-8-(1-phényl-5-propyl-1H-pyrazole-4-carbonyl)-1-oxa-3,8-diazaspiro[4,5]décan-2-one
407. la 3-naphtalén-2-ylméthyl-8-(1-phényl-5-propyl-1H-pyrazole-4-carbonyl)-1-oxa-3,8-diazaspiro[4,5]décan-2-one
408. la 8-(1-phényl-5-propyl-1H-pyrazole-4-carbonyl)-3-(4-trifluorométhylsulfanylbenzyl)-1-oxa-3,8-diazaspiro[4,5]décan-2-one
409. le 3-[8-(2-chloropyridine-4-carbonyl)-2-oxo-1-oxa-3,8-diazaspiro[4,5]déc-3-ylméthyl]-benzonitrile
410. la 8-(2-chloropyridine-4-carbonyl)-3-(3-méthoxybenzyl)-1-oxa-3,8-diazaspiro[4,5]décan-2-one
411. la 8-(5-tert-butyl-2-méthylfuranne-3-carbonyl)-3-(4-fluorobenzyl)-1-oxa-3,8-diazaspiro[4,5]décan-2-one
412. le 3-[8-(5-tert-butyl-2-méthylfuranne-3-carbonyl)-2-oxo-1-oxa-3,8-diazaspiro[4,5]déc-3-ylméthyl]-benzonitrile
413. le 2-[8-(5-tert-butyl-2-méthylfuranne-3-carbonyl)-2-oxo-1-oxa-3,8-diazaspiro[4,5]déc-3-ylméthyl]-benzonitrile
414. le 4-[8-(5-tert-butyl-2-méthylfuranne-3-carbonyl)-2-oxo-1-oxa-3,8-diazaspiro[4,5]déc-3-ylméthyl]-benzonitrile
415. la 8-(5-tert-butyl-2-méthylfuranne-3-carbonyl)-3-(2-trifluorométhylbenzyl)-1-oxa-3,8-diazaspiro[4,5]décan-2-one
416. la 8-(5-tert-butyl-2-méthylfuranne-3-carbonyl)-3-naphtalén-2-ylméthyl-1-oxa-3,8-diazaspiro[4,5]décan-2-one
417. la 3-biphényl-2-ylméthyl-8-(5-tert-butyl-2-méthylfuranne-3-carbonyl)-1-oxa-3,8-diazaspiro[4,5]décan-2-one
418. la 3-(3-bromobenzyl)-8-(5-tert-butyl-2-méthylfuranne-3-carbonyl)-1-oxa-3,8-diazaspiro[4,5]décan-2-one
419. la 8-(benzo[1,2,5]oxadiazole-5-carbonyl)-3-(3,4-difluorobenzyl)-1-oxa-3,8-diazaspiro[4,5]décan-2-one
420. la 8-(benzo[1,2,5]oxadiazole-5-carbonyl)-3-(4-trifluorométhylbenzyl)-1-oxa-3,8-diazaspiro[4,5]décan-2-one
421. le 3-[8-(2-méthyl-6-trifluorométhylpyridine-3-carbonyl)-2-oxo-1-oxa-3,8-diazaspiro[4,5]déc-3-ylméthyl]-benzonitrile
422. le 2-[8-(2-méthyl-6-trifluorométhylpyridine-3-carbonyl)-2-oxo-1-oxa-3,8-diazaspiro[4,5]déc-3-ylméthyl]-benzonitrile
423. le 4-[8-(2-méthyl-6-trifluorométhylpyridine-3-carbonyl)-2-oxo-1-oxa-3,8-diazaspiro[4,5]déc-3-ylméthyl]-benzonitrile
424. la 8-(2-méthyl-6-trifluorométhylpyridine-3-carbonyl)-3-(2-trifluorométhylbenzyl)-1-oxa-3,8-diazaspiro[4,5]décan-2-one
425. la 3-(3,4-difluorobenzyl)-8-(2-méthyl-6-trifluorométhylpyridine-3-carbonyl)-1-oxa-3,8-diazaspiro[4,5]décan-2-one
426. la 3-(3-bromobenzyl)-8-(2-méthyl-6-trifluorométhylpyridine-3-carbonyl)-1-oxa-3,8-diazaspiro[4,5]décan-2-one
427. la 8-(6-chloro-2H-chromène-3-carbonyl)-3-(3,4-difluorobenzyl)-1-oxa-3,8-diazaspiro[4,5]décan-2-one
428. la 8-(2-chloropyridine-4-carbonyl)-3-(2-fluorobenzyl)-1-oxa-3,8-diazaspiro[4,5]décan-2-one
429. la 8-(2-chloropyridine-4-carbonyl)-3-naphtalén-2-ylméthyl-1-oxa-3,8-diazaspiro[4,5]décan-2-one
430. la 8-acétyl-3-(2-fluorobenzyl)-1-oxa-3,8-diazaspiro[4,5]décan-2-one
431. la 8-acétyl-3-(3-bromobenzyl)-1-oxa-3,8-diazaspiro[4,5]décan-2-one
et
432. la 3-(2-méthoxy-5-nitrobenzyl)-8-(3-phénoxypropyl)-1-oxa-3,8-diazaspiro[4,5]décan-2-one ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

17. Procédé pour la préparation de composés substitués de 1-oxa-3,8-diazaspiro[4,5]-décan-2-one de formule générale I selon l'une ou plusieurs des revendications 1 à 16, **caractérisé en ce qu'**on transforme au moins un composé de formule générale II, 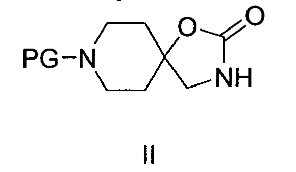 où PG représente un groupe de protection, de préférence un groupe tert-butyloxycarbonyle ou benzyloxycarbonyle, dans un milieu réactionnel, en présence d'au moins une base, de préférence en présence d'au moins un sel d'hydrure de métal, de manière particulièrement préférée en présence d'hydrure de potassium et/ou de sodium avec au moins un composé de formule générale R¹-(CH₂)ₙ-X, où R¹ et n présentent la signification selon l'une ou plusieurs des revendications 1 à 16 et X représente un groupe partant, de préférence un atome d'halogène, de manière particulièrement préférée un atome de chlore ou de brome, en au moins un composé de formule générale III, 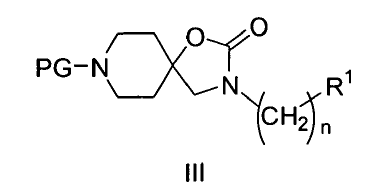 où
R¹, PG et n présentent la signification susmentionnée et ce composé est le cas échéant purifié et/ou isolé,
et on transforme au moins un composé de formule générale III par dissociation du groupe de protection, de préférence du groupe tert-butoxycarbonyle dans un milieu réactionnel, de préférence en présence d'au moins un acide ou en présence d'au moins une base ou par dissociation du groupe benzyloxycarbonyle dans un milieu réactionnel, de préférence en présence d'hydrogène et d'un catalyseur, de préférence du palladium sur carbone, en au moins un composé de formule générale IV, le cas échéant sous forme d'un sel correspondant, de préférence sous forme d'un chlorhydrate correspondant, 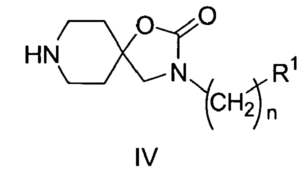 où
R¹ et n présentent la signification susmentionnée et ce composé est le cas échéant purifié et/ou isolé,
et on transforme au moins un composé de formule générale IV dans un milieu réactionnel avec au moins un isothiocyanate de formule générale R³-N=C=S, où R³ a la signification selon l'une ou plusieurs des revendications 1 à 16, le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, en au moins un composé de formule générale I, où R¹ et n ont la signification susmentionnée et R² représente -C(=S)-NH-R³, où R³ a la signification susmentionnée, et ce composé est le cas échéant purifié et/ou isolé ;
ou on transforme au moins un composé de formule générale IV dans un milieu réactionnel avec au moins un isocyanate de formule générale R⁴-N=C=O, où R⁴ a la signification selon l'une ou plusieurs des revendications 1 à 16, le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, en au moins un composé de formule générale I, où R¹ et n ont la signification susmentionnée et R² représente -C(=O)-NH-R⁴, où R⁴ a la signification susmentionnée, et ce composé est le cas échéant purifié et/ou isolé ;
ou on transforme au moins un composé de formule générale IV dans un milieu réactionnel le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un dérivé d'acide sulfonique de formule générale R⁵-S (=O) ₂-X, où R⁵ a la signification selon l'une ou plusieurs des revendications 1 à 16 et X représente un groupe partant, de préférence un atome d'halogène, de manière particulièrement préférée un atome de chlore ou de brome, en au moins un composé de formule générale I, où R¹ et n ont la signification susmentionnée et R² représente -S(=O)₂-R⁵, où R⁵ a la signification susmentionnée, et ce composé est le cas échéant purifié et/ou isolé ;
ou on transforme au moins un composé de formule générale IV dans un milieu réactionnel le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un composé de formule générale X-(CH₂)-C(=O)-NHR⁶, où R⁶ a la signification selon l'une ou plusieurs des revendications 1 à 16 et X représente un groupe partant, de préférence un atome d'halogène, de manière particulièrement préférée un atome de chlore ou de brome, en au moins un composé de formule générale I, où R¹ et n ont la signification susmentionnée et R² représente - (CH₂) -C(=O)-NH-R⁶, où R⁶ a la signification susmentionnée, et ce composé est le cas échéant purifié et/ou isolé ;
ou on transforme au moins un composé de formule générale IV dans un milieu réactionnel le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un composé de formule générale X-(CH₂)-Dₐₐ-(CH₂)_{bb}-E_{cc}-(CH₂)_{dd}-R⁷, où R⁷, D, E, aa, bb, cc et dd ont la signification selon l'une ou plusieurs des revendications 1 à 16 et X représente un groupe partant, de préférence un atome d'halogène, de manière particulièrement préférée un atome de chlore ou de brome, en au moins un composé de formule générale I, où R¹ et n ont la signification susmentionnée et R² représente -(CH₂)-Dₐₐ-(CH₂)_{bb}-E_{cc}-(CH₂)_{dd}-R⁷, où R⁷, D, E, aa, bb, cc et dd ont la signification susmentionnée, et ce composé est le cas échéant purifié et/ou isolé ;
ou on transforme au moins un composé de formule générale IV dans un milieu réactionnel le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un dérivé d'acide carboxylique de formule générale R⁸-C(=O)-X, où R⁸ a la signification selon l'une ou plusieurs des revendications 1 à 16 et X représente un groupe partant, de préférence un atome d'halogène, de manière particulièrement préférée un atome de chlore ou de brome, en au moins un composé de formule générale I, où R¹ et n ont la signification susmentionnée et R² représente -C(=O)-R⁸, où R⁸ a la signification susmentionnée, et ce composé est le cas échéant purifié et/ou isolé ;
ou on transforme au moins un composé de formule générale IV dans un milieu réactionnel en présence d'au moins un réactif de couplage, le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un dérivé d'acide carboxylique de formule générale R⁸-C(=O)-OH, où R⁸ a la signification selon l'une ou plusieurs des revendications 1 à 16, en au moins un composé de formule générale I, où R¹ et n ont la signification susmentionnée et R² représente -C(=O)-R⁸, où R⁸ a la signification susmentionnée, et ce composé est le cas échéant purifié et/ou isolé ;
ou on transforme au moins un composé de formule générale IV dans un milieu réactionnel le cas échéant en présence d'au moins une base, de préférence en présence d'au moins une base choisie dans le groupe constitué par la triéthylamine, la (4,4)-diméthylaminopyridine, la pyridine, la diisopropyléthylamine et la N-méthylmorpholine, avec au moins un dérivé d'acide sulfonique de formule générale X-S(=O)₂-NR⁹R¹⁰, où R⁹ et R¹⁰ ont la signification selon l'une ou plusieurs des revendications 1 à 16 et X représente un groupe partant, de préférence un atome d'halogène, de manière particulièrement préférée un atome de chlore ou de brome, en au moins un composé de formule générale I, où R¹ et n ont la signification susmentionnée et R² représente -S(=O)₂-NR⁹NR¹⁰, où R⁹ et R¹⁰ ont la signification susmentionnée, et ce composé est le cas échéant purifié et/ou isolé.

18. Médicament contenant au moins un composé selon l'une ou plusieurs des revendications 1 à 16 ainsi que le cas échéant un ou plusieurs adjuvants physiologiquement acceptables.

19. Médicament selon la revendication 18 pour la prophylaxie et/ou le traitement de la douleur, de préférence de la douleur choisie dans le groupe constitué par la douleur aiguë, la douleur chronique, la douleur viscérale et la douleur neuropathique.

20. Médicament selon la revendication 18 pour la prophylaxie et/ou le traitement d'une ou de plusieurs maladies choisies dans le groupe constitué par les troubles de l'absorption alimentaire, de préférence choisis dans le groupe constitué par la boulimie, l'anorexie, l'obésité et la cachexie ; les maladies de rétention d'eau ; les migraines ; l'hémicrânie paroxystique chronique ; les dépressions ; l'incontinence urinaire ; la toux ; l'asthme ; le glaucome ; les acouphènes ; les inflammations ; les maladies de neurodégénérescence, de préférence choisies dans le groupe constitué par la maladie de Parkinson, de Huntington, d'Alzheimer et la sclérose en plaques ; les dysfonctions cognitives, de préférence les troubles de la mémoire ; les états déficitaires cognitifs (le syndrome du déficit d'attention, ADS) ; l'épilepsie ; la catalepsie ; la narcolepsie ; la diarrhée ; la gastrite ; les ulcères d'estomac ; le prurit ; les états d'anxiété ; les attaques de panique ; la schizophrénie ; les ischémies cérébrales ; les spasmes musculaires ; les crampes ; le syndrome de reflux gastro-oesophagique ; l'abus d'alcool et/ou de drogues, de préférence de la nicotine ou de la cocaïne, et/ou de médicaments ; la dépendance de l'alcool et/ou des drogues, de préférence de la nicotine ou de la cocaïne, et/ou des médicaments, de préférence pour la prophylaxie et/ou la diminution de phénomènes de sevrage lors de la dépendance de l'alcool et/ou des drogues, de préférence de la nicotine ou de la cocaïne, et/ou des médicaments ; pour la prophylaxie et/ou la diminution d'un développement d'une tolérance par rapport aux médicaments et/ou aux drogues, en particulier aux médicaments à base d'opiacés ; pour la régulation de l'absorption des aliments ; pour la modulation de l'activité de mouvement, pour supprimer le réflexe de miction ; pour la régulation du système cardiovasculaire, de préférence pour la vasodilatation des artères ; pour l'anesthésie locale ; pour l'augmentation de la vigilance ; pour l'augmentation de la libido ; pour la diurèse et/ou pour l'antinatriurèse.

21. Utilisation d'au moins un composé selon l'une ou plusieurs des revendications 1 à 16 pour la préparation d'un médicament destiné à la prophylaxie et/ou au traitement de la douleur, de préférence de la douleur choisie dans le groupe constitué par la douleur aiguë, la douleur chronique, la douleur viscérale et la douleur neuropathique.

22. Utilisation d'au moins un composé selon l'une ou plusieurs des revendications 1 à 16 pour la préparation d'un médicament destiné à la prophylaxie et/ou au traitement d'une ou de plusieurs maladies choisies dans le groupe constitué par les troubles de l'absorption alimentaire, de préférence choisis dans le groupe constitué par la boulimie, l'anorexie, l'obésité et la cachexie ; les maladies de rétention d'eau ; les migraines ; l'hémicrânie paroxystique chronique ; les dépressions ; l'incontinence urinaire ; la toux ; l'asthme ; le glaucome ; les acouphènes ; les inflammations ; les maladies de neurodégénérescence, de préférence choisies dans le groupe constitué par la maladie de Parkinson, de Huntington, d'Alzheimer et la sclérose en plaques ; les dysfonctions cognitives, de préférence les troubles de la mémoire ; les états déficitaires cognitifs (le syndrome du déficit d'attention, ADS) ; l'épilepsie ; la catalepsie ; la narcolepsie ; la diarrhée ; la gastrite ; les ulcères d'estomac ; le prurit ; les états d'anxiété ; les attaques de panique ; la schizophrénie ; les ischémies cérébrales ; les spasmes musculaires ; les crampes ; le syndrome de reflux gastro-oesophagique ; l'abus d'alcool et/ou de drogues, de préférence de la nicotine ou de la cocaïne, et/ou de médicaments ; la dépendance de l'alcool et/ou des drogues, de préférence de la nicotine ou de la cocaïne, et/ou des médicaments, de préférence à la prophylaxie et/ou la diminution de phénomènes de sevrage lors de la dépendance de l'alcool et/ou des drogues, de préférence de la nicotine ou de la cocaïne, et/ou des médicaments ; à la prophylaxie et/ou la diminution d'un développement d'une tolérance par rapport aux médicaments et/ou aux drogues, en particulier aux médicaments à base d'opiacés ; à la régulation de l'absorption des aliments ; à la modulation de l'activité de mouvement, à la suppression du réflexe de miction ; à la régulation du système cardiovasculaire, de préférence à la vasodilatation des artères ; à l'anesthésie locale ; à l'augmentation de la vigilance ; à l'augmentation de la libido ; à la diurèse et/ou à l'antinatriurèse.
